# EUROPÄISCHE PATENTSCHRIFT

(11) **EP 2 914 596 B1**
(45) Veröffentlichungstag und Bekanntmachung des Hinweises auf die Patenterteilung: **21.06.2017**
(21) Anmeldenummer: 13789237.8
(22) Anmeldetag: 04.11.2013
(51) Int. Cl.: C07D 471/04, A61K 31/437, A61P 7/02, A61P 9/00, A61P 9/10, A61P 9/12, A61P 13/12, A61K 45/06, A61K 31/444, A61K 31/5377

(54) **HYDROXY-SUBSTITUIERTE IMIDAZO[1,2-A]PYRIDINCARBOXAMIDE UND IHRE VERWENDUNG ALS STIMULATOREN DER LÖSLICHEN GUANYLATCYCLASE**
HYDROXY SUBSTITUTED IMIDAZO[1,2-A]PYRIDINE CARBOXAMIDES AND THEIR USE AS STIMULATORS OF SOLUBLE GUANYLATE CYCLASE
IMIDAZO[1,2-A]PYRIDINE CARBOXAMIDES À SUBSTITUTION HYDROXY ET LEUR UTILISATION EN TANT QUE STIMULATEURS DE LA GUANYLATE CYCLASE SOLUBLE

(30) Priorität: 05.11.2012 EP 12191200; 07.03.2013 US 201313789208
(43) Veröffentlichungstag der Anmeldung: 09.09.2015
(73) Patentinhaber: Bayer Pharma Aktiengesellschaft, 13353 Berlin (DE)
(72) Erfinder: VAKALOPOULOS, Alexandros, 40721 Hilden (DE); HARTUNG, Ingo, 13158 Berlin (DE); FOLLMANN, Markus, 50859 Köln (DE); JAUTELAT, Rolf, 42781 Haan (DE); STRAUB, Alexander, 42117 Wuppertal (DE); HAßFELD, Jorma, 40221 Düsseldorf (DE); LINDNER, Niels, 42115 Wuppertal (DE); SCHNEIDER, Dirk, 42115 Wuppertal (DE); WUNDER, Frank, 42117 Wuppertal (DE); STASCH, Johannes-Peter, 42651 Solingen (DE); REDLICH, Gorden, 44799 Bochum (DE); LI, Volkhart Min-Jian, 42553 Velbert (DE); BECKER-PELSTER, Eva-Maria, 42327 Wuppertal (DE); KNORR, Andreas, 40699 Erkrath (DE)
(74) Vertreter: BIP Patents
(86) Internationale Anmeldenummer: PCT/EP2013/072908
(87) Internationale Veröffentlichungsnummer: WO 2014/068104

(56) Entgegenhaltungen:
- WO-A1-2011/141409
- DATABASE WPI Week 201301 Thomson Scientific, London, GB; AN 2012-Q85627 XP002716569, & WO 2012/165399 A1 (ASTELLAS PHARMA INC) 6. Dezember 2012 (2012-12-06) & WO 2012/165399 A1 (ASTELLAS PHARMA INC [JP]; KOGA YUJI [JP]; MAENO KYOICHI [JP]; SATO IPP) 6. Dezember 2012 (2012-12-06)

## Beschreibung

Die vorliegende Anmeldung betrifft neue substituierte Imidazo[1,2-a]pyridin-3-carboxamide, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorphosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Unter anderem in EP 0 266 890-A1, WO 89/03833-A1, JP 01258674-A [vgl. Chem. Abstr. 112:178986], WO 96/34866-A1, EP 1 277 754-A1, WO 2006/015737-A1, WO 2008/008539-A2, WO 2008/082490-A2, WO 2008/134553-A1, WO 2010/030538-A2 und WO 2011/113606-A1 sind verschiedene Imidazo[1,2-a]pyridin-Derivate beschrieben, die zur Behandlung von Erkrankungen verwendet werden können.

Aufgabe der vorliegenden Erfindung war die Bereitstellung neuer Substanzen, die als Stimulatoren der löslichen Guanylatcyclase wirken, und als solche zur Behandlung und/oder Prophylaxe von Krankheiten geeignet sind.

Offenbarungsgegenstand sind Verbindungen der allgemeinen Formel (I) 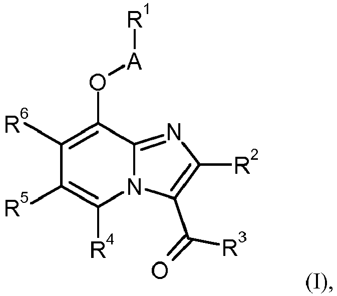 in welcher
- A: für CH₂, CD₂ oder CH(CH₃) steht,
- R¹: für (C₄-C₆)-Alkyl, (C₃-C₇)-Cycloalkyl oder Phenyl steht, wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy, Difluormethoxy und Trifluormethoxy substituiert sein kann oder an zwei benachbarten Kohlenstoffatomen des Phenyls mit einer Difluormethylendioxy-Brücke substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 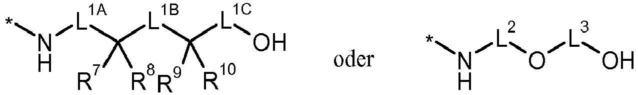 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- R⁷: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R⁸: für Wasserstoff oder (C₁-C₄)-Alkyl steht,
worin (C₁-C₄)-Alkyl mit Hydroxy substituiert sein kann,
oder
- R⁷ und R⁸: zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
   und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,

R¹⁰ für Wasserstoff oder (C₁-C₄)-Alkyl steht,
worin (C₁-C₄)-Alkyl mit Hydroxy substituiert sein kann,
oder
- R⁹ und R¹⁰: zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass die Reste R⁷ und R⁹ nicht beide gleichzeitig für Phenyl stehen,
oder
- R⁷ und R⁹: zusammen mit den Kohlenstoffatomen, an die sie jeweils gebunden sind, sowie der Gruppe L^{1B} einen 5- bis 10-gliedrigen Carbocyclus bilden,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
L² für geradkettiges (C₂-C₄)-Alkandiyl steht,
L³ für geradkettiges (C₂-C₄)-Alkandiyl steht,
- R⁴: für Wasserstoff steht,
- R⁵: für Wasserstoff, Halogen, Cyano, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Offenbarungsgegenstand sind Verbindungen der allgemeinen Formel (I) 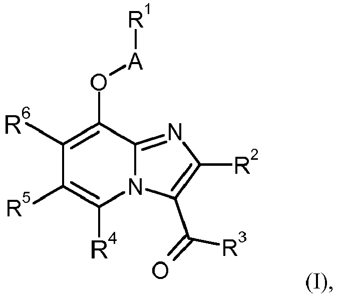 in welcher
- A: für CH₂, CD₂ oder CH(CH₃) steht,
- R¹: für (C₄-C₆)-Alkyl, (C₃-C₇)-Cycloalkyl, Pyridyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 bis 3 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann oder an 2 benachbarten Kohlenstoffatomen des Phenyls mit einer Difluormethylendioxy-Brücke substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 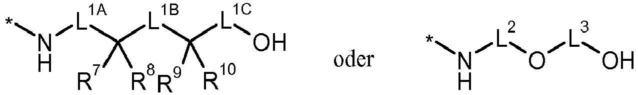
steht, wobei
- *: für die Anknüpfstelle an die Carbonylgruppe steht,
- L^{1A}: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- L^{1B}: für eine Bindung oder (C₁-C₄)-Alkandiyl steht,
- L^{1C}: für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- R⁷: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl, (C₁-C₄)-Alkoxy und (C₁-C₄)-Alkylsulfonyl substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R⁸: für Wasserstoff oder (C₁-C₄)-Alkyl steht,
worin (C₁-C₄)-Alkyl mit Hydroxy substituiert sein kann,
oder
- R⁷ und R⁸: zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁰: für Wasserstoff oder (C₁-C₄)-Alkyl steht, worin (C₁-C₄)-Alkyl mit Hydroxy substituiert sein kann,
oder
R⁹ und R¹⁰ zusammen mit dem Kohlenstoffatom, an das sie gebunden sind, einen 3-bis 7-gliedrigen Carbocyclus oder einen 4- bis 7-gliedriger Heterocyclus bilden,
   worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
mit der Maßgabe, dass die Reste R⁷ und R⁹ nicht beide gleichzeitig für Phenyl stehen, oder
R⁷ und R⁹ zusammen mit den Kohlenstoffatomen, an die sie jeweils gebunden sind, sowie der Gruppe L^{1B} einen 5- bis 10-gliedrigen Carbocyclus bilden,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
   L² für geradkettiges (C₂-C₄)-Alkandiyl steht,
   L³ für geradkettiges (C₂-C₄)-Alkandiyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Offenbarungsgegenstand sind Verbindungen der allgemeinen Formel (I) 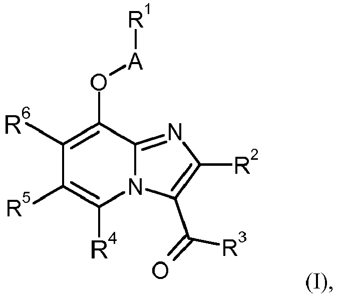 in welcher
- A: für CH₂, CD₂ oder CH(CH₃) steht,
- R¹: für Phenyl steht,
wobei Phenyl an 2 benachbarten Kohlenstoffatomen des Phenyls mit einer Difluormethylendioxy-Brücke substituiert ist,
- R²: für Wasserstoff, (C₁-C₄)-Alkyl, Cyclopropyl, Monofluormethyl, Difluormethyl oder Trifluormethyl steht,
- R³: für eine Gruppe der Formel 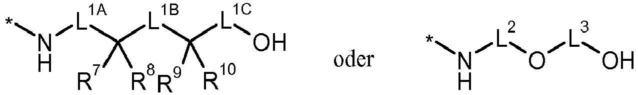 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
- R⁷: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl, (C₁-C₄)-Alkoxy und (C₁-C₄)-Alkylsulfonyl substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R⁸: für Wasserstoff oder (C₁-C₄)-Alkyl steht,
worin (C₁-C₄)-Alkyl mit Hydroxy substituiert sein kann,
oder
R⁷ und R⁸ zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
- R⁹: für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁰: für Wasserstoff oder (C₁-C₄)-Alkyl steht,
worin (C₁-C₄)-Alkyl mit Hydroxy substituiert sein kann,
oder
R⁹ und R¹⁰ zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können, mit der Maßgabe, dass die Reste R⁷ und R⁹ nicht beide gleichzeitig für Phenyl stehen,
oder
R⁷ und R⁹ zusammen mit den Kohlenstoffatomen, an die sie jeweils gebunden sind, sowie der Gruppe L^{1B} einen 5- bis 10-gliedrigen Carbocyclus bilden,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
   L² für geradkettiges (C₂-C₄)-Alkandiyl steht,
   L³ für geradkettiges (C₂-C₄)-Alkandiyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Offenbarungsgegenstand sind Verbindungen der allgemeinen Formel (I) 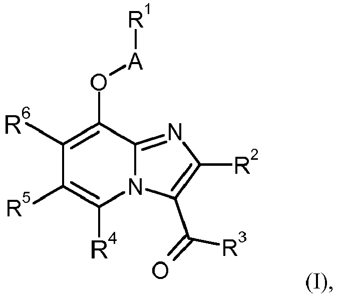 in welcher
- A: für CH₂, CD₂ oder CH(CH₃) steht,
- R¹: für (C₄-C₆)-Alkyl, (C₃-C₇)-Cycloalkyl, Pyridyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 bis 3 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann oder an 2 benachbarten Kohlenstoffatomen des Phenyls mit einer Difluormethylendioxy-Brücke substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 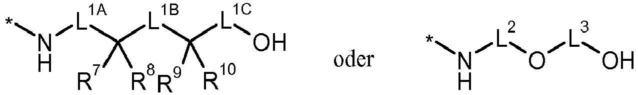 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl, (C₁-C₄)-Alkoxy und (C₁-C₄)-Alkylsulfonyl substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R⁸: für Wasserstoff oder (C₁-C₄)-Alkyl steht,
worin (C₁-C₄)-Alkyl mit Hydroxy substituiert sein kann,
oder
R⁷ und R⁸ zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
- R¹⁰: für Wasserstoff oder (C₁-C₄)-Alkyl steht,
worin (C₁-C₄)-Alkyl mit Hydroxy substituiert sein kann, oder
R⁹ und R¹⁰ zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können, mit der Maßgabe, dass die Reste R⁷ und R⁹ nicht beide gleichzeitig für Phenyl stehen,
oder
R⁷ und R⁹ zusammen mit den Kohlenstoffatomen, an die sie jeweils gebunden sind, sowie der Gruppe L^{1B} einen 5- bis 10-gliedrigen Carbocyclus bilden,
mit der Maßgabe, dass gleichzeitig nicht mehr als eines der Reste-Paare R⁷ und R⁸, R⁹ und R¹⁰ bzw. R⁷ und R⁹ einen Carbo- oder Heterocyclus bildet,
L² für geradkettiges (C₂-C₄)-Alkandiyl steht,
L³ für geradkettiges (C₂-C₄)-Alkandiyl steht,
- R⁴: für Wasserstoff steht,
- R⁵: für Monofluormethyl, Difluormethyl, Trifluormethyl, Cyclopropyl, Ethinyl, Morpholinyl oder Pyrolidinyl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Gegenstand der vorliegenden Erfindung sind Verbindungen der allgemeinen Formel (I) 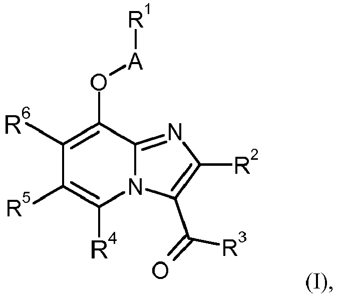 in welcher
- A: für CH₂, CD₂ oder CH(CH₃) steht,
- R¹: für (C₄-C₆)-Alkyl, (C₃-C₇)-Cycloalkyl, Pyridyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 bis 3 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann oder an 2 benachbarten Kohlenstoffatomen des Phenyls mit einer Difluormethylendioxy-Brücke substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 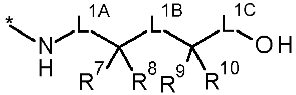 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für (C₁-C₆)-Alkyl oder (C₂-C₆)-Alkinyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy und Benzyloxy substituiert ist,
worin Benzyloxy mit 1 bis 3 Substituenten Halogen substituiert ist,
und
worin darüber hinaus (C₁-C₆)-Alkyl mit Hydroxy substituiert sein kann,
R⁸ für Wasserstoff oder (C₁-C₄)-Alkyl steht,
worin (C₁-C₄)-Alkyl mit Hydroxy substituiert sein kann,
R⁹ für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
R¹⁰ für Wasserstoff oder (C₁-C₄)-Alkyl steht,
worin (C₁-C₄)-Alkyl mit Hydroxy substituiert sein kann,
oder
R⁹ und R¹⁰ zusammen mit dem Kohlenstoffatom, an das sie gebunden sind, einen 3-bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁴ für Wasserstoff steht,
R⁵ für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

Gegenstand der vorliegenden Erfindung sind Verbindungen der allgemeinen Formel (I) 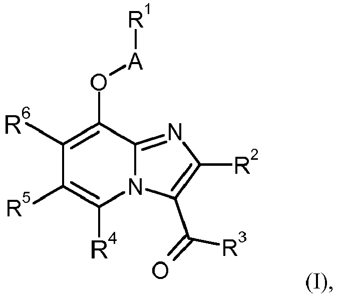 in welcher
- A: für CH₂, CD₂ oder CH(CH₃) steht,
- R¹: für (C₄-C₆)-Alkyl, (C₃-C₇)-Cycloalkyl, Pyridyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 bis 3 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann oder an 2 benachbarten Kohlenstoffatomen des Phenyls mit einer Difluormethylendioxy-Brücke substituiert sein kann,
- R²: für Wasserstoff, (C₁-C₄)-Alkyl, Cyclopropyl, Monofluormethyl, Difluormethyl oder Trifluormethyl steht,
- R³: für eine Gruppe der Formel 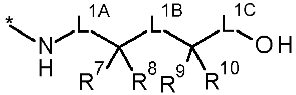 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl, (C₁-C₄)-Alkoxy und (C₁-C₄)-Alkylsulfonyl substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
R⁸ für Wasserstoff oder (C₁-C₄)-Alkyl steht,
worin (C₁-C₄)-Alkyl mit Hydroxy substituiert sein kann,
oder
R⁷ und R⁸ zusammen mit dem Kohlenstoffatom, an das sie gebunden sind, einen 3-bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für (C₁-C₆)-Alkyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Phenoxy und Benzyloxy substituiert ist,
worin Phenoxy mit 1 bis 3 Substituenten Halogen substituiert ist,
worin Benzyloxy mit 1 bis 3 Substituenten Halogen substituiert ist,

R¹⁰ für Wasserstoff oder (C₁-C₄)-Alkyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff, Cyano oder Halogen steht,
sowie ihre *N*-Oxide, Salze, Solvate, Salze der *N*-Oxide und Solvate der *N*-Oxide und Salze.

Offenbarungsgegenstand sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl.
Carbocyclus bzw Cycloalkyl steht im Rahmen der Erfindung für einen mono- oder bicyclischen, gesättigten oder teilweise ungesättigten Carbocyclus mit der jeweils angegeben Anzahl an RingKohlenstoffatomen und bis zu 3 Doppelbindungen. Beispielhaft und vorzugsweise seien genannt: Cyclopropyl, Cyclobutyl, Cyclopentyl, Cyclohexyl, Cycloheptyl, Cyclopentenyl, Cyclohexenyl, Cyclohexadienyl, Cycloheptenyl, Cycloheptadienyl, Indanyl, Tetralinyl.

Alkenyl steht im Rahmen der Erfindung für einen linearen oder verzweigten Alkenylrest mit 2 bis 6 Kohlenstoffatomen und einer oder zwei Doppelbindungen. Bevorzugt ist ein linearer oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.

Alkinyl steht im Rahmen der Erfindung für einen linearen oder verzweigten Alkinylrest mit 2 bis 6 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.

Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl und Butan-2,3-diyl.

Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.

Alkoxycarbonyl stehen im Rahmen der Erfindung für einen linearen oder verzweigten Alkoxyrest mit 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Alkylthio steht im Rahmen der Erfindung für eine Thio-Gruppe mit einem linearen oder verzweigten Alkylsubstituenten, der 1 bis 4 Kohlenstoffatome aufweist. Beispielhaft und vorzugsweise seien genannt: Methylthio, Ethylthio, n-Propylthio, Isopropylthio, n-Butylthio und *tert.-*Butylthio.

Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.

Ein 4- bis 7-gliedriger Heterocyclus steht im Rahmen der Erfindung für einen monocyclischen, gesättigten Heterocyclus mit insgesamt 4 bis 7 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.

Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl.

Halogen schließt im Rahmen der Erfindung Fluor, Chlor, Brom und Iod ein. Bevorzugt sind Chlor oder Fluor.

In der Formel der Gruppe, für die R³ bzw. R¹ stehen kann, steht der Endpunkt der Linie, an dem das Zeichen * und # steht, nicht für ein Kohlenstoffatom beziehungsweise eine CH₂-Gruppe, sondern ist Bestandteil der Bindung zu dem jeweils bezeichneten Atom, an das R³ bzw. R¹ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt sind im Rahmen der vorliegenden Offenbarung Verbindungen der Formel (I), in welcher
- A: für CH₂, CD₂ oder CH(CH₃) steht,
- R¹: für (C₄-C₆)-Alkyl, (C₄-C₆)-Cycloalkyl oder Phenyl steht,
wobei (C₄-C₆)-Alkyl bis zu sechsmal mit Fluor substituiert sein kann,
wobei (C₄-C₆)-Cycloalkyl mit 1 bis 2 Substituenten unabhängig voneinander ausgewählt aus der Gruppe Fluor, Trifluormethyl und Methyl substituiert sein kann,
und
wobei Phenyl mit 1 bis 4 Substituenten unabhängig voneinander ausgewählt aus der Gruppe Fluor, Chlor, Brom, Difluormethyl, Trifluormethyl und Methyl substituiert sein kann oder an zwei 2 benachbarten Kohlenstoffatomen des Phenyls mit einer Difluor-methylendioxy-Brücke substituiert sein kann,
- R²: für Wasserstoff, (C₁-C₄)-Alkyl, Trifluormethyl oder Cyclopropyl steht,
- R³: für eine Gruppe der Formel 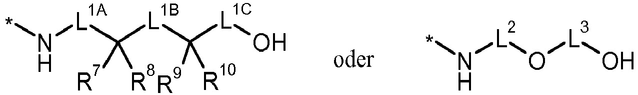 steht, wobei
* für die Anknüpfstelle an die Carbonylgruppe steht,
- L^{1A}: für eine Bindung oder Methylen steht,
- L^{1B}: für eine Bindung, Methylen oder 1,2-Ethandiyl steht,
L^{1C} für eine Bindung oder Methylen steht,
worin Methylen mit 1 bis 2 Substituenten unabhängig voneinander ausgewählt aus der Gruppe Trifluormethyl und (C₁-C₄)-Alkyl substituiert sein kann,
R⁷ für Wasserstoff, Trifluormethyl, (C₁-C₆)-Alkyl, (C₂-C₄)-Alkenyl, (C₃-C₆)-Cycloalkyl, (C₁-C₄)-Alkoxycarbonyl, 5- oder 6-gliedriges Heteroaryl oder Phenyl steht,
worin (C₁-C₆)-Alkyl mit 1 bis 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, Methoxy und Ethoxy substituiert sein können,
R⁸ für Wasserstoff, Methyl oder Ethyl steht,
worin Methyl und Ethyl mit Hydroxy substituiert sein können,
oder
R⁷ und R⁸ zusammen mit dem Kohlenstoffatom, an das sie gebunden sind, einen 3- bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann,
R⁹ für Wasserstoff, Trifluormethyl, (C₁-C₆)-Alkyl, (C₂-C₄)-Alkenyl, (C₃-C₆)-Cycloalkyl, (C₁-C₄)-Alkoxycarbonyl, 5- oder 6-gliedriges Heteroaryl oder Phenyl steht,
worin (C₁-C₆)-Alkyl mit 1 bis 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, Methoxy und Ethoxy substituiert sein können,
R¹⁰ für Wasserstoff, Methyl oder Ethyl steht,
worin Methyl und Ethyl mit Hydroxy substituiert sein können,
oder
R⁹ und R¹⁰ zusammen mit dem Kohlenstoffatom, an das sie gebunden sind, einen 3- bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann,
mit der Maßgabe, dass die Reste R⁷ und R⁹ nicht beide gleichzeitg für Phenyl stehen,
oder
R⁷ und R⁹ zusammen mit den Kohlenstoffatomen, an die sie jeweils gebunden sind, sowie der Gruppe L^{1B} einen 5- bis 10-gliedrigen Carbocyclus bilden,
mit der Maßgabe, dass gleichzeitig nicht mehr als eines der Reste-Paare R⁷ und R⁸, R⁹ und R¹⁰ bzw. R⁷ und R⁹ einen Carbocyclus bildet,
- L²: für 1,2-Ethandiyl steht,
- L³: für 1,2-Ethandiyl steht,
R⁴ für Wasserstoff steht,
R⁵ für Wasserstoff, Fluor, Chlor, Brom, Cyano, Difluormethyl, Trifluormethyl, Methyl, Ethyl, Cyclopropyl, Ethinyl, Morpholinyl oder Pyrrolidinyl steht,
R⁶ für Wasserstoff oder Fluor steht,
sowie ihre *N*-Oxide, Salze, Solvate, Salze der *N*-Oxide und Solvate der *N*-Oxide und Salze.

Besonders bevorzugt sind im Rahmen der vorliegenden Offenbarung Verbindungen der Formel (I), in welcher
A für CH₂ steht,
R¹ für eine Phenyl-Gruppe der Formel 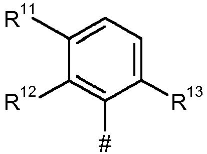 steht, wobei
# für die Anknüpfstelle an A steht,
   und
R¹¹, R¹² und R¹³unabhängig voneinander für Wasserstoff, Fluor oder Chlor stehen,
mit der Maßgabe, dass mindestens zwei der Reste R¹¹, R¹², R¹³ von Wasserstoff verschieden sind,
R² für Methyl steht,
R³ für eine Gruppe der Formel 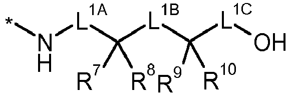 steht, wobei
- *: für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung steht,
R⁷ für Wasserstoff, Trifluormethyl, (C₁-C₆)-Alkyl oder Phenyl steht,
worin (C₁-C₆)-Alkyl mit 1 oder 2 Substituenten unabhängig voneinander ausgewählt aus der Gruppe Fluor, Trifluormethyl, Hydroxy, (C₁-C₄)-Alkoxy und Phenyl substituiert sein kann,
und
wobei die oben genannten Phenyl-Gruppen mit 1 oder 2 Substituenten unabhängig voneinander ausgewählt aus Fluor und Chlor substituiert sein können,
R⁸ für Wasserstoff, Methyl oder Ethyl steht,
R⁹ für Wasserstoff, Trifluormethyl, (C₁-C₆)-Alkyl oder Phenyl steht,
worin (C₁-C₆)-Alkyl mit 1 bis 2 Substituenten unabhängig voneinander ausgewählt aus der Gruppe Fluor, Trifluormethyl, Hydroxy, (C₁-C₄)-Alkoxy und Phenyl substituiert sein kann,
und
wobei die oben genannten Phenyl-Gruppen mit 1 oder 2 Substituenten unabhängig voneinander ausgewählt aus Fluor und Chlor substituiert sein können,
R¹⁰ für Wasserstoff, Methyl oder Ethyl steht,
oder
R⁹ und R¹⁰ zusammen mit dem Kohlenstoffatom, an das sie gebunden sind, einen 3- bis 6-gliedrigen Carbocyclus bilden,
mit der Maßgabe, dass die Reste R⁷ und R⁹ nicht beide gleichzeitg für Phenyl stehen,
oder
R⁷ und R⁹ zusammen mit den Kohlenstoffatomen, an die sie jeweils gebunden sind, sowie der Gruppe L^{1B} einen 5- bis 9-gliedrigen Carbocyclus bilden,
mit der Maßgabe, dass gleichzeitig nicht mehr als eines der Reste-Paare R⁹ und R¹⁰ bzw. R⁷ und R⁹ einen Carbocyclus bildet,
R⁴ für Wasserstoff steht,
R⁵ für Wasserstoff, Fluor, Chlor, Cyano, Methyl oder Ethyl steht,
R⁶ für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Offenbarung Verbindungen der Formel (I), in welcher
- A: für CH₂, CD₂ oder CH(CH₃) steht,
- R¹: für Phenyl steht,
wobei Phenyl an 2 benachbarten Kohlenstoffatomen des Phenyls mit einer Difluormethylendioxy-Brücke substituiert ist,
- R²: für Wasserstoff, (C₁-C₄)-Alkyl, Trifluormethyl oder Cyclopropyl steht,
- R³: für eine Gruppe der Formel 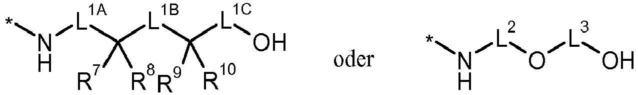 steht, wobei
* für die Anknüpfstelle an die Carbonylgruppe steht,
L^{1A} für eine Bindung oder Methylen steht,
L^{1B} für eine Bindung, Methylen oder 1,2-Ethandiyl steht,
L^{1C} für eine Bindung oder Methylen steht,
worin Methylen mit 1 bis 2 Substituenten unabhängig voneinander ausgewählt aus der Gruppe Trifluormethyl und (C₁-C₄)-Alkyl substituiert sein kann,
R⁷ für Wasserstoff, Trifluormethyl, (C₁-C₆)-Alkyl, (C₂-C₄)-Alkenyl, (C₃-C₆)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, Methoxy und Ethoxy substituiert sein können,
R⁸ für Wasserstoff, Methyl oder Ethyl steht,
worin Methyl und Ethyl mit Hydroxy substituiert sein können,
oder
R⁷ und R⁸ zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann,
R⁹ für Wasserstoff, Trifluormethyl, (C₁-C₆)-Alkyl, (C₂-C₄)-Alkenyl, (C₃-C₆)-Cycloalkyl, (C₁-C₄)-Alkoxycarbonyl, 5- oder 6-gliedriges Heteroaryl oder Phenyl steht,
worin (C₁-C₆)-Alkyl mit 1 bis 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können, worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
   und
worin Phenyl und 5- oder 6-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, Methoxy und Ethoxy substituiert sein können,

R¹⁰ für Wasserstoff, Methyl oder Ethyl steht,
worin Methyl und Ethyl mit Hydroxy substituiert sein können, oder
R⁹ und R¹⁰ zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann, mit der Maßgabe, dass die Reste R⁷ und R⁹ nicht beide gleichzeitg für Phenyl stehen,
oder
R⁷ und R⁹ zusammen mit den Kohlenstoffatomen, an die sie jeweils gebunden sind, einen 5- bis 10-gliedrigen Carbocyclus bilden, mit der Maßgabe, dass gleichzeitig nicht mehr als eines der Reste-Paare R⁷ und R⁸, R⁹ und R¹⁰ bzw. R⁷ und R⁹ einen Carbocyclus bildet,
L² für 1,2-Ethandiyl steht,
L³ für 1,2-Ethandiyl steht,
- R⁴: für Wasserstoff steht,
- R⁵: für Wasserstoff, Fluor, Chlor, Brom, Cyano, Monofluormethyl, Difluormethyl, Trifluormethyl, Methyl, Ethyl, Cyclopropyl, Ethinyl, Morpholinyl oder Pyrolidinyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Offenbarung Verbindungen der Formel (I), in welcher
- A: für CH₂ steht,
- R¹: für Phenyl steht,
wobei Phenyl an 2 benachbarten Kohlenstoffatomen des Phenyls mit einer Difluormethylendioxy-Brücke substituiert ist,
- R²: für Methyl steht,
- R³: für eine Gruppe der Formel 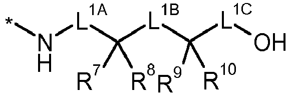 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung steht,
R⁷ für Wasserstoff, Trifluormethyl, (C₁-C₆)-Alkyl oder Phenyl steht,
worin (C₁-C₆)-Alkyl mit Hydroxy, (C₁-C₄)-Alkoxy oder Phenyl substituiert sein kann,
oder
worin (C₁-C₆)-Alkyl bis zu fünffach mit Fluor substituiert sein kann,
und
wobei die oben genannten Phenyl-Gruppen mit 1 oder 2 Substituenten unabhängig voneinander ausgewählt aus Fluor und Chlor substituiert sein können,
R⁸ für Wasserstoff, Methyl oder Ethyl steht, oder
R⁷ und R⁸ zusammen mit dem Kohlenstoffatom, an das sie gebunden sind, einen 3-bis 6-gliedrigen Carbocyclus bilden,
R⁹ für Wasserstoff, Trifluormethyl, (C₁-C₆)-Alkyl oder Phenyl steht,
worin (C₁-C₆)-Alkyl mit (C₁-C₄)-Alkoxy oder Phenyl substituiert sein kann,
oder
worin (C₁-C₆)-Alkyl bis zu fünffach mit Fluor substituiert sein kann,
und
wobei die oben genannten Phenyl-Gruppen mit 1 oder 2 Substituenten unabhängig voneinander ausgewählt aus Fluor und Chlor substituiert sein können,
R¹⁰ für Wasserstoff, Methyl oder Ethyl steht, oder
R⁹ und R¹⁰ zusammen mit dem Kohlenstoffatom, an das sie gebunden sind, einen 3-bis 6-gliedrigen Carbocyclus bilden, mit der Maßgabe, dass die Reste R⁷ und R⁹ nicht beide gleichzeitg für Phenyl stehen,
- R⁴: für Wasserstoff steht,
- R⁵: für Wasserstoff, Fluor, Chlor, Cyano, Methyl oder Ethyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für Phenyl steht,
wobei Phenyl an 2 benachbarten Kohlenstoffatomen des Phenyls mit einer Difluormethylendioxy-Brücke substituiert ist,
- R²: für Methyl steht,
- R³: für eine Gruppe der Formel 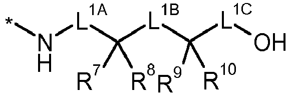 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff oder (C₁-C₆)-Alkyl steht,
worin (C₁-C₆)-Alkyl bis zu fünffach mit Fluor substituiert sein kann,
R⁸ für Wasserstoff steht,
R⁹ für Wasserstoff oder Methyl steht,
R¹⁰ für Wasserstoff oder Methyl steht,
- R⁴: für Wasserstoff steht,
- R⁵: für Wasserstoff, Chlor, Methyl oder Ethyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Offenbarung Verbindungen der Formel (I), in welcher
- A: für CH₂, CD₂ oder CH(CH₃) steht,
- R¹: für (C₄-C₆)-Alkyl, (C₄-C₆)-Cycloalkyl, Pyridyl oder Phenyl steht,
wobei (C₄-C₆)-Alkyl bis zu sechsmal mit Fluor substituiert sein kann,
wobei (C₄-C₆)-Cycloalkyl mit 1 bis 2 Substituenten unabhängig voneinander ausgewählt aus der Gruppe Fluor, Trifluormethyl und Methyl substituiert sein kann,
wobei Pyridyl mit 1 oder 2 Substituenten unabhängig voneinander ausgewählt aus der Gruppe Fluor, Trifluormethyl und Methyl substituiert sein kann
und
wobei Phenyl mit 1 bis 4 Substituenten unabhängig voneinander ausgewählt aus der Gruppe Fluor, Chlor, Brom, Difluormethyl, Trifluormethyl, Cyclopropyl und Methyl substituiert sein kann oder an zwei benachbarten Kohlenstoffatomen des Phenyls mit einer Difluormethylendioxy-Brücke substituiert sein kann,
- R²: für Wasserstoff, (C₁-C₄)-Alkyl, Trifluormethyl oder Cyclopropyl steht,
- R³: für eine Gruppe der Formel 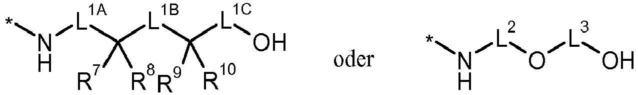 steht, wobei
* für die Anknüpfstelle an die Carbonylgruppe steht,
L^{1A} für eine Bindung oder Methylen steht,
L^{1B} für eine Bindung, Methylen oder 1,2-Ethandiyl steht,
L^{1C} für eine Bindung oder Methylen steht,
worin Methylen mit 1 bis 2 Substituenten unabhängig voneinander ausgewählt aus der Gruppe Trifluormethyl und (C₁-C₄)-Alkyl substituiert sein kann,
R⁷ für Wasserstoff, Trifluormethyl, (C₁-C₆)-Alkyl, (C₂-C₄)-Alkenyl, (C₃-C₆)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können, worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, Methoxy und Ethoxy substituiert sein können,
R⁸ für Wasserstoff, Methyl oder Ethyl steht,
worin Methyl und Ethyl mit Hydroxy substituiert sein können, oder
R⁷ und R⁸ zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann,
R⁹ für Wasserstoff, Trifluormethyl, (C₁-C₆)-Alkyl, (C₂-C₄)-Alkenyl, (C₃-C₆)-Cycloalkyl, (C₁-C₄)-Alkoxycarbonyl, 5- oder 6-gliedriges Heteroaryl oder Phenyl steht,
worin (C₁-C₆)-Alkyl mit 1 bis 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann, und
worin Phenyl und 5- oder 6-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, Methoxy und Ethoxy substituiert sein können,
R¹⁰ für Wasserstoff, Methyl oder Ethyl steht,
worin Methyl und Ethyl mit Hydroxy substituiert sein können, oder
R⁹ und R¹⁰ zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann, mit der Maßgabe, dass die Reste R⁷ und R⁹ nicht beide gleichzeitg für Phenyl stehen,
oder
R⁷ und R⁹ zusammen mit den Kohlenstoffatomen, an die sie jeweils gebunden sind, einen 5- bis 10-gliedrigen Carbocyclus bilden, mit der Maßgabe, dass gleichzeitig nicht mehr als eines der Reste-Paare R⁷ und R⁸, R⁹ und R¹⁰ bzw. R⁷ und R⁹ einen Carbocyclus bildet,
L² für 1,2-Ethandiyl steht,
L³ für 1,2-Ethandiyl steht,
- R⁴: für Wasserstoff steht,
- R⁵: für Cyclopropyl, Ethinyl, Morpholinyl oder Pyrolidinyl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Offenbarung Verbindungen der Formel (I), in welcher
- A: für CH₂ steht,
- R¹: für eine Phenyl-Gruppe der Formel 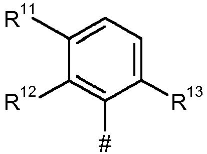 steht, wobei
# für die Anknüpfstelle an A steht,
und
R¹¹, R¹² und R¹³ unabhängig voneinander für Wasserstoff, Fluor oder Chlor stehen, mit der Maßgabe, dass mindestens zwei der Reste R¹¹, R¹², R¹³ von Wasserstoff verschieden sind,
oder
für eine Pyridyl-Gruppe der Formel 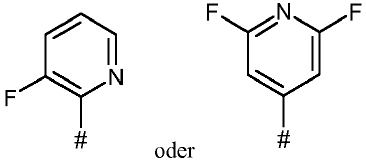 steht,
- R²: für Methyl steht,
- R³: für eine Gruppe der Formel 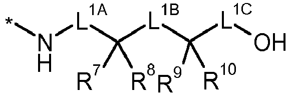 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung steht,
R⁷ für Wasserstoff, Trifluormethyl, (C₁-C₆)-Alkyl oder Phenyl steht,
worin (C₁-C₆)-Alkyl mit Hydroxy, (C₁-C₄)-Alkoxy oder Phenyl substituiert sein kann,
oder
worin (C₁-C₆)-Alkyl bis zu fünffach mit Flour substituiert sein kann,
und
wobei die oben genannten Phenyl-Gruppen mit 1 oder 2 Substituenten unabhängig voneinander ausgewählt aus Fluor und Chlor substituiert sein können,
R⁸ für Wasserstoff, Methyl oder Ethyl steht, oder
R⁷ und R⁸ zusammen mit dem Kohlenstoffatom, an das sie gebunden sind, einen 3-bis 6-gliedrigen Carbocyclus bilden,
R⁹ für Wasserstoff, Trifluormethyl, (C₁-C₆)-Alkyl oder Phenyl steht,
worin (C₁-C₆)-Alkyl mit (C₁-C₄)-Alkoxy oder Phenyl substituiert sein kann,
oder
worin (C₁-C₆)-Alkyl bis zu fünffach mit Fluor substituiert sein kann,
und
wobei die oben genannten Phenyl-Gruppen mit 1 oder 2 Substituenten unabhängig voneinander ausgewählt aus Fluor und Chlor substituiert sein können,
R¹⁰ für Wasserstoff, Methyl oder Ethyl steht, oder
R⁹ und R¹⁰ zusammen mit dem Kohlenstoffatom, an das sie gebunden sind, einen 3-bis 6-gliedrigen Carbocyclus bilden, mit der Maßgabe, dass die Reste R⁷ und R⁹ nicht beide gleichzeitg für Phenyl stehen,
oder
R⁷ und R⁹ zusammen mit den Kohlenstoffatomen, an die sie jeweils gebunden sind, sowie der Gruppe L^{1B} einen 5- bis 9-gliedrigen Carbocyclus bilden, mit der Maßgabe, dass gleichzeitig nicht mehr als eines der Reste-Paare R⁹ und R¹⁰ bzw. R⁷ und R⁹ einen Carbocyclus bildet,
- R⁴: für Wasserstoff steht,
- R⁵: für Cyclopropyl, Ethinyl, Morpholinyl oder Pyrolidinyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für eine Phenyl-Gruppe der Formel 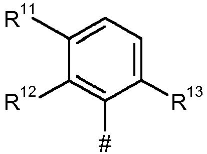 steht, wobei
# für die Anknüpfstelle an A steht,
und
R¹¹, R¹² und R¹³ unabhängig voneinander für Wasserstoff oder Fluor stehen, mit der Maßgabe, dass mindestens zwei der Reste R¹¹, R¹², R¹³ von Wasserstoff verschieden sind,
oder
für eine Pyridyl-Gruppe der Formel 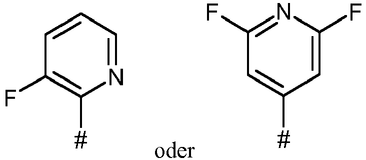 steht,
- R²: für Methyl steht,
- R³: für eine Gruppe der Formel 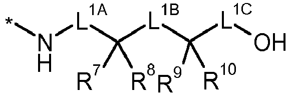 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff oder (C₁-C₆)-Alkyl steht,
worin (C₁-C₆)-Alkyl bis zu fünffach mit Fluor substituiert sein kann,
R⁸ für Wasserstoff steht,
R⁹ für Wasserstoff oder Methyl steht,
R¹⁰ für Wasserstoff oder Methyl steht,
- R⁴: für Wasserstoff steht,
- R⁵: für Cyclopropyl, Ethinyl, Morpholinyl oder Pyrolidinyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Offenbarung Verbindungen der Formel (I), in welcher
- A: für CH₂, CD₂ oder CH(CH₃) steht,
- R¹: für (C₄-C₆)-Alkyl, (C₄-C₆)-Cycloalkyl, Pyridyl oder Phenyl steht,
wobei (C₄-C₆)-Alkyl bis zu sechsmal mit Fluor substituiert sein kann,
wobei (C₄-C₆)-Cycloalkyl mit 1 bis 2 Substituenten unabhängig voneinander ausgewählt aus der Gruppe Fluor, Trifluormethyl und Methyl substituiert sein kann,
wobei Pyridyl mit 1 oder 2 Substituenten unabhängig voneinander ausgewählt aus der Gruppe Fluor, Trifluormethyl und Methyl substituiert sein kann
und
wobei Phenyl mit 1 bis 4 Substituenten unabhängig voneinander ausgewählt aus der Gruppe Fluor, Chlor, Brom, Difluormethyl, Trifluormethyl, Cyclopropyl und Methyl substituiert sein kann oder an zwei benachbarten Kohlenstoffatomen des Phenyls mit einer Difluormethylendioxy-Brücke substituiert sein kann,
- R²: für Wasserstoff, (C₁-C₄)-Alkyl, Trifluormethyl oder Cyclopropyl steht,
- R³: für eine Gruppe der Formel 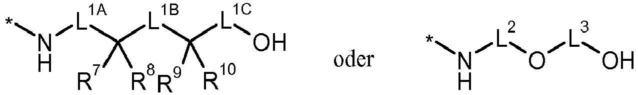 steht, wobei
* für die Anknüpfstelle an die Carbonylgruppe steht,
L^{1A} für eine Bindung oder Methylen steht,
L^{1B} für eine Bindung, Methylen oder 1,2-Ethandiyl steht,
L^{1C} für eine Bindung oder Methylen steht,
worin Methylen mit 1 bis 2 Substituenten unabhängig voneinander ausgewählt aus der Gruppe Trifluormethyl und (C₁-C₄)-Alkyl substituiert sein kann,
R⁷ für (C₁-C₆)-Alkyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy substituiert ist,
und
worin darüber hinaus (C₁-C₆)-Alkyl mit Hydroxy substituiert sein kann,
R⁸ für Wasserstoff oder Methyl steht,
R⁹ für Wasserstoff, Trifluormethyl, (C₁-C₆)-Alkyl, (C₂-C₄)-Alkenyl, (C₃-C₆)-Cycloalkyl, (C₁-C₄)-Alkoxycarbonyl, 5- oder 6-gliedriges Heteroaryl oder Phenyl steht,
worin (C₁-C₆)-Alkyl mit 1 bis 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, Methoxy und Ethoxy substituiert sein können,
R¹⁰ für Wasserstoff, Methyl oder Ethyl steht,
worin Methyl und Ethyl mit Hydroxy substituiert sein können, oder
R⁹ und R¹⁰ zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann,
L² für 1,2-Ethandiyl steht,
L³ für 1,2-Ethandiyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung Verbindungen der Formel (I), in welcher
- A: für CH₂ steht,
- R¹: für eine Phenyl-Gruppe der Formel 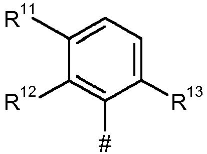 steht, wobei
# für die Anknüpfstelle an A steht, und
R¹¹, R¹² und R¹³ unabhängig voneinander für Wasserstoff, Fluor oder Chlor stehen, mit der Maßgabe, dass mindestens zwei der Reste R¹¹, R¹², R¹³ von Wasserstoff verschieden sind,
oder
für eine Pyridyl-Gruppe der Formel 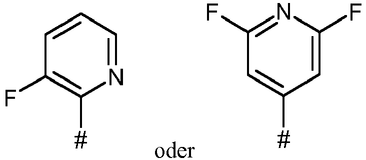 steht,
- R²: für Methyl steht,
- R³: für eine Gruppe der Formel 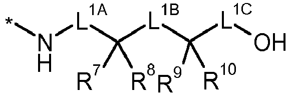 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung steht,
R⁷ für (C₁-C₆)-Alkyl steht,
worin (C₁-C₆)-Alkyl bis zu fünffach mit Fluor substituiert ist,
und
worin darüber hinaus (C₁-C₆)-Alkyl mit Hydroxy substituiert sein kann,
R⁸ für Wasserstoff steht,
R⁹ für Wasserstoff, Trifluormethyl, (C₁-C₆)-Alkyl oder Phenyl steht,
worin (C₁-C₆)-Alkyl mit (C₁-C₄)-Alkoxy oder Phenyl substituiert sein kann,
oder
worin (C₁-C₆)-Alkyl bis zu fünffach mit Fluor substituiert sein kann,
und
wobei die oben genannten Phenyl-Gruppen mit 1 oder 2 Substituenten unabhängig voneinander ausgewählt aus Fluor und Chlor substituiert sein können,
R¹⁰ für Wasserstoff, Methyl oder Ethyl steht,
oder
R⁹ und R¹⁰ zusammen mit dem Kohlenstoffatom, an das sie gebunden sind, einen 3-bis 6-gliedrigen Carbocyclus bilden,
- R⁴: für Wasserstoff steht,
- R⁵: für Wasserstoff, Fluor, Chlor, Cyano, Methyl oder Ethyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für eine Phenyl-Gruppe der Formel 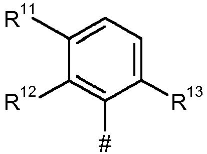 steht, wobei
# für die Anknüpfstelle an A steht, und
R¹¹, R¹² und R¹³ unabhängig voneinander für Wasserstoff oder Fluor stehen, mit der Maßgabe, dass mindestens zwei der Reste R¹¹, R¹², R¹³ von Wasserstoff verschieden sind,
oder
für eine Pyridyl-Gruppe der Formel 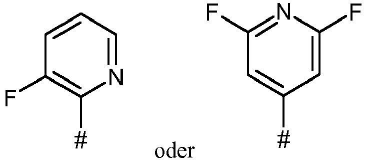 steht,
- R²: für Methyl steht,
- R³: für eine Gruppe der Formel 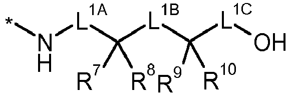 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für (C₁-C₆)-Alkyl steht,
worin (C₁-C₆)-Alkyl bis zu fünffach mit Fluor substituiert ist,
R⁸ für Wasserstoff steht,
R⁹ für Wasserstoff oder Methyl steht,
R¹⁰ für Wasserstoff oder Methyl steht,
- R⁴: für Wasserstoff steht,
- R⁵: für Wasserstoff, Chlor oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Offenbarung Verbindungen der Formel (I), in welcher
- A: für CH₂, CD₂ oder CH(CH₃) steht,
- R¹: für (C₄-C₆)-Alkyl, (C₄-C₆)-Cycloalkyl, Pyridyl oder Phenyl steht,
wobei (C₄-C₆)-Alkyl bis zu sechsmal mit Fluor substituiert sein kann,
wobei (C₄-C₆)-Cycloalkyl mit 1 bis 2 Substituenten unabhängig voneinander ausgewählt aus der Gruppe Fluor, Trifluormethyl und Methyl substituiert sein kann,
wobei Pyridyl mit 1 oder 2 Substituenten unabhängig voneinander ausgewählt aus der Gruppe Fluor, Trifluormethyl und Methyl substituiert sein kann
und
wobei Phenyl mit 1 bis 4 Substituenten unabhängig voneinander ausgewählt aus der Gruppe Fluor, Chlor, Brom, Difluormethyl, Trifluormethyl, Cyclopropyl und Methyl substituiert sein kann oder an zwei benachbarten Kohlenstoffatomen des Phenyls mit einer Difluormethylendioxy-Brücke substituiert sein kann,
- R²: für Wasserstoff, (C₁-C₄)-Alkyl, Trifluormethyl oder Cyclopropyl steht,
- R³: für eine Gruppe der Formel 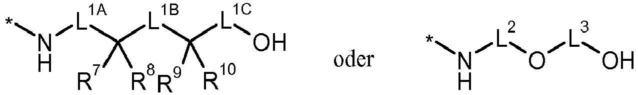 steht, wobei
* für die Anknüpfstelle an die Carbonylgruppe steht,
L^{1A} für eine Bindung oder Methylen steht,
L^{1B} für eine Bindung, Methylen oder 1,2-Ethandiyl steht,
L^{1C} für eine Bindung oder Methylen steht,
worin Methylen mit 1 bis 2 Substituenten unabhängig voneinander ausgewählt aus der Gruppe Trifluormethyl und (C₁-C₄)-Alkyl substituiert sein kann,
R⁷ für Wasserstoff, Trifluormethyl, (C₁-C₆)-Alkyl, (C₂-C₄)-Alkenyl, (C₃-C₆)-Cycloalkyl, (C₁-C₄)-Alkoxycarbonyl, 5- oder 6-gliedriges Heteroaryl oder Phenyl steht,
worin (C₁-C₆)-Alkyl mit 1 bis 2 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 oder 2 Substituenten ausgewählt aus Fluor und Chlor substituiert sein können,
worin (C₃-C₆)-Cycloalkyl mit 1 oder 2 Substituenten unabhängig voneinander ausgewählt aus der Gruppe Fluor, Trifluormethyl, Methyl und Ethyl substituiert sein kann,
und
worin Phenyl und 5- oder 6-gliedriges Heteroaryl mit 1 bis 3 Substituenten unabhängig voneinander ausgewählt aus der Gruppe Fluor, Chlor, Cyano, Trifluormethyl, (C₁-C₄)-Alkyl, Methoxy und Ethoxy substituiert sein können,
R⁸ für Wasserstoff, Methyl oder Ethyl steht,
worin Methyl und Ethyl mit Hydroxy substituiert sein können, oder
R⁷ und R⁸ zusammen mit dem Kohlenstoffatom, an das sie gebunden sind, einen 3-bis 6-gliedrigen Carbocyclus bilden,
worin der 3- bis 6-gliedrige Carbocyclus mit 1 oder 2 Substituenten unabhängig voneinander ausgewählt aus der Gruppe Fluor und Methyl substituiert sein kann,
R⁹ für (C₁-C₆)-Alkyl steht,
worin (C₁-C₆)-Alkyl mit 1 bis 3 Substituenten unabhängig voneinander ausgewählt aus der Gruppe Fluor, Difluormethyl, Trifluormethyl und Benzyloxy substituiert ist,
worin Benzyloxy mit 1 oder 2 Substituenten Fluor substituiert ist,

R¹⁰ für Wasserstoff, Methyl oder Ethyl steht,
L² für 1,2-Ethandiyl steht,
L³ für 1,2-Ethandiyl steht,
- R⁴: für Wasserstoff steht,
- R⁵: für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
- R⁶: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Offenbarung Verbindungen der Formel (I), in welcher
- A: für CH₂ steht,
- R¹: für eine Phenyl-Gruppe der Formel 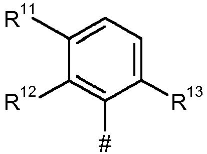 steht, wobei
# für die Anknüpfstelle an A steht, und
R¹¹, R¹² und R¹³ unabhängig voneinander für Wasserstoff, Fluor oder Chlor stehen, mit der Maßgabe, dass mindestens zwei der Reste R¹¹, R¹², R¹³ von Wasserstoff verschieden sind,
oder
für eine Pyridyl-Gruppe der Formel 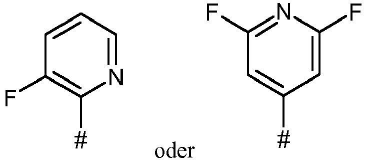 steht,
- R²: für Methyl steht,
- R³: für eine Gruppe der Formel 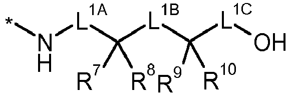 steht, wobei
* für die Anknüpfstelle an die Carbonylgruppe steht,
L^{1A} für eine Bindung steht,
L^{1B} für eine Bindung, Methylen oder 1,2-Ethandiyl steht,
L^{1C} für eine Bindung steht,
R⁷ für Wasserstoff, Trifluormethyl, (C₁-C₆)-Alkyl oder Phenyl steht,
worin (C₁-C₆)-Alkyl mit Hydroxy, (C₁-C₄)-Alkoxy oder Phenyl substituiert sein kann,
oder
worin (C₁-C₆)-Alkyl bis zu fünffach mit Fluor substituiert sein kann,
und
wobei die oben genannten Phenyl-Gruppen mit 1 oder 2 Substituenten unabhängig voneinander ausgewählt aus Fluor und Chlor substituiert sein können,
R⁸ für Wasserstoff, Methyl oder Ethyl steht, oder
R⁷und R⁸ zusammen mit dem Kohlenstoffatom, an das sie gebunden sind, einen 3-bis 6-gliedrigen Carbocyclus bilden,
R⁹ für (C₁-C₆)-Alkyl steht,
worin (C₁-C₆)-Alkyl bis zu fünffach mit Fluor substituiert ist,
oder
worin (C₁-C₆)-Alkyl mit Benzyloxy substituiert ist,
worin Benzyloxy mit 1 bis 2 Substituenten Fluor substituiert ist,

R¹⁰ für Wasserstoff oder Methyl steht,
- R⁴: für Wasserstoff steht,
- R⁵: für Wasserstoff, Fluor, Chlor, Cyano, Methyl oder Ethyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung Verbindungen der Formel (I), in welcher
- A: für CH₂ steht,
- R¹: für eine Phenyl-Gruppe der Formel 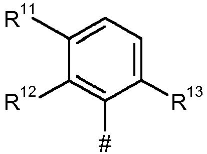 steht, wobei
# für die Anknüpfstelle an A steht, und
R¹¹, R¹² und R¹³ unabhängig voneinander für Wasserstoff oder Fluor stehen, mit der Maßgabe, dass mindestens zwei der Reste R¹¹, R¹², R¹³ von Wasserstoff verschieden sind,
oder
für eine Pyridyl-Gruppe der Formel 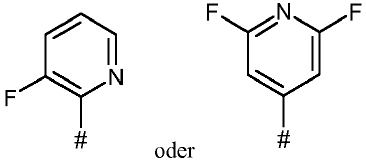 steht,
- R²: für Methyl steht,
- R³: für eine Gruppe der Formel 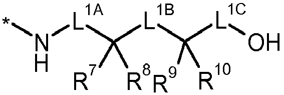 steht, wobei
* für die Anknüpfstelle an die Carbonylgruppe steht,
L^{1A} für eine Bindung steht,
L^{1B} für eine Bindung steht,
L^{1C} für eine Bindung steht,
R⁷ für Wasserstoff oder (C₁-C₆)-Alkyl steht,
R⁸ für Wasserstoff steht,
R⁹ für (C₁-C₆)-Alkyl steht,
worin (C₁-C₆)-Alkyl bis zu fünffach mit Fluor substituiert ist,
R¹⁰ für Wasserstoff steht,
- R⁴: für Wasserstoff steht,
- R⁵: für Wasserstoff, Chlor oder Methyl steht,
- R⁶: für Wasserstoff steht,
sowie ihre *N*-Oxide, Salze, Solvate, Salze der *N*-Oxide und Solvate der *N*-Oxide und Salze.

Besonders bevorzugt ist auch *ent*-8-[(3-Fluorpyridin-2-yl)methoxy]-2,6-dimethyl-*N-*(7,7,7-trifluor-2-hydroxy-2-methylheptan-3-yl)imidazo[1,2-a]pyridin-3-carboxamid (Enantiomer B) mit der folgenden Formel (I), 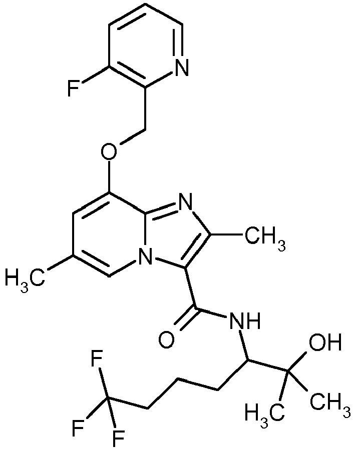 oder eines ihrer *N*-Oxide, Salze, Solvate, Salze der *N*-Oxide und Solvate der *N*-Oxide und Salze.

Besonders bevorzugt ist auch *ent*-8-[(3-Fluorpyridin-2-yl)methoxy]-2,6-dimethyl-N-(6,6,7,7,7-pentafluor-2-hydroxy-2-methylheptan-3-yl)imidazo[1,2-a]pyridin-3-carboxamid (Enantiomer B) mit der folgenden Formel (I), 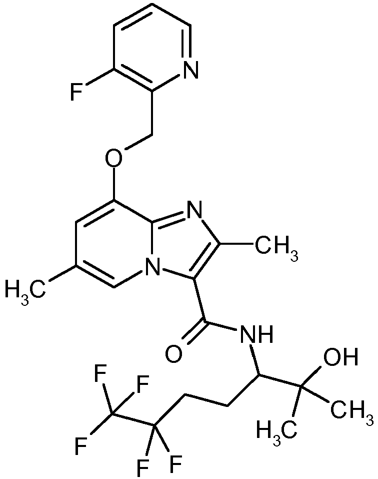 oder eines ihrer *N*-Oxide, Salze, Solvate, Salze der *N*-Oxide und Solvate der *N*-Oxide und Salze.

Besonders bevorzugt ist auch *ent*-6-Chlor-8-[(3-fluorpyridin-2-yl)methoxy]-2-methyl-N-(7,7,7-trifluor-2-hydroxy-2-methylheptan-3-yl)imidazo[1,2-a]pyridin-3-carboxamid (Enantiomer A) mit der folgenden Formel (I), 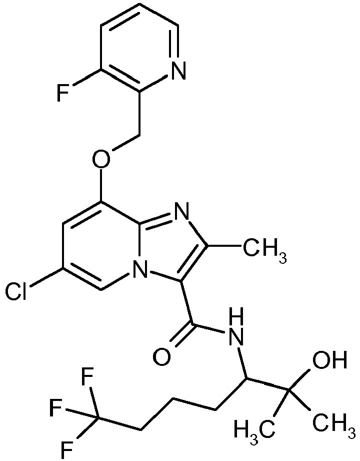 oder eines ihrer *N*-Oxide, Salze, Solvate, Salze der *N*-Oxide und Solvate der *N*-Oxide und Salze.

Besonders bevorzugt ist auch ent-6-Chlor-8-[(3-fluorpyridin-2-yl)methoxy]-2-methyl-N-(6,6,7,7,7-pentafluor-2-hydroxy-2-methylheptan-3-yl)imidazo[1,2-a]pyridin-3-carboxamid (Enantiomer A) mit der folgenden Formel (I), 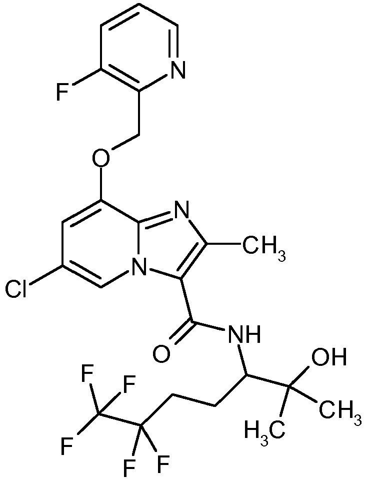 oder eines ihrer *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
R¹ für eine Gruppe der Formel 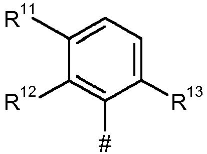 steht, wobei
- #: für die Anknüpfstelle an A steht,
und
- R¹¹, R¹² und R¹³: unabhängig voneinander für Wasserstoff, Fluor oder Chlor stehen,
mit der Maßgabe, dass mindestens zwei der Reste R¹¹, R¹², R¹³ von Wasserstoff verschieden sind, sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
R¹ für eine Pyridyl-Gruppe der Formel 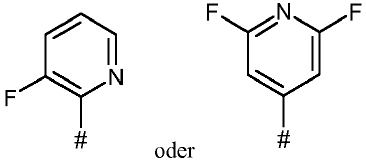 steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
R² für Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 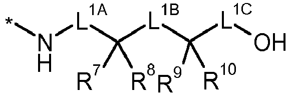 steht, wobei
* für die Anknüpfstelle an die Carbonylgruppe steht,
- L^{1A}: für eine Bindung steht,
- L^{1B}: für eine Bindung, Methylen oder 1,2-Ethandiyl steht,
L^{1C} für eine Bindung oder Methylen steht,
worin Methylen mit 1 oder 2 Substituenten unabhängig voneinander ausgewählt aus der Gruppe Trifluormethyl, (C₁-C₄)-Alkyl, Cyclopropyl und Cyclobutyl substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für eine Gruppe der Formel 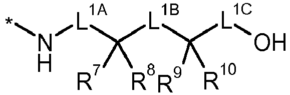 steht, wobei
* für die Anknüpfstelle an die Carbonylgruppe steht,
- R⁸: für Wasserstoff steht,
und
- R¹⁰: für Wasserstoff, Methyl oder Ethyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
R³ für eine Gruppe der Formel 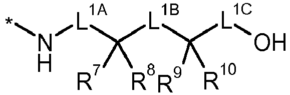 steht, wobei
- *: für die Anknüpfstelle an die Carbonylgruppe steht,
- L^{1A}: für eine Bindung steht,
- L^{1B}: für eine Bindung, Methylen oder 1,2-Ethandiyl steht,
- L^{1C}: für eine Bindung steht,
- R⁷: für (C₁-C₆)-Alkyl steht,
worin (C₁-C₆)-Alkyl bis zu fünffach mit Fluor substituiert ist,
- R⁸: für Wasserstoff steht,
- R⁹: für Wasserstoff oder Methyl steht,
- R⁸: für Wasserstoff oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
R³ für eine Gruppe der Formel 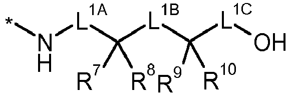 steht, wobei
- *: für die Anknüpfstelle an die Carbonylgruppe steht,
- L^{1A}: für eine Bindung steht,
- L^{1B}: für eine Bindung, Methylen oder 1,2-Ethandiyl steht,
- L^{1C}: für eine Bindung steht,
- R⁷: für Wasserstoff oder (C₁-C₆)-Alkyl steht,
- R⁸: für Wasserstoff steht,
- R⁹: für (C₁-C₆)-Alkyl steht,
worin (C₁-C₆)-Alkyl bis zu fünffach mit Fluor substituiert ist,
- R¹⁰: für Wasserstoff oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
R⁶ für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
R⁵ für Wasserstoff, Fluor, Chlor oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
R⁵ für Cyclopropyl, Ethinyl, Morpholinyl oder Pyrolidinyl steht,
sowie ihre *N*-Oxide, Salze, Solvate, Salze der *N*-Oxide und Solvate der *N*-Oxide und Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 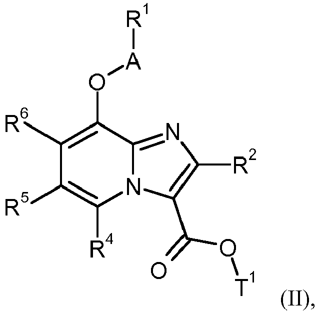
   in welcher A, R¹, R², R⁴, R⁵ und R⁶ jeweils die oben angegebenen Bedeutungen haben und
   T¹ für (C₁-C₄)-Alkyl oder Benzyl steht,
   in einem inerten Lösungsmittel in Gegenwart einer geeigneten Base oder Säure zu einer Carbonsäure der Formel (III) 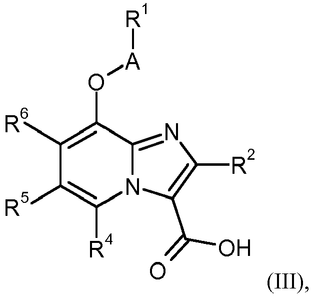 in welcher A, R¹, R², R⁴, R⁵ und R⁶ jeweils die oben angegebenen Bedeutungen haben,
   umsetzt und diese in der Folge in einem inerten Lösungsmittel unter Amidkupplungsbedingungen mit einem Amin der Formel (IV-A) 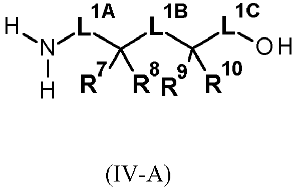 in welchem L^{1A}, L^{1B}, L^{1C}, R⁷, R⁸, R⁹ und R¹⁰ jeweils die oben angegebenen Bedeutungen haben, umsetzt
   oder
[B] eine Verbindung der Formel (III-B) 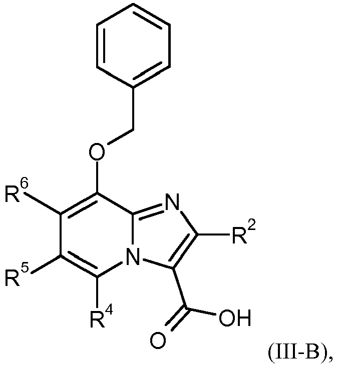 in welcher R², R⁴, R⁵ und R⁶ jeweils die oben angegebenen Bedeutungen haben,
   in einem inerten Lösungsmittel unter Amidkupplungsbedingungen mit einem Amin der Formel (IV-A) zu einer Verbindung der Formel (I-A) 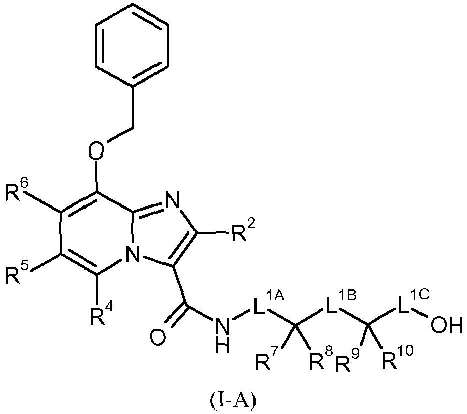 in welcher R², R⁴, R⁵, R⁶, L^{1A}, L^{1B}, L^{1C}, R⁷, R⁸, R⁹ und R¹⁰ jeweils die oben angegebenen Bedeutungen haben,
   umsetzt, von dieser im Folgenden nach dem Fachmann bekannten Methoden die Benzylgruppe abspaltet und die resultierende Verbindung der Formel (V-A) 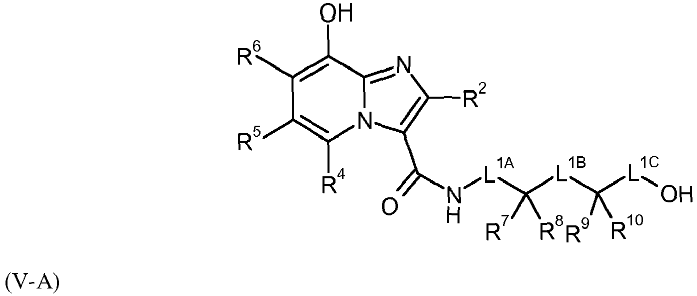 in welcher R², R⁴, R⁵, R⁶, L^{1A}, L^{1B}, L^{1C}, R⁷, R⁸, R⁹ und R¹⁰ jeweils die oben angegebenen Bedeutungen haben,
   in einem inerten Lösungsmittel in Gegenwart einer geeigneten Base mit einer Verbindung der Formel (VI) 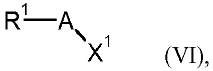 in welcher A und R¹ die oben angegebene Bedeutung haben und
   X¹ für eine geeignete Abgangsgruppe, insbesondere Chlor, Brom, Iod, Mesylat, Triflat oder Tosylat, steht,
   umsetzt,
   und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (I-A) bilden eine Teilmenge der erfindungsgemäßen Verbindungen der Formel (I).

Die beschriebenen Herstellverfahren können durch die folgenden Syntheseschemata (Schema 1 und 2) beispielhaft verdeutlicht werden: 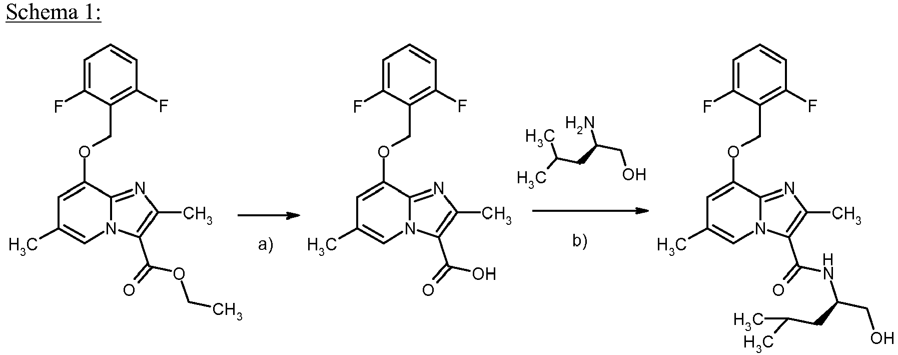 [a): Lithiumhydroxid, THF/Methanol/H₂O, RT; b): TBTU, 4-Methylmorpholin, DMF, RT]. 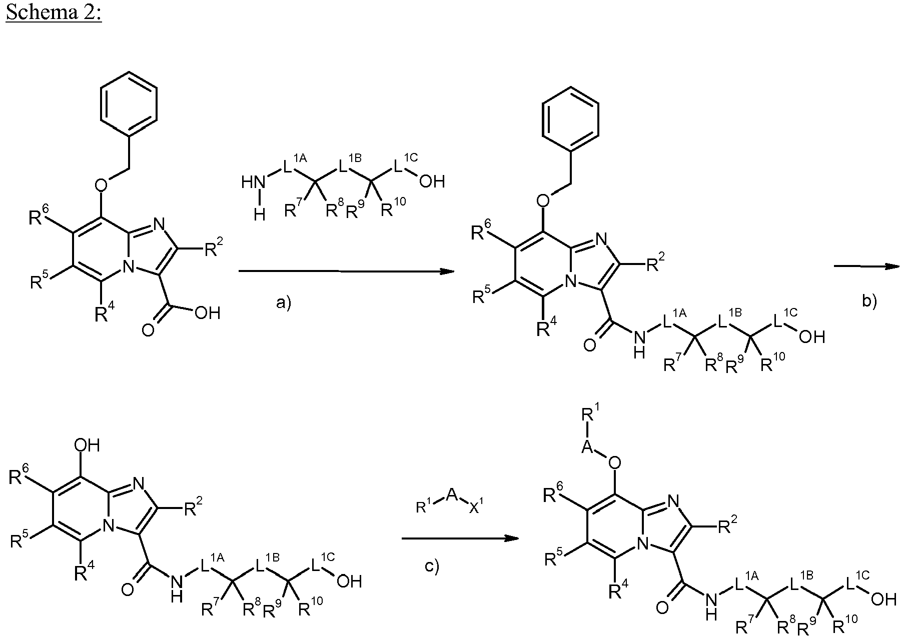 [a): TBTU, N-Methylmorpholin, DMF; b): H₂, Pd/C, Essigsäureethylester; c): Cs₂CO₃, DMF].

Die Verbindungen der Formeln (IV) und (VI) sind kommerziell erhältlich, literaturbekannt oder können in Analogie zu literaturbekannten Verfahren hergestellt werden.

Inerte Lösungsmittel für die Verfahrensschritte (III) + (IV) → (I), (III-A) + (IV-A) → (I-A) und (III-B) + (IV) → (I-B) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N-*Dimethylformamid, *N,N'*-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung in den Verfahrensschritte (III) + (IV) → (I), (III-A) + (IV-A) → (I-A) und (III-B) + (IV) → (I-B) eignen sich beispielsweise Carbodiimide wie *N,N'-*Diethyl-, *N,N'*-Dipropyl-, *N,N'*-Diisopropyl-, *N,N'*-Dicyclohexylcarbodiimid (DCC) oder *N*-(3-Dimethylaminopropyl)-*N'*-ethylcarbodiimid-Hydrochlorid (EDC), Phosgen-Derivate wie *N,N'-*Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid (T3P), 1-Chlor-*N,N*,2-trimethylprop1-en-1-amin, Cyanophosphonsäurediethylester, Bis-(2-oxo-3-oxazolidinyl)-phosphorylchlorid, Benzotriazol-1-yloxy-tris(dimethylamino)phosphonium-hexafluorphosphat, Benzotriazol-1-yloxy-tris(pyrrolidino)phosphonium-hexafluorphosphat (PyBOP), *O*-(Benzotriazol-1-yl)-*N,N,N,N'*-tetramethyluronium-tetrafluorborat (TBTU), *O*-(Benzotriazol-1-yl)-*N,N,N,N'*-tetramethyluronium-hexafluorphosphat (HBTU), 2-(2-Oxo-1-(2*H*)-pyridyl)-1,1,3,3-tetramethyluronium-tetrafluorborat (TPTU), *O*-(7-Azabenzotriazol-1-yl)-*N,N,N,N'*-tetramethyl-uronium-hexafluorphosphat (HATU) oder *O*-(1*H*-6-Chlorbenzotriazol-1-yl)-1,1,3,3-tetramethyl-uronium-tetrafluorborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N-*Diisopropylethyl-amin. Bevorzugt wird TBTU in Verbindung mit N-Methylmorpholin, HATU in Verbindung mit *N,N*-Diisopropylethylamin oder 1-Chlor-*N,N*,2-trimethylprop-1-en-1amin verwendet.

Die Kondensationen (III) + (IV) → (I), (III-A) + (IV-A) → (I-A) und (III-B) + (IV) → (I-B) werden im Allgemeinen in einem Temperaturbereich von -20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Alternativ kann die Carbonsäure der Formel (III) auch zunächst in das entsprechende Carbonsäurechlorid überführt werden und dieses dann direkt oder in einer separaten Umsetzung mit einem Amin der Formel (IV) zu den erfindungsgemäßen Verbindungen umgesetzt werden. Die Bildung von Carbonsäurechloriden aus Carbonsäuren erfolgt nach den dem Fachmann bekannten Methoden, beispielsweise durch Behandlung mit Thionylchlorid, Sulfurylchlorid oder Oxalylchlorid in Gegenwart einer geeigneten Base, beispielsweise in Gegenwart von Pyridin, sowie optional unter Zusatz von Dimethylformamid, optional in einem geeigneten inerten Lösemittel.

Die Hydrolyse der Ester-Gruppe T¹ der Verbindungen der Formel (II) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren. Im Falle der Benzylester erfolgt die Esterspaltung bevorzugt hydrogenolytisch mit Palladium auf Aktivkohle oder Raney-Nickel.

Als inerte Lösungsmittel eignen sich für diese Reaktion Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt.

Als Basen für die Ester-Hydrolyse sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Inerte Lösungsmittel für den Verfahrensschritt (V) + (VI) → (I) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N-*Dimethylformamid, *N,N-*Dimethylacetamid, Dimethylsulfoxid, *N,N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dimethylformamid oder Dimethylsulfoxid verwendet.

Als Basen für den Verfahrensschritt (V) + (VI) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)-amid oder Lithiumdiisopropylamid, oder organische Amine wie Triethylamin, *N*-Methylmorpholin, *N*-Methylpiperidin, *N,N-*Diisopropylethylamin, Pyridin, 1,5-Diazabicyclo[4.3.0]non-5-en (DBN), 4-(*N,N-*Dimethylamino)-pyridin (DMAP), 1,8-Diazabicyclo[5.4.0]undec-7-en (DBU) oder 1,4-Diazabicyclo[2.2.2]octan (DABCO^{®}). Bevorzugt wird Kaliumcarbonat, Cäsiumcarbonat oder Natriummethanolat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Gegebenenfalls vorhandene funktionelle Gruppen - wie insbesondere Amino-, Hydroxy- und Carboxylgruppen - können bei den zuvor beschriebenen Verfahrensschritten, falls zweckmäßig oder erforderlich, auch in temporär geschützter Form vorliegen. Die Einführung und Entfernung solcher Schutzgruppen erfolgt hierbei nach üblichen Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Bei Vorhandensein mehrerer geschützter Gruppen kann deren Wiederfreisetzung gegebenenfalls simultan in einer Eintopf-Reaktion oder auch in separaten Reaktionsschritten vorgenommen werden.

Als Amino- Schutzgruppe wird bevorzugt *tert*.-Butoxycarbonyl (Boc) oder Benzyloxycarbonyl (Z) verwendet. Als Schutzgruppe für eine Hydroxy- oder Carboxyl-Funktion wird vorzugsweise *tert.-*Butyl oder Benzyl eingesetzt. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Diethylether, Dichlormethan oder Essigsäure durchgeführt; gegebenenfalls kann die Abspaltung auch ohne ein zusätzliches inertes Lösungsmittel erfolgen. Im Falle von Benzyl und Benzyloxycarbonyl als Schutzgruppe können diese auch Hydrogenolyse in Gegenwart eines Palladium-Katalysators entfernt werden. Die Abspaltung der genannten Schutzgruppen kann gegebenenfalls simultan in einer Eintopf-Reaktion oder in separaten Reaktionschritten vorgenommen werden.

Die Abspaltung der Benzylgruppe im Reaktionsschritt (I-A) → (V-A) und (I-B) → (V-B) erfolgt hierbei nach üblichen, aus der Schutzgruppenchemie bekannten Methoden, vorzugsweise durch Hydrogenolyse in Gegenwart von eines Palladiumkatalysators, wie beispielsweise Palladium auf Aktivkohle, in einem inerten Lösungsmittel, wie beispielsweise Ethanol oder Essigsäureethylester [siehe auch z.B. T.W. Greene und P.G.M. Wuts, Protective Groups in Organic Synthesis, Wiley, New York, 1999].

Die Verbindungen der Formel (II) sind literaturbekannt oder können hergestellt werden, indem eine Verbindung der Formel (VII) 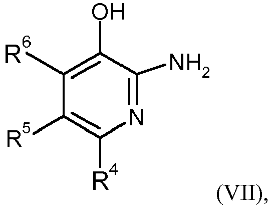 in welcher R⁴, R⁵ und R⁶ die oben angegebene Bedeutung hat,
in einem inerten Lösungsmittel in Gegenwart einer geeigneten Base mit einer Verbindung der Formel (VI) zu einer Verbindung der Formel (VIII) 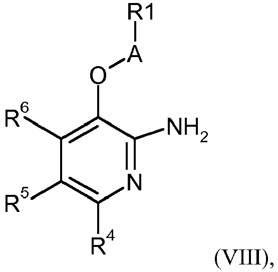 in welcher R¹ R⁴, R⁵ und R⁶ jeweils die oben angegebenen Bedeutungen haben,
umgesetzt wird, und diese anschliessend in einem inerten Lösungsmittel mit einer Verbindung der Formel (IX) 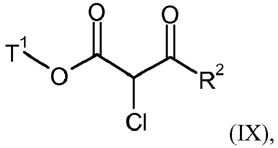 in welcher R² und T¹ jeweils die oben angegebenen Bedeutungen haben,
umgesetzt wird.

Das beschriebene Verfahren wird durch das nachfolgende Schema (Schema 3) beispielhaft verdeutlicht: 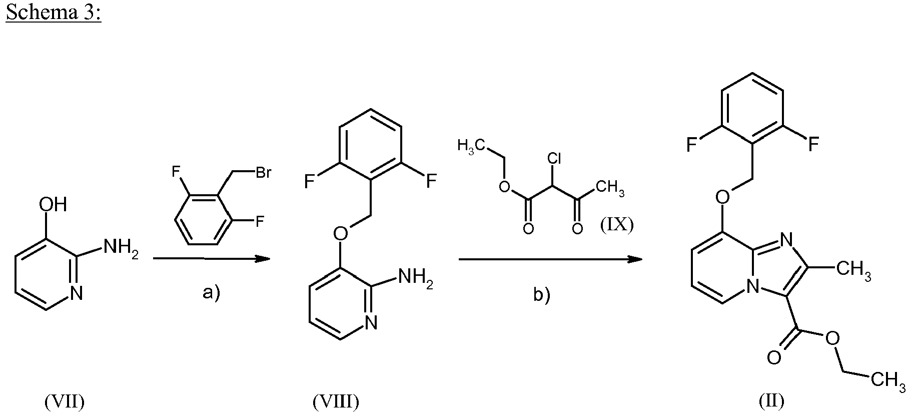 [a): i) NaOMe, MeOH, RT; ii) DMSO, RT; b): EtOH, Molekularsieb, Rückfluß].

Die gezeigte Synthesesequenz kann dahingehend modifiziert werden, dass die jeweiligen Reaktionsschritte in einer veränderten Reihenfolge durchlaufen werden. Ein Beispiel für eine solche modifizierte Synthesesequenz ist in Schema 4 gezeigt. 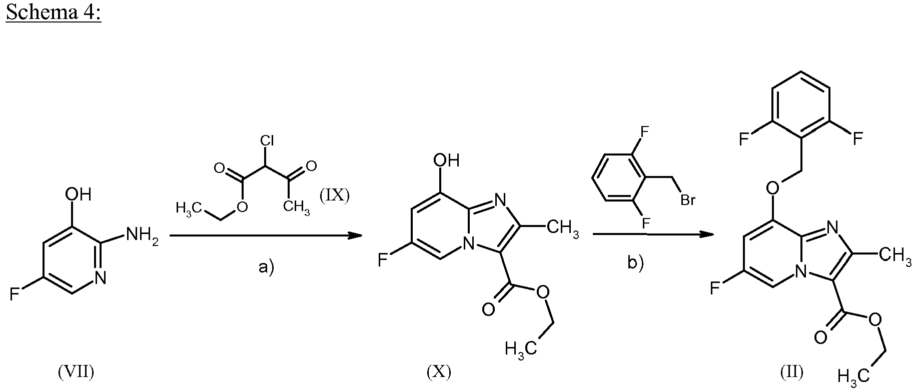 [a): EtOH, Molekularsieb, 80°C; b): b) Cs₂CO₃, DMF, 50°C].

Inerte Lösungsmittel für den Ringschluss zum Imidazo[1,2-a]pyridin-Grundgerüst (VIII) + (IX) → (II) bzw. (VII) + (IX) → (X) sind die üblichen organischen Lösungsmittel. Hierzu gehören bevorzugt Alkohole wie Methanol, Ethanol, n-Propanol, Isopropanol, n-Butanol, n-Pentanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, 1,2-Dichlorethan, Dimethylformamid, Acetonitril oder Dimethylsulfoxid. Ebenso ist es möglich, Gemische der genannten Lösungsmittel einzusetzen. Bevorzugt wird Ethanol verwendet.

Der Ringschluss erfolgt im Allgemeinen in einem Temperaturbereich von +50°C bis +150°C, bevorzugt bei +50°C bis +100°C, gegebenenfalls in einer Mikrowelle.

Der Ringschluss (VIII) + (IX) → (II) bzw. (VII) + (IX) → (X) erfolgt optional in Gegenwart wasserziehender Reaktionszusätze, beispielsweise in Gegenwart von Molekularsieb (4Å Porengröße) oder mittels Wasserabscheider. Die Umsetzung (VIII) + (IX) → (II) bzw. (VII) + (IX) → (X) erfolgt unter Verwendung eines Überschusses des Reagenzes der Formel (IX), beispielsweise mit 1 bis 20 Äquivalenten des Reagenzes (IX), gegebenenfalls unter Zusatz von Basen (wie z.B. Natriumhydrogencarbonat) wobei die Zugabe dieses Reagenzes einmalig oder in mehreren Portionen erfolgen kann.

Alternativ zu den in den Schemata 1 bis 4 gezeigten Einführungen von R¹ durch Umsetzung der Verbindungen (V), (VII) oder (X) mit Verbindungen der Formel (VI), ist es ebenso möglich - wie in Schema 5 gezeigt - diese Zwischenverbindungen mit Alkoholen der Formel (XI) unter Bedingungen der Mitsunobu-Reaktion umzusetzen.

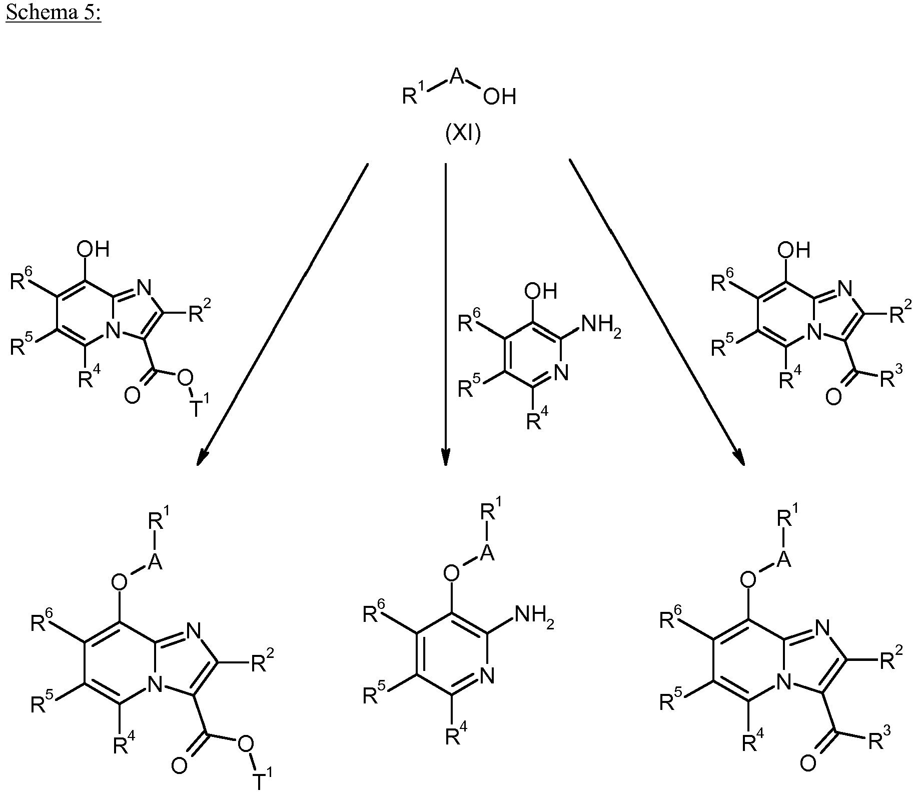

Typische Reaktionsbedingungen für derartige Mitsunobu-Kondensationen von Phenolen mit Alkoholen finden sich in der Fachliteratur, z.B. Hughes, D.L. Org. React. 1992, 42, 335; Dembinski, R. Eur. J. Org. Chem. 2004, 2763. Typischerweise wird mit einem Aktivierungsreagenz, z.B. Diethylazodicarboxylat (DEAD) oder Diisopropylazodicarboxylat (DIAD), sowie einem Phosphinreagenz, z.B. Triphenylphosphin oder Tributylphosphin, in einem inerten Lösemittel, z.B. THF, Dichlormethan, Toluol oder DMF, bei einer Temperatur zwischen 0 °C und dem Siedepunkt des verwendeten Lösemittels umgesetzt.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die erfindungsgemäßen Verbindungen besitzen wertvolle pharmakologische Eigenschaften und können zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antünflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Desweiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
antithrombotisch wirkende Mittel, beispielhaft und vorzugsweise aus der Gruppe der Thrombozytenaggregationshemmer, der Antikoagulantien oder der profibrinolytischen Substanzen;
den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: Berechnet
- br.: Verbreitertes Signal (NMR Kupplungsmuster)
- δ: Verschiebung im NMR Spektrum (Angabe in ppm)
- d: Dublett (NMR-Kupplungsmuster)
- DCI: direkte chemische Ionisation (bei MS)
- DMAP: 4*-N,N-*Dimethylaminopyridin
- DMF: Dimethylformamid
- DMSO: Dimethylsulfoxid
- d. Th.: der Theorie (bei Ausbeute)
- *ent*: Enantiomerenrein
- eq.: Äquivalent(e)
- ESI: Elektrospray-Ionisation (bei MS)
- Et: Ethyl
- gef.: Gefunden
- h: Stunde(n)
- HATU: *O*-(7-Azabenzotriazol-1-yl)-*N,N,N'N'-*tetramethyluroniumhexafluorphosphat
- HPLC: Hochdruck-, Hochleistungsflüssigchromatographie
- HRMS: hochaufgelöste Massenspektrometrie
- konz.: Konzentriert
- 1, L: Liter
- LC/MS: Flüssigchromatographie-gekoppelte Massenspektrometrie
- LiHMD S: Lithiumhexamethyldisilazid
- m: Multiplett
- Me: Methyl
- min: Minute(n)
- MS: Massenspektrometrie
- NMR: Kernresonanzspektrometrie
- Pd₂dba₃: Tris-(dibenzylidenaceton)-dipalladium
- Ph: Phenyl
- q: Quartett (NMR Kupplungsmuster)
- quint.: Quintett (NMR Kupplungsmuster)
- *rac*: Racemisch
- RT: Raumtemperatur
- Rt: Retentionszeit (bei HPLC)
- s: Singulett (NMR Kupplungsmuster)
- t: Triplett (NMR Kupplungsmuster)
- THF: Tetrahydrofuran
- TFA: Trifluoressigsäure
- TBTU: (Benzotriazol-1-yloxy)bisdimethylaminomethyliumfluoroborat
- UV: Ultraviolett-Spektrometrie
- v/v: Volumen zu Volumen-Verhältnis (einer Lösung)
- Xantphos: 4,5-Bis(diphenylphosphino)-9,9-dimethylxanthene
- XPHOS: Dicyclohexyl-(2',4',6'-triisopropylbiphenyl-2-yl)-phosphin

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Angaben zu Kopplungsmustern in NMR-Spektren sind beschreibender Natur, Kopplungsmuster höherer Ordnung werden nicht als solche beschrieben.

### LC/MS- und HPLC-Methoden:

### Methode 1 (LC-MS):

Instrument: Waters ACQUITY SQD UPLC System; Säule: Waters Acquity UPLC HSS T3 1,8µ 50 x 1mm; Eluent A: 11 Wasser + 0.25 ml 99%ige Ameisensäure , Eluent B: 1 1 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 2 (LC-MS):

Instrument: Micromass Quattro Premier mit Waters UPLC Acquity; Säule: Thermo Hypersil GOLD 1.9 µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen:50°C; UV-Detektion: 210 nm.

### Methode 3 (LC-MS):

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98% A - 0.9 min 25% A - 1.0 min 5% A - 1.4 min 5% A - 1.41 min 98% A - 1.5 min 98% A; Ofen: 40°C; Fluss: 0.600 ml/min; UV-Detektion: DAD; 210 nm.

### Methode 5 (LC-MS):

Instrument MS: Waters ZQ 2000; Instrument HPLC: Agilent 1100, 2-Säulen-Schaltung, Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A - 0.2 min 95% A - 1.8 min 25% A - 1.9 min 10% A - 2.0 min 5% A - 3.2 min 5% A - 3.21 min 100% A - 3.35 min 100% A; Ofen: 40°C; Fluss: 3.0 ml/min; UV-Detektion: 210 nm.

### Methode 6 (präparative HPLC):

Säule: Macherey-Nagel VP 50/21 Nucleosil 100-5 C18 Nautilus. Flußrate: 25 ml/min. Gradient: A = Acetonitril, B= Wasser + 0.1 % Ameisensäure, 0 min 10% A ; 2.00 min 10% A ; 6.00 min 90% A ; 7.00 min 90% A ; 7.10 min 10% A ; 8 min 10% A; UV-Detektion: 220 nm

### Methode 7 (präparative HPLC):

Säule: Phenomenex Gemini C18; 110A, AXIA, 5 µm, 21.2 X 50 mm 5micron; Gradient: A = Wasser + 0.1 % konz. Ammoniak , B = Acetonitril, 0 min = 10% B, 2 min = 10% B, 6 min = 90% B, 7 min = 90% B, 7.1 min = 10% B, 8 min = 10% B, Flußrate 25 ml/min, UV-Detektion 220 nm.

### Methode 8 (präparative HPLC):

Säule: Axia Gemini 5 µ C18 110 A, 50 x 21,5 mm, P/NO: 00B-4435-P0-AX, S/NO: 35997-2, Gradient: A=Wasser + 0.1 % konz. wässriger Ammoniak , B = Acetonitril, 0 min = 30 % B, 2 min = 30% B, 6 min = 100% B, 7 min = 100% B, 7,1 Min = 30% B, 8 Min=30% B, Flußrate 25 ml/min, UV-Detektion 220 nm.

### Methode 9 (präparative HPLC):

Säule: Macherey-Nagel VP 50/21 Nucleosil 100-5 C18 Nautilus. Flussrate: 25 ml/min. Gradient: A = Wasser + 0.1 % Ameisensäure, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 10 (präparative HPLC):

Säule: Macherey-Nagel VP 50/21 Nucleosil 100-5 C18 Nautilus. Flussrate: 25 ml/min. Gradient: A = Wasser + 0.1 % konz. aq. Ammoniak, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 11 (präparative HPLC):

Instrument MS: Waters, Instrument HPLC: Waters (Säule Waters X-Bridge C18, 18 mm x 50 mm, 5 µm, Eluent A: Wasser + 0.05% Triethylamin, Eluent B: Acetonitril (ULC) + 0.05% Triethylamin, Gradient: 0.0 min 95%A - 0.15 min 95%A - 8.0 min 5%A - 9.0 min 5%A; Fluss: 40 ml/min; UV-Detektion: DAD; 210 - 400 nm).

### bzw.:

Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäuren, Eluent B: Acetonitril (ULC) + 0.05% Ameisensäure, Gradient: 0.0 min 95%A - 0.15 min 95%A - 8.0 min 5%A - 9.0 min 5%A; Fluss: 40 ml/min; UV-Detektion: DAD; 210 - 400 nm).

### Methode 12 (LC-MS):

Instrument MS: Waters SQD; Instrument HPLC: Waters UPLC; Saeule: Zorbax SB-Aq (Agilent), 50 mm x 2.1 mm, 1.8 µm; Eluent A: Wasser + 0.025% Ameisensaeure, Eluent B: Acetonitril (ULC) + 0.025% Ameisensaeure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 13 (DCI-MS):

Gerät: DSQ II; Thermo Fisher-Scientific; DCI mit NH₃, Fluss: 1.1 ml/min; Quellentemeperatur: 200°C; Ionisierungsenergie 70 eV; DCI-Heizfaden bis 800°C aufheizen; Mass-Range 80-900.

### Methode 14 (GC-MS):

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 15 (MS):

Gerät: Waters ZQ; Ionisierungsart: ESI (+); Laufmittel; Acetonitril/Wasser.

### Methode 16 (GC-MS):

Instrument: Thermo DFS, Trace GC Ultra; Säule: Restek RTX-35, 15 m x 200 µm x 0.33 µm; konstanter Fluss mit Helium: 1.20 ml/min; Ofen: 60°C; Inlet: 220°C; Gradient: 60°C, 30°C/min → 300°C (3.33 min halten).

### Methode 17 (LC-MS):

Instrument: Waters ACQUITY SQD UPLC System; Säule: Waters Acquity UPLC HSS T3 1.8 µ 30 x 2 mm; Eluent A: 1 1 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 18 (LC-MS)

Instrument: Micromass Quattro Premier mit Waters UPLC Acquity; Säule: Thermo Hypersil GOLD 1.9 µ 50 x 1 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 19 (MS):

Instrument: Waters ZQ 2000; Elektrospray-Ionisierung; Eluent A: 1 1 Wasser + 0.25 ml 99%ige Ameisensäure, Eluent B: 1 1 Acetonitril + 0.25 ml 99%ige Ameisensäure; 25% A, 75% B; Fluss: 0.25 ml/min.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische bzw. saure Funktionalität enthalten. Ein solches Salz kann durch verschiedene dem Fachmann bekannte Methoden in die entsprechende freie Base bzw. Säure überführt werden.

Salze können unter- oder überstöchiometrisch vorliegen, insbesondere bei Vorliegen eines Amins oder einer Carbonsäure. Zusätzlich können bei den vorliegenden Imidazopyridinen unter sauren Bedingungen stets Salze, auch unterstöchiometrisch, vorliegen, ohne dass diese im ¹H-NMR erkenntlich sind und ohne besondere Angabe und Kennzeichnung dieser in den jeweiligen IUPAC-Namen und Strukturformeln.

Alle Angaben in 1H-NMR-Spektren geben die Chemischen Verschiebungen δ in ppm an.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung.

### Ausgangsverbindungen und Intermediate:

### Beispiel 1A

### Ethyl-8-(benzyloxy)-2-methylimidazo[1,2-a]pyridin-3-carboxylat

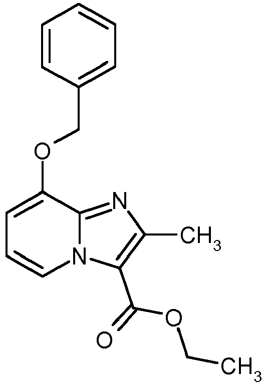

25 g (124.8 mmol) 2-Amino-3-benzyloxypyridin wurde in 781 ml Ethanol gelöst, mit 102.7 g (624.2 mmol) Ethyl-2-chloracetoacetat und zwei Esslöffeln 4A Molsieb versetzt, und dann wurde das Reaktionsgemisch für 2 Tage zum Rückfluß erhitzt (Badtemperatur 100°C). Der Ansatz wurde eingeengt, und am Rotationsverdampfer mit Trockeneiskühlung wurde das überschüssige Ethyl-2-chloracetoacetat abdestilliert. Der Rückstand wurde über eine Kieselgelchromatographie gereinigt (Laufmittel Cyclohexan:Essigsäureethylester - Gradient 9:1, 4:1). Es wurden 20.81 g der Zielverbindung (54% d. Th., Reinheit 99%) erhalten.
LC-MS (Methode 2): Rₜ = 1.12 min
MS (ESpos): m/z = 311 (M+H)⁺
¹H-NMR (400 MHz, DMSO-d₆): δ = 1.35 (t, 3H), 2.59 (s, 3H), 4.34 (q, 2H), 5.32 (s, 2H), 7.01-7.09 (m, 2H), 7.33-7.48 (m, 3H), 7.52 (d, 2H), 8.81-8.86 (m, 1H).

### Beispiel 2A

### 8-(Benzyloxy)-2-methylimidazo[1,2-a]pyridin-3-carbonsäure

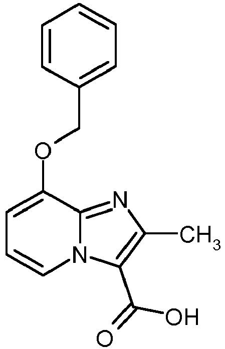

Eine Lösung von 15.7 g (50.59 mmol) Ethyl-8-(benzyloxy)-2-methylimidazo[1,2-a]pyridin-3-carboxylat in 253 ml Dioxan wurde mit 253 ml 2N Natronlauge versetzt und 14 Stunden bei Raumtemperatur gerührt. Dann wurde der Ansatz mit 101 ml 6N Salzsäure versetzt. Der entstandene Feststoff wurde abfiltriert, mit Wasser und mit Methyl-tert.-butylether nachgewaschen und dann über Nacht im Vakuumtrockenschrank bei 40 °C getrocknet. So erhielt man 15.49 g (108% d. Th.) 8-(Benzyloxy)-2-methylimidazo[1,2-a]pyridin-3-carbonsäure als farblosen Feststoff. Die Ausbeute liegt über 100% aufgrund von Kristallwasser (¹H-NMR).
LC-MS (Methode 1): Rₜ = 0.66 min
MS (ESpos): m/z = 283.0 (M+H)⁺
¹H-NMR (400 MHz, DMSO-d₆): δ = 2.67 (s, 3H), 3.2 - 3.8 (sehr breiter Wasserpeak), 5.41 (s, 2H), 7.30 (m, 1H), 7.35 - 7.48 (m, 4H), 7.57 (d, 2H), 9.02 (d, 1H).

### Beispiel 3A

### Ethyl 8-hydroxy-2-methylimidazo[1,2-a]pyridin-3-carboxylat

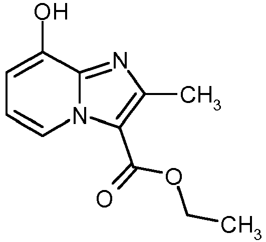

31.45 g (101.3 mmol) Ethyl-8-(benzyloxy)-2-methylimidazo[1,2-a]pyridin-3-carboxylat wurden in 2 1 Essigsäureethylester gelöst, mit 3.15 g 10%iger Pd/Kohle versetzt und 5 h bei RT und Normaldruck mit Wasserstoff gerührt. Der Ansatz wurde über Kieselgur filtriert, gut mit Essigsäureethylester/Methanol nachgewaschen und das Filtrat zur Trockene eingedampft. Es wurden 21.94 g der Zielverbindung (98% d. Th., Reinheit 99%) erhalten.
LC-MS (Methode 1): Rₜ = 0.61 min
MS (ESpos): m/z = 221 (M+H)⁺
¹H-NMR (400 MHz, DMSO-d₆): δ = 1.36 (t, 3H), 2.60 (s, 3H), 4.36 (q, 2H), 6.78 (d, 1H), 6.98 (t, 1H), 8.73 (d, 1H), 10.60 (br s, 1H).

### Beispiel 4A

### 3-[(2,6-Difluorbenzyl)oxy]pyridin-2-amin

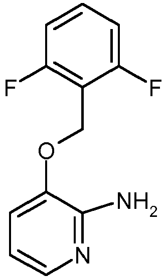

51 g Natriummethanolat (953 mmol, 1.05 Äquivalente) wurden in 1000 mL Methanol bei RT vorgelegt, mit 100 g 2-Amino-3-hydroxypyridin (908 mmol, 1 Äquivalent) versetzt und 15 min bei RT gerührt. Die Reaktionsmischung wurde am Vakuum aufkonzentriert, der Rückstand in 2500 mL DMSO aufgenommen und mit 197 g 2,6-Difluorbenzylbromid (953 mmol, 1.05 Äquivalente) versetzt. Nach 4 h bei RT wurde das Reaktionsgemisch auf 20 L Wasser gegossen, für 15 min gerührt und der Feststoff abfiltriert. Der Feststoff wurde mit 1 L Wasser sowie 100 mL Isopropanol und 500 mL Petrolether nachgewaschen und im Hochvakuum getrocknet. Es wurden 171 g der Titelverbindung (78% d. Th) erhalten.
¹H-NMR (400 MHz, DMSO-d₆): δ = 5.10 (s, 2 H); 5.52 (br. s, 2 H), 6.52 (dd, 1 H); 7.16 - 7.21 (m, 3 H); 7.49 - 7.56 (m, 2 H).

### Beispiel 5A

### Ethyl-8-[(2,6-difluorbenzyl)oxy]-2-methylimidazo[1,2-a]pyridin-3-carboxylat

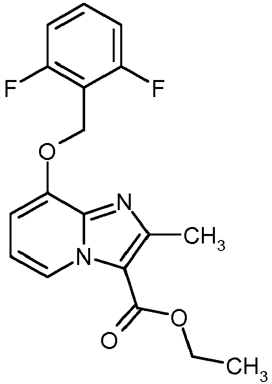

170 g 3-[(2,6-Difluorbenzyl)oxy]pyridin-2-amin (Beispiel 4A; 719 mmol, 1 Äquivalent) wurden in 3800 mL Ethanol vorgelegt und mit 151 g gepulvertem Molsieb 3Å sowie 623 g Ethyl-2-chloracetoacetat (3.6 mol, 5 Äquivalente) versetzt. Die resultierende Reaktionsmischung wurde für 24 h zum Rückfluß erhitzt, anschließend über Kieselgur abfiltriert und am Vakuum aufkonzentriert. Nach längerem Stehen (48h) bei RT fiel ein Feststoff aus. Dieser wurde filtriert, dreimal mit wenig Isopropanol aufgerührt und jeweils abfiltriert und abschließend mit Diethylether gewaschen. Es wurden 60.8 g (23.4% d. Th.) der Titelverbindung erhalten. Die vereinigte Mutterlauge der Filtrationsschritte wurde an Kieselgel mit Cyclohexan/Diethylether als Eluent chromatographiert und lieferte weitere 46.5 g (18.2 % d. Th.; Gesamtausbeute: 41.6% d. Th.) der Titelverbindung.
LC-MS (Methode 1): Rₜ = 1.01 min
MS (ESpos): m/z = 347 (M+H)⁺
¹H-NMR (400 MHz, DMSO-d₆): δ = 1.36 (t, 3 H); 2.54 (s, 3 H; verdeckt durch DMSO-Signal); 4.36 (q, 2 H); 5.33 (s, 2 H); 7.11 (t, 1 H); 7.18 - 7.27 (m, 3 H); 7.59 (quint, 1 H); 8.88 (d, 1 H).

### Beispiel 6A

### 8-[(2,6-Difluorbenzyl)oxy]-2-methylimidazo[1,2-a]pyridin-3-carbonsäure

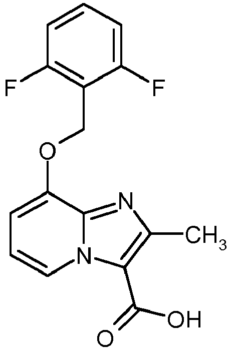

107 g Ethyl-8-[(2,6-difluorbenzyl)oxy]-2-methylimidazo[1,2-a]pyridin-3-carboxylat (Beispiel 5A; 300 mmol, 1 Äquivalent) wurde in 2.8 L THF/Methanol (1:1) gelöst, mit 1.5 L 1N wässriger Lithiumhydroxid-Lösung (1.5 mol, 5 Äquivalente) versetzt und bei RT für 16 h gerührt. Die organischen Lösemittel wurden am Vakuum entfernt und die resultierende wässrige Lösung im Eisbad mit 1N Salzsäure auf pH 3-4 eingestellt. Der resutierende Feststoff wurde abfiltriert, mit Wasser und Isopropanol nachgewaschen und im Vakuum getrocknet. Es wurden 92 g (95% d. Th.) der Titelverbindung erhalten.
LC-MS (Methode 1): Rₜ = 0.62 min
MS (ESpos): m/z = 319.1 (M+H)⁺
¹H-NMR (400 MHz, DMSO-d₆): δ = 2.55 (s, 3 H; überlagert durch DMSO-Signal); 5.32 (s, 2 H); 7.01 (t, 1 H); 7.09 (d, 1 H); 7.23 (t, 2 H); 7.59 (quint, 1 H); 9.01 (d, 1 H).

### Beispiel 7A

### 3-(Cyclohexylmethoxy)pyridin-2-amin

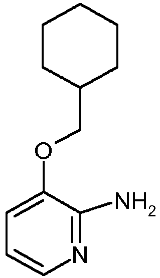

96 g Natriumhydroxid (45%; 1081 mmol, 1 Äquivalente) wurden in 1170 mL Methanol bei RT vorgelegt, mit 119 g 2-Amino-3-hydroxypyridin (1080 mmol, 1 Äquivalent) versetzt und 10 min bei RT gerührt. Die Reaktionsmischung wurde am Vakuum aufkonzentriert, der Rückstand in 2900 mL DMSO aufgenommen und mit 101 g Cyclohexylmethylbromid (1135 mmol, 1.05 Äquivalente) versetzt. Nach 16 h bei RT wurde das Reaktionsgemisch in 6 L Wasser eingerührt und die wässrige Lösung zweimal mit je 2 L Essigsäureethylester extrahiert. Die vereinigten organischen Phasen wurden mit je 1 L gesättigter wässriger Natriumhydrogencarbonat-Lösung und Wasser gewaschen, getrocknet, filtriert und eingeengt. Der Rückstand wurde mit 500 mL Pentan verrührt, abfiltriert und am Vakuum getrocknet. Es wurden 130 g (58.3% d. Th.) der Titelverbindung erhalten.
LC-MS (Methode 3): Rₜ = 1.41 min
MS (ESpos): m/z = 207.1 (M+H)⁺

### Beispiel 8A

### Ethyl-8-(cyclohexylmethoxy)-2-methylimidazo[1,2-a]pyridin-3-carboxylat

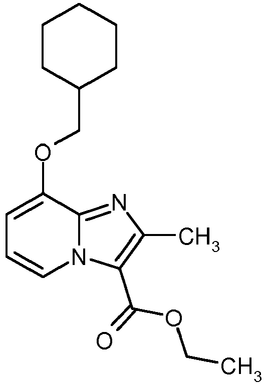

130 g 3-(Cyclohexylmethoxy)pyridin-2-amin (Beispiel 7A; 630 mmol, 1 Äquivalent) wurden in 3950 mL Ethanol vorgelegt und mit 436 mL Ethyl-2-chloracetoacetat (3.2 mol, 5 Äquivalente) versetzt. Die resultierende Reaktionsmischung wurde für 24 h am Rückfluß erhitzt und anschließend im Vakuum aufkonzentriert. Das so erhaltene Rohprodukt wurde an Kieselgel mit Cyclohexan/Diethylether als Eluent chromatographiert, und lieferte 66.2 g (33.2 % d. Th.) der Titelverbindung.
LC-MS (Methode 1): Rₜ = 1.17 min
MS (ESpos): m/z = 317.1 (M+H)⁺
¹H-NMR (400 MHz, DMSO-d₆): δ = 1.02-1.31 (m, 5 H); 1.36 (t, 3 H); 1.64 - 1.77 (m, 3 H); 1.79 - 1.90 (m, 3 H); 2.60 (s, 3 H); 3.97 (d, 2 H); 4.35 (q, 2 H); 6.95 (d, 1 H); 7.03 (t, 1 H); 8.81 (d, 1 H).

### Beispiel 9A

### 8-(Cyclohexylmethoxy)-2-methylimidazo[1,2-a]pyridin-3-carbonsäure

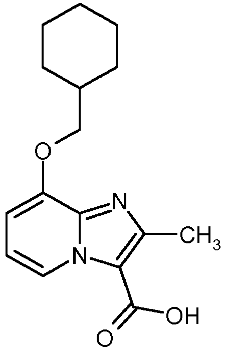

50 g Ethyl-8-(cyclohexylmethoxy)-2-methylimidazo[1,2-a]pyridin-3-carboxylat (Beispiel 8A; 158 mmol, 1 Äquivalent) wurden in 600 mL Dioxan gelöst, mit 790 mL 2 N Natronlauge (1.58 mol, 10 Äquivalente) versetzt und bei RT für 16 h gerührt. Der Ansatz wurde mit 316 mL 6 N Salzsäure versetzt und auf ca. 1/5 des Gesamtvolumens eingeengt. Der resutierende Feststoff wurde abfiltriert, mit Wasser und tert.-Butylmethylether nachgewaschen und im Vakuum getrocknet. Es wurden 35 g (74% d. Th.) der Titelverbindung erhalten.
LC-MS (Methode 1): Rₜ = 0.81 min
MS (ESpos): m/z = 289.0 (M+H)⁺
¹H-NMR (400 MHz, DMSO-d₆): δ = 1.03-1.44 (m, 5 H); 1.64 - 1.78 (m, 3 H); 1.81 - 1.92 (m, 3 H); 2.69 (s, 3 H); 4.07 (d, 2 H); 7.30 - 7.36 (m, 2 H); 9.01 (d, 1 H).

### Beispiel 10A

### 5-Fluor-2-nitropyridin-3-ol

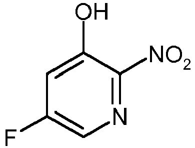

5 g 5-Fluorpyridin-3-ol (44 mmol, 1 Äquivalent) wurden unter Eiskühlung in 43 mL konzentrierter Schwefelsäure gelöst und bei 0 °C innerhalb von 5 min mit 2.8 mL konzentrierter Salpetersäure versetzt. Die Reaktion wurde auf RT erwärmt und über Nacht gerührt. Der Ansatz wurde auf 100 g Eis gegeben und für 30 min nachgerührt. Der entstandene Feststoff wurde abfiltriert und im Vakuum getrocknet. Es wurden 5.6 g (81% d. Th.) der Titelverbindung erhalten und ohne weitere Aufreinigung in die Folgereaktion eingesetzt.
LC-MS (Methode 1): Rₜ = 0.45 min
MS (ESneg): m/z = 156.9 (M-H)-
¹H-NMR (400 MHz, DMSO-d₆): δ = 7.5 (dd, 1 H); 8.08 (d, 1 H); 12.2 (br. s, 1 H).

### Beispiel 11A

### 2-Amino-5-fluorpyridin-3-ol

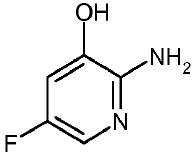

5.6 g 5-Fluor-2-nitropyridin-3-ol (Beispiel 10A; 36 mmol) wurden in 2 L Ethanol gelöst, mit einer katalytischen Menge Palladium auf Aktivkohle (10%-ig) versetzt und für 16 h unter Wasserstoff-Normaldruck hydriert. Der Ansatz wurde über Kieselgur abfiltriert und das Filtrat eingeengt (Produktcharge 1). Der Rückstand wurde mit Methanol solange nachgespült bis das Filtrat keine gelbliche Färbung mehr aufwies. Das Filtrat wurde eingeengt und ergab eine zweite Produktcharge. Insgesamt wurden 4.26 g (85% d. Th.) der Titelverbindung erhalten.
LC-MS (Methode 1): Rₜ = 0.17 min
MS (ESpos): m/z = 128.9 (M+H)⁺
¹H-NMR (400 MHz, DMSO-d₆): δ = 5.4 (br. s, 2 H); 6.8 (dd, 1 H); 7.4 (d, 1 H).

### Beispiel 12A

### Ethyl-6-fluor-8-hydroxy-2-methylimidazo[1,2-a]pyridin-3-carboxylat

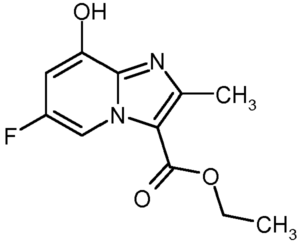

3.2 g 2-Amino-5-fluorpyridin-3-ol (Beispiel 11A; 25 mmol, 1 Äquivalent) wurden in 155 mL Ethanol vorgelegt, mit 1.5 g gepulvertem Molsieb 3Å und 20.6 g Ethyl-2-chloracetoacetat (125 mmol, 5 Äquivalente) versetzt und über Nacht am Rückfluß erhitzt. Die Reaktionslösung wurde aufkonzentriert und chromatographiert (Biotage Isolera Four; SNAP Cartridge KP-Sil 50 g; Cyclohexan-Essigsäureethylester Gradient; nachfolgend Dichlormethan-Methanol-Gradient). Das Rohprodukt wurde in wenig Methanol angelöst und mit tert.-Butylmethylether versetzt. Der erhaltene Feststoff wurde abfiltriert und mit tert.-Butylmethylether nachgespült. Es wurden 570 mg (10% d. Th.) der Titelverbindung erhalten.
LC-MS (Methode 1): Rₜ = 0.77 min
MS (ESpos): m/z = 239.2 (M+H)⁺
¹H-NMR (400 MHz, DMSO-d₆): δ = 1.39 (t, 3 H); 2.64 (s, 3 H); 4.40 (q, 2 H); 7.20 (br. d, 1 H); 8.9 (dd, 1 H); 12.5 (br., 1 H).

### Beispiel 13A

### Ethyl-8-[(2,6-difluorbenzyl)oxy]-6-fluor-2-methylimidazo[1,2-a]pyridin-3-carboxylat

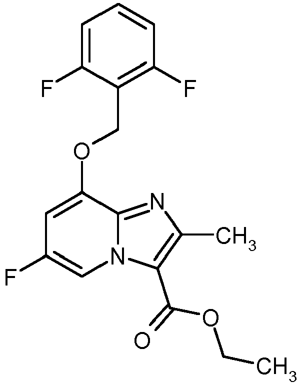

560 mg Ethyl-6-fluor-8-hydroxy-2-methylimidazo[1,2-a]pyridin-3-carboxylat (Beispiel 12A; 2.4 mmol, 1.0 Äquivalent), 1.7 g Cäsiumcarbonat (5.17 mmol, 2.2 Äquivalente) und 535 mg 2,6-Difluorbenzylbromid (2.6 mmol, 1.1 Äquivalente) wurden in 34 mL trockenem DMF vorgelegt und für 15 min auf 50°C erwärmt. Der Ansatz wurde mit Wasser versetzt und 30 min nachgerührt. Der Feststoff wurde abfiltriert und mit Wasser nachgewaschen. Es wurden 560 mg der Titelverbindung (65% d. Th.) erhalten.
LC-MS (Methode 1): Rₜ = 1.18 min
MS (ESpos): m/z = 365.1 (M+H)⁺
¹H-NMR (400 MHz, DMSO-d₆): δ = 1.37 (t, 3 H); 2.55 (s, 3 H; überlagert durch DMSO-Signal); 4.38 (q, 2 H); 5.89 (s, 2 H); 7.23 (t, 2 H); 7.44 (dd, 1 H); 7.60 (quint., 1 H); 8.90 (dd, 1 H).

### Beispiel 14A

### 8-[(2,6-Difluorbenzyl)oxy]-6-fluor-2-methylimidazo[1,2-a]pyridin-3-carbonsäure

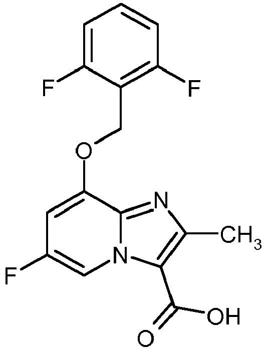

550 mg Ethyl-8-[(2,6-difluorbenzyl)oxy]-6-fluor-2-methylimidazo[1,2-a]pyridin-3-carboxylat (Beispiel 13A; 1.5 mmol, 1 Äquivalent) wurden in 64 mL THF und 12 mL Methanol gelöst , mit 7.5 mL 1N wässriger Lithiumhydroxid-Lösung versetzt und über Nacht bei RT gerührt. Dann wurde mit 8 mL 1N Salzsäure versetzt und aufkonzentriert. Der entstandene Feststoff wurde abfiltriert und mit Wasser nachgewaschen. Es wurden 429 mg der Titelverbindung (80% d. Th.) erhalten.
LC-MS (Methode 2): Rₜ = 0.90 min
MS (ESpos): m/z = 337.1 (M+H)⁺
¹H-NMR (400 MHz, DMSO-d₆): δ = 2.54 (s, 3 H; überlagert durch DMSO-Signal); 5.84 (s, 2 H); 7.23 (t, 2 H); 7.40 (dd, 1 H); 7.51 (quint., 1 H); 8.92 (dd, 1 H); 13.28 (br. s, 1 H).

### Beispiel 15A

### 5-Chlor-2-nitropyridin-3-ol

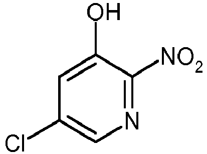

30 g 5-Chlorpyridin-3-ol (232 mmol, 1 Äquivalent) wurden unter Eiskühlung in 228 mL konzentrierter Schwefelsäure gelöst und bei 0 °C langsam mit 24 mL konzentrierter Salpetersäure versetzt. Die Reaktion wurde auf RT erwärmt und über Nacht weitergerührt. Der Ansatz wurde in ein Eis/Wasser-Gemisch eingerührt und für 30 min nachgerührt. Der Fesstoff wurde abfiltriert, mit kaltem Wasser nachgewaschen und an der Luft getrocknet. Es wurden 33 g (82% d. Th.) der Titelverbindung erhalten und ohne weitere Aufreinigung in die Folgereaktion eingesetzt.
LC-MS (Methode 1): Rₜ = 0.60 min
MS (ESneg): m/z = 172.9/174.9 (M-H)⁻
¹H-NMR (400 MHz, DMSO-d₆): δ = 7.71 (d, 1 H); 8.10 (d, 1 H); 12.14 (br. 1 H).

### Beispiel 16A

### 5-Chlor-3-[(2,6-difluorbenzyl)oxy]-2-nitropyridin

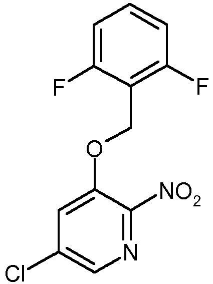

33 g 5-Chlor-2-nitropyridin-3-ol (Beispiel 15A; 189 mmol, 1 Äquivalent) und 61.6 g Cäsiumcarbonat (189 mmol, 1 Äquivalent) wurden in 528 mL DMF vorgelegt, mit 40.4 g 2,6-Difluorbenzylbromid (189 mmol, 1 Äquivalent) versetzt und bei RT über Nacht gerührt. Das Reaktionsgemisch wurde in Wasser/1N Salzsäure eingerührt, der Feststoff abfiltriert, mit Wasser nachgewaschen und an der Luft getrocknet. Es wurden 54.9 g (97% d. Th.) der Titelverbindung erhalten.
¹H-NMR (400 MHz, DMSO-d₆): δ = 5.46 (s, 2 H); 7.22 (t, 2 H); 7.58 (quint., 1 H); 8.28 (d, 1 H); 8.47 (d, 1 H).

### Beispiel 17A

### 5-Chlor-3-[(2,6-difluorbenzyl)oxy]pyridin-2-amin

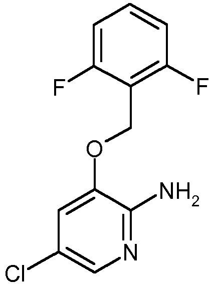

59.7 g 5-Chlor-3-[(2,6-difluorbenzyl)oxy]-2-nitropyridin (Beispiel 16A; 199 mmol, 1 Äquivalent) wurden in 600 mL Ethanol vorgelegt, mit 34.4 g Eisenpulver (616 mmol, 3.1 Äquivalente) versetzt und zum Rückfluß erhitzt. Es wurden langsam 152 mL konzentrierte Salzsäure zugetropft und dann für weitere 30 Minuten am Rückfluß gekocht. Das Reaktionsgemisch wurde abgekühlt und in ein Eis-Wassergemisch eingerührt. Das resultierende Gemisch wurde mit Natriumacetat auf pH 5 eingestellt, der Feststoff abfiltriert, mit Wasser nachgewaschen und an der Luft und anschließend im Vakuum bei 50 °C getrocknet. Es wurden 52.7 g (98% d. Th.) der Titelverbindung erhalten.
LC-MS (Methode 1): Rₜ = 0.93 min
MS (ESpos): m/z = 271.1/273.1 (M+H)⁺
¹H-NMR (400 MHz, DMSO-d₆): δ = 5.14 (s, 2 H); 5.82 (br. s, 2 H); 7.20 (t, 2 H); 7.35 (d, 1 H); 7.55 (quint., 1 H); 7.56 (d, 1 H).

### Beispiel 18A

### Ethyl-6-chlor-8-[(2,6-difluorbenzyl)oxy]-2-methylimidazo[1,2-a]pyridin-3-carboxylat

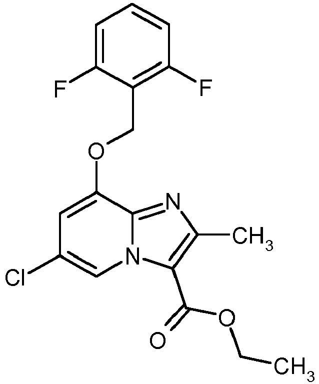

40 g 5-Chlor-3-[(2,6-difluorbenzyl)oxy]pyridin-2-amin (Beispiel 17A; 147.8 mmol; 1 Äquivalent) wurden in 800 mL Ethanol vorgelegt, mit 30 g gepulvertem Molsieb 3Å und 128 g Ethyl-2-chloracetoacetat (739 mmol, 5 Äquivalente) versetzt und über Nacht zum Rückfluß erhitzt. Das Reaktionsgemisch wurde aufkonzentriert, der Rückstand in Essigsäureethylester aufgenommen und filtriert. Die Essigsäureethylester-Phase wurde mit Wasser gewaschen, getrocknet, filtriert und eingeengt. Es wurden 44 g (78% d. Th.) der Titelverbindung erhalten.
LC-MS (Methode 1): Rₜ = 1.27 min
MS (ESpos): m/z = 381.2/383.2 (M+H)⁺
¹H-NMR (400 MHz, DMSO-d₆): δ = 1.36 (t, 3 H); 2.54 (s, 3 H; verdeckt durch DMSO-Signal); 4.37 (q, 2 H); 5.36 (s, 2 H); 7.26 (t, 2 H); 7.38 (d, 1 H); 7.62 (quint., 1 H); 8.92 (d, 1 H).

### Beispiel 19A

### 6-Chlor-8-[(2,6-difluorbenzyl)oxy]-2-methylimidazo[1,2-a]pyridin-3-carbonsäure

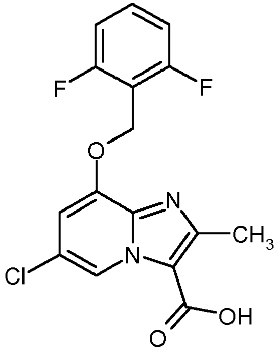

44 g Ethyl-6-chlor-8-[(2,6-difluorbenzyl)oxy]-2-methylimidazo[1,2-a]pyridin-3-carboxylat (Beispiel 18A; 115.5 mmol, 1 Äquivalent) wurden in 550 mL THF und 700 mL Methanol gelöst, mit 13.8 g Lithiumhydroxid (gelöst in 150 mL Wasser; 577 mmol, 5 Äquivalente) versetzt und über Nacht bei RT gerührt. Dann wurde mit 1N Salzsäure versetzt und aufkonzentriert. Der entstandene Feststoff wurde abfiltriert und mit Wasser nachgewaschen. Es wurden 34 g der Titelverbindung (84% d. Th.) erhalten.
LC-MS (Methode 2): Rₜ = 1.03 min
MS (ESpos): m/z = 353.0/355.0 (M+H)⁺
¹H-NMR (400 MHz, DMSO-d₆): δ = 2.54 (s, 3 H; überlagert von DMSO-Signal); 5.36 (s, 2 H); 7.26 (t, 2 H); 7.34 (d, 1 H); 7.61 (quint., 1 H); 8.99 (d, 1 H); 13.36 (br. s, 1 H).

### Beispiel 20A

### 5-Brom-3-[(2,6-difluorbenzyl)oxy]pyridin-2-amin

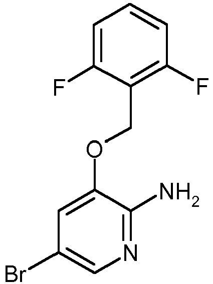

32.6 g 3-[(2,6-Difluorbenzyl)oxy]pyridin-2-amin (Beispiel 4A; 138 mmol, 1 Äquivalent) wurden in 552 mL 10%ige Schwefelsäure suspendiert und auf 0°C gekühlt. 8.5 mL Brom (165 mmol, 1.2 Äquivalente) wurden in 85 mL Essigsäure gelöst und dann innerhalb von 90 min zur eisgekühlten Reaktionslösung getropft. Nach erfolgter Zugabe wurde 90 min bei 0°C nachgerührt, anschließend mit 600 mL Essigsäureethylester verdünnt und die wässrige Phase abgetrennt. Die wässrige Phase wurde mit Essigsäureethylester nachextrahiert, die organischen Phasen wurden vereinigt, mit gesättigter wässriger Natriumhydrogencarbonat-Lösung gewaschen, getrocknet und eingeengt. Der Rückstand wurde in Dichlormethan gelöst und an Kieselgel chromatographiert (Petrolether/Essigsäureethylester Gradient als Eluent). Es wurden 24 g (55% d. Th.) der Titelverbindung erhalten. LC-MS (Methode 1): Rₜ = 0.96 min
MS (ESpos): m/z = 315.1/317.1 (M+H)^{+ 1}H-NMR (400 MHz, DMSO-d₆): δ = 5.14 (s, 2 H); 5.83 (br. s, 2 H); 7.20 (t, 2 H); 7.42 (d, 1 H); 7.54 (quint., 1 H); 7.62 (d, 1 H).

### Beispiel 21A

### Ethyl-6-brom-8-[(2,6-difluorbenzyl)oxy]-2-methylimidazo[1,2-a]pyridin-3-carboxylat

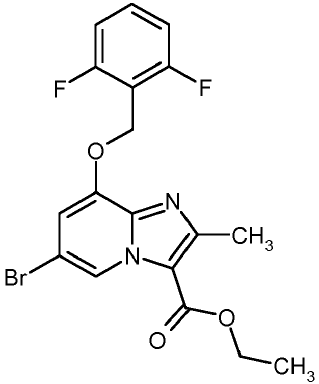

24 g 5-Brom-3-[(2,6-difluorbenzyl)oxy]pyridin-2-amin (Beispiel 20A; 76.2 mmol; 1 Äquivalent) wurden in 400 mL Ethanol vorgelegt, mit 16 g gepulvertem Molsieb 3Å und 52.7 mL Ethyl-2-chloracetoacetat (380.8, 5 Äquivalente) versetzt und über Nacht zum Rückfluß erhitzt. Es wurden weitere 8 g Molsieb zugegeben und für weitere 24 h zum Rückfluß erhitzt. Das Reaktionsgemisch wurde aufkonzentriert, der Rückstand in Dichlormethan aufgenommen und an Kieselgel chromatographiert (Dichlormethan/Methanol 20:1 als Eluent). Die produkthaltigen Fraktionen wurden eingeengt, der Rückstand in 100 mL Diethylether 30 min verrührt, abfiltriert, mit wenig Diethylether gewaschen und getrocknet. Es wurden 15 g (45% d. Th.) der Titelverbindung erhalten.
LC-MS (Methode 2): Rₜ = 1.43 min
MS (ESpos): m/z = 414.9/416.8 (M+H)⁺
¹H-NMR (400 MHz, DMSO-d₆): δ = 1.36 (t, 3 H); 2.54 (s, 3 H; verdeckt durch DMSO-Signal); 4.37 (q, 2 H); 5.36 (s, 2 H); 7.25 (t, 2 H); 7.42 (d, 1 H); 7.61 (quint., 1 H); 9.00 (d, 1 H).

### Beispiel 22A

### 6-Brom-8-[(2,6-difluorbenzyl)oxy]-2-methylimidazo[1,2-a]pyridin-3-carbonsäure

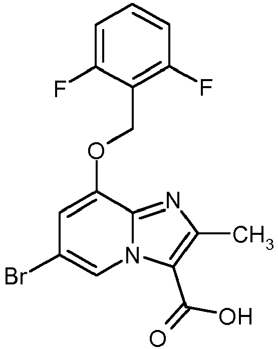

1.5 g Ethyl-6-brom-8-[(2,6-difluorbenzyl)oxy]-2-methylimidazo[1,2-a]pyridin-3-carboxylat (Beispiel 21A; 3.5 mmol, 1 Äquivalent) wurden in 72 mL THF/Methanol 5:1 gelöst, mit 17.6 mL 1N wässriger Lithiumhydroxid-Lösung (17.6 mmol, 5 Äquivalente) versetzt, auf 40 °C erwärmt und für 6 h bei dieser Temperatur gerührt. Der Ansatz wurde mit 6N Salzsäure auf pH 4 gestellt und aufkonzentriert. Der entstandene Feststoff wurde mit Wasser versetzt, ausgerührt, abfiltriert, mit Wasser nachgewaschen und im Vakuum getrocknet. Es wurden 1.24 g der Titelverbindung (88% d. Th.) erhalten.
LC-MS (Methode 1): Rₜ = 0.93 min
MS (ESpos): m/z = 397.0/399.1 (M+H)⁺
¹H-NMR (400 MHz, DMSO-d₆): δ = 2.54 (s, 3 H; überlagert von DMSO-Signal); 5.36 (s, 2 H); 7.25 (t, 2 H); 7.40 (d, 1 H); 7.61 (quint., 1 H); 9.06 (d, 1 H); 13.35 (br. s, 1 H).

### Beispiel 23A

### Ethyl-8-[(2,6-difluorbenzyl)oxy]-2,6-dimethylimidazo[1,2-a]pyridin-3-carboxylat

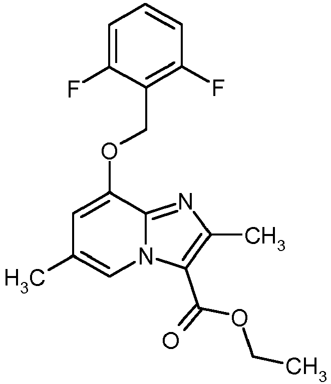

20.00 g (85.38 mmol) Ethyl-8-hydroxy-2,6-dimethylimidazo[1,2-a]pyridin-3-carboxylat Beispiel 239A wurden zusammen mit 19.44 g (93.91 mmol) 2,6-Difluorbenzylbromid und 61.20 g (187.83 mmol) Cäsiumcarbonat in 1.18 L DMF vorgelegt und 5 h bei 60°C gerührt. Dann wurde die Reaktionslösung in 6.4 L 10%ige Natriumchlorid-Lösung gegossen und anschließend zweimal mit Essigsäureethylester extrahiert. Die vereinigten organischen Phasen wurden mit 854 mL 10%ige Natriumchlorid-Lösung gewaschen, getrocknet, eingeengt und über Nacht im Hochvakuum bei RT getrocknet. Es wurden 28.2 g (92% d. Th.; Reinheit ca. 90%) der Titelverbindung erhalten.
LC-MS (Methode 1): Rₜ = 1.05 min
MS (ESpos): m/z = 361.1 (M+H)⁺
¹H-NMR (400 MHz, DMSO-d₆): δ = 1.38 (t, 3 H); 2.36 (s, 3 H); 4.35 (q, 2 H); 5.30 (s, 2 H); 7.10 (s, 1 H); 7.23 (t, 2 H); 7.59 (quint., 1 H); 8.70 (s, 1 H).

### Beispiel 24A

### 8-[(2,6-Difluorbenzyl)oxy]-2,6-dimethylimidazo[1,2-a]pyridin-3-carbonsäure

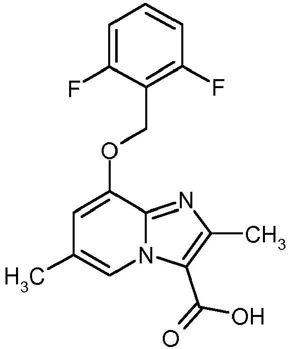

220 mg Ethyl-8-[(2,6-difluorbenzyl)oxy]-2,6-dimethylimidazo[1,2-a]pyridin-3-carboxylat (Beispiel 23A; 0.524 mmol, 1 Äquivalent) wurden in 7 mL THF/Methanol 1:1 gelöst, mit 2.6 mL 1N wässriger Lithiumhydroxid-Lösung (2.6 mmol, 5 Äquivalente) versetzt und für 16 h bei RT gerührt. Der Ansatz wurde aufkonzentriert und der Rückstand mit 1N Salzsäure sauer gestellt. Der entstandene Feststoff wurde ausgerührt, abfiltriert, mit Wasser nachgewaschen und im Vakuum getrocknet. Es wurden 120 mg der Titelverbindung (60% d. Th.) erhalten.
LC-MS (Methode 1): Rₜ = 0.68 min
MS (ESpos): m/z = 333.1 (M+H)⁺
¹H-NMR (400 MHz, DMSO-d₆): δ = 2.34 (s, 3 H); 5.28 (s, 2 H); 7.09 (s, 1 H); 7.23 (t, 2 H); 7.58 (quint., 1 H); 8.76 (s, 1 H); 13.1 (br. s, 1 H).

### Beispiel 25A

### 8-[(2,6-Difluorbenzyl)oxy]-2-methylimidazo[1,2-a]pyridin-3-carbonylchlorid-Hydrochlorid

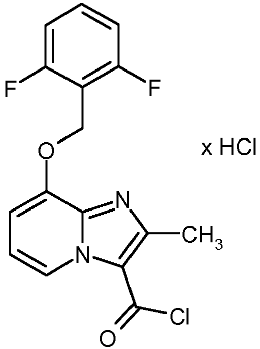

2.0 g (6.28 mmol) 8-[(2,6-Difluorbenzyl)oxy]-2-methylimidazo[1,2-a]pyridin-3-carbonsäure wurden in abs. THF vorgelegt, mit 4 Tropfen DMF versetzt und dann wurden 3.19 g (25.14 mmol) Oxalsäuredichlorid zugetropft. Das Reaktionsgemisch wurde 3 h bei RT gerührt. Es wurden nochmals 0.80 g (6.29 mmol) Oxalsäuredichlorid zugegeben und die Reaktion wurde weitere 4 h bei RT gerührt. Das Reaktionsgemisch wurde eingeengt, dreimal mit Toluol eingeengt und der Rückstand wurde im Hochvakuum getrocknet. Es wurden 2.43 g der Titelverbindung (103% d. Th.) erhalten.
DCI-MS (Methode 13): MS (ESpos): m/z = 437 (M-HCl+H)⁺

### Beispiel 26A

### Ethyl-2-chlor-3-cyclopropyl-3-oxopropanoat

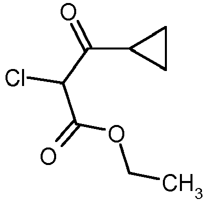

3.1 mL Sulfurylchlorid (38.2 mmol, 1.05 Äquivalente) wurden in 21 mL Dichlormethan vorgelegt und auf dem Wasserbad tropfenweise mit 5.68 g Ethyl-3-cyclopropyl-3-oxopropanoat (36.4 mmol) versetzt. Die Reaktionsmischung wurde 2 h bei RT gerührt, anschließend mit Wasser, 5%-iger wässriger Natriumhydrogencarbonat-Lösung und gesättigter wässriger Natriumchlorid-Lösung gewaschen, über Magnesiumsulfat getrocknet und eingeengt. Das Rohprodukt (6.8 g) wurde ohne weitere Aufreinigung in die Folgeumsetzung eingesetzt.

### Beispiel 27A

### Ethyl-2-cyclopropyl-8-[(2,6-difluorbenzyl)oxy]imidazo[1,2-a]pyridin-3-carboxylat

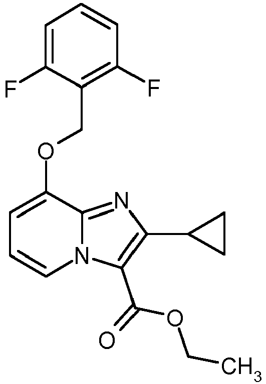

1.69 g 3-[(2,6-Difluorbenzyl)oxy]pyridin-2-amin (Beispiel 4A; 7.13 mmol, 1 Äquivalent) wurden in 44.4 mL Ethanol vorgelegt, mit 425 mg gepulvertem Molsieb 3Å und 6.8 g Ethyl-2-chlor-3-cyclopropyl-3-oxopropanoat (Rohprodukt aus Beispiel 26A) versetzt. Die resultierende Reaktionsmischung wurde für 48 h zum Rückfluß erhitzt, anschließend aufkonzentriert und der Rückstand chromatographiert (Cyclohexan/Essigsäureethylester als Laufmittel). Die produkthaltigen Fraktionen wurden vereinigt und aufkonzentriert. Der so erhaltene Rückstand wurde in Methanol, Dimethylsulfoxid und Wasser aufgenommen, der sich bildende Feststoff abfiltiert und getrocknet. Es wurden 410 mg (15.4% d. Th.) Titelverbindung erhalten.
LC-MS (Methode 1): Rₜ = 1.22 min
MS (ESpos): m/z = 373.2 (M+H)⁺
¹H-NMR (400 MHz, DMSO-d₆): δ = 0.95 - 1.05 (m, 4 H); 1.39 (t, 3 H); 2.36 (s, 3 H); 2.70 - 2.80 (m, 1 H); 4.39 (q, 2 H); 5.30 (s, 2 H); 7.08 (t, 1 H); 7.15 (d, 1 H); 7.20 (t, 2 H); 7.59 (quint., 1 H); 8.88 (d, 1 H).

### Beispiel 28A

### 2-Cyclopropyl-8-[(2,6-difluorbenzyl)oxy]imidazo[1,2-a]pyridin-3-carbonsäure

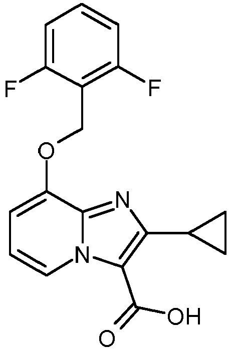

410 mg Ethyl-2-cyclopropyl-8-[(2,6-difluorbenzyl)oxy]imidazo[1,2-a]pyridin-3-carboxylat (Beispiel 27A, 1.1 mmol, 1 Äquivalent) wurden in 15 mL Methanol/Tetrahydrofuran (1:1) vorgelegt und mit 5.5 mL einer 1N wässrigen Lithiumhydroxidlösung (5.5 mmol, 5 Äquivalente) versetzt. Die Reaktionsmischung wurde über Nacht bei RT gerührt und anschließend wurden erneut 5.5 mL 1N wässriger Lithiumhydroxidlösung addiert und für eine weitere Nacht bei RT gerührt. Der Ansatz wurde aufkonzentriert, der Rückstand in Wasser aufgenommen und mit 1N Salzsäure sauer gestellt. Das ausgefallene Produkt wurde abfiltriert und am Hochvakuum getrocknet. Es wurden 293 mg (77% d. Th.) der Titelverbindung erhalten.
LC-MS (Methode 1): Rₜ = 0.83 min
MS (ESpos): m/z = 345.2 (M+H)⁺
¹H-NMR (400 MHz, DMSO-d₆): δ = 0.95 - 1.02 (m, 4 H); 2.80 (quint., 1 H); 5.30 (s, 2 H); 7.02 (t, 1 H); 7.15 (d, 1 H); 7.22(t, 2 H); 7.59 (quint., 1 H); 8.92 (s, 1 H); 13.3 (br. s, 1 H).

### Beispiel 29A

### Ethyl-2-chlor-3-oxopropanoat

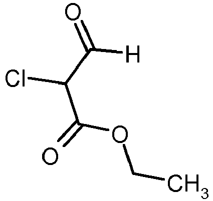

139 mL einer 21%igen Natriumethylat-Lösung in Ethanol (371 mmol, 0.91 Äquivalente) wurden in 200 mL Diethylether vorgelegt und bei RT mit einer Lösung bestehend aus 43.7 mL Chloressigsäureethylester (408 mmol, 1 Äquivalent) und 32.9 mL Ameisensäureethylester (408 mmol, 1 Äquivalent) in 150 mL Diethylether tropfenweise versetzt. Die Reaktionsmischung wurde über Nacht gerührt, der entstandene Feststoff wurde abfiltriert und mit Diethylether gewaschen. Der Feststoff wurde in Wasser gelöst und die wässrige Phase unter Eisbadkühlung mit konzentrierter Salzsäure auf pH 4 eingestellt. Es wurde mehrfach mit Diethylether extrahiert, die vereinigten organischen Phasen wurden mit gesättigter wässriger Natriumchlorid-Lösung gewaschen, mit Magnesiumsulfat getrocknet, filtiert und eingeengt. Das erhaltene Rohprodukt (8.2 g) wurde am Hochvakuum von Lösungsmittelresten befreit und ohne weitere Aufreinigung in die Folgereaktion eingesetzt.

### Beispiel 30A

### Ethyl-8-[(2,6-difluorbenzyl)oxy]imidazo[1,2-a]pyridin-3-carboxylat

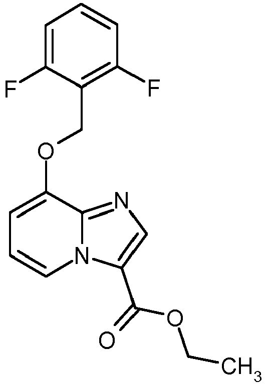

1.93 g 3-[(2,6-difluorbenzyl)oxy]pyridin-2-amin (Beispiel 4A; 8.2 mmol, 1 Äquivalent) wurden in 50 mL Ethanol vorgelegt und mit 8.2 g Ethyl-2-chlor-3-oxopropanoat (75% Reinheit, Rohprodukt aus Beispiel 29A, 40.8 mmol, 5 Äquivalente) versetzt. Die resultierende Reaktionsmischung wurde über Nacht am Rückfluß erhitzt. Die Reaktionslösung wurde eingeengt, das erhaltene Rohprodukt über 340 g Kieselgel (Biotage Isolera) chromatographiert (Laufmittel: Cyclohexan:Essigsäureethylester Gradient; R_{f}-Wert Produkt in Cyclohexan:Essigsäureethylester 2:1 = 0.36). Die Produktfraktionen wurden vereinigt, eingeengt und der erhaltene Rückstand mit Diisopropylether ausgerührt. Der Feststoff wurde abfiltriert und im Hochvakuum getrocknet. Es wurden 2.02 g der Titelverbindung (71% d. Th.) erhalten
LC-MS (Methode 1): Rₜ = 1.08 min
MS (ESpos): m/z = 333.1 (M+H)⁺
¹H-NMR (400 MHz, DMSO-d₆): δ = 1.35 (t, 3 H); 4.39 (q, 2 H); 5.35 (s, 2 H); 7.15 - 7.28 (m, 4 H); 7.58 (quint., 1 H); 8.18 (s, 1 H); 8.90 (d, 1 H).

### Beispiel 31A

### 8-[(2,6-Difluorbenzyl)oxy]imidazo[1,2-a]pyridin-3-carbonsäure

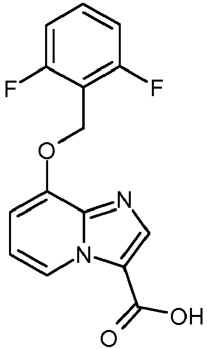

1 g Ethyl-8-[(2,6-difluorbenzyl)oxy]imidazo[1,2-a]pyridin-3-carboxylat (Beispiel 30A, 3 mmol, 1 Äquivalent) wurden in 60 mL Methanol/Tetrahydrofuran (5:1) vorgelegt, mit 15 mL einer 1N wässrigen Lithiumhydroxidlösung (15 mmol, 5 Äquivalente) versetzt, auf 40 °C erwärmt und für 4 h bei dieser Temperatur gerührt. Der Ansatz wurde abgekühlt und unter Eiskühlung mit 6N Salzsäure auf pH=4 eingestellt. Die organischen Lösungsmittel wurden am Rotationsverdampfer entfernt, das ausfallende Produkt wurde mit Wasser versetzt und abfiltriert. Der Filterkuchen wurde mit Wasser nachgewaschen und im Hochvakuum getrocknet. Es wurden 797 mg (87% d. Th.) der Titelverbindung erhalten.
LC-MS (Methode 1): Rₜ = 0.66 min
MS (ESpos): m/z = 305.1 (M+H)⁺
1H-NMR (400 MHz, DMSO-d6): δ = 5.38 (s, 2 H); 7.10 - 7.28 (m, 4 H); 7.59 (quint., 1 H); 8.12 (s, 1 H); 8.92 (s, 1 H); 13.1 (br. s, 1 H).

### Beispiel 32A

### Ethyl-8-[(2,6-difluorbenzyl)oxy]-2-methyl-6-(pyrrolidin-1-yl)imidazo[1,2-a]pyridin-3-carboxylat

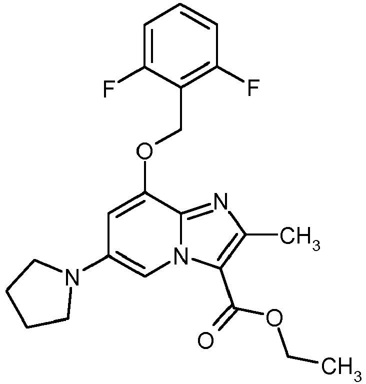

500 mg Ethyl-6-brom-8-[(2,6-difluorbenzyl)oxy]-2-methylimidazo[1,2-a]pyridin-3-carboxylat (1.18 mmol, 1 Äquivalent), 43 mg Tris-(dibenzylidenaceton)-dipalladium (0.047 mmol, 4 mol%), 158 mg Natrium-tert-butylat (1.65 mmol, 1.4 Äquivalente), 67 mg XPHOS (0.141 mmol, 12 mol%) und 294 µL Pyrrolidin (3.5 mmol, 3 Äquivalente) wurden in 30 mL trockenem Toluol gelöst und in einem auf 100 °C vorgewärmten Ölbad zur Reaktion gebracht. Nach 16 h bei dieser Temperatur wurde die Reaktionsmischung abgekühlt, über Kieselgur filtriert, aufkonzentriert und chromatograhiert (Biotage Isolera Four; Cyclohexan-Essigsäureethylester-Gradient als Eluent). Es wurden 100 mg (19% d. Th.) der Titelverbindung erhalten.
LC-MS (Methode 1): Rₜ = 1.08 min
MS (ESpos): m/z = 416.2 (M+H)⁺
¹H-NMR (400 MHz, DMSO-d₆): δ = 1.34 (t, 3 H); 1.95 - 2.04 (m, 4 H); 2.55 (s, 3 H; durch DMSO-Signal verdeckt); 3.21 - 3.29 (m, 4 H); 4.31 (q, 2 H); 5.38 (s, 2 H); 6.80 (s, 1 H); 7.22 (t, 2 H); 7.58 (quint., 1 H); 8.13 (s, 1 H).

### Beispiel 33A

### 8-[(2,6-Difluorbenzyl)oxy]-2-methyl-6-(pyrrolidin-1-yl)imidazo[1,2-a]pyridin-3-carbonsäure

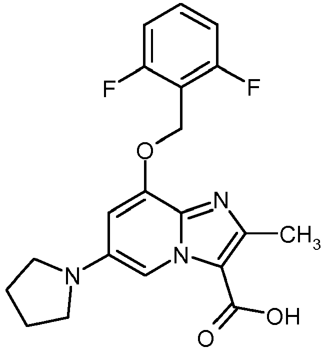

90 mg Ethyl-8-[(2,6-difluorbenzyl)oxy]-2-methyl-6-(pyrrolidin-1-yl)imidazo[1,2-a]pyridin-3-carboxylat (Beispiel 32A; 0.217 mmol, 1 Äquivalent) wurden in 6 mL THF/Methanol 5:1 gelöst, mit 1.1 mL 1N wässriger Lithiumhydroxid-Lösung (1.1 mmol, 5 Äquivalente) versetzt, auf 40 °C erwärmt und für 20 h bei dieser Temperatur gerührt. Der Ansatz wurde abgekühlt, mit 6 N Salzsäure auf pH 4 angesäuert und aufkonzentriert. Der entstandene Feststoff wurde mit Wasser versetzt, ausgerührt, abfiltriert, mit Wasser nachgewaschen und im Vakuum getrocknet. Es wurden 87 mg der Titelverbindung (93% d. Th.) erhalten.
LC-MS (Methode 1): Rₜ = 0.83 min
MS (ESpos): m/z = 388.2 (M+H)⁺
¹H-NMR (400 MHz, DMSO-d₆): δ = 2.00 - 2.08 (m, 4 H); 2.60 (s, 3 H); 3.30 - 3.38 (m, 4 H); 5.52 (s, 2 H); 7.24 (s, 1 H); 7.25 (t, 2 H); 7.60 (quint., 1 H); 8.30 (s, 1 H).

### Beispiel 34A

### Ethyl-8-[(2,6-difluorbenzyl)oxy]-2-methyl-6-(morpholin-4-yl)imidazo[1,2-a]pyridin-3-carboxylat

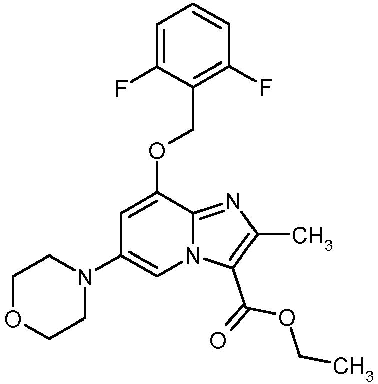

500 mg Ethyl-6-brom-8-[(2,6-difluorbenzyl)oxy]-2-methylimidazo[1,2-a]pyridin-3-carboxylat (1.18 mmol, 1 Äquivalent), 43 mg Tris-(dibenzylidenaceton)-dipalladium (0.047 mmol, 4 mol%), 158 mg Natrium-tert-butylat (1.65 mmol, 1.4 Äquivalente), 67 mg XPHOS (0.141 mmol, 12 mol%) und 307 µL Morpholin (3.5 mmol, 3 Äquivalente) wurden in 30 mL trockenem Toluol gelöst und in einem auf 100 °C vorgewärmten Ölbad zur Reaktion gebracht. Nach 16 h bei dieser Temperatur wurde die Reaktionsmischung abgekühlt, über Kieselgur filtriert, aufkonzentriert und chromatograhiert (Biotage Isolera Four; Cyclohexan-Essigsäureethylester-Gradient als Eluent). Es wurden 352 mg (63% d. Th.) der Titelverbindung erhalten.
LC-MS (Methode 1): Rₜ = 1.05 min
MS (ESpos): m/z = 432.2 (M+H)⁺
¹H-NMR (400 MHz, DMSO-d₆): δ = 1.35 (t, 3 H); 2.55 (s, 3 H; durch DMSO-Signal verdeckt); 3.08 - 3.13 (m, 4 H); 3.75 - 3.80 (m, 4 H); 4.31 (q, 2 H); 5.30 (s, 2 H); 7.20 (s, 1 H); 7.23 (t, 2 H); 7.59 (quint., 1 H); 8.40 (s, 1 H).

### Beispiel 35A

### 8-[(2,6-Difluorbenzyl)oxy]-2-methyl-6-(morpholin-4-yl)imidazo[1,2-a]pyridin-3-carbonsäure

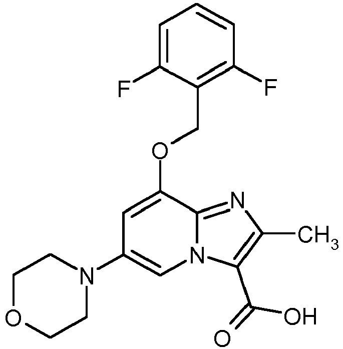

400 mg Ethyl-8-[(2,6-difluorbenzyl)oxy]-2-methyl-6-(pyrrolidin-1-yl)imidazo[1,2-a]pyridin-3-carboxylat (Beispiel 34A; 0.927 mmol, 1 Äquivalent) wurden in 24 mL THF/Methanol 5:1 gelöst, mit 4.6 mL 1N wässrige Lithiumhydroxid-Lösung (4.6 mmol, 5 Äquivalente) versetzt, auf 40 °C erwärmt und für 4 h bei dieser Temperatur gerührt. Der Ansatz wurde abgekühlt, mit 6 N Salzsäure auf pH 4 angesäuert und aufkonzentriert. Der Rückstand wurde mit Wasser versetzt und mehrfach mit Dichlormethan extrahiert. Die vereinigten organischen Phasen wurden mit gesättigter wässriger Natriumchlorid-Lösung gewaschen, getrocknet, filtriert und eingeengt. Es wurden 145 mg der Titelverbindung (35% d. Th.) erhalten, welches ohne weitere Aufreinigung weiter umgesetzt wurde.
LC-MS (Methode 1): Rₜ = 0.72 min
MS (ESpos): m/z = 404.2 (M+H)⁺
¹H-NMR (400 MHz, DMSO-d₆): δ = 2.55 (s, 3 H; von DMSO-Signal überlagert); 3.10 - 3.20 (m, 4 H); 3.75 - 3.82 (m, 4 H); 5.38 (s, 2 H); 7.23 (t, 2 H); 7.25 (s, 1 H); 7.58 (quint., 1 H); 8.48 (s, 1 H).

### Beispiel 36A

### 6-Chlor-8-[(2,3-difluorbenzyl)oxy]-2-methylimidazo[1,2-a]pyridin-3-carbonsäure

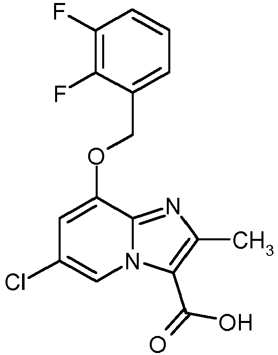

### Schritt a): 2-Amino-5-chlorpyridin-3-ol

Nitroreduktion von 5-Chlor-2-nitropyridin-3-ol (Beispiel 15A) in Analogie zur Herstellung von Beispiel 11A zu 2-Amino-5-chlorpyridin-3-ol; 84% Ausbeute (enthielt 33% dechloriertes Produkt).
LC-MS (Methode 1): Rₜ = 0.20 min
MS (ESpos): m/z = 144.9/146.9 (M+H)⁺

### Schritt b): 5-Chlor-3-[(2,6-difluorbenzyl)oxylpyridin-2-amin

Umsetzung von 2-Amino-5-chlorpyridin-3-ol mit 1.1 Äquivalenten 2,3-Difluorbenzylbromid und 2.2 Äquivalenten Cäsiumcarbonat in DMF (15 min bei 50 °C), wässrige Aufarbeitung, Extraktion mit Essigsaäureethylester und nachfolgende Chromatographie des organischen Rückstandes (Gradient: Cyclohexan/Essigsäureethylester 8:1 bis Essigsäureethylester pur) zu 5-Chlor-3-[(2,6-difluorbenzyl)oxy]pyridin-2-amin; 10% Ausbeute.
LC-MS (Methode 1): Rₜ = 0.94 min
MS (ESpos): m/z = 271.0/273.0 (M+H)⁺

### Schritt c): Ethyl-6-chlor-8-[(2,3-difluorbenzyl)oxy]-2-methylimidazo[1,2-a]pyridin-3-carboxylat

Cyclisierung (in Analogie zur Herstellung von Beispiel 18A) zu Ethyl-6-chlor-8-[(2,3-difluorbenzyl)oxy]-2-methylimidazo[1,2-a]pyridin-3-carboxylat; 48% Ausbeute.
LC-MS (Methode 1): Rₜ = 1.25 min
MS (ESpos): m/z = 381.1/383.0 (M+H)⁺

### Schritt d): 6-Chlor-8-[(2,3-difluorbenzyl)oxy]-2-methylimidazo[1,2-a]pyridin-3-carbonsäure

Esterverseifung (in Analogie zur Herstellung von Beispiel 19A) zu 6-Chlor-8-[(2,3-difluorbenzyl)oxy]-2-methylimidazo[1,2-a]pyridin-3-carbonsäure; 67% Ausbeute.
LC-MS (Methode 1): Rₜ = 0.87 min
MS (ESpos): m/z = 353.1/355.1 (M+H)⁺
¹H-NMR (400 MHz, DMSO-d₆): δ = 2.54 (s, 3 H; überlagert von DMSO-Signal); 5.41 (s, 2 H); 7.27 (s, 1 H); 7.25 - 7.31 (m, 1 H); 7.43 - 7.55 (m, 2 H); 8.99 (s, 1 H); 13.39 (br. s, 1 H).

### Beispiel 37A

### Ethyl-8-[(2,6-difluorbenzyl)oxy]-2-methyl-6-[(trimethylsilyl)ethinyl]imidazo[1,2-a]pyridin-3-carboxylat

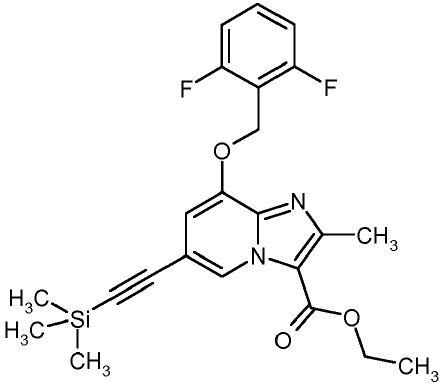

1 g Ethyl-6-brom-8-[(2,6-difluorbenzyl)oxy]-2-methylimidazo[1,2-a]pyridin-3-carboxylat (Beispiel 21A; 2.35 mmol; 1 Äquivalent) wurden in 52 mL Dioxan/Diisopropylethylamin 1:1 vorgelegt und mit 165 mg Dichlorbis(triphenylphosphin)-Palladium(II) (0.24 mmol, 0.1 Äquivalente) und 45 mg Kupfer(I)-Iodid (0.24 mmol, 0.1 Äquivalente) versetzt. 1.3 mL Trimethylsilylacetylen (9.4 mmol, 4 Äquivalente) wurden langsam bei RT zugetropft. Die resultierende Reaktionsmischung wurde für 16 h bei 50 °C gerührt. Anschließend wurde eingeengt, mit Wasser versetzt und dreimal mit Essigsäureethylester extrahiert. Die vereinten organischen Phasen wurden zweimal mit 10%iger wässriger Natriumthiosulfatlösung und anschließend mit gesättigter Natriumchloridlösung gewaschen, über Magnesiumsulfat getrocknet und das Filtrat eingeengt. Der Rückstand wurde in Methanol gelöst und per präparativer HPLC aufgereinigt (Säule: Nucleodur C18 Gravity 50 x 200 mm, 10 µm, Gradient: A=Wasser + 0,1% konzentrierte wässrige Ammoniak-Lösung. , B = Acetonitril, 0 Min = 30 % B, 5 Min = 30% B, 23 Min = 100% B, 28 Min = 100% B, 28,2 Min = 30% B, 34 Min=30% B, Flußrate 110 ml/Min, Wellenlänge 220 nm). Es wurden 726 mg Feststoff erhalten (89% Reinheit, 62% d. Th.).
LC-MS (Methode 1): Rₜ = 1.48 min
MS (ESpos): m/z = 443.3 (M+H)⁺
¹H-NMR (400 MHz, DMSO-d₆): δ = 0.3 (s, 9 H), 1.36 (t, 3 H), 2.54 (s, 3 H; verdeckt durch DMSO-Signal), 4.37 (q, 2 H), 5.33 (s, 2 H), 7.20 - 7.28 (m, 3 H), 7.61 (quint., 1 H), 8.93 (s, 1 H).

### Beispiel 38A

### Ethyl-8-[(2,6-difluorbenzyl)oxy]-6-ethinyl-2-methylimidazo[1,2-a]pyridin-3-carboxylat

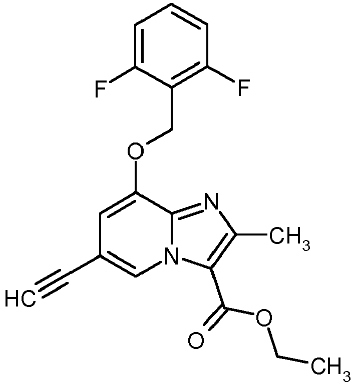

720 mg Ethyl-8-[(2,6-difluorbenzyl)oxy]-2-methyl-6-[(trimethylsilyl)ethinyl]imidazo[1,2-a]pyridin-3-carboxylat (Beispiel 37A; 1.63 mmol; 1 Äquivalent) wurden in 16 mL Methanol vorgelegt und mit 674 mg Kaliumcarbonat (4.81 mmol, 3 Äquivalente) versetzt. Die resultierende Reaktionsmischung wurde für 2 h bei Raumtemperatur gerührt. Der Reaktionsansatz wurde anschließend eingeengt, mit Wasser versetzt und dreimal mit Essigsäureethylester extrahiert. Die vereinten organischen Phasen wurden mit gesättigter Natriumchloridlösung gewaschen, über Magnesiumsulfat getrocknet und das Filtrat eingeengt. Es wurden 587.7 mg Rohprodukt erhalten (enthielt zu 35% den Methylester der Zielverbindung; quantitative Ausbeute), welches ohne weitere Aufreinigung in die nächste Umsetzung eingesetzt worden ist.
LC-MS (Methode 1): Rₜ = 1.22 min (Methylester: 1.14 min)
MS (ESpos): m/z = 371.2 (M+H)⁺ (Ethylester: 357.1)
¹H-NMR (400 MHz, DMSO-d₆): δ = 1.36 (t, 3 H), 2.54 (s, 3 H; verdeckt durch DMSO-Signal), 4.38 (q, 2 H), 4.40 (s, 1 H), 5.33 (s, 2 H), 7.20 - 7.28 (m, 3 H), 7.61 (quint., 1 H), 8.93 (s, 1 H).

### Beispiel 39A

### 8-[(2,6-Difluorbenzyl)oxy]-6-ethinyl-2-methylimidazo[1,2-a]pyridin-3-carbonsäure

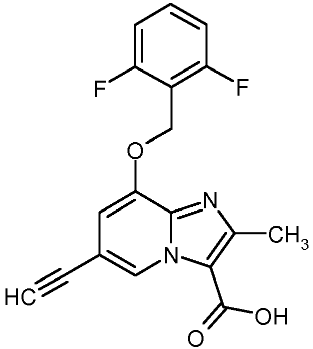

580 mg Ethyl-8-[(2,6-difluorbenzyl)oxy]-6-ethinyl-2-methylimidazo[1,2-a]pyridin-3-carboxylat (Beispiel 38A; enthielt zu 35% den Methylester) wurden in 30 mL Tetrahydrofuran und 5 mL Methanol vorgelegt und mit 7.8 mL 1N wässriger Lithiumhydroxid-Lösung versetzt. Die resultierende Reaktionsmischung wurde für 6 h bei Raumtemperatur gerührt und anschließend für 16 h bei 0 °C gelagert. Dann wurde mit 6 N Salzsäure sauer gestellt und eingeengt. Der sich bildene Feststoff wurde abfiltriert, mit wenig Wasserr verrührt und erneut abfiltriert. Nach Trocknen im Vakuum wurden 521 mg gewünschtes Produkt erhalten (quantitative Ausbeute).
LC-MS (Methode 1): Rₜ = 0.87 min
MS (ESpos): m/z = 343.1 (M+H)⁺
¹H-NMR (400 MHz, DMSO-d₆): δ = 2.54 (s, 3 H; verdeckt durch DMSO-Signal), 4.40 (s, 1 H), 5.33 (s, 2 H), 7.20 - 7.28 (m, 3 H), 7.61 (quint., 1 H), 9.06 (s, 1 H).

### Beispiel 40A

### Ethyl-6-cyclopropyl-8-[(2,6-difluorbenzyl)oxy]-2-methylimidazo[1,2-a]pyridin-3-carboxylat

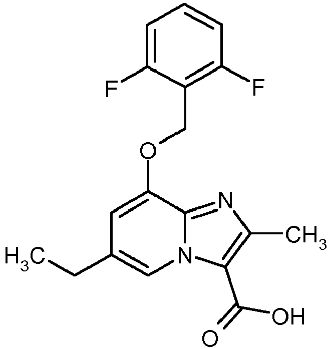

150 mg 8-[(2,6-Difluorbenzyl)oxy]-6-ethinyl-2-methylimidazo[1,2-a]pyridin-3-carbonsäure (Beispiel 39A) wurden in 50 mL Ethanol und 20 mL Essigsäureethylester vorgelegt, mit einer Spatelspitze Pd/C (10%ig) versetzt und bei Normaldruck für 4 h hydriert. Die Reaktionsmischung wurde filtriert und der erhaltene Rückstand (137 mg Feststoff; 95% Reinheit, 86% d. Th.) ohne Aufreinigung weiter umgesetzt.
LC-MS (Methode 1): Rₜ = 0.80 min
MS (ESpos): m/z = 347.1 (M+H)⁺
¹H-NMR (400 MHz, DMSO-d₆): δ = 1.22 (t, 3 H), 2.54 (s, 3 H; verdeckt durch DMSO-Signal), 2.69 (q, 2 H), 5.31 (s, 2 H), 7.09 (s, 1 H), 7.23 (t, 2 H), 7.61 (quint., 1 H), 8.80 (s, 1 H).

### Beispiel 41A

### Ethyl-6-cyclopropyl-8-[(2,6-difluorbenzyl)oxy]-2-methylimidazo[1,2-a]pyridin-3-carboxylat

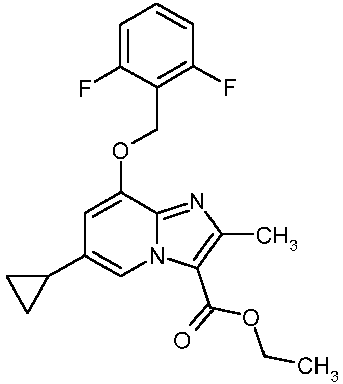

500 mg Ethyl-6-brom-8-[(2,6-difluorbenzyl)oxy]-2-methylimidazo[1,2-a]pyridin-3-carboxylat (Beispiel 21A; 1.18 mmol; 1 Äquivalent), 131 mg Cyclopropanboronsäure (1.53 mmol, 1.3 Äquivalente), 873 mg Kaliumphosphat (4.12 mmol, 3.5 Äquivalente), 33 mg Tricyclohexylphosphin (0.12 mmol, 0.1 Äquivalente) und 13 mg Palladium(II)acetat (0.059 mmol, 5 mol%) wurden in 10.5 mL Toluol/Wasser 20:1 vorgelegt und für 16 h bei 80 °C gerührt. Es wurde anschließend über Kieselgel filtriert und eingeengt. Der Rückstand wurde über eine 50g Kieselgelkartusche chromatographiert (Biotage Isolera, Cyclohexan-Essigsäureethylester-Gradient als Eluent). Es wurden 473 mg eines gelblichen Feststoffes erhalten (98% Reinheit, quantitative Ausbeute).
LC-MS (Methode 1): Rₜ = 1.17 min
MS (ESpos): m/z = 387.3 (M+H)⁺
¹H-NMR (500 MHz, DMSO-d₆): δ = 0.78 - 0.82 (m, 2 H), 0.95 - 1.05 (m, 2 H), 1.36 (t, 3 H), 2.05 - 2.10 (m, 1 H), 2.54 (s, 3 H; verdeckt durch DMSO-Signal), 4.37 (q, 2 H), 5.33 (s, 2 H), 6.80 (s, 1 H), 7.23 (t, 2 H), 7.59 (quint., 1 H), 8.70 (s, 1 H)

### Beispiel 42A

### 6-Cyclopropyl-8-[(2,6-difluorbenzyl)oxy]-2-methylimidazo[1,2-a]pyridin-3-carbonsäure

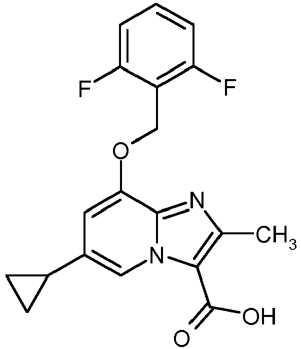

470 mg Ethyl-6-cyclopropyl-8-[(2,6-difluorbenzyl)oxy]-2-methylimidazo[1,2-a]pyridin-3-carboxylat (Beispiel 41A) wurden in 36 mL Tetrahydrofuran/Methanol 5:1 vorgelegt und mit 6.1 ml 1N wässriger Lithiumhydroxid-Lösung versetzt. Es wurde für 4h bei 40 °C gerührt und anschließend mit 6 N Salzsäure sauer gestellt und eingeengt. Der Rückstand wurde in Wasser ausgenommen und mehrfach mit Dichlormethan extrahiert. Die vereinten organischen Phasen wurden mit gesättigter wässriger Natriumchlorid-Lösung gewaschen, mit Magnesiumsulfat getrocknet, filtriert und das Filtrat eingeengt. Es wurden 470 mg des gewünschten Produktes (90% Reinheit; 97% d. Th.) erhalten und ohne weitere Aufreinigung weiter umgesetzt.
LC-MS (Methode 2): Rₜ = 0.92 min
MS (ESpos): m/z = 359.1 (M+H)⁺
¹H-NMR (400 MHz, DMSO-d₆): δ = 0.88 - 0.92 (m, 2 H), 1.05 - 1.10 (m, 2 H), 2.12 - 2.20 (m, 1 H), 2.60 (s, 3 H), 4.40 (s, 1 H), 5.40 (s, 2 H), 7.14 (s, 1 H), 7.22 (t, 2 H), 7.61 (quint., 1 H), 8.90 (s, 1 H).

### Beispiel 43A

### 2,6-Difluorphenyl)(²H₂)methanol

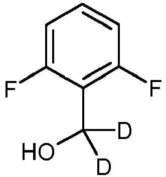

1.00 g (5.81 mmol) Methyl-2,6-difluorbenzoat wurde bei 0°C in 20 ml THF vorgelegt und mit 11.62 ml (11.62 mmol) Lithiumaluminiumdeuterid [= LiAlD₄ =Lithiumtetrahydrido(²H₄)aluminate] (1 M Lösung in THF) tropfenweise versetzt. Es wurde 1 h in einem langsam auftauenden Eisbad gerührt. Die Reaktionslösung wurde nacheinander mit 0.58 ml Wasser, 0.58 ml 2 N wässriger Natriumhydroxid-Lösung und 1.16 ml Wasser versetzt. Der entstandene Niederschlag wurde abfiltriert und gut mit THF gewaschen. Das Filtrat wurde eingeengt und der Rückstand im Hochvakuum getrocknet. Es wurden 0.743 g des Produktes (88% d. Th., Reinheit ca. 90%) erhalten und ohne Reinigung weiter umgesetzt.
LC-MS (Methode 14): Rₜ = 2.38 min
MS (EIpos): m/z = 146 (M)⁺
¹H-NMR (400 MHz, DMSO-d₆): δ = 5.20 (s, 1H), 7.08 (t, 2H), 7.39 (quint, 1H).

### Beispiel 44A

### Ethyl-8-{[(2,6-difluorphenyl)(²H₂)methyl]oxy}-2-methylimidazo[1,2-a]pyridin-3-carboxylat

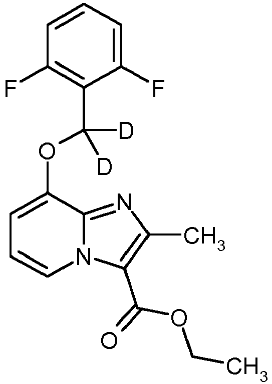

493 mg (2.24 mmol) Ethyl 8-hydroxy-2-methylimidazo[1,2-a]pyridin-3-carboxylat (Beispiel 3 A) wurden in 13.5 ml trockenem Toluol vorgelegt und anschließend erst mit 490 mg (3.35 mmol) 2,6-Difluorphenyl)(²H₂)methanol (Beispielverbindung 43A) und 938 mg (3.58 mmol) Triphenylphosphin und dann mit 769 mg (3.58 mmol) Azodicarbonsäurediisopropylester versetzt und über Nacht bei RT gerührt. Die Reaktionslösung wurde eingeengt und der Rückstand auf Kieselgel gezogen und per Chromatographie (Laufmittel: Cyclohaxan/Essigsäureethylester = 8:1) gereinigt. Es wurden 467 mg der Zielverbindung (60% d. Th., Reinheit 100%) erhalten.
LC-MS (Methode 2): Rₜ = 1.16 min
MS (ESpos): m/z = 349 (M+H)⁺
¹H-NMR (400 MHz, DMSO-d₆): δ = 1.36 (t, 3H), 2.54 (s, 3H), 4.36 (q, 2H), 7.11 (t, 1H); 7.18-7.27 (m, 3H); 7.59 (quint, 1H); 8.88 (d, 1H).

### Beispiel 45A

### 8-{[(2,6-Difluorphenyl)(²H₂)methyl]oxy}-2-methylimidazo[1,2-a]pyridin-3-carbonsäure

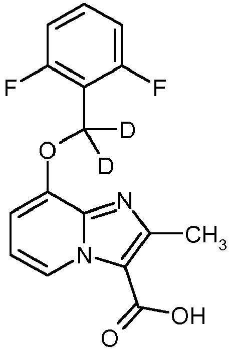

1.57 g (4.52 mmol) Ethyl-8-{[(2,6-difluorphenyl)(²H₂)methyl]oxy}-2-methylimidazo[1,2-a]pyridin-3-carboxylat (Beispielverbindung 44A) wurde in 96 ml THF:Methanol (5:1) gelöst, mit 22.6 ml (22.6 mmol) 1 N wässriger Lithiumhydroxid-Lösung versetzt und über Nacht bei RT gerührt. Der Ansatz wurde unter Kühlung mit 2 N wässriger Salzsäure-Lösung auf pH 4 eingestellt und die organischen Lösemittel wurden im Vakuum entfernt. Der resutierende Feststoff wurde abfiltriert, mit Wasser gewaschen und im Vakuum getrocknet. Es wurden 1.29 g (89% d. Th., Reinheit 99%) des Produktes erhalten.
LC-MS (Methode 2): Rₜ = 0.76 min
MS (ESpos): m/z = 321 (M+H)⁺
¹H-NMR (400 MHz, DMSO-d₆): δ = 2.55 (s, 3H), 7.01 (t, 1H), 7.09 (d, 1H), 7.23 (t, 2H), 7.59 (quint, 1H), 8.92 (d, 1H), 13.08 (br s, 1H).

### Beispiel 46A

### 8-Hydroxy-N-[(2R)-1-hydroxyhexan-2-yl]-2-methylimidazo[1,2-a]pyridin-3-carboxamid

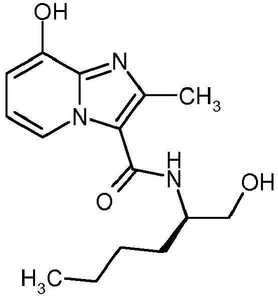

Eine Lösung von 3.5 g (9.18 mmol) 8-(Benzyloxy)-N-[(2R)-1-hydroxyhexan-2-yl]-2-methylimidazo[1,2-a]pyridin-3-carboxamid in 250 ml Essigsäureethylester wurde mit 350 mg 10%iger Palladium/Kohle versetzt und bei Raumtemperatur und Normaldruck mit Wasserstoff beschickt. Nach 3 h wurde über Kieselgur filtriert, intensiv mit Essigsäureethylester/Methanol nachgewaschen und das Filtrat zur Trockene eingedampft. Das erhaltene Rohprodukt wurde mit Methyl-tert.-butylether versetzt, ausgerührt und anschließend wurde der Feststoff abfiltriert und bei 40°C im Hochvakkum getrocknet. So erhielt man 2.1 g (79% d. Th.) 8-Hydroxy-N-[(2R)-1-hydroxyhexan-2-yl]-2-methylimidazo[1,2-a]pyridin-3-carboxamid
LC-MS (Methode 1): Rₜ = 0.61 min
MS (ESpos): m/z = 292.1 (M+H)⁺
¹H-NMR (400 MHz, DMSO-d₆): δ = 0.88 (t, 3H), 1.25 - 1.70 (m, 6H), 2.55 (s, 3H), 3.45 (m, 2H), 3.98 (m, 1H), 4.72 (s br, 1H), 6.59 (d, 1H), 6.79 (t, 1H). 7.45 (d, 1H), 8.40 (d, 1H).
Spezifischer Drehwert (589 nm, 20.1 °C, c = 0.315 g/100 ml) in Methanol: + 16.9°

### Beispiel 47A

### 8-Hydroxy-N-[(2R)-1-hydroxybutan-2-yl]-2-methylimidazo[1,2-a]pyridin-3-carboxamid

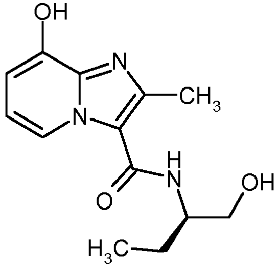

Die Herstellung erfolgte analog zu Beispiel 46A durch Pd/C-vermittelte Hydrierung ausgehend von 3.5 g (ca. 9.72 mmol) 8-(Benzyloxy)-N-[(2R)-1-hydroxybutan-2-yl]-2-methylimidazo[1,2-a]pyridin-3-carboxamid. Man erhielt 1.2 g (58% d. Th.) der Titelverbindung.
LC-MS (Methode 1): Rₜ = 0.30 min
MS (ESpos): m/z = 264.1 (M+H)⁺
¹H-NMR (400 MHz, DMSO-d₆): δ = 0.93 (t, 3H), 1.48 (m, 1H), 1.70 (m, 1H), 2.53 (s, 3H), 3.48 (m, 2H), 3.92 (m, 1H), 4.72 (br, 1H), 6.59 (d, 1H), 6.79 (t, 1H), 7.45 (d, 1H), 8.40 (d, 1H).
Spezifischer Drehwert (589 nm, 20.1 °C, c = 0.285 g/100 ml) in Methanol: + 23.0°

Die folgenden Syntheseintermediate wurden in Analogie zu Beispiel 46A hergestellt:

**Tabelle 1A:**

| **Beispiel** | **IUPAC-Name / Struktur (Ausbeute)** | **Analytische Daten** |
|---|---|---|
| **48A** | 8-Hydroxy-N-[2-(1-hydroxycyclopentyl)ethyl]-2-methylimidazo[1,2-a]pyridin-3-carboxamid | LC-MS (Methode 2): Rₜ = 0.62 min |
| | 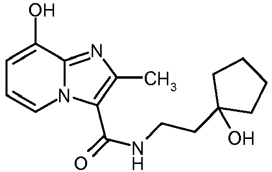 | MS (ESpos): m/z = 304.2 (M+H)⁺ |
| | (82% d. Th.) | |
| **49A** | N-(1,3-Dihydroxypropan-2-yl)-8-hydroxy-2-methylimidazo[1,2-a]pyridin-3-carboxamid | LC-MS (Methode 3): Rₜ = 0.70 min |
| | 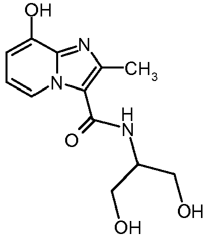 | MS (ESpos): m/z = 266.1 (M+H)⁺ |
| | (46% d. Th.) | |

### Beispiel 50A

### Methly-8-(cyclohexylmethoxy)-2-ethylimidazo[1,2-a]pyridin-3-carboxylat

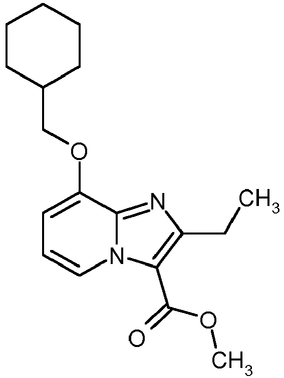

Eine Lösung von 100 mg (0.485 mmol) 3-(Cyclohexylmethoxy)pyridin-2-amin und 399 mg (2.42 mmol) Methyl-2-chlor-3-oxopentanoat in 4 ml Ethanol wurde über Nacht zum Rückfluss erhitzt. Danach wurde der Ansatz abgekühlt, eingeengt und der erhaltene Rückstand über präparative HPLC (Methode 6) aufgereinigt. Dadurch erhielt man 26 mg (17% d.Th.) Methyl-8-(cyclohexylmethoxy)-2-ethylimidazo[1,2-a]pyridin-3-carboxylat.
LC-MS (Methode 2): Rₜ = 1.31 min
MS (ESpos): m/z = 317.3 (M+H)⁺
¹H-NMR (400 MHz, DMSO-d₆): δ = 1.07 - 1.17 (m, 2 H), 1.16 - 1.34 (m, 3 H), 1.24 (t, 3 H), 1.65 - 1.76 (m, 3 H), 1.84 - 1.90 (m, 3 H), 2.50 (durch DMSO-Signal überdeckt, s, 3 H), 3.00 (q, 2 H), 3.89 (s, 3 H), 3.98 (d, 2 H), 6.96 (d, 1 H), 7.04 (t, 1 H), 8.82 (d, 1 H).

Die folgenden Syntheseintermediate in Tabelle 2A wurden in Analogie zu Beispiel 50A durch Umsetzung der entsprechenden alkoxylierten Aminopyridine mit den folgenden Kondensationspartnern erhalten:
Beispiel 51A: Kondensation mit Ethyl-2-chlor-3-keto-4,4,4-trifluorbutyrat in Ethanol;
Beispiel 52A: Kondensation mit Ethyl-2-chlor-3-oxohexanoat in Ethanol;
Beispiel 53A und Beispiel 54A: Kondensation mit Ethyl-2-chlor-3-oxobutanoat in Ethanol;
Beispiel 55A: Kondensation mit Methyl-2-chlor-3-oxopentanoat in Ethanol;
Beispiel 56A: Kondensation mit Ethyl-2-chlor-3-oxopropanoat in Ethanol.

**Tabelle 2A:**

| **Beispiel** | **IUPAC-Name / Struktur (Ausbeute)** | **Analytische Daten** |
|---|---|---|
| **51A** | Ethyl-8-(cyclohexylmethoxy)-2-trifluormethylimidazo[1,2-a]pyridin-3-carboxylat | LC-MS (Methode 2): Rₜ = 1.62 min |
| | 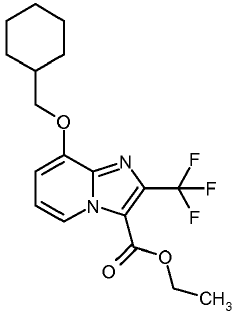 | MS (ESpos): m/z = 371.2 (M+H)⁺ |
| | (31% d. Th.) | |
| **52A** | Ethyl-8-(cyclohexylmethoxy)-2-propylimidazo[1,2-a]pyridin-3-carboxylat | LC-MS (Methode 2): Rₜ = 1.46 min |
| | 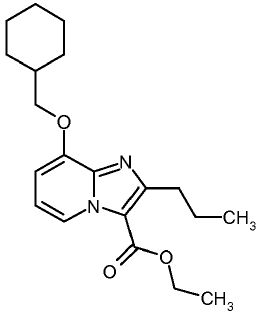 | MS (ESpos): m/z = 345.4 (M+H)+ |
| | (15% d. Th.) | |
| **53A** | Ethyl-8-(cyclohexylmethoxy)-2-methylimidazo[1,2-a]pyridin-3-carboxylat | LC-MS (Methode 1): Rₜ = 1.18 min |
| | 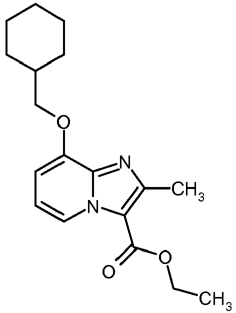 | MS (ESpos): m/z = 317.1 (M+H)+ |
| | (56% d. Th.) | |
| **54A** | Ethyl-8-(cyclobutylmethoxy)-2-methylimidazo[1,2-a]pyridin-3-carboxylat | LC-MS (Methode 1): Rₜ = 1.01 min |
| | 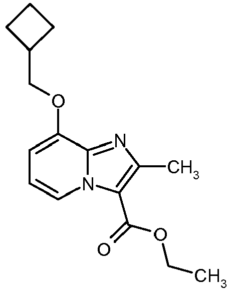 | MS (ESpos): m/z = 289 (M+H)+ |
| | (57% d. Th.) | |
| **55A** | Methyl-8-(cyclobutylmethoxy)-2-ethylimidazo[1,2-a]pyridin-3-carboxylat | LC-MS (Methode 2): Rₜ = 1.12 min |
| | 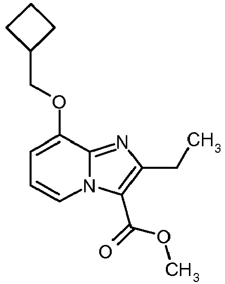 | MS (ESpos): m/z = 289.2 (M+H)+ |
| | (20% d. Th.) | |
| **56A** | Ethyl-8-(cyclohexylmethoxy)imidazo[1,2-a]pyridin-3-carboxylat | LC-MS (Methode 2): Rt = 1.36 min |
| | 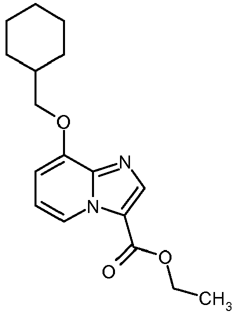 | MS (ESpos): m/z = 303.0 (M+H)+ |
| | (33% d. Th.) | |

### Beispiel 57A

### 8-(Cyclohexylmethoxy)-2-ethylimidazo[1,2-a]pyridin-3-carbonsäure

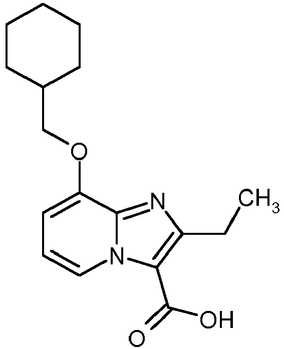

Eine Lösung von 26 mg (0.082 mmol) Ethyl-8-(cyclohexylmethoxy)-2-ethylimidazo[1,2-a]pyridin-3-carboxylat in 1.15 ml Dioxan wurde mit 0.8 ml 2N Natronlauge versetzt und über Nacht bei Raumtemperatur gerührt. Dann wurde der Ansatz mit 1.5 ml 6N wässriger Salzsäure angesäuert, mit 5 ml Dichlormethan verdünnt und über eine Extrelut^{®}-Kartusche filtriert. Die Kartusche wurde mit 30ml Dichlormethan nachgewaschen, das Filtrat eingedampft und der entstehende Rückstand im HV getrocknet. So erhielt man 24 mg (97% d.Th.) 8-(Cyclohexylmethoxy)-2-ethylimidazo[1,2-a]pyridin-3-carbonsäure als Rohprodukt, das so in die Folgereaktion eingesetzt wurde.
LC-MS (Methode 1): Rₜ = 0.87 min
MS (ESpos): m/z = 303.2 (M+H)⁺

Die folgenden Syntheseintermediate in Tabelle 3A wurden in Analogie zu Beispielverbindung 57A durch basische Hydrolyse der entsprechenden Ester hergestellt:

**Tabelle 3A:**

| **Beispiel** | **IUPAC-Name / Struktur (Ausbeute)** | **Analytische Daten** |
|---|---|---|
| **58A** | 8-(Cyclohexylmethoxy)-2-trifluormethylimidazo[1,2-a]pyridin-3-carbonsäure | LC-MS (Methode 1): Rₜ = 1.15 min |
| | 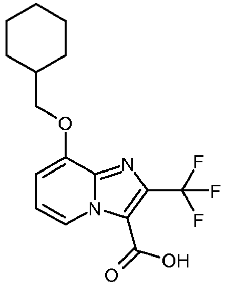 | MS (ESpos): m/z = 343.1 (M+H)⁺ |
| | (99% d. Th.) | |
| **59A** | 8-(Cyclohexylmethoxy)-2-propylimidazo[1,2-a]pyridin-3-carbonsäure | LC-MS (Methode 1): Rₜ = 0.94 min |
| | 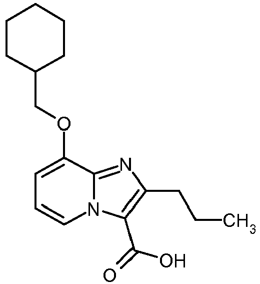 | MS (ESpos): m/z = 317.3 (M+H)⁺ |
| | (73% d. Th.) | |
| **60A** | 8-(Cyclohexylmethoxy)-2-methylimidazo[1,2-a]pyridin-3-carbonsäure | LC-MS (Methode 1): Rₜ = 0.82 min |
| | 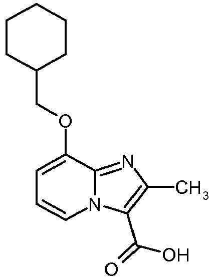 | MS (ESpos): m/z = 289.1 (M+H)⁺ |
| | (ca. 100% d. Th.) | |
| **61A** | 8-(Cyclobutylmethoxy)-2-methylimidazo[1,2-a]pyridin-3-carbonsäure | LC-MS (Methode 3): Rₜ = 1.67 min |
| | 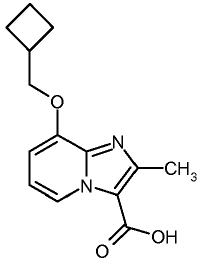 | MS (ESpos): m/z = 289.2 (M+H)⁺ |
| | (ca. 62% d. Th.) | |
| **62A** | 8-(Cyclobutylmethoxy)-2-ethylimidazo[1,2-a]pyridin-3-carbonsäure | LC-MS (Methode 1): Rₜ = 0.73 min |
| | 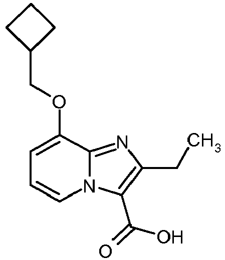 | MS (ESpos): m/z = 275.1 (M+H)⁺ |
| | (57% d. Th.) | |
| **63A** | 8-(Cyclohexylmethoxy)imidazo[1,2-a]pyridin-3-carbonsäure | LC-MS (Methode 2): Rₜ = 0.90 min |
| | 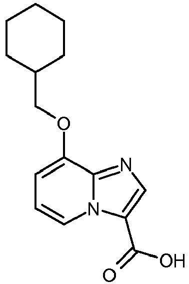 | MS (ESpos): m/z = 275.2 (M+H)⁺ |
| | (77% d. Th.) | |

### Beispiel 64A

### 8-[(2,6-Difluorbenzyl)oxy]-2-methyl-N-[(2R)-1-oxohexan-2-yl]imidazo[1,2-a]pyridin-3-carboxamid

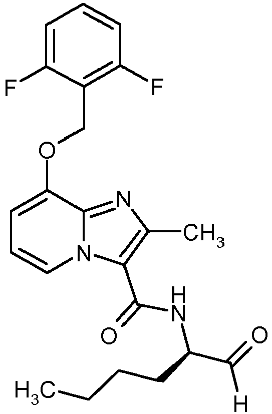

655 mg 1,1,1-Triacetoxy-1,1-dihydro-1,2-benziodoxol-3(1*H*)-on (Dess-Martin-Periodinan; 97%, Aldrich; 1.5 mmol) wurden in 10 mL wasserfreiem Dichlormethan vorgelegt und auf ca. -30 °C abgekühlt. Zu dieser Suspension wurden nacheinander 83 µl (1.03 mmol) Pyridin und 430 mg 8-[(2,6-Difluorbenzyl)oxy]-N-[(2R)-1-hydroxyhexan-2-yl]-2-methylimidazo[1,2-a]pyridin-3-carboxamid (1.03 mmol) in 20 mL Dichlormethan gegeben. Die Reaktions-mischung wurde auf Raumtemperatur erwärmt und weitere 4h gerührt. Dann wurde unter Eiskühlung mit 1N wässriger Natronlauge versetzt und dreimal mit Essigsäureethylester extrahiert. Die vereinten organischen Phasen wurden mit gesättigter wässriger Natriumchlorid-Lösung gewaschen, über Magnesiumsulfat getrocknet, abfiltriert und eingeengt. Der erhaltene Rückstand wurde ohne weitere Aufreinigung in Folgereaktionen eingesetzt.
LC-MS (Methode1): Rₜ = 1.0 min
MS (ESpos): m/z = 416 (M+H)⁺
¹H-NMR (400 MHz, DMSO-d6): δ = 0.88 (t, 3H), 1.25 - 1.50 (m, 4H), 1.61 - 1.71 (m, 1 H); 1.85 - 1.91 (m, 1 H); 2.57 (s, 3 H); 4.31 - 4.36 (m, 1 H); 5.32 (s, 2 H); 6.95 (t, 1 H); 7.03 (d, 1 H); 7.23 (t, 2 H); 7.59 (quint., 1 H); 8.21 (d, 1 H); 8.57 (d, 1 H); 9.56 (s, 1 H).

### Beispiel 65A

### Methyl-N-({8-[(2,6-difluorbenzyl)oxy]-2-methylimidazo[1,2-a]pyridin-3-yl}carbonyl)-L-norleucinat

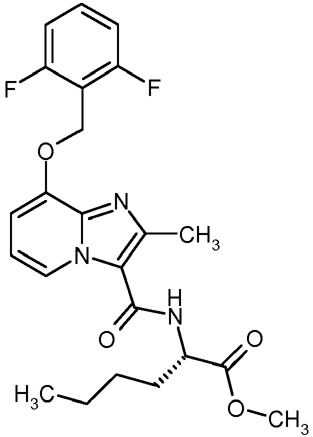

500 mg 8-[(2,6-Difluorbenzyl)oxy]-2-methylimidazo[1,2-a]pyridin-3-carbonsäure (Beispiel 6A; 1.57 mmol, 1 Äquivalent) wurden in 10 mL wasserfreiem Dichlormethan vorgelegt und nacheinander mit 313 mg Methyl-L-norleucinat Hydrochlorid (1.73 mmol, 1.1 Äquivalente), 554 mg Benzotriazol-1-yloxy)bisdimethylaminomethyliumfluoroborat (TBTU; 1.73 mmol, 1.1 Äquivalente) und 0.864 mL 4-Methylmorpholin (7.85 mmol, 5 Äquivalente) versetzt. Die Reaktionsmischung wurde 4h bei Raumtemperatur gerührt. Dann wurde mit Dichlormethan verdünnt, mit gesättigter wässriger Natriumhydrogencarbonat-Lösung, mit Wasser und abschließend mit gesättigter wässriger Natriumchloridlösung gewaschen, getrocknet und eingeengt. Der erhaltene Rückstand wurde über eine 100 g Kieselgel-Kartusche chromatographiert (Biotage Isolera; Cyclohexan-Essigsäureethylester Gradient als Eluent). Es wurden 644 mg der Zielverbindung (91% d. Th.) erhalten.
LC-MS (Methode1): Rₜ = 1.05 min
MS (ESpos): m/z = 446.2 (M+H)⁺
¹H-NMR (400 MHz, DMSO-d6): δ = 0.88 (t, 3H), 1.25 - 1.50 (m, 4H), 1.71 - 1.91 (m, 2 H), 2.57 (s, 3 H; verdeckt durch DMSO-Signal), 3.79 (s, 3 H), 4.45 (q, 1 H), 5.32 (s, 2 H), 6.91 (t, 1 H), 7.01 (d, 1 H), 7.21 (t, 2 H), 7.59 (quint., 1 H), 8.21 (d, 1 H), 8.51 (d, 1 H).
spezifischer Drehwert: -15.9 ° (c = 0.445 g in 100 mL Methanol, 100 mm Schichtdicke, 20.2 °C, 589 nM Wellanlänge).

### Beispiel 66A

### Methyl-N-({8-[(2,6-difluorbenzyl)oxy]-2-methylimidazo[1,2-a]pyridin-3-yl}carbonyl)-D-norleucinat

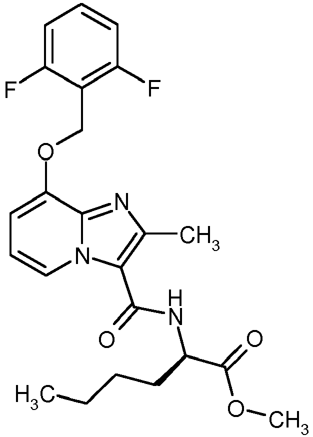

2 g 8-[(2,6-Difluorbenzyl)oxy]-2-methylimidazo[1,2-a]pyridin-3-carbonsäure (Beispiel 6A; 6.28 mmol, 1 Äquivalent) wurden in 40 mL wasserfreiem Dichlormethan vorgelegt und nacheinander mit 1.26 g Methyl-DL-norleucinat Hydrochlorid (6.91 mmol, 1.1 Äquivalente), 2.2 g Benzotriazol-1-yloxy)bisdimethylaminomethyliumfluoroborat (TBTU; 6.91 mmol, 1.1 Äquivalente) und 3.45 mL 4-Methylmorpholin (31.4 mmol, 5 Äquivalente) versetzt. Die Reaktionsmischung wurde 4h bei Raumtemperatur gerührt. Dann wurde mit Dichlormethan verdünnt, mit gesättigter wässriger Natriumhydrogencarbonat-Lösung, mit Wasser und abschließend mit gesättigter wässriger Natriumchloridlösung gewaschen, getrocknet und eingeengt. Der erhaltene Rückstand wurde über eine 340 g Kieselgel-Kartusche chromatographiert (Biotage Isolera; Cyclohexan-Essigsäureethylester Gradient als Eluent). Es wurden 2.75 g der Zielverbindung (97% d. Th.) erhalten. Das Racemat wurde in 40 mL Ethanol aufgelöst und durch chirale HPLC in die Enantiomere aufgetrennt (Daicel Chiralpak AD-H 5 µm 250x20 mm; 15 mL/min Flußrate, Eluent Ethanol, 220 nm Detektionswellenlänge, 2.5 mL Injektionsvolumen, 45 °C Temperatur).

### Enantiomer A: Entspricht Beispiel 65A

1.16 g Produkt (41% d. Th.)
LC-Analytik (Säule 250x4.6 mm gefüllt mit Daicel Chiralpak AD-H, 5 µM; Fluß 1.0 mL/min, Temperatur 40 °C, Eluent: Ethanol) Rₜ = 19.12 min; 99 % ee
spezifischer Drehwert: -15.3 ° (c = 0.33 g in 100 mL Methanol, 100 mm Schichtdicke, 19.6 °C, 589 nM Wellanlänge).

### Enantiomer B: Entspricht Enantiomer des Beispiels 65A

1.18 g Produkt (42% d. Th.)
LC-Analytik (Säule 250x4.6 mm gefüllt mit Daicel Chiralpak AD-H, 5 µM; Fluß 1.0 mL/min, Temperatur 40 °C, Eluent: Ethanol) Rₜ = 41.01 min; 99 % ee
spezifischer Drehwert: +15.1 ° (c = 0.445 g in 100 mL Methanol, 100 mm Schichtdicke, 19.9 °C, 589 nM Wellanlänge).
¹H-NMR (400 MHz, DMSO-d6): δ = 0.88 (t, 3H), 1.25 - 1.50 (m, 4H), 1.71 - 1.91 (m, 2 H), 2.57 (s, 3 H; verdeckt durch DMSO-Signal), 3.70 (s, 3 H), 4.45 (q, 1 H), 5.31 (s, 2 H), 6.91 (t, 1 H), 7.01 (d, 1 H), 7.21 (t, 2 H), 7.59 (quint., 1 H), 8.25 (d, 1 H), 8.51 (d, 1 H).

### Beispiel 67A

### (2R)-2-Aminobutan-1,4-diol

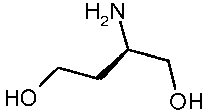

500 mg (R)-2-Cbz-aminobutan-1,4-diol wurden in 250 mL Ethanol gelöst, mit einer Spatelspitze Pd/C (10%ig) versetzt und bei Normaldruck für 90 Minuten mit Wasserstoff hydriert. Die Reaktionsmischung wurde durch Kieselgur filtiert, der Filterkuchen mit Ethanol nachgewaschen und das Filtrat eingeengt. Es wurden 199 mg (91% d. Th.) der Zielverbindung erhalten, die ohne weitere Aufreinigung weiter umgesetzt worden ist.
¹H-NMR (400 MHz, DMSO-d6): δ = 1.22 - 1.32 (m, 1 H), 1.46 - 1.55 (m, 1 H), 2.70 - 2.76 (m, 1 H), 3.12 (dd, 1 H), 3.30 (dd, 1 H), 3.49 (t, 2 H).

### Beispiel 68A

### (2R)-2-Aminopentan-1,5-diol

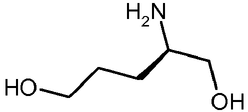

500 mg (R)-2-Cbz-aminopentan-1,5-diol wurden in 250 mL Ethanol gelöst, mit einer Spatelspitze Pd/C (10%ig) versetzt und bei Normaldruck für 90 Minuten mit Wasserstoff hydriert. Die Reaktionsmischung wurde durch Kieselgur filtiert, der Filterkuchen mit Ethanol nachgewaschen und das Filtrat eingeengt. Es wurden 183 mg (78% d. Th.) der Zielverbindung erhalten, die ohne weitere Aufreinigung weiter umgesetzt worden ist.
¹H-NMR (400 MHz, DMSO-d6): δ = 1.00 - 1.15 (m, 1 H), 1.30 - 1.65 (m, 3 H), 2.55 (m, 1 H; teilweise überdeckt durch DMSO-Signal), 3.10 (dd, 1 H), 3.28 (dd, 1 H), 3.40 (t, 2 H; teilweise überdeckt durch Wasser-Signal), 4.50 (br.s, 1 H).

### Repräsentative Arbeitsvorschrift 1a

### Reduktion von Aminosäuren unter Verwendung von Lithiumborhydrid und Chlortrimethylsilan.

1.7-2.5 Äquivalente Lithiumborhydrid wurden in THF vorgelegt (ca. 0.1-0.5 M bezogen auf die Aminosäure) und mit 3.4-5.0 Äquivalenten Chlortrimethylsilan (bei 0°C oder RT) versetzt und fünf bis 30 Minuten bei RT gerührt. Anschließend wurde 1 Äquivalent der Aminosäure vorsichtig portionsweise bei 0°C oder RT zugegeben und das Reaktionsgemisch wurde 1-2 d bei RT gerührt. Beispielhafte Aufarbeitung der Reaktionsmischung: Es wurde Methanol zugegeben und das Gemisch wurde eingeengt. Der Rückstand wurde mit einer 20%igen wässrigen Kaliumhydroxid-Lösung versetzt und dreimal mit Dichlormethan extrahiert. Die vereinigten org. Phasen wurden über Natriumsulfat getrocknet, filtriert und eingeengt.

### Beispiel 69A

### 2-Amino-4,4,4-trifluorbutan-1-ol

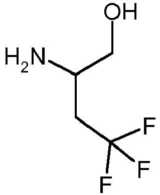

0.32 ml (0.65 mmol) Lithiumborhydrid (2 M in THF) wurden in 0.5 ml trockenem THF vorgelegt, mit 0.16 ml (1.28 mmol) Chlortrimethylsilan bei RT versetzt und 5 min bei RT gerührt. Anschließend wurden 50 mg (0.26 mmol) 2-Amino-4,4,4-trifluorbutansäurehydrochlorid (1:1) portionsweise zugegeben und das Reaktionsgemisch wurde über Nacht bei RT gerührt. Zum Reaktionsgemisch wurden 0.5 ml Methanol gegeben und das Gemisch wurde eingeengt. Der Rückstand wurde mit 0.6 ml einer 20%igen wässrigen Kaliumhydroxid-Lösung versetzt und dreimal mit Dichlormethan extrahiert. Die vereinigten organischen Phasen wurden über Natrimsulfat getrocknet, filtriert und eingeengt. Es wurden 33 mg der Zielverbindung (88% d. Th.) erhalten.
DCI-MS (Methode 13): MS (ESpos): m/z = 144 (M+H)⁺
¹H-NMR (400 MHz, DMSO-d₆): δ = 2.08-2.20 (m, 1H), 2.22-2.38 (m, 1H), 3.25-3.32 (m, 1H), 3.39-3.44 (m, 1H), 3.59-3.65 (m, 1H).

In Analogie zu Beispiel 69A wurden die in Tabelle 4A gezeigten Beispielverbindungen hergestellt, indem Lithiumborhydrid (1.7-2.5 Äquivalente) und Chlortrimethylsilan (3.4-5 Äquivalente) mit den entsprechenden, kommerziell erhältlichen Aminosäuren unter den beschriebenen Reaktionsbedingungen umgesetzt worden sind:

**Tabelle 4A:**

| **Beispiel** | **IUPAC-Name / Struktur (Ausbeute)** | **Analytische Daten** |
|---|---|---|
| **70A** | 2-Amino-5,5,5-trifluorpentan-1-ol | DCI-MS (Methode 13): |
| | 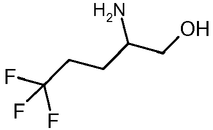 | MS (ESpos): m/z = 158 (M+H)⁺ |
| | (65% d. Th.) | |
| **71A** | 2-Amino-6,6,6-trifluorhexan-1-ol | DCI-MS (Methode 13): |
| | 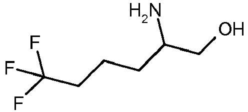 | MS (ESpos): m/z = 172 (M+H)⁺ |
| | (75% d. Th.) | |
| **72A** | 2-Amino-3,3,3-trifluorpropan-1-ol | LC-MS (Methode 13): |
| | 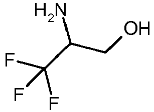 | MS (ESpos): m/z = 130 (M+H)⁺ |
| | (63% d. Th.) | |
| **73A** | 2-Amino-6,6,6-trifluorhexan-1,5-diolhydrochlorid (1:1) | LC-MS (Methode 13): MS (ESpos): m/z = 188 (M-HCl+H)⁺ |
| | 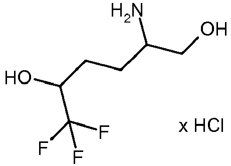 | |
| | (ca. 90% d. Th.) | |
| **74A** | 2-Amino-2-[4-(methylsulfonyl)phenyl]ethanol | LC-MS (Methode 2): |
| | 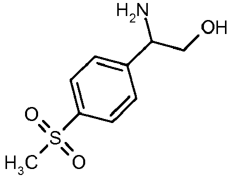 | MS (ESpos): m/z = 216 (M+H)⁺ |
| | (ca. 98% d. Th., Reinheit ca. 80%) | |

### Beispiel 75A

### Methyl 2-amino-4,4,4-trifluorbutanoat Hydrochlorid (1:1)

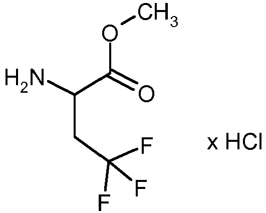

1.186 g (6.127 mmol) 2-Amino-4,4,4-trifluorbutansäurehydrochlorid (1:1) wurden in 11.6 ml mit Chlorwasserstoff gesättigtem Methanol vorgelegt und 4 h unter Rückfluss gerührt. Die Reaktionslösung wurde eingeengt und im Hochvakuum getrocknet. Es wurden 1.275 g der Zielverbindung (100% d. Th.) erhalten.
DCI-MS (Methode 13): MS (ESpos): m/z = 172 (M-HCl+H)⁺
¹H-NMR (400 MHz, DMSO-d₆): δ = 2.90-3.08 (m, 2H), 3.78 (s, 3H), 3.41 (t, 1H), 8.89 (br s, 3H).

In Analogie zu Beispiel 75A wurden die in Tabelle 5A gezeigten Beispielverbindungen hergestellt, indem Chlorwasserstoff in Methanol mit den entsprechenden, kommerziell erhältlichen Aminosäuren unter den beschriebenen Reaktionsbedingungen umgesetzt worden sind:

**Tabelle 5A:**

| **Beispiel** | **IUPAC-Name / Struktur (Ausbeute)** | **Analytische Daten** |
|---|---|---|
| **76A** | Methyl 5,5,5-trifluornorvalinat Hydrochlorid (1:1) | DCI-MS (Methode 15): |
| | 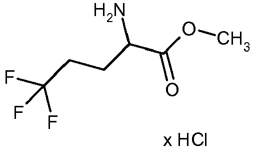 | MS (ESpos): m/z = 186 (M+H)⁺ |
| | (94% d. Th.) | |
| **77A** | Methyl-6,6,6-trifluornorleucinat Hydrochlorid (1:1) | DCI-MS (Methode 13): MS (ESpos): m/z = 200 (M-HCl+H)⁺ |
| | 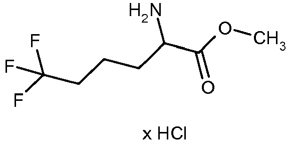 | ¹H-NMR (400 MHz, DMSO-d₆): δ = 1.48-1.73 (m, 2H), 1.82-1.96 (m, 2H), 2.24-2.38 (m, 2H), 3.76 (s, 3H), 4.06-4.12 (m, 1H), 8.54-8.70 (br s, 3H). |
| | (100% d. Th.) | |

### Repräsentative Arbeitsvorschrift 2a

### Amidbildung unter Verwendung von TBTU als Kupplungsreagenz.

1 Äquivalent der zu kuppelnden Carbonsäure, 1.1 - 1.5 Äquivalente (Benzotriazol-1-yloxy)bisdimethylaminomethyliumfluoroborat (TBTU) und 3-6 Äquivalente 4-Methylmorpholin wurden in DMF oder Dichlormethan (ca. 0.1-0.2 M bezogen auf die zu kuppelnde Carbonsäure) vorgelegt und anschließend wurden 1.1 bis 1.5 Äquivalente des zu kuppelnden Amins zugesetzt und über Nacht bei Raumtemperatur gerührt.

Beispielhafte Aufarbeitung der Reaktionsmischung: Die Reaktionslösung wurde mit Wasser versetzt, der entstandene Niederschlag noch 0.5-1.0 h ausgerührt, abfiltriert, gut mit Wasser gewaschen und über Nacht im Hochvakuum getrocknet. Alternativ wurde das Reaktionsrohgemisch direkt aufkonzentriert und per präparativer HPLC weiter aufgereinigt undüber Nacht im Hochvakuum getrocknet.

### Beispiel 78A

### Methyl-N-({8-[(2,6-difluorbenzyl)oxy]-2-methylimidazo[1,2-a]pyridin-3-yl}carbonyl)-beta-alaninat

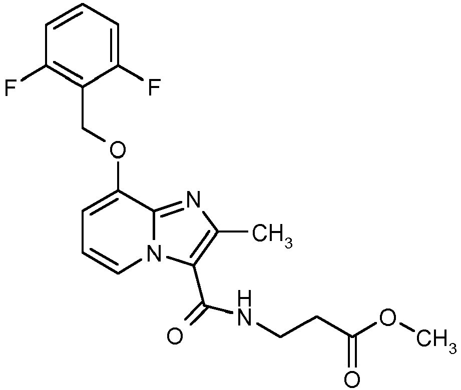

300 mg (0.94 mmol) 8-[(2,6-Difluorbenzyl)oxy]-2-methylimidazo[1,2-a]pyridin-3-carbonsäure, 454 mg (1.41 mmol) (Benzotriazol-1-yloxy)bisdimethylaminomethyliumfluoroborat (TBTU) und 477 mg (4.71 mmol) 4-Methylmorpholin wurden in 6.0 ml DMF vorgelegt. Nach 10 min bei RT wurden 158 mg (1.13 mmol) Methyl-beta-alaninat-Hydrochlorid zugesetzt und es wurde über Nacht bei Raumtemperatur gerührt. Die Reaktionslösung wurde mit ca. 48 ml Wasser versetzt, der entstandene Niederschlag noch 30 min ausgerührt, abfiltriert, mit Diethylether gewaschen und über Nacht im Hochvakuum getrocknet. Es wurden 334 mg der Zielverbindung (88% d. Th.) erhalten.
LC-MS (Methode 1): Rₜ = 0.78 min
MS (ESpos): m/z = 404 (M+H)⁺
¹H-NMR (400 MHz, DMSO-d₆): δ = 2.48 (s, 3H), 2.63 (t, 2H), 3.58 (q, 2H), 3.63 (s, 3H), 5.30 (s, 2H), 6.92 (t, 1H), 7.01 (d, 1H), 7.23 (t, 2H), 7.59 (quint, 1H), 7.94 (t, 1H), 8.62 (d, 1H).

In Analogie zu Beispielverbindung 78A wurden die in Tabelle 6A gezeigten Beispielverbindungen hergestellt, indem 8-[(2,6-Difluorbenzyl)oxy]-2-methylimidazo[1,2-a]pyridin-3-carbonsäure mit den entsprechenden, kommerziell erhältlichen Aminen in DMF oder Dichlormethan unter den in der repräsentativen Arbeitsvorschrift 2a beschriebenen Reaktionsbedingungen umgesetzt worden ist:

**Tabelle 6A:**

| **Beispiel** | **IUPAC-Name / Struktur (Ausbeute)** | **Analytische Daten** |
|---|---|---|
| **79A** | *rac*-Methyl 2-[({8-[(2,6-difluorbenzyl)oxy]-2-methylimidazo[1,2-a]pyridin-3-yl}carbonyl)amino]-4,4,4-trifluorbutanoat | LC-MS (Methode 1): Rₜ = 0.95 min |
| | | MS (ESpos): m/z = 472 (M+H)⁺ |
| | 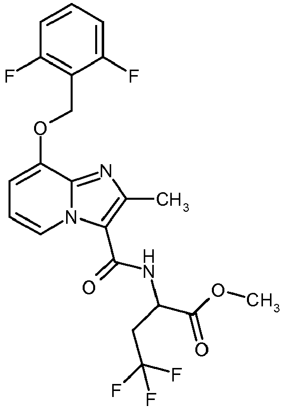 | ¹H-NMR (400 MHz, DMSO-d₆): δ = 2.50 (s, 3H), 2.88-3.04 (m, 2H), 3.72 (s, 3H), 4.80-4.87 (m, 1H), 5.31 (s, 2H), 6.98 (t, 1H), 7.07 (d, 1H), 7.22 (t, 2H), 7.59 (quint, 1H), 8.49 (d, 1H), 8.58 (d, 1H). |
| | (80% d. Th.) | |
| **80A** | *rac*-Methyl-*N-*({8-[(2,6-difluorbenzyl)oxy]-2-methylimidazo[1,2-a]pyridin-3-yl}carbonyl)-5,5,5-trifluornorvalinat | LC-MS (Methode 3): Rₜ = 2.05 min |
| | | MS (ESpos): m/z = 486 (M+H)⁺ |
| | 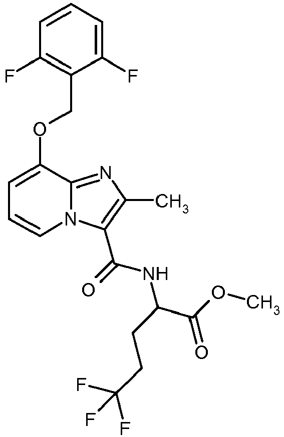 | ¹H-NMR (400 MHz, DMSO-d₆): δ = 1.97-2.18 (m, 2H), 2.30-2.54 (m, 2H), darunter bei 2.50 (s, 3H), 3.71 (s, 3H), 4.59-4.67 (m, 1H), 5.32 (s, 2H), 6.96 (t, 1H), 7.04 (d, 1H), 7.22 (t, 2H), 7.60 (quint, 1H), 8.34 (d, 1H), 8.55 (d, 1H). |
| | (91% d. Th.) | |
| **81A** | *rac*-Methyl-*N-*({8-[(2,6-difluorbenzyl)oxy]-2-methylimidazo[1,2-a]pyridin-3-yl}carbonyl)-6,6,6-trifluornorleucinat | LC-MS (Methode 1): Rₜ = 1.04 min |
| | | MS (ESpos): m/z = 500 (M+H)⁺ |
| | 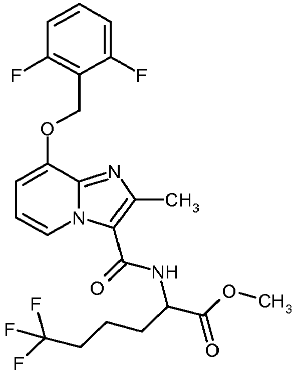 | ¹H-NMR (400 MHz, DMSO-d₆): δ = 1.59-1.69 (m, 2H), 1.80-2.01 (m, 2H), 2.20-2.43 (m, 2H), 2.50 (s, 3H), 3.70 (s, 3H), 4.49-4.57 (m, 1H), 5.31 (s, 2H), 6.94 (t, 1H), 7.03 (d, 1H), 7.22 (t, 2H), 7.60 (quint, 1H), 8.33 (d, 1H), 8.49 (d, 1H). |
| | (95% d. Th.) | |
| **82A** | *rac*-Methyl (4-chlorphenyl)[({8-[(2,6-difluorobenzyl)oxy]-2-methylimidazo[1,2-a]pyridin-3-yl}carbonyl)amino]acetat | LC-MS (Methode 1): Rₜ = 1.10 min |
| | | MS (ESpos): m/z = 500 (M+H)⁺ |
| | 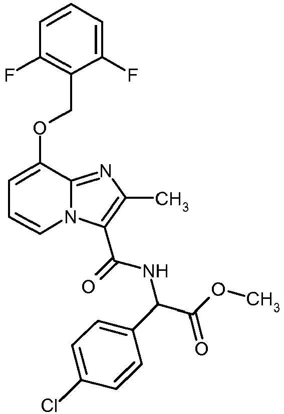 | ¹H-NMR (400 MHz, DMSO-d₆): δ = 2.50 (s, 3H), 3.70 (s, 3H), 5.31 (s, 2H), 5.71 (d, 1H), 6.98 (t, 1H), 7.03 (d, 1H), 7.22 (t, 2H), 7.49 (d, 2H), 7.53-7.63 (m, 3H), 8.53 (d, 1H), 8.73 (d, 1H). |
| | (72% d. Th.) | |
| **83A** | *rac*-Methyl [({8-[(2,6-difluorbenzyl)oxy]-2-methylimidazo[1,2-a]pyridin-3-yl}carbonyl)amino](4-fluorphenyl)acetat | LC-MS (Methode 1): Rₜ = 1.04 min |
| | | MS (ESpos): m/z = 484 (M+H)⁺ |
| | 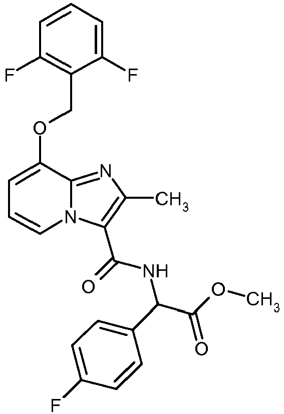 | ¹H-NMR (400 MHz, DMSO-d₆): δ = 2.50 (s, 3H), 3.69 (s, 3H), 5.31 (s, 2H), 5.70 (d, 1H), 6.96 (t, 1H), 7.03 (d, 1H), 7.19-7.28 (m, 4H), 7.53-7.63 (m, 3H), 8.53 (d, 1H), 8.72 (d, 1H). |
| | (84% d. Th.) | |

### Beispiel 84A

### 3-(Benzyloxy)-5-brompyridin-2-amin

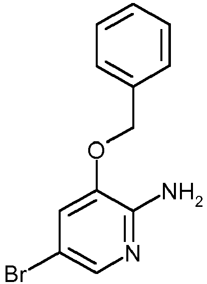

200 g (1 mol) 2-Amino-3-benzyloxypyridin wurden in 4 l Dichlormethan vorgelegt und bei 0°C innerhalb von 30 min mit einer Lösung aus 62 mL (1.2 mol) Brom in 620 mL Dichlormethan versetzt. Nach beendeter Zugabe wurde die Reaktionslösung 60 min bei 0°C gerührt. Dann wurde das Gemisch mit ca. 4 l gesättigter wässriger Natriumhydrogencarbonat-Lösung versetzt. Die organische Phase wurde abgetrennt und eingeengt. Der Rückstand wurde mittels Kieselgelsäulechromatographie (Petrolether:Essigsäurethylester 6:4) gereinigt und die Produktfraktionen wurden eingeengt. Man erhielt 214 g (77% d. Th.) der Titelverbindung.
LC-MS (Methode 1): Rₜ = 0.92 min
MS (ESpos): m/z = 279 (M+H)⁺
¹H-NMR (400 MHz, DMSO-d₆): δ = 5.16 (s, 2H), 5.94 - 6.00 (m, 2H), 7.26 - 7.29 (m, 1H), 7.31 - 7.36 (m, 1H), 7.37 - 7.43 (m, 2H), 7.47-7.52 (m, 2H), 7.57 - 7.59 (m, 1H).

### Beispiel 85A

### Ethyl-8-(benzyloxy)-6-brom-2-methylimidazo[1,2-a]pyridin-3-carboxylat

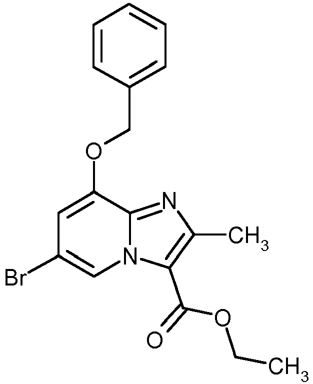

Unter Argon wurden 200 g (0.72 mol) 3-(Benzyloxy)-5-brompyridin-2-amin Beispiel 84A, 590 g (3.58 mol) Ethyl-2-chloracetoacetat und 436 g 3A Molsieb in 61 Ethanol suspendiert und 72 h bei RF gekocht. Die Reaktionsmischung wurde über Kieselgur abfiltriert und eingeengt. Der Rückstand wurde mittels Kieselgelchromatographie (Petrolether:Essigsäureethylester 9:1, anschließend 6:4) gereinigt und die Produktfraktionen wurden eingeengt. Man erhielt 221 g (79% d. Th.) der Zielverbindung.
LC-MS (Methode 17): Rₜ = 1.31 min
MS (ESpos): m/z = 389 (M+H)⁺
¹H-NMR (400 MHz, DMSO-d₆): δ = 1.36 (t, 3 H), 2.58 (s, 3 H), 4.32 - 4.41 (m, 2 H), 5.33 (s, 2 H), 7.28 - 7.32 (m, 1 H), 7.36 - 7.47 (m, 3 H), 7.49 - 7.54 (m, 2 H), 8.98 (d, 1 H).

### Beispiel 86A

### Ethyl-8-(benzyloxy)-2,6-dimethylimidazo[1,2-a]pyridin-3-carboxylat

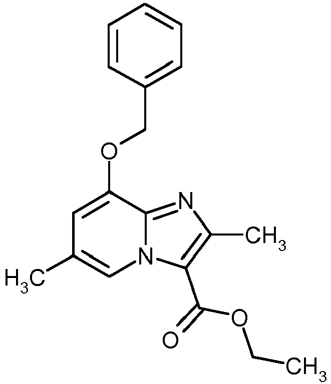

105 g (270 mmol) Ethyl-8-(benzyloxy)-6-brom-2-methylimidazo[1,2-a]pyridin-3-carboxylat Beispiel 85A wurden unter Argon in 4.2 1 1,4-Dioxan suspendiert und nacheinander mit 135.4 g (539 mmol, Reinheit 50%) Trimethylboroxin, 31.2 g (27 mmol) Tetrakis(triphenylphosphin)-palladium(0) und 78.3 g (566 mmol) Kaliumcarbonat versetzt und 8 h unter Rückfluss gerührt. Die auf RT abgekühlte Reaktionsmischung wurde über Kieselgel vom Niederschlag abfiltriert und das Filtrat wurde eingeengt. Der Rückstand wurde in Dichlormethan gelöst und mittels Kieselgelchromatographie (Dichlormethan:Essigsäureethylester = 9:1) gereinigt. Man erhielt 74 g (84.6% d. Th.; Reinheit 100%) der Zielverbindung.
LC-MS (Methode 17): Rₜ = 1.06 min
MS (ESpos): m/z = 325 (M+H)⁺
¹H-NMR (400 MHz, DMSO-d₆): δ = 1.35 (t, 3 H), 2.34 (br. s, 3 H), 2.56 (s, 3 H), 4.31 - 4.38 (m, 2 H), 5.28 (br. s, 2 H), 6.99 - 7.01 (m, 1 H), 7.35 - 7.47 (m, 3 H), 7.49 - 7.54 (m, 2 H), 8.68 - 8.70 (m, 1 H).

### Beispiel 87A

### Ethyl-8-hydroxy-2,6-dimethylimidazo[1,2-a]pyridin-3-carboxylat

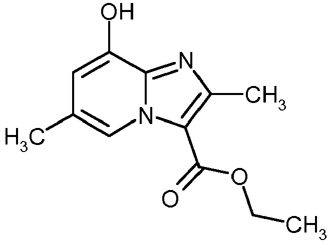

74 g (228 mmol) Ethyl-8-(benzyloxy)-2,6-dimethylimidazo[1,2-a]pyridin-3-carboxylat Beispiel 86A wurden in 1254 ml Dichlormethan und 251 ml Ethanol vorgelegt und unter Argon mit 20.1 g 10%igem Palladium auf Aktivkohle (wasserfeucht 50%) versetzt. Das Reaktionsgemisch wurde über Nacht bei RT und Normaldruck hydriert. Die Reaktionsmischung wurde über Kieselgur abfiltriert und eingeengt. Der Rohprodukt wurde mittels Kieselgelchromatographie (Dichlormethan:Methanol = 95:5) gereinigt. Man erhielt 50.4 g (94% d. Th.) der Zielverbindung.
DCI-MS: (Methode 13) (ESpos): m/z = 235.2 (M+H)⁺
¹H-NMR (400 MHz, DMSO-d₆): δ = 1.35 (t, 3 H), 2.27 (s, 3 H), 2.58 (s, 3 H), 4.30 - 4.38 (m, 2 H), 6.65 (d, 1 H), 8.59 (s, 1 H), 10.57 (br. s, 1H).

### Beispiel 88A

### Ethyl-2,6-dimethyl-8-(3-methylbutoxy)imidazo[1,2-a]pyridin-3-carboxylat

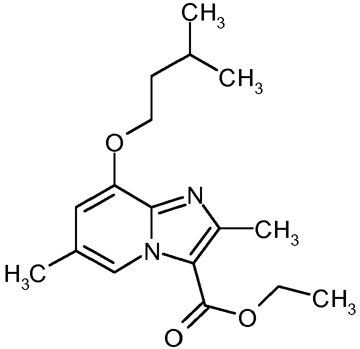

2.0 g (8.5 mmol) Ethyl-8-hydroxy-2,6-dimethylimidazo[1,2-a]pyridin-3-carboxylat Beispiel 87A wurden in 122.3 mL DMF vorgelegt und mit 1.23 mL (9.4 mmol) 1-Iod-3-methyl-butan sowie 6.12 g (18.8 mmol) Cäsiumcarbonat versetzt. Es wurde 40 min bei 60°C gerührt. Das auf RT abgekühlte Reaktionsgemisch wurde mit 900 mL Wasser versetzt und 1 h bei RT gerührt. Der ausgefallene Feststoff wurde abfiltriert, mit Wasser gewaschen und am Hochvakuum getrocknet. Man erhielt 2.25 g (84% d. Th.; Reinheit 97%) der Titelverbindung.
LC-MS (Methode 17): Rₜ = 1.12 min
MS (ESpos): m/z = 305 (M+H)⁺
¹H-NMR (400 MHz, DMSO-d₆): δ = 0.96 (d, 6H), 1.35 (t, 3H), 1.70 (q, 2H), 1.77 - 1.89 (m, 1H), 2.33 (s, 3H), 2.56 (s, 3H), 4.17 (t, 2H), 4.34 (q, 2H), 6.88 (s, 1H), 8.64 (s, 1H).

### Beispiel 89A

### 2,6-Dimethyl-8-(3-methylbutoxy)imidazo[1,2-a]pyridin-3-carbonsäure

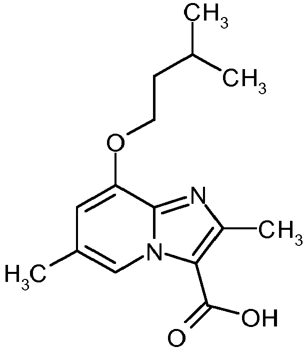

2.25 g (7.4 mmol) Ethyl-2,6-dimethyl-8-(3-methylbutoxy)imidazo[1,2-a]pyridin-3-carboxylat Beispiel 88A wurden in 157 mL THF/Methanol (5:1) vorgelegt, mit 37 mL (37 mmol) 1N wässriger Lithiumhydroxid-Lösung versetzt und die Reaktionsmischung wurde über das Wochenende bei RT gerührt. Dann wurde auf 0°C abgekühlt, mit 6 N wässriger Salzsäure auf pH 4 angesäuert und am Rotationsverdampfer vom organischen Lösungsmittel befreit. Der ausgefallene Feststoff wurde abfiltriert, mit Wasser gewaschen und am Hochvakuum getrocknet. Man erhielt 1.64 g (80% d. Th.; Reinheit 100%) der Titelverbindung.
LC-MS (Methode 1): Rₜ = 0.71 min
MS (ESpos): m/z = 277 (M+H)⁺
¹H-NMR (400 MHz, DMSO-d₆): δ = 0.96 (d, 6H), 1.70 (q, 2H), 1.78 - 1.89 (m, 1H), 2.32 (s, 3H), 2.56 (s, 3H), 4.17 (t, 2H), 6.85 (s, 1H), 8.69 (s, 1H), 12.86 - 13.08 (m, 1H).

### Beispiel 90A

### Ethyl-2,6-dimethyl-8-[4,4,4-trifluor-3-(trifluormethyl)butoxy]imidazo[1,2-a]pyridin-3-carboxylat

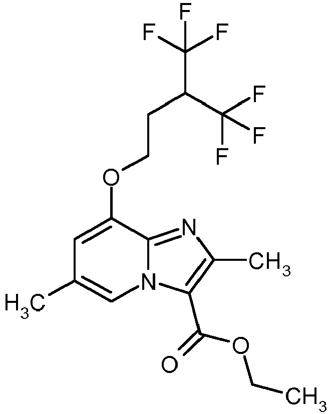

1.89 g (8.07 mmol) Ethyl-8-hydroxy-2,6-dimethylimidazo[1,2-a]pyridin-3-carboxylat Beispiel 87A wurden in 60 mL DMF vorgelegt, mit 7.89 g (24.2 mmol) Cäsiumcarbonat und 2.30 g (8.88 mmol) 4,4,-Trifluoro-3-(trifluoromethyl)butylbromid versetzt und das Reaktionsgemisch wurde 90 min bei RT gerührt. Dann wurde mit 60 ml Wasser versetzt, der ausgefallene Feststoff abfiltriert und der Filterrückstand mit 100 ml Wasser und zweimal mit 20 ml tert.-Butylmethylether nachgewaschen. Der aus dem Filtrat ausgefallene Niederschlag wurde abfiltriert und mit Mutterlauge nachgewaschen. Beide Filterrückstände wurden mit 50 ml Essigsäureethylester aufgenommen. Die Lösung wurde am Rotationsverdampfer eingeengt und der Rückstand über Nacht im Vakuum getrocknet. Es wurden 2.25 g der Zielverbindung (95% Reinheit, 64% d. Th.) erhalten.
LC-MS (Methode 1): Rₜ = 1.16 min
MS (ESpos): m/z = 413 (M+H)⁺
¹H-NMR (400 MHz, DMSO-d₆): δ = 1.36 (t, 3H), 2.34 (s, 3H), 2.32-2.38 (m, 2H), 2.58 (s, 3H), 4.18-4.30 (m, 1H), 4.31-4.38 (m, 4H), 6.93 (s, 1H), 8.71 (s, 1H).

### Beispiel 91A

### 2,6-Dimethyl-8-[4,4,4-trifluor-3-(trifluormethyl)butoxy]imidazo[1,2-a]pyridin-3-carbonsäure

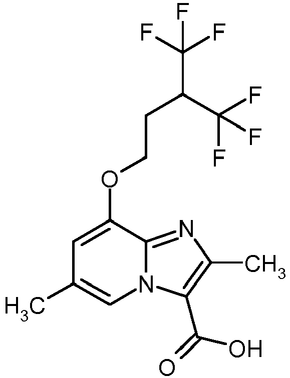

1.95 g (4.73 mmol) Ethyl-2,6-dimethyl-8-[4,4,4-trifluor-3-(trifluormethyl)butoxy]imidazo[1,2-a]pyridin-3-carboxylat Beispiel 90A wurden in 30 ml Methanol vorgelegt, mit 3.28 g (10.4 mmol) Bariumhydroxid-Octahydrat versetzt und 3 Tage bei RT gerührt. Die Suspension wurde mit 30 ml Wasser verdünnt und mit 1 M wässriger Salzsäure auf pH 6 gestellt. Der Feststoff wurde abfiltriert, mit 50 ml Wasser gewaschen und bei 70°C 2 h im Vakuum getrocknet. Es wurden 1.64 g der Zielverbindung (90% Reinheit, 81 % d. Th.) erhalten.
LC-MS (Methode 1): Rₜ = 0.78 min
MS (ESpos): m/z = 385 (M+H)⁺
¹H-NMR (400 MHz, DMSO-d₆): δ = 2.29 (s, 3H), 2.28-2.37 (m, 2H), 2.56 (s, 3H), 4.22-4.35 (m, 3H), 6.74 (s, 1H), 8.99 (s, 1H), Säure-OH nicht sichtbar.

### Beispiel 92A

### rac-Ethyl-8-[1-(2,6-difluorphenyl)ethoxy]-2,6-dimethylimidazo[1,2-a]pyridin-3-carboxylat

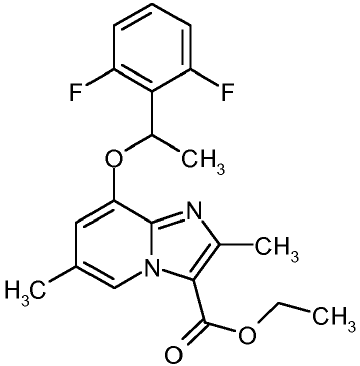

5.50 g (23.5 mmol) Ethyl-8-hydroxy-2,6-dimethylimidazo[1,2-a]pyridin-3-carboxylat Beispiel 87A wurden mit 4.46 g (28.2 mmol) 1-(2,6-Difluorphenyl)ethanol, 5.35 mL (27.0 mmol) Azodicarbonsäurediisopropylester und 7.08 g (27.0 mmol) Triphenylphosphin in 141 mL THF gelöst und 2 h bei RT gerührt. Die Reaktionsmischung wurde mit 0.70 mL (3.5 mmol) Azodicarbonsäurediisopropylester und 0.62 g (2.3 mmol) Triphenylphosphin versetzt und die Reaktionslösung wurde 1 h bei RT gerührt. Der ausgefallene Feststoff wurde abfiltriert und am Hochvakuum getrocknet. Man erhielt 4.6 g (52.8% d. Th.; Reinheit 100%) der Titelverbindung. Das Fitrat wurde eingeengt und zweimal mittels Kieselgelchromatographie (Cyclohexan:Essigsäureethylester-Gradient = 8:1 nach 4:1) gereinigt. Alle produkthaltigen Fraktionen wurden nochmals mittels präparativer HPLC gereinigt (RP18 Säule, Laufmittel: Acetonitril/Wasser-Gradient unter Zusatz von 0.1% TFA). Man erhielt nochmals 2.16 g (25% d. Th.) der Zielverbindung.
LC-MS (Methode 1): Rₜ = 1.08 min
MS (ESpos): m/z = 375 (M+H)⁺
¹H-NMR (400 MHz, DMSO-d₆): δ = 1.34 (t, 3H), 1.79 (d, 3H), 2.25 (s, 3H), 2.58 (s, 3H), 4.33 (q, 2H), 6.17 (q, 1H), 6.73 (s, 1H), 7.06 - 7.16 (m, 2H), 7.37 - 7.48 (m, 1H), 8.67 (s, 1H).

### Beispiel 93A

### ent-Ethyl-8-[1-(2,6-difluorphenyl)ethoxy]-2,6-dimethylimidazo[1,2-a]pyridin-3-carboxylat (Enantiomer B)

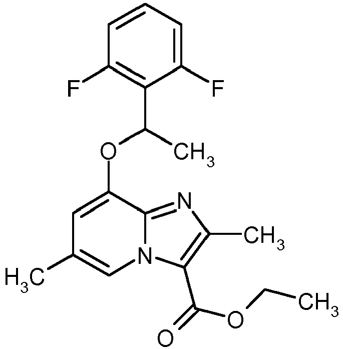

6.8 g Beispiel 92A wurden durch präparative Trennung an chiraler Phase in die Enantiomere getrennt [Säule: Daicel Chiralpak AD-H, 5 µm, 250 x 30 mm, Eluent: 70% Iso-Hexan, 30% Ethanol, Fluß: 50 ml/min; 40°C, Detektion: 210 nm].

### Enantiomer B:

Ausbeute: 2.7 g (98.4% ee)
Rt= 5.18 min [Daicel Chiralpak AD-H, 5µm, 250 x 4.6 mm; Eluent: 70% Iso-Hexan, 30% Ethanol; Fluss 1.0 ml/min; 30°C; Detektion: 220 nm].

### Beispiel 94A

### ent-8-[1-(2,6-Difluorphenyl)ethoxy]-2,6-dimethylimidazo[1,2-a]pyridin-3-carbonsäure (Enantiomer B)

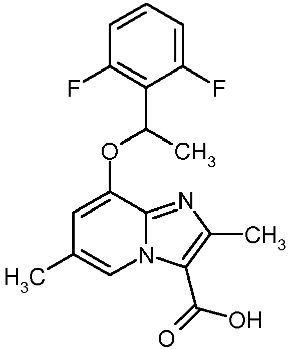

2.58 g (6.9 mmol) ent-Ethyl-8-[1-(2,6-difluorphenyl)ethoxy]-2,6-dimethylimidazo[1,2-a]pyridin-3-carboxylat Beispiel 93A (Enantiomer B) wurde in 154 mL THF/Methanol (5:1) gelöst, mit 34.5 mL (34.5 mmol) 1 N wässriger Lithiumhydroxid-Lösung versetzt und 5 h bei 40°C gerührt. Die auf RT abgekühlte Reaktionsmischung wurde mit 6 N wässriger Salzsäure-Lösung angesäuert und dann eingeengt. Der Feststoff wurde abfiltriert, mit Wasser gewaschen und am Hochvakuum getrocknet. Man erhielt 2.26 g (95% d. Th.; Reinheit 100%) der Titelverbindung.
LC-MS (Methode 1): Rₜ = 0.74 min
MS (ESpos): m/z = 347 (M+H)⁺
¹H-NMR (400 MHz, DMSO-d₆): δ = 1.79 (d, 3H), 2.24 (s, 3H), 2.57 (s, 3H), 6.16 (q, 1H), 6.67 (s, 1H), 7.06 - 7.16 (m, 2H), 7.38 - 7.48 (m, 1H), 8.74 (s, 1H), 12.24 - 13.90 (br. s, 1H).

### Beispiel 95A

### rac-Methyl-N-[(benzyloxy)carbonyl]norleucinat

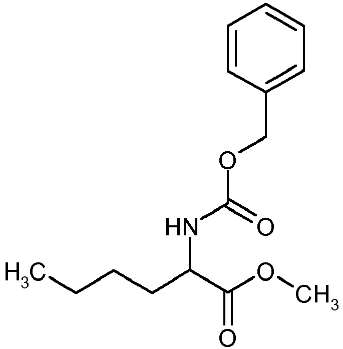

12 g (66.1 mmol) *rac*-Methylnorleucinathydrochlorid wurden in 974 ml Wasser/THF (8:1) vorgelegt und mit 28.3 g (204.8 mmol) Kaliumcarbonat versetzt. Das Reaktionsgemisch wurde auf 0°C abgekühlt. 12.3 ml (72.7 mmol) Chlorameisensäurebenzylester wurden langsam zugetropft und die Reaktionsmischung über Nacht bei RT gerührt. Das Gemisch wurde mit 480 ml Wasser verdünnt und dreimal mit Dichlormethan extrahiert. Die vereinigten organischen Phasen wurden über Natriumsulfat getrocknet, abfiltriert und eingeengt. Der Rückstand wurde mittels Kieselgelchromatographie (Cyclohexan/Essigsäureethylester = 4:1) gereinigt. Man erhielt 18.0 g (97.6% d. Th., Reinheit 100%) der Zielverbindung.
LC-MS (Methode 1): Rₜ = 1.10 min.
MS (ESIpos): m/z = 280 (M+H)⁺.
¹H-NMR (400 MHz, DMSO-d₆): δ = 0.79 - 0.90 (m, 3H), 1.21 - 1.35 (m, 4H), 1.52 - 1.73 (m, 2H), 3.63 (s, 3H), 3.95 - 4.05 (m, 1H), 4.97 - 5.11 (m, 2H), 7.24 - 7.42 (m, 5H), 7.74 (d, 1H).

### Beispiel 96A

### rac-Benzyl-(2-hydroxy-2-methylheptan-3-yl)carbamat

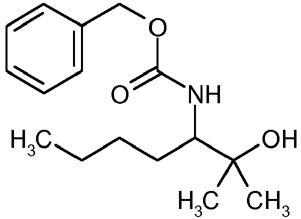

16.87 g (60.4 mmol) *rac*-Methyl-*N*-[(benzyloxy)carbonyl]norleucinat Beispiel 95A wurden unter Argon in 584 ml THF vorgelegt. Das Reaktionsgemisch wurde auf 0°C abgekühlt, 70.5 ml (211.4 mmol) 3 M Methylmagnesiumbromid in Diethylether wurden zugetropft und es wurde 15 min bei 0°C nachgerührt. Dann ließ man langsam auf RT kommen und rührte über Nacht bei Raumtemperatur. Das Reaktionsgemisch wurde vorsichtig mit 1 N wässriger Salzsäure angesäuert, die Reaktionslösung mit Celite versetzt und der Feststoff abfiltriert. Es wurde gut mit THF gewaschen und das Filtrat eingeengt. Der Rückstand wurde zwischen Dichlormethan und Wasser verteilt, die organische Phase zweimal mit Wasser gewaschen, über Natriumsulfat getrocknet, filtriert und eingeengt. Der Rückstand wurde mittels Kieselgel-Chromatographie (Cyclohexan/Essigsäureethylester = 9:1 nach 7:3) gereinigt und die Produktfraktionen wurden eingeengt. Man erhielt 13.46 g (80% d. Th.,) der Zielverbindung.
LC-MS (Methode 1): Rₜ = 0.98 min.
MS (ESIpos): m/z = 280 (M+H)⁺.
¹H-NMR (400 MHz, DMSO-d₆): δ = 0.80 - 0.89 (m, 3H), 0.98 (s, 3H), 1.05 (s, 3H), 1.09 - 1.37 (m, 5H), 1.58 - 1.74 (m, 1H), 3.25 - 3.32 (m, 1H), 4.24 (s, 1H), 4.99 - 5.08 (m, 2H), 6.85 (d, 1H), 7.26 - 7.40 (m, 5H).

### Beispiel 97A

### ent-Benzyl-(2-hydroxy-2-methylheptan-3-yl)carbamat (Enantiomer A)

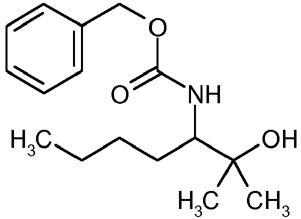

15.85 g Beispiel 96A wurden durch präparative Trennung an chiraler Phase in die Enantiomere getrennt [Säule: Daicel Chiralpak AD-H, 5 µm, 250 x 20 mm, Eluent: 80% iso-Hexan, 20% Ethanol, Fluß: 20 ml/min; 35°C, Detektion: 210 nm].

### Enantiomer A:

Ausbeute: 5.43 g (97% ee)
Rt= 5.93 min [Daicel Chiralpak AD-H, 5µm, 250 x 4.6 mm; Eluent: 80% iso-Hexan, 20% Ethanol; Fluss 1.0 ml/min; 40°C; Detektion: 220 nm].

### Beispiel 98A

### ent-3-Amino-2-methylheptan-2-olhydrochlorid (Enantiomer A)

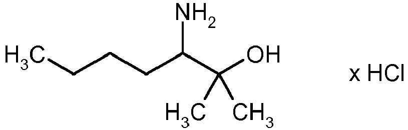

1.0 g (3.58 mmol) *ent*-Benzyl-(2-hydroxy-2-methylheptan-3-yl)carbamat (Enantiomer A) Beispiel 97A wurden unter Argon in Ethanol (25 ml) vorgelegt, mit 381 mg (0.36 mmol, 10% rein) Palladium auf Aktivkohle und 10.9 ml (107.38 mmol) Cyclohexen versetzt und die Reaktionsmischung 3 h bei RF gerührt. Das Gemisch wurde über einen Millipore^{®}-Filter filtriert und es wurde mit Ethanol nachgewaschen. Das Filtrat wurde mit 3.6 ml (7.16 mmol) 2 N wässriger Salzsäure in Diethylether versetzt, dann eingeengt und am Hochvakuum getrocknet. Man erhielt 801 mg (123% d. Th.) der Zielverbindung. Das Produkt wurde ohne weitere Reinigung in die nächste Reaktion eingesetzt.
DCI-MS (Methode 13): m/z = 146 (M-HCl+H)⁺
¹H-NMR (400 MHz, DMSO-d₆): δ = 0.88 (t, 3H), 1.07 (s, 3H), 1.18 (s, 3H), 1.21 - 1.58 (m, 6H), 2.73 -2.83 (m, 1H), 7.69 - 7.84 (m, 2H).

### Beispiel 99A

### rac-Methyl-6,6,6-trifluornorleucinat Hydrochlorid

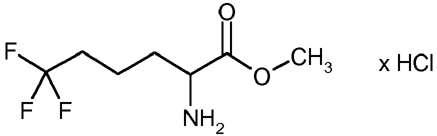

2.70 g (14.58 mmol) *rac*-6,6,6-Trifluornorleucin wurden in 27.6 ml gesättigter Salzsäure in Methanol vorgelegt und 4 h unter Rückfluss gerührt. Dann wurden nochmals 10 ml gesättigte Salzsäure in Methanol zu der Reaktionslösung gegeben und weitere 4 h bei Rückfluss gerührt. Die Reaktionslösung wurde eingeengt und der Rückstand am Hochvakuum getrocknet. Es wurden 3.77 g der Zielverbindung (99% d. Th.; Reinheit 90%) erhalten.
DCI-MS (Methode 13): (ESpos): m/z = 200 (M-HCl+H)⁺
¹H-NMR (400 MHz, DMSO-d6): δ = 1.48-1.73 (m, 2H), 1.80-1.96 (m, 2H), 2.24-2.38 (m, 2H), 3.76 (s, 3H), 4.06-4.14 (m, 1H), 8.49-8.68 (br. s, 3H).

### Beispiel 100A

### rac-Methyl-N-[(benzyloxy)carbonyl]-6,6,6-trifluornorleucinat

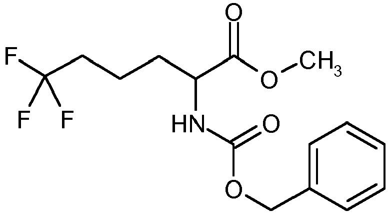

3.77 g (14.4 mmol, Reinheit ca. 90%) *rac*-Methyl-6,6,6-trifluornorleucinat Hydrochlorid Beispiel 99A wurden in 212 ml Wasser/THF (8:1) vorgelegt und mit 6.17 g (44.6 mmol) Kaliumcarbonat versetzt. Das Reaktionsgemisch wurde auf 0°C abgekühlt und es wurden 2.68 ml (15.8 mmol) Chlorameisensäurebenzylester langsam zugetropft und dann über Nacht bei RT gerührt. Das Gemisch wurde mit 100 ml Wasser verdünnt und dreimal mit Dichlormethan extrahiert. Die vereinigten organischen Phasen wurden über Natriumsulfat getrocknet, abfiltriert und eingeengt. Der Rückstand wurde mittels Kieselgelchromatographie (Cyclohexan/Essigsäureethylester 4:1) gereinigt. Man erhielt 3.64 g (76% d. Th., Reinheit 100%) der Zielverbindung.
LC-MS (Methode 18): Rₜ = 2.32 min.
MS (ESIpos): m/z = 334 (M+H)⁺.
¹H-NMR (400 MHz, DMSO-d₆): δ = 1.47 - 1.59 (m, 2H), 1.61 - 1.72 (m, 1H), 1.73 - 1.85 (m, 1H), 2.14 - 2.34 (m, 2H), 3.64 (s, 3H), 4.04 - 4.12 (m, 1H), 5.04 (s, 2H), 7.25 - 7.40 (m, 5H), 7.81 (d, 1 H).

### Beispiel 101A

### rac-Benzyl-(7,7,7-trifluor-2-hydroxy-2-methylheptan-3-yl)carbamat

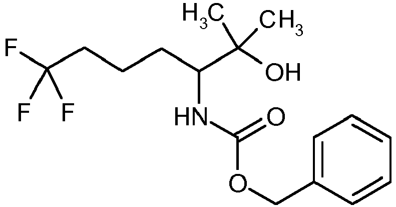

3.23 g (9.70 mmol) *rac*-Methyl-N-[(benzyloxy)carbonyl]-6,6,6-trifluornorleucinat Beispiel 100A wurde unter Argon in 94 ml THF vorgelegt. Das Reaktionsgemisch wurde auf 0°C abgekühlt, 11.32 ml (33.96 mmol) 3M Methylmagnesiumbromid in Diethylether wurden zugetropft und se wurde 15 min bei 0°C nachgerührt. Man ließ langsam auf RT kommen und rührte über Nacht bei Raumtemperatur. Das Reaktionsgemisch wurde vorsichtig mit gesättigter, wässriger Ammoniumchlorid-Lösung und anschließend mit Celite versetzt. Der Feststoff wurde abfiltriert, gut mit THF gewaschen und das Filtrat wurde eingeengt. Der wässrige Rückstand wurde zwischen Dichlormethan und Wasser verteilt. Die organische Phase wurde noch zweimal mit Wasser gewaschen, über Natriumsulfat getrocknet, filtriert und das Filtrat eingeengt. Der Rückstand wurde mittels Kieselgel-Chromatographie (Cyclohexan/Essigsäureethylester 7:3) gereinigt und die Produktfraktionen wurden eingeengt. Man erhielt 2.83 g (87% d. Th., Reinheit 97%) der Zielverbindung.
LC-MS (Methode 17): Rₜ = 1.02 min.
MS (ESIpos): m/z = 334 (M+H)⁺.
¹H-NMR (400 MHz, DMSO-d₆): δ = 0.99 (s, 3H), 1.06 (s, 3H), 1.25 - 1.45 (m, 2H), 1.47 - 1.60 (m, 1H), 1.67 - 1.80 (m, 1H), 2.06 - 2.35 (m, 2H), 3.29 - 3.32 (m, 1H, teilweise verdeckt durch Wasser-Peak), 4.32 (s, 1H), 5.05 (q, 2H), 6.95 (d, 1H), 7.26 - 7.38 (m, 5H).

### Beispiel 102A

### ent-Benzyl-(7,7,7-trifluor-2-hydroxy-2-methylheptan-3-yl)carbamat (Enantiomer A)

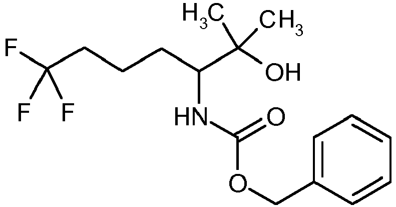

1.91 g Beispiel 101A wurden durch präparative Trennung an chiraler Phase in die Enantiomere getrennt [Säule: Daicel Chiralpak AY-H, 5 µm, 250 x 20 mm, Eluent: 90% iso-Hexan, 10% Ethanol, Fluß: 15 ml/min; 35°C, Detektion: 220 nm].

### Enantiomer A:

Ausbeute: 766 mg (99% ee)
Rₜ= 5.12 min [Daicel Chiralpak AY-H, 5µm, 250 x 4.6 mm; Eluent: 90% iso-Hexan, 10% Ethanol; Fluss 1.0 ml/min; 30°C; Detektion: 220 nm].

### Beispiel 103A

### rac-3-Amino-7,7,7-trifluor-2-methylheptan-2-olhydrochlorid

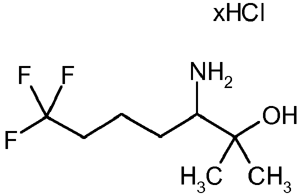

1.0 g (3.0 mmol) *rac*-Benzyl-(7,7,7-trifluor-2-hydroxy-2-methylheptan-3-yl)carbamat Beispiel 101A wurde unter Argon in Ethanol (21 ml) vorgelegt, mit 319 mg (0.30 mmol, 10% rein) Palladium auf Aktivkohle und 9.1 ml (90.0 mmol) Cyclohexen versetzt und die Reaktionsmischung über Nacht bei RF gerührt. Das Gemisch wurde über einen Millipore^{®}-Filter filtriert und es wurde mit Ethanol nachgewaschen. Das Filtrat wurde mit 3 ml (6.0 mmol) 2 N Salzsäure in Diethylether versetzt, eingeengt und am Hochvakuum getrocknet. Man erhielt 785 mg (111% d. Th.) der Zielverbindung. Das Produkt wurde ohne weitere Reinigung in die nächste Reaktion eingesetzt.
MS (Methode 13): m/z = 200 (M-HCl+H)⁺.
¹H-NMR (400 MHz, DMSO-d₆): δ = 1.08 (s, 3H), 1.19 (s, 3H), 1.40 - 1.59 (m, 2H), 1.60 - 1.82 (m, 2H), 2.15 - 2.41 (m, 2H), 2.80 - 2.91 (m, 1H), 5.17 - 5.35 (br. s, 1H), 7.65 - 7.93 (br. s, 2H).

### Beispiel 104A

### ent-3-Amino-7,7,7-trifluor-2-methylheptan-2-olhydrochlorid (Enantiomer A)

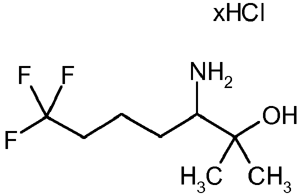

765 mg (2.30 mmol) *ent*-Benzyl-(7,7,7-trifluor-2-hydroxy-2-methylheptan-3-yl)carbamat (Enantiomer A) Beispiel 102A wurden unter Argon in Ethanol (16.1 ml) vorgelegt, mit 244 mg (0.23 mmol, 10% rein) Palladium auf Aktivkohle und 7.0 ml (68.85 mmol) Cyclohexen versetzt und die Reaktionsmischung 3 h bei RF gerührt. Das Gemisch wurde über einen Millipore^{®}-Filter filtriert und mit Ethanol gewaschen. Das Filtrat wurde mit 2.3 ml (4.59 mmol) 2 N Salzsäure in Diethylether versetzt, eingeengt und am Hochvakuum getrocknet. Man erhielt 559 mg (99% d. Th.) der Zielverbindung.
DCI-MS (Methode 13): m/z = 200 (M-HCl+H)⁺.
¹H-NMR (400 MHz, DMSO-d₆): δ = 1.08 (s, 3H), 1.19 (s, 3H), 1.40 - 1.59 (m, 2H), 1.60 - 1.69 (m, 1H), 1.70 - 1.82 (m, 1H), 2.15 - 2.27 (m, 1H), 2.28 - 2.42 (m, 1H), 2.80 - 2.91 (m, 1H), 5.17 - 5.35 (br. s, 1H), 7.73 - 7.97 (br. s, 2H).

### Beispiel 105A

### (3,3-Difluorcyclobutyl)methylmethansulfonat

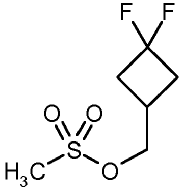

1.35 g (11.06 mmol) (3,3-Difluorcyclobutyl)methanol wurden in 41.8 ml abs. Dichlormethan vorgelegt, mit 3.08 ml (22.11 mmol) Triethylamin und 1.03 ml (13.27 mmol) Methansulfonsäurechlorid versetzt und über Nacht bei Raumtemperatur gerührt. Das Reaktionsgemisch wurde mit Wasser versetzt und die wässrige Phase zweimal mit Dichlormethan extrahiert. Die vereinten organischen Phasen mit gesättigter, wässriger Natriumchloridlösung gewaschen, über Natriumsulfat getrocknet, filtriert und das Filtrat eingeengt. Man erhielt 2.37 g (qunatitative Ausbeute) der Zielverbindung.
DCI-MS (Methode 16): Rₜ = 4.18 min. m/z = 218 (M+NH₄)⁺.
¹H-NMR (400 MHz, DMSO-d₆): δ = 2.34 - 2.59 (m, 3H), 2.62 - 2.74 (m, 2H), 3.21 (s, 3H), 4.26 (d, 2H).

### Beispiel 106A

### Ethyl-8-[(3,3-difluorcyclobutyl)methoxy]-2,6-dimethylimidazo[1,2-a]pyridin-3-carboxylat

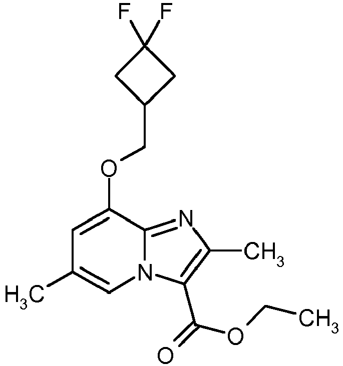

1.85 g (7.89 mmol) Ethyl-8-hydroxy-2,6-dimethylimidazo[1,2-a]pyridin-3-carboxylat Beispiel 87A und 2.37 g (9.47 mmol) (3,3-Difluorcyclobutyl)methylmethansulfonat Beispiel 105A wurden in 104 mL DMF vorgelegt und mit 10.28 g (31.56 mmol) Cäsiumcarbonat versetzt. Die Reaktionsmischung wurde über Nacht bei 60°C gerührt. Nach dem Abkühlen wurde das Reaktionsgemisch filtriert, der Feststoff gut mit Essigsäureethylester gewaschen, das Filtrat eingeengt und der Rückstand mit ca. 150 ml Wasser versetzt. Der entstandene Feststoff wurde abfiltriert und im Hochvakuum getrocknet. Man erhielt 2.51 g (89% d. Th.; Reinheit 95%) der Titelverbindung.
LC-MS (Methode 1): Rₜ = 1.00 min
MS (ESpos): m/z = 339 (M+H)⁺
¹H-NMR (400 MHz, DMSO-d₆): δ = 1.35 (t, 3H), 2.32 (s, 3H), 2.42 - 2.60 (m, 5H), 2.62 - 2.84 (m, 3H), 4.22 (d, 2H), 4.33 (q, 2H), 6.90 (s, 1H), 8.68 (s, 1H).

### Beispiel 107A

### 8-[(3,3-Difluorcyclobutyl)methoxy]-2,6-dimethylimidazo[1,2-a]pyridin-3-carbonsäure

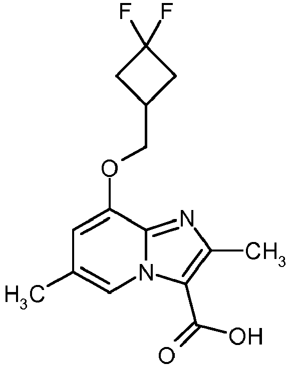

2.39 g (7.06 mmol) Ethyl-8-[(3,3-difluorcyclobutyl)methoxy]-2,6-dimethylimidazo[1,2-a]pyridin-3-carboxylat Beispiel 106A wurden in 151 mL THF/Methanol (5:1) gelöst, mit 35.3 mL (35.3 mmol) 1 N wässriger Lithiumhydroxid-Lösung versetzt und 2 d bei RT gerührt. Die Reaktionsmischung wurde mit 1 N wässriger Salzsäure-Lösung auf pH 4 angesäuert und eingeengt. Der Feststoff wurde abfiltriert, mit Wasser gewaschen und am Hochvakuum getrocknet. Man erhielt 1.63 g (71% d. Th.; Reinheit 95%) der Titelverbindung.
LC-MS (Methode 1): Rₜ = 0.63 min
MS (ESpos): m/z = 311 (M+H)⁺
¹H-NMR (500 MHz, DMSO-d₆): δ = 2.32 (s, 3H), 2.42 - 2.60 (m, 5H), 2.62 - 2.82 (m, 3H), 4.22 (d, 2H), 6.87 (s, 1H), 8.71 (s, 1H), 12.93 (br. s, 1H).

### Beispiel 108A

### 4-Fluor-2-nitropyridin-3-ol

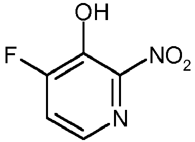

500 mg (3.43 mmol) 4-Fluorpyridin-3-olhydrochlorid wurden unter Eiskühlung vorsichtig in 3.2 ml konzentrierter Schwefelsäure gelöst und bei 0°C langsam mit 0.21 ml konzentrierter Salpetersäure versetzt. Die Reaktion wurde auf RT erwärmt und über Nacht gerührt. Es wurde auf 10 g Eis gegeben und unter Eiskühlung wurden 6 ml 45%ige Natronlauge zugetropft. Der entstandene Feststoff wurde abfiltriert und anschließend über Nacht im Vakuum getrocknet. Es wurden 191 mg (36% d. Th.) der Titelverbindung erhalten.
LC-MS (Methode 1): Rₜ = 0.36 min
MS (ESneg): m/z = 157 (M-H)-
¹H-NMR (400 MHz, DMSO-d₆): δ = 7.69 (dd, 1 H); 7.95 - 8.01 (m, 1 H); 11.97 (br. s, 1 H).

### Beispiel 109A

### 2-Amino-4-fluorpyridin-3-ol

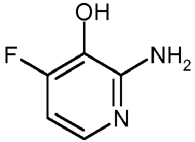

90 mg (0.57 mmol) 4-Fluor-2-nitropyridin-3-ol Beispiel 108A wurden unter Argon in 30 ml Ethanol gelöst und mit einer Spatelspitze Palladium auf Aktivkohle (10%ig) versetzt. Das Gemisch wurde 1.5 h bei RT unter Normaldruck hydriert. Das Reaktionsgemisch wurde über Kieselgel abfiltriert und mit viel Ethanol nachgewaschen. Die Lösung wurde eingeengt und getrocknet. Es wurden 56 mg (77% d. Th.) der Titelverbindung erhalten.
LC-MS (Methode 1): Rₜ = 0.16 min
MS (ESpos): m/z = 129 (M+H)⁺
¹H-NMR (400 MHz, DMSO-d₆): δ = 5.78 (br. s, 2 H); 6.42 (dd, 1 H); 7.37 - 7.43 (m, 1 H); 9.47 (br. s, 1 H).

### Beispiel 110A

### 3-[(2,6-Difluorbenzyl)oxy]-4-fluorpyridin-2-amin

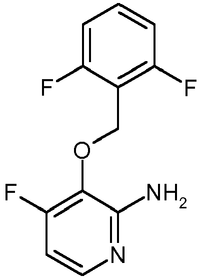

55 mg (0.43 mmol) 2-Amino-4-fluorpyridin-3-ol Beispiel 109A, 98 mg (0.47 mmol) 2-(Brommethyl)-1,3-difluorbenzol und 308 mg (0.95 mmol) Cäsiumcarbonat wurden in 1 ml trockenem DMF vorgelegt und für 15 min in einem auf 50°C erwärmten Ölbad erhitzt. Dann wurde abfiltriert und mittels einer präparativen HPLC (Methode 9) gereinigt. Es wurden 70 mg der Titelverbindung (64% d. Th.) erhalten.
LC-MS (Methode 1): Rₜ = 0.70 min
MS (ESpos): m/z = 255 (M+H)⁺
¹H-NMR (400 MHz, DMSO-d₆): δ = 5.06 (s, 2 H); 6.04 (br. s, 2 H); 6.42 (dd, 1 H); 7.08 - 7.16 (m, 2 H); 7.45 - 7.54 (m, 1 H); 7.62 - 7.69 (m, 1 H).

### Beispiel 111A

### Ethyl-8-[(2,6-difluorbenzyl)oxy]-7-fluor-2-methylimidazo[1,2-a]pyridin-3-carboxylat

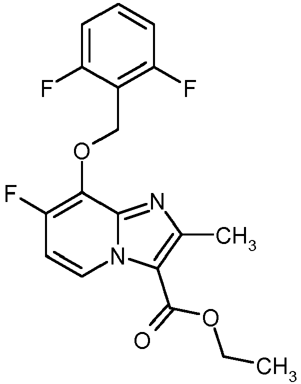

500 mg (1.97 mmol) 3-[(2,6-Difluorbenzyl)oxy]-4-fluorpyridin-2-amin Beispiel 110A wurden unter Argon in 10 ml Ethanol vorgelegt und mit 500 mg gepulvertem Molsieb 4Å und 3.24 g (19.67 mmol) Ethyl-2-chloracetoacetat versetzt. Die resultierende Reaktionsmischung wurde für 48 h zum Rückfluß erhitzt. Alle flüchtigen Komponeneten wurden weitesgehend am Trockeneisrotationsverdampfer bei einer Wasserbadtemperatur von 85°C eingedampft. Das Rohprodukt wurde mittels Kieselgel-Chromatographie gereinigt (Laufmittel: Cyclohexan/Essigsäureethylester = 9/1 isokratisch). Es wurden 368 mg (39% d. Th.; Reinheit ca. 76%) der Titelverbindung erhalten.
LC-MS (Methode 1): Rₜ = 1.19 min
MS (ESpos): m/z = 365 (M+H)⁺
¹H-NMR (400 MHz, DMSO-d₆): δ = 1.37 (t, 3 H); 2.62 (s, 3 H); 4.38 (q, 2 H); 5.60 (s, 2 H); 7.09 - 7.22 (m, 3 H); 7.47 - 7.56 (m, 1 H); 8.98 (dd, 1 H).

### Beispiel 112A

### 8-[(2,6-Difluorbenzyl)oxy]-7-fluor-2-methylimidazo[1,2-a]pyridin-3-carbonsäure

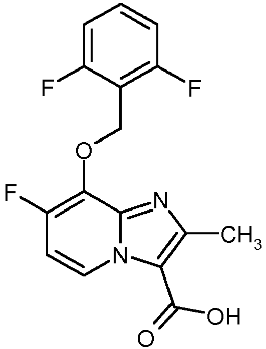

365 mg (0.76 mmol; Reinheit ca. 76%) Ethyl-8-[(2,6-difluorbenzyl)oxy]-7-fluor-2-methylimidazo[1,2-a]pyridin-3-carboxylat Beispiel 111A wurden in 16.6 ml THF/Ethanol (5:1) gelöst, mit 1.14 ml (1.14 mmol) 1 N wässriger Lithiumhydroxid-Lösung versetzt und über Nacht bei RT gerührt. Es wurden nochmals 2.67 ml (2.67 mmol) 1 N wässrige Lithiumhydroxid-Lösung hinzugegeben und über Nacht bei RT gerührt. Es wurde eingedampft und die wässrige Phase wurde mit 6 N Salzsäure auf pH 4 angesäuert. Es entstand ein Niederschlag und die Suspension wurde mit Eiswasser gekühlt. Der Feststoff wurde abfiltriert und im Hochvakuum getrocknet. Es wurden 236 mg der Zielverbindung (87% d. Th., Reinheit 94%) erhalten.
LC-MS (Methode 1): Rₜ = 0.83 min
MS (ESpos): m/z = 337 (M+H)⁺
¹H-NMR (400 MHz, DMSO-d₆): δ = 2.62 (s, 3 H); 5.60 (s, 2 H); 7.09 - 7.18 (m, 3 H); 7.47 - 7.55 (m, 1 H); 9.04 (dd, 1 H); 13.22 (br. s, 1 H).

### Beispiel 113A

### 3,5-Difluorisonicotinaldehyd

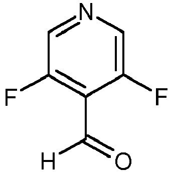

Unter Argon wurden bei -70°C zu 15.4 ml Diisopropylamin (110 mmol, 1.1 Äquivalente) in 23 ml THF 44 ml 2.5 M n-Butyllithium-Lösung in n-Hexan (110 mmol, 1.1 Äquivalente) langsam zugetropft. Die entstandene Lösung wurde auf 0°C erwärmt und bei dieser Temperatur 30 min gerührt. Dann wurde das Reaktionsgemisch auf -70°C gebracht, mit 23 ml THF verdünnt und tropfenweise mit 11.5 g 3,5-Difluorpyridin (100 mmol, 1 Äquivalent), gelöst in 72 ml THF, versetzt. Es wurde 30 min bei -70°C nachgerührt. Dann tropfte man 12.4 ml Methylformiat (200 mmol, 2 Äquivalente), gelöst in 23 ml THF, langsam zu. Nach 1.5 h bei -70°C wurde die Reaktionslösung rasch in 230 ml gesättigte, wässrige Natriumhydrogencarbonat-Lösung gegossen und mit insgesamt 460 ml Essigsäureethylester extrahiert. Die vereinten organischen Phasen wurden zweimal mit je 115 ml gesättigter, wässriger Natriumhydrogencarbonat-Lösung und zweimal mit gesättigter wässriger Natriumchlorid-Lösung gewaschen, über Natriumsulfat getrocknet und einrotiert. Es wurden 11.6 g (81% d. Th.) der Titelverbindung erhalten und direkt weiter umgesetzt.
GC-MS (Methode 14): Rₜ = 1.82 min
MS (ESpos): m/z = 144.0 (M+H)⁺
¹H-NMR (400 MHz, DMSO-d₆): δ = 8.75 (br. s, 2 H), 10.24 (br. s, 1 H).

### Beispiel 114A

### (3,5-Difluorpyridin-4-yl)methanol

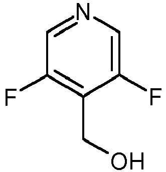

3.68 g Natriumborhydrid (97.3 mmol, 1.2 Äquivalente) wurden in 200 ml Methanol bei RT mit 11.60 g 3,5-Difluorisonicotinaldehyd (Beispiel 113A, 81 mmol, 1 Äquivalent), gelöst in 100 ml Methanol, versetzt. Nach beendeter Gasentwicklung (ca. 2 h) wurde mit 200 ml gesättigter, wässriger Natriumchlorid-Lösung versetzt und zweimal mit je 200 ml Essigsäureethylester extrahiert. Die vereinigten organischen Phasen wurden über Natriumsulfat getrocknet und eingeengt. Es wurden 9.5 g (81% d. Th.) der Titelverbindung erhalten.
LC-MS (Methode 2): Rₜ = 0.28 min
MS (ESpos): m/z = 146 (M+H)⁺
¹H-NMR (400 MHz, DMSO-d₆): δ = 4.56 (d, 2 H), 5.56 (t, 1 H), 8.51 (s, 2 H).

### Beispiel 115A

### 4-(Chlormethyl)-3,5-difluorpyridin

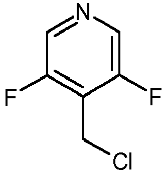

Unter Argon wurden 5.0 g (3,5-Difluorpyridin-4-yl)methanol (Beispiel 114A, 34.5 mmol, 1 Äquivalent) in 100 ml Dichlormethan bei -20°C vorgelegt und nacheinander mit 5.7 ml Diisopropylethylamin (34.5 mmol, 1 Äquivalent) und 2.95 ml Methansulfonsäurechlorid (37.9 mmol, 1.1 Äquivalente) versetzt. Es wurde auf RT erwärmt und 16 h bei RT und dann 3 h bei 40°C gerührt. Dann wurde die Reaktionslösung eingeengt und je zweimal mit 50 ml Toluol versetzt und wieder eingeengt. Es wurden 13 g (230% d. Th.) als Rohprodukt erhalten und ohne Reinigung weiter umgesetzt.

### Beispiel 116A

### Ethyl 8-[(3-fluoropyridin-2-yl)methoxy]-2-methylimidazo[1,2-a]pyridin-3-carboxylat

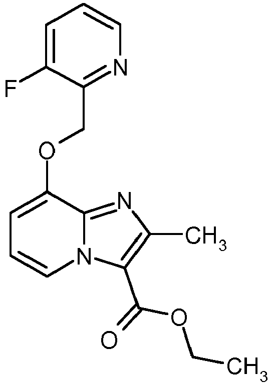

### Varainte A:

4.18 g Ethyl 8-hydroxy-2-methylimidazo[1,2-a]pyridin-3-carboxylat (Beispiel 3A, 19 mmol) wurden in 265 ml abs. DMF gelöst, mit 3.80 g 2-(Chlormethyl)-3-fluorpyridin-Hydrochlorid (20.88 mmol, kommerziell erhältlich; zusätzlich beschrieben in: US5593993, 1997; WO2007/2181 A2, 2007) und 18.55 g Cäsiumcarbonat (56.94 mmol) versetzt und anschließend über Nacht bei 60°C gerührt. Nach dem Abkühlen wurde das Reaktionsgemisch filtriert, der Niederschlag mit Essigsäureethylester gewaschen, das Filtrat eingeengt und der Rückstand mittels Kieselgel-Chromatographie gereinigt (Laufmittel: Cyclohexan:Essigsäureethylester = 1:3). Es wurden 4.66 g (73% d. Th.) der Zielverbindung erhalten.
MS (ESpos): m/z = 330 (M+H)⁺
¹H-NMR (400 MHz, DMSO-d₆): δ = 1.36 (t, 3H), 2.61 (s, 3H), 4.38 (q, 2H), 4.50 (br s, 1H), 5.49 (s, 2H), 7.20 (t, 1H), 7.32 (d, 1H), 7.57-7.61 (m, 1H), 7.87 (t, 1H), 8.49 (d, 1H), 8.90 (d, 1H).

### Variante B:

### Ethyl 8-[(3-fluoropyridin-2-yl)methoxy]-2-methylimidazo[1,2-a]pyridin-3-carboxylat Trifluoracetat

144 mg Ethyl 8-hydroxy-2-methylimidazo[1,2-a]pyridin-3-carboxylat (Beispiel 3A, 0.65 mmol) wurden in 3.9 ml THF gelöst, mit 100 mg (3-Fluorpyridin-2-yl)methanol (0.79 mmol), 189 mg Triphenylphosphin (0.72 mmol) und anschließend mit 0.15 ml Azodicarbonsäurediisopropylester (0.72 mmol) versetzt. Das Reaktionsgemisch wurde über Nacht bei RT gerührt und anschließend wurde mittels präparativer HPLC gereinigt (RP18 Säule, Laufmittel: Acetonitril/Wasser-Gradient unter Zusatz von 0.1% TFA). Es wurden 198 mg (68% d. Th., Reinheit 99%) der Zielverbindung erhalten.
LC-MS (Methode 1): Rₜ = 0.84 min

### Beispiel 117A

### 8-[(3-Fluoropyridin-2-yl)methoxy]-2-methylimidazo[1,2-a]pyridin-3-carbonsäure Hydrochlorid

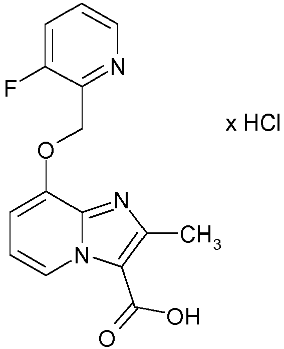

4.66 g Ethyl 8-[(3-fluoropyridin-2-yl)methoxy]-2-methylimidazo[1,2-a]pyridin-3-carboxylat (Beispiel 116A, (14.2 mmol) wurden in 304 ml THF/MeOH (5/1) gelöst, mit 70.8 ml 1 N wässriger Lithiumhydroxid-Lösung (70.8 mmol) versetzt und über Nacht bei 40°C gerührt. Das Reaktionsgemisch wurde mit 1 N wässriger Salzsäure sauer gestellt (ca. pH 3-4) und die Lösung wurde aufkonzentriert. Der entstandene Niederschlag wurde mit Eiswasser gekühlt, anschließend abgesaugt und im Vakuum getrocknet. Es wurden 3.97 g des Produktes (83% d. Th.) erhalten.
LC-MS (Methode 1): Rₜ = 0.46 min
MS (ESpos): m/z = 302 (M+H)⁺
¹H-NMR (400 MHz, DMSO-d₆): δ = 2.50 (s, 3H, verborgen unter DMSO-Signal), 5.42 (s, 2H), 7.02 (t, 1H), 7.13 (d, 1H), 7.56-7.62 (m, 1H), 7.84 (t, 1H), 8.49 (d, 1H), 8.89 (d, 1H), 13.08 (br. s, 1 H).

### Beispiel 118A

### Ethyl-8-[(3,5-difluorpyridin-4-yl)methoxy]-2-methylimidazo[1,2-a]pyridin-3-carboxylat

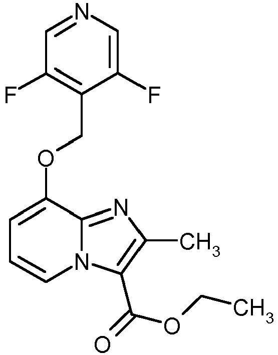

1.0 g Ethyl 8-hydroxy-2-methylimidazo[1,2-a]pyridin-3-carboxylat (Beispiel 3A, 4.54 mmol, 1.0 Äquvivalente) wurde in 100 ml DMF bei RT mit 1.86 g 4-(Chlormethyl)-3,5-difluorpyridin (Beispiel 115A, 9.1 mmol, 2.0 Äquivalente) und 4.44 g Cäsiumcarbonat (13.6 mmol, 3 Äquivalente) versetzt und 1 h bei 60°C gerührt. Dann wurde mit 500 ml Wasser verdünnt und zweimal mit je 300 ml Essigsäureethylester extrahiert. Die vereinigten, organischen Phasen wurden mit 400 ml gesättigter, wässriger Natriumchlorid-Lösung gewaschen, über Natriumsulfat getrocknet und einrotiert. Der erhaltene Rückstand wurde über eine Kieselgelsäule gereinigt (Eluent: Cyclohexan/Essigsäureethylester 7:1 nach 1:1). Es wurden 900 mg (54% d. Th.) und 200 mg (13% d. Th.; Reinheit ca. 85%) der Titelverbindung erhalten.
LC-MS (Methode 2): Rₜ = 0.91 min
MS (ESpos): m/z = 348 (M+H)⁺
¹H-NMR (400 MHz, DMSO-d₆): δ = 1.36 (t, 3 H), 2.57 (s, 3 H), 4.31 - 4.40 (m, 2 H), 5.45 (s, 2 H), 7.12 (t, 1H), 7.17 (d, 1H), 8.67 (s, 2 H), 8.89 (d, 1H).

### Beispiel 119A

### 8-[(3,5-Difluorpyridin-4-yl)methoxy]-2-methylimidazo[1,2-a]pyridin-3-carbonsäure

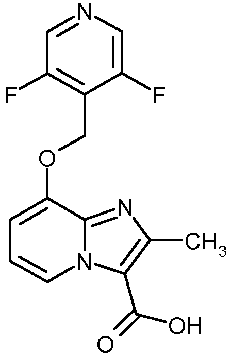

1.1 g Ethyl-8-[(3,5-difluorpyridin-4-yl)methoxy]-2-methylimidazo[1,2-a]pyridin-3-carboxylat (Beispiel 118A, 2.9 mmol, Reinheit ca. 90%, 1 Äquivalent) wurden in 54 ml Methanol/THF (1/1) vorgelegt und anschließend mit 14.3 ml 1 N wässriger Lithiumhydroxid-Lösung (14.3 mmol, 5 Äquivalente) versetzt. Es wurde über Nacht bei RT gerührt. Dann wurden Methanol und THF im Vakuum entfernt, der Rückstand mit Wasser verdünnt und mit 1 N wässriger Salzsäure sauer gestellt. Der entstandene Feststoff wurde abfiltriert und im Vakuum getrocknet. Es wurden 0.57 g (58% d. Th.; Reinheit 92%) der Titelverbindung erhalten.
LC-MS (Methode 1): Rₜ = 0.45 min
MS (ESpos): m/z = 320 (M+H)⁺
¹H-NMR (400 MHz, DMSO-d₆): δ = 2.59 (s, 3 H), 5.48 (s, 2 H), 7.17 (t, 1 H), 7.27 (d, 1 H), 8.67 (s, 2 H), 8.98 (d, 1 H), [weiteres Signal unter Lösungsmittel-Peaks verbogen].

### Beispiel 120A

### Ethyl-8-[(3-fluorpyridin-2-yl)methoxy]-2,6-dimethylimidazo[1,2-a]pyridin-3-carboxylat

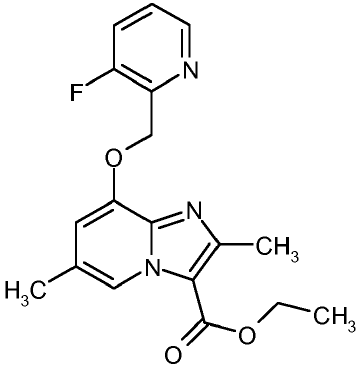

16.92 g (72.2 mmol) Ethyl-8-hydroxy-2,6-dimethylimidazo[1,2-a]pyridin-3-carboxylat aus Beispiel 87A wurden in 956 ml DMF mit 15.78 g (86.7 mmol) 2-(Chlormethyl)-3-fluorpyridin-Hydrochlorid (kommerziell erhältlich; zusätzlich beschrieben in: US5593993 A1, 1997; WO2007/2181 A2, 2007) und 94.06 g (288.9 mmol) Cäsiumcarbonat versetzt. Das Reaktionsgemisch wurde über Nacht bei 60°C gerührt. Das auf RT abgekühlte Reaktionsgemisch wurde filtriert, mit Essigsäureethylester gewaschen und das Filtrat eingeengt. Der Rückstand wurde mit ca. 500 ml Wasser versetzt, der ausgefallene Feststoff abfiltriert und im Hochvakuum getrocknet. Man erhielt 24.1 g (93% d. Th.) der Zielverbindung.
LC-MS (Methode 1): Rₜ = 0.84 min
MS (ESpos): m/z = 344 (M+H)⁺
¹H-NMR (400 MHz, DMSO-d₆): δ = 1.35 (t, 3H), 2.35 (s, 3H), 2.54 (s, 3H, verdeckt vom DMSO-Signal), 4.35 (q, 2H), 5.40 (s, 2H), 7.08 (s, 1H), 7.55 - 7.62 (m, 1H), 7.82 - 7.89 (m, 1H), 8.48 - 8.52 (m, 1H), 8.70 (s, 1H).

### Beispiel 121A

### 8-[(3-Fluorpyridin-2-yl)methoxy]-2,6-dimethylimidazo[1,2-a]pyridin-3-carbonsäure Hydrochlorid

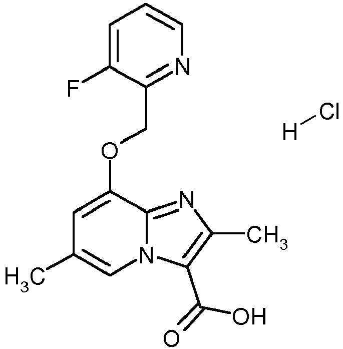

24.06 g (70.1 mmol) Ethyl-8-[(3-fluorpyridin-2-yl)methoxy]-2,6-dimethylimidazo[1,2-a]pyridin-3-carboxylat aus Beispiel 120A wurden in 1.51 THF/Methanol (5:1) vorgelegt, mit 350.4 ml (350.4 mmol) 1 N wässriger Lithiumhydroxid-Lösung versetzt und die Reaktionsmischung wurde 2.5 h bei 40°C gerührt. Nach dem Abkühlen wurde mit 1 N wässriger Salzsäure auf ca. pH 4 eingestellt und die Lösung wurde im Vakuum von THF/Methanol befreit. Der Rückstand wurde abgekühlt, der ausgefallene Feststoff abfiltriert und im Vakuum getrocknet. Es wurden 22.27 g (100% d. Th.) der Titelverbindung erhalten.
LC-MS (Methode 1): Rₜ = 0.55 min
MS (ESpos): m/z = 316 (M+H)⁺
¹H-NMR (400 MHz, DMSO-d₆): δ = 2.34 (s, 3H), 2.53 (s, 3H, verdeckt vom DMSO-Signal), 5.38 - 5.42 (m, 2H), 7.06 (s, 1H), 7.56 - 7.62 (m, 1H), 7.82 - 7.89 (m, 1H), 8.48 - 8.52 (m, 1H), 8.74 (s, 1H), 13.02 (br. s, 1H).

### Beispiel 122A

### rac-Methyl-6,6,6-trifluor-N-({8-[(3-fluorpyridin-2-yl)methoxy]-2,6-dimethylimidazo[1,2-a]pyridin-3-yl}carbonyl)norleucinat

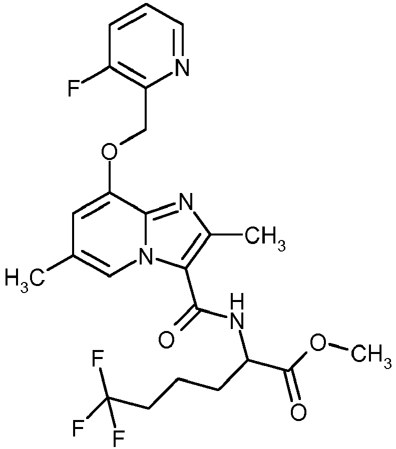

136 mg 8-[(3-Fluorpyridin-2-yl)methoxy]-2,6-dimethylimidazo[1,2-a]pyridin-3-carbonsäureHydrochlorid (Beispiel 121A; 0.39 mmol, 1 Äquivalent) wurden mit 132 mg Benzotriazol-1-yloxy)bisdimethylaminomethyliumfluoroborat (TBTU, 0.46 mmol, 1.2 Äquivalente) und 0.30 ml 4-Methylmorpholin (273 mg, 2.7 mmol, 7 Äquivalente) in 2.2 ml DMF vorgelegt. Bei RT wurde mit 100 mg *rac*-Methyl-6,6,6-trifluornorleucinat Hydrochlorid (Beispiel 77A, 0.41 mmol, 1.2 Äquivalente) versetzt und über Nacht bei RT gerührt. Dann wurde mit 20 ml Wasser versetzt, 30 min bei RT gerührt, der entstandene Feststoff wurde abfiltriert, mit Wasser gewaschen und im Vakuum getrocknet. Es wurden 141 mg (68% d. Th.; Reinheit 93%) der Zielverbindung erhalten.
LC-MS (Methode 1): Rₜ = 0.91 min
MS (ESpos): m/z = 497 (M+H)⁺

In Analogie zu Beispielverbindung 122A wurden die in Tabelle 7A gezeigten Beispielverbindungen hergestellt, indem 8-[(3-Fluoropyridin-2-yl)methoxy]-2-methylimidazo[1,2-a]pyridin-3-carbonsäure Hydrochlorid aus Beispiel 117A mit den entsprechenden zuvor beschriebenen Aminen in DMF oder Dichlormethan unter den in der repräsentativen Arbeitsvorschrift 2a beschriebenen Reaktionsbedingungen umgesetzt worden sind:

**Tabelle 7A:**

| **Beispiel** | **IUPAC**-**Name / Struktur (Ausbeute)** | **Analytische Daten** |
|---|---|---|
| **123A** | *rac*-Methyl-4,4,4-trifluor-2-[({8-[(3-fluorpyridin-2-yl)methoxy]-2-methylimidazo[1,2-a]pyridin-3-yl}carbonyl)amino]butanoat | LC-MS (Methode 1): Rₜ = 0.80 min |
| | | MS (ESpos): m/z = 455.1 (M+H)⁺ |
| | 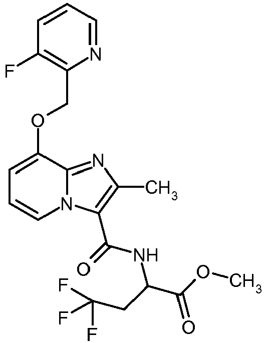 | ¹H-NMR (400 MHz, DMSO-d₆): δ = 2.87-3.03 (m, 2 H), 3.72 (s, 3 H), 4.78-4.86 (m, 1 H), 5.42 (s, 2 H), 6.94 (t, 1 H), 7.05 (d, 1H), 7.54 -7.62 (m, 1H), 7.84 (t, 1H), 8.47 - 8.52 (m, 2 H), 8.56 (d, 1 H) [weiteres Signal unter DMSO-Peak verborgen]. |
| | (50% d. Th.) | |
| **124A** | *rac*-Methyl-6,6,6-trifluor-*N*-({8-[(3-fluorpyridin-2-yl)methoxy]-2-methylimidazo[1,2-a]pyridin-3-yl} carbonyl)norleucinat | LC-MS (Methode 1): Rₜ = 0.92 min |
| | | MS (ESpos): m/z = 483 (M+H)⁺ |
| | 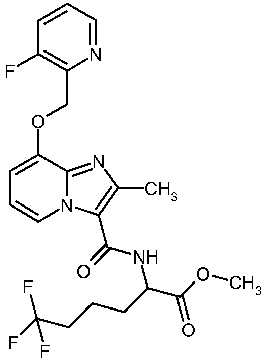 | ¹H-NMR (400 MHz, DMSO-d₆): δ = |
| | | 1.58-1.70 (m, 2H), 1.80-2.03 (m, 2H), 2.20-2.44 (m, 2H), 2.50 (s, 3H, unter DMSO-Peak verborgen), 3.70 (s, 3H), 4.49-4.57 (m, 1H), 5.42 (s, 2H), 6.92 (t, 1H), 7.02 (d, 1H), 7.56-7.61 (m, 1H), 7.84 (t, 1H), 8.34 (d, 1H), 8.46-8.51 (m, 2H). |
| | (68% d. Th.) | |

### Beispiel 125A

### rac-Methyl-4,4,4-trifluor-2-[({8-[(3-fluorpyridin-2-yl)methoxy]-2,6-dimethylimidazo[1,2-a]pyridin-3-yl}carbonyl)amino]butanoat (Racemat)

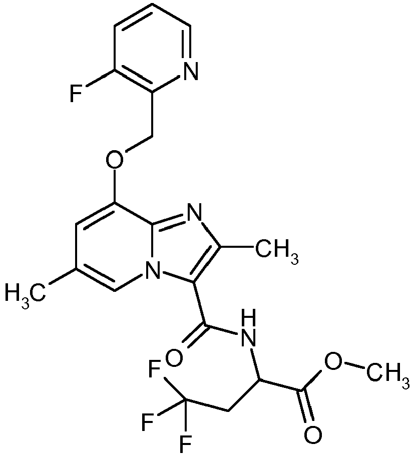

250 mg (0.71 mmol) 8-[(3-Fluorpyridin-2-yl)methoxy]-2,6-dimethylimidazo[1,2-a]pyridin-3-carbonsäure-Hydrochlorid aus Beispiel 121A wurden in 4.1 ml DMF vorgelegt und mit 274 mg (0.85 mmol) TBTU und 0.47 ml (4.26 mmol) 4-Methylmorpholin versetzt. Anschließend wurden 221 mg (1.07 mmol) Methyl-2-amino-4,4,4-trifluorbutanoat Hydrochlorid aus Beispiel 75A zugegeben und die Reaktionsmischung wurde über Nacht bei RT gerührt. Das Gemisch wurde mit ca. 32 ml Wasser versetzt. Der ausgefallene Feststoff wurde abfiltriert, mit Wasser gewaschen und im Hochvakuum getrocknet. Man erhielt 298 mg (68% d. Th., Reinheit 76%) der Zielverbindung.
LC-MS (Methode 1): Rₜ = 0.84 min
MS (ESIpos): m/z = 469 (M+H)⁺.

### Beispiel 126A

### 5-Chlor-3-[(3-fluorpyridin-2-yl)methoxy]-2-nitropyridin

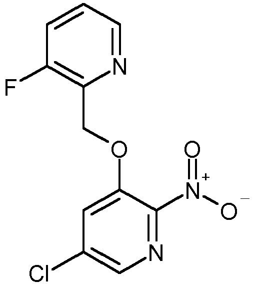

20.0 g (114.6 mmol) 5-Chlor-2-nitropyridin-3-ol aus Beispiel 15A und 56.0 g (171.9 mmol) Cäsiumcarbonat wurden in 319 ml DMF vorgelegt. 17.51 g (120.3 mmol) 2-(Chlormethyl)-3-fluorpyridin (kommerziell erhältlich; zusätzlich beschrieben in: K. Weidmann et al. Journal of Medicinal Chemistry 1992, 35, 438-450; US5593993, 1997; WO2007/2181 A2, 2007) wurden zugegeben und das Reaktionsgemisch wurde über Nacht bei RT gerührt. Es wurden 6.0 g (41.2 mmol) 2-(Chlormethyl)-3-fluorpyridin hinzugegeben und das Gemisch wurde 24 h bei RT gerührt. Anschließend wurden nochmals 6.0 g (41.2 mmol) 2-(Chlormethyl)-3-fluorpyridin und 5.0 g (15.3 mmol) Cäsiumcarbonat hinzugegeben und 12 h bei 60°C gerührt. Das Reaktionsgemisch wurde vorsichtig zu 2.3 1 0.5 M wässriger Salzsäure gegeben. Es wurde dreimal mit je 500 ml Essigsäureethylester extrahiert. Die vereinigten organischen Phasen wurden mit 500 ml gesättigter, wässriger Natriumchloridlösung gewaschen, getrocknet und im Vakuum eingeengt. Das Rohprodukt wurde mittels Kieselgelchromatographie gereinigt (Laufmittel: Cyclohexan/Essigsäureethylester-Gradient: 9/1 bis 7/3). Es wurden 29.8 g (92% d. Th.) der Zielverbindung erhalten.
LC-MS (Methode 1): Rₜ = 0.94 min.
MS (ESIpos): m/z = 284 (M+H)⁺.
¹H-NMR (400 MHz, DMSO-d₆): δ = 5.59 (d, 2H), 7.53 - 7.60 (m, 1H), 7.80 - 7.87 (m, 1H), 8.26 (d, 1H), 8.40 - 8.47 (m, 2H).

### Beispiel 127A

### 5-Chlor-3-[(3-fluorpyridin-2-yl)methoxy]pyridin-2-amin

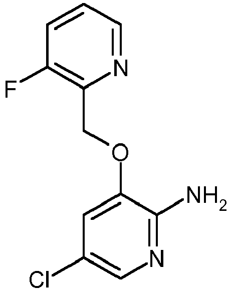

29.8 g (105.1 mmol) 5-Chlor-3-[(3-fluorpyridin-2-yl)methoxy]-2-nitropyridin aus Beispiel 126A wurden unter Argon in 317 ml Ethanol vorgelegt. 18.2 g (325.7 mmol) Eisenpulver wurden zugegeben und das Reaktionsgemisch wurde zum Rückfluss erhitzt. 80.4 ml konz. Salzsäure wurden langsam zugetropft und das Gemisch wurde weiter 6 h unter Rückfluss erhitzt. Das Reaktionsgemisch wurde mit 33%iger wässriger Ammoniaklösung alkalisch gestellt und anschließend im Vakuum eingeengt. Nach Reinigung durch Kieselgelchromatographie (Laufmittel: Dichlormethan/Methanol-Gradient: 95/5 bis 90/10) wurden 25.0 g (94% d. Th.) der Zielverbindung erhalten.
LC-MS (Methode 1): Rₜ = 0.70 min
MS (ESIpos): m/z = 254 (M+H)⁺
¹H-NMR (400 MHz, DMSO-d₆): δ = 5.27 (d, 2H), 5.87 (br. s, 2H), 7.32 - 7.35 (m, 1H), 7.51 - 7.58 (m, 2H), 7.77 - 7.85 (m,1H, 7.45 - 7.50 (m,1H.

### Beispiel 128A

### Ethyl-6-chlor-8-[(3-fluorpyridin-2-yl)methoxy]-2-methylimidazo[1,2-a]pyridin-3-carboxylat

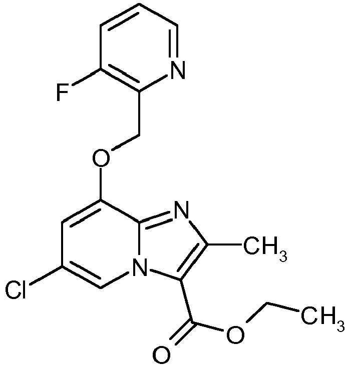

3.00 g (11.83 mmol) 5-Chlor-3-[(3-fluorpyridin-2-yl)methoxy]pyridin-2-amin aus Beispiel 127A und 9.73 g (59.13 mmol) Ethyl-2-chlor-3-oxobutanoat wurden in 72 ml Ethanol gelöst und zusammen mit 4.5 g 3 Å Molsieb unter Rückfluss für 6 Tage gerührt. Das Gemisch wurde abgekühlt, filtriert und das Filtrat im Vakuum eingeengt. Der erhaltene Rückstand wurde mittels Kieselgel-Chromatographie gereinigt (Laufmittel: Cyclohexan/Essigsäureethylester-Gradient = 4/1 nach 2/1). Es wurden 2.0 g (46% d. Th.) der Zielverbindung erhalten.
LC-MS (Methode 1): Rₜ = 1.07 min
MS (ESIpos): m/z = 364 (M+H)⁺
¹H-NMR (400 MHz, DMSO-d₆): δ = 1.36 (t, 3H), 2.56 (s, 3H; überlagert mit Lösungsmittelpeak), 4.37 (q, 2H), 5.48 (d, 2H), 7.36 (d, 1H), 7.57 - 7.63 (m, 1H), 7.83 - 7.90 (m, 1H), 8.50 (d, 1H), 8.92 (d, 1H).

### Beispiel 129A

### 6-Chlor-8-[(3-fluorpyridin-2-yl)methoxy]-2-methylimidazo[1,2-a]pyridin-3-carbonsäure

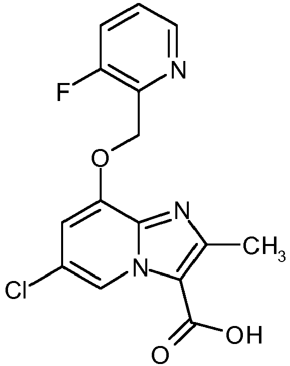

2.0 g (5.62 mmol) Ethyl-6-chlor-8-[(3-fluorpyridin-2-yl)methoxy]-2-methylimidazo[1,2-a]pyridin-3-carboxylat aus Beispiel 128A wurden in 110 ml THF/Methanol (5/1) mit 28.1 ml (28.1 mmol) 1 M Lithiumhydroxid-Lösung versetzt und 2.5 h bei 40°C gerührt. Das auf RT abgekühlte Reaktionsgemisch wurde mit 6 N wässriger Salzsäure auf ca. pH 4 gestellt, das Lösungsmittel zur Hälfte eingeengt, der ausgefallene Feststoff abgesaugt und im Vakuum getrocknet. Man erhielt 1.97 g (102% d. Th.) der Zielverbindung (möglicherweise anteilig als Hydrochlorid-Salz).
LC-MS (Methode 1): Rₜ = 0.65 min
MS (ESIpos): m/z = 336 (M+H)⁺
¹H-NMR (400 MHz, DMSO-d₆): δ = 5.43 - 5.51 (m, 2H), 7.32 (d, 1H), 7.57 - 7.63 (m, 1H), 7.83 - 7.91 (m, 1H), 8.48 - 8.54 (m, 1H), 8.96 - 9.00 (m, 1H), 13.36 (br. s, 1H), [weiteres Signal unter Lösungsmittelpeak].

### Beispiel 130A

### Ethyl-8-[(3,5-difluorpyridin-4-yl)methoxy]-2,6-dimethylimidazo[1,2-a]pyridin-3-carboxylat

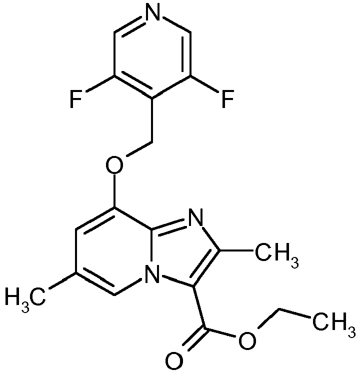

5.0 g (21.34 mmol) Ethyl-8-hydroxy-2,6-dimethylimidazo[1,2-a]pyridin-3-carboxylat aus Beispiel 87A und 3.83 g (23.48 mmol) 4-(Chlormethyl)-3,5-difluorpyridin aus Beispiel 115A wurden in 306 ml abs. DMF vorgelegt und mit 20.8 g (64.03 mmol) Cäsiumcarbonat versetzt. Das Reaktionsgemisch wurde über Nacht bei 60°C gerührt. Das auf RT abgekühlte Reaktionsgemisch wurde filtriert, mit Essigsäureethylester gewaschen und eingeengt. Der Rückstand wurde mittels Kieselgel-Chromatographie (Cyclohexan/Essigsäureethylester-Gradient = 4:1 nach 2:1) gereinigt. Man erhielt 5.40 g (70% d. Th.) der Zielverbindung.
LC-MS (Methode 17): Rₜ = 0.96 min
MS (ESIpos): m/z = 362 (M+H)⁺
¹H-NMR (400 MHz, DMSO-d₆): δ = 1.35 (t, 3H), 2.36 (s, 3H), 2.51 (s, 3H; überlagert mit Lösungsmittelsignal), 4.35 (q, 2H), 5.40 - 5.46 (m, 2H), 7.09 (s, 1H), 8.68 (s, 2H), 8.73 (s, 1H).

### Beispiel 131A

### 8-[(3,5-Difluorpyridin-4-yl)methoxy]-2,6-dimethylimidazo[1,2-a]pyridin-3-carbonsäure

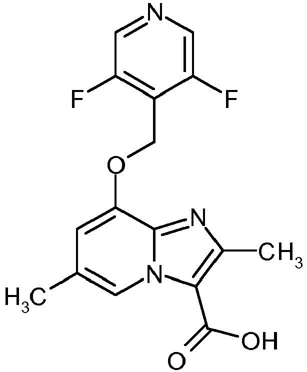

5.34 g (14.78 mmol) Ethyl-8-[(3,5-difluorpyridin-4-yl)methoxy]-2,6-dimethylimidazo[1,2-a]pyridin-3-carboxylat aus Beispiel 130A wurden in 160 ml Dioxan vorgelegt, mit 147.8 ml (147.8 mmol) 1 M wässrige Natriumhydroxid-Lösung versetzt und über Nacht bei RT gerührt. Das auf RT abgekühlte Reaktionsgemisch wurde mit 1 N wässriger Salzsäure auf ca. pH 4 gestellt, das Lösungsmittel zur Hälfte eingeengt, der ausgefallene Feststoff abgesaugt und im Vakuum getrocknet. Man erhielt 4.61 g (93% d. Th.) der Zielverbindung.
LC-MS (Methode 1): Rₜ = 0.58 min
MS (ESIpos): m/z = 334 (M+H)⁺
¹H-NMR (400 MHz, DMSO-d₆): δ = 2.36 (s, 3H), 2.51 (s, 3H; überlagert mit Lösungsmittelsignal), 5.41 - 5.46 (m, 2H), 7.08 (s, 1H), 8.68 (s, 2H), 8.79 (s, 1H), 13.09 (br. s, 1H).

### Beispiel 132A

### 3,3,4,4,4-Pentafluorbutyltrifluormethansulfonat

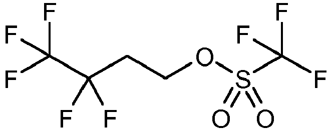

Unter Argon wurden 198.49 g (703.51 mmol) Trifluormethansulfonsäureanhydrid vorgelegt. Der Kolben wurde in ein 70 °C heißes Ölbad getaucht und auf 56 °C Innentemperatur erhitzt. 88.2 ml (738.68 mmol) 3,3,4,4,4-Pentafluorobutanol wurden innerhalb von 35 min zum Reaktionsgemisch zugetropft. Es wurde zwei Stunden bei 70-73 °C Badtemperatur und 69 °C Innentemperatur gerührt. Das Reaktionsgemisch wurde im Vakuum eingeengt und der Rückstand in 1500 ml Dichlormethan aufgenommen. Es wurde einmal mit 300 ml kaltem Wasser, einmal mit 300 ml kalter, wässriger gesättigter Natriumhydrogencarbonat-Lösung und einmal mit 300 ml kaltem Wasser gewaschen. Die organische Phase wurde mit Magnesiumsulfat getrocknet, abfiltriert und eingeengt. Man erhielt 192.86 g (92.6% d. Th.) der Zielverbindung.
¹H-NMR (400 MHz, DMSO-d₆): δ = 2.71 - 2.89 (m, 2H), 4.58 (t, 2H).

### Beispiel 133A

### rac-Methyl-5,5,6,6,6-pentafluornorleucinat Hydrochlorid (Racemat)

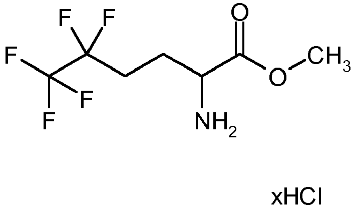

Unter Argon wurden 132 g (521.0 mmol) Methyl-N-(diphenylmethylen)glycinat [beschrieben in: WO2010/123792 A1, 2010, 11-13; zusätzlich: kommerziell erhältlich] in 1000 ml THF (wasserfrei) vorgelegt und auf - 40 °C abgekühlt. 625.2 ml (625.2 mmol) Bis-(trimethylsilyl)-lithiumamid (1 M in THF) wurden innerhalb von 30 min zugetropft. Nach 10 min wurde das Kältebad durch ein Wassereisbad ersetzt und man ließ innerhalb von 35 min die Innentemperatur auf 0 °C ansteigen. 192.86 g (651.25 mmol) 3,3,4,4,4-Pentafluorbutyltrifluormethansulfonat aus Beispiel 132A gelöst in 400 ml THF, wurden bei 0°C in die Reaktionslösung zugetropft. Nach 10 min wurde das Kältebad entfernt und das Gemisch wurde 3 Tage bei RT gerührt. Anschließend wurde die Reaktionsmischung auf 0 °C abgekühlt und tropfenweise mit 410 ml (1.33 mol) 3 N wässriger Salzsäure versetzt. Das Kältebad wurde entfernt und die Reaktionslösung wurde zwei Stunden bei RT gerührt. Das Gemisch wurde anschließend eingeengt. Man erhielt 141.5 g der Zielverbindung als Rohgemisch, welche ohne weitere Reinigung in die Folgestufe eingesetzt wurde.

### Beispiel 134A

### rac-Methyl-N-[(benzyloxy)carbonyl]-5,5,6,6,6-pentafluornorleucinat (Racemat)

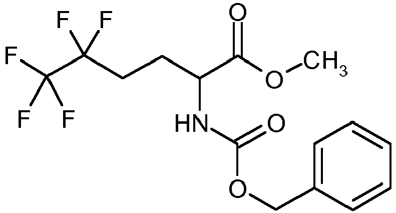

141.5 g (520.99 mmol) *rac*-Methyl-5,5,6,6,6-pentafluornorleucinathydrochlorid aus Beispiel 133A wurden unter Argon in 850 ml THF und 850 ml Wasser aufgenommen und vorsichtig mit 223.2 g (1.62 mol) Kaliumcarbonat bei RT versetzt. Anschließend wurden 82 ml (573.09 mmol) Chlorameisensäurebenzylester zugetropft und die Suspension über Nacht bei RT gerührt. Das Reaktionsgemisch wurde zweimal mit 500 ml Essigsäureethylester extrahiert, die organische Phase wurde mit Magnesiumsulfat getrocknet, abfiltriert und eingeengt. Der Rückstand wurde in 50 ml Dichlormethan verdünnt und mittels Kieselgel-Chromatographie (Laufmittel: Cyclohexan/Essigsäureethylester-Gradient: 9/1 nach 4/1) gereinigt. Die isolierte Produktfraktion wurde nochmals mittels präparativer HPLC [Daiso C18 10µm Bio 300 x 100mm, neutral; Eluent: Acetonitril/Wasser-Gradient; Fluss: 250 ml/min; Temperatur: RT; Wellenlänge: 210 nm) gereinigt. Man erhielt 27.4 g (14% d. Th.) der Zielverbindung.
LC-MS (Methode 1): Rₜ = 1.09 min
MS (ESIpos): m/z = 370 (M+H)⁺
¹H-NMR (400 MHz, DMSO-d₆): δ = 1.78 - 1.91 (m, 1H), 1.93 - 2.05 (m, 1H), 2.10 - 2.30 (m, 1H), 2.30 - 2.46 (m, 1H), 3.66 (s, 3H), 4.18 - 4.26 (m, 1H), 5.05 (s, 2H), 7.27 - 7.40 (m, 5H), 7.89 (d, 1 H).

### Beispiel 135A

### rac-Benzyl-(6,6,7,7,7-pentafluor-2-hydroxy-2-methylheptan-3-yl)carbamat (Racemat)

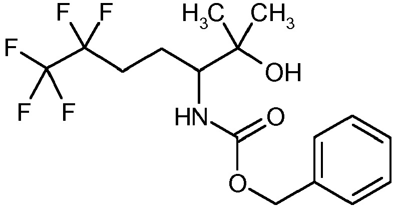

1.7 g (3.68 mmol, Reinheit 80%) *rac*-Methyl-N-[(benzyloxy)carbonyl]-5,5,6,6,6-pentafluornorleucinat (Racemat) aus Beispiel 134A wurde unter Argon in THF vorgelegt und auf 0°C abgekühlt. 4.3 ml (12.89 mmol) 3 M Methylmagnesiumbromid-Lösung in Diethylether wurden zugetropft und es wurde 15 min bei 0°C nachgerührt. Man ließ das Gemisch langsam auf RT erwärmen und rührte über Nacht bei Raumtemperatur. Dann wurde vorsichtig mit gesättigter wässriger Ammoniumchlorid-Lösung versetzt und zur Hälfte eingeengt. Der Rückstand wurde zwischen Dichlormethan und Wasser verteilt, die organische Phase zweimal mit Wasser gewaschen, über Natriumsulfat getrocknet, filtriert und eingeengt. Der Rückstand wurde mittels Kieselgel-Chromatographie (Cyclohexan/Essigsäureethylester = 10:1 nach 7:3) gereinigt. Man erhielt 1.31 g (96% d. Th.) der Zielverbindung.
LC-MS (Methode 1): Rₜ = 1.03 min.
MS (ESIpos): m/z = 370 (M+H)⁺.
¹H-NMR (400 MHz, DMSO-d₆): δ = 1.01 (s, 3H), 1.08 (s, 3H), 1.43 - 1.56 (m, 1H), 1.92 - 2.01 (m, 1H), 2.01 - 2.19 (m, 2H), 3.36 - 3.44 (m, 1H), 4.48 (s, 1H), 4.99 - 5.12 (m, 2H), 7.11 (d, 1H), 7.27 - 7.38 (m, 5H).

### Beispiel 136A

### rac-3-Amino-6,6,7,7,7-pentafluor-2-methylheptan-2-ol Hydrochlorid (Racemat)

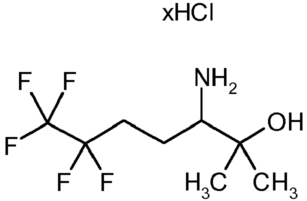

100 mg (0.27 mmol) *rac*-Benzyl-(6,6,7,7,7-pentafluor-2-hydroxy-2-methylheptan-3-yl)carbamat (Racemat) aus Beispiel 135A wurden in 1.9 ml Ethanol vorgelegt und mit 29 mg (0.027 mmol) 10%-igem Palladium/Kohle und 0.82 ml (8.12 mmol) Cyclohexen versetzt. Das Reaktionsgemisch wurde 4 h unter Rückfluss gerührt. Das Reaktionsgemisch wurde über einen Millipore-Filter filtriert und gut mit Ethanol gewaschen. Das Filtrat wurde mit 0.27 ml 2 N ChlorwasserstoffLösung in Diethylether versetzt, eingeengt und im Hochvakuum getrocknet. Es wurden 66 mg (90% d. Th.) der Zielverbindung erhalten.
MS (Methode 19): m/z = 236 (M-HCl+H)⁺

### Beispiel 137A

### ent-Benzyl-(6,6,7,7,7-pentafluor-2-hydroxy-2-methylheptan-3-yl)carbamat (Enantiomer A)

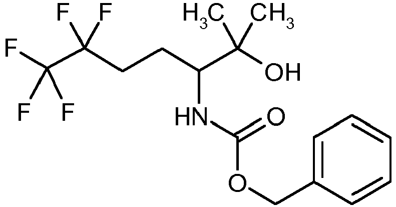

1.31 g *rac*-Benzyl-(6,6,7,7,7-pentafluor-2-hydroxy-2-methylheptan-3-yl)carbamat aus Beispiel 135A wurden durch präparative Trennung an chiraler Phase in die Enantiomere getrennt [Säule: Daicel Chiralpak AY-H, 5 µm, 250 x 20 mm, Eluent: 90% iso-Hexan, 10% Ethanol, Fluß: 15 ml/min; 35°C; Detektion: 220 nm].
Enantiomer A: 459 mg (99% ee)
Rₜ= 4.31 min [Daicel Chiralpak AY-H, 5µm, 250 x 4.6 mm; Eluent: 90% iso-Hexan, 10% Ethanol; Fluss: 1.0 ml/min; 30°C; Detektion: 220 nm].

### Beispiel 138A

### ent-3-Amino-6,6,7,7,7-pentafluor-2-methylheptan-2-ol Hydrochlorid (Enantiomer A)

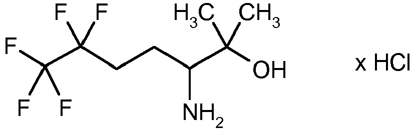

455 mg (1.23 mmol) *ent*-Benzyl-(6,6,7,7,7-pentafluor-2-hydroxy-2-methylheptan-3-yl)carbamat (Enantiomer A) aus Beispiel 137A wurden in 8.6 ml Ethanol vorgelegt, mit 131 mg (0.123 mmol) 10%-igem Palladium auf Kohle und 3.74 ml (36.96 mmol) Cyclohexen versetzt und 3 h unter Rückfluss gerührt. Das Reaktionsgemisch wurde über einen Millipore-Filter filtriert und mit Ethanol gewaschen. Das Filtrat wurde mit 1.23 ml 2 N Chlorwasserstoff in Diethylether versetzt, eingeengt und im Hochvakuum getrocknet. Es wurden 335 mg (98% d. Th.) der Zielverbindung erhalten.
MS (Methode 19): m/z = 236 (M-HCl+H)⁺
¹H-NMR (400 MHz, DMSO-d₆): δ = 1.11 (s, 3H), 1.22 (s, 3H), 1.58 - 1.72 (m, 1H), 1.80 - 1.92 (m, 1H), 2.27 - 2.46 (m, 2H, teilweise verdeckt durch DMSO-Peak), 2.94 - 3.04 (m, 1H), 5.35 (s, 1H), 7.80 - 8.01 (m, 3H).

### Beispiel 139A

### rac-Methyl-2-amino-6,6,7,7,7-pentafluorheptanoathydrochlorid (Racemat)

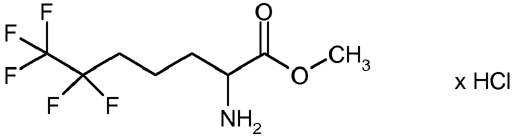

Unter Argon wurden 15 g (59.2 mmol) Methyl-N-(diphenylmethylen)glycinat [beschrieben in: WO2010/123792 A1, 2010; p. 11-13] in 127 ml 1,4-Dioxan vorgelegt, auf 0°C abgekühlt und mit 68.1 ml (68.1 mmol) 1 N Kalium-*tert*-butylat-Lösung in THF versetzt. Die Reaktionslösung wurde 1 h bei 0°C gerührt, anschließend mit 21.2 g (78.7 mmol) 4,4,5,5,5-Pentafluorpentylmethansulfonat [kommerziell erhältlich; zusätzlich beschrieben in: H. Kimura et al. Chemistry and Biology 2010, 17, 18-27] versetzt und über Nacht bei 50°C gerührt. Das Gemisch wurde auf RT abgekühlt, mit 59.2 ml (118.4 mmol) 2 N Salzsäure in Diethylether und 2.1 ml (118.4 mmol) Wasser versetzt und über Nacht bei RT kräftig gerührt. Das Reaktionsgemisch wurde eingeengt und der Rückstand im Hochvakuum getrocknet. Man erhielt ca. 17 g der Zielverbindung als Rohgemisch, welche ohne weitere Reinigung in die Folgestufe eingesetzt wurde.
MS (Methode 19): m/z = 250 (M-HCl+H)⁺

### Beispiel 140A

### rac-Methyl-2-{[(benzyloxy)carbonyl]amino}-6,6,7,7,7-pentafluorheptanoat (Racemat)

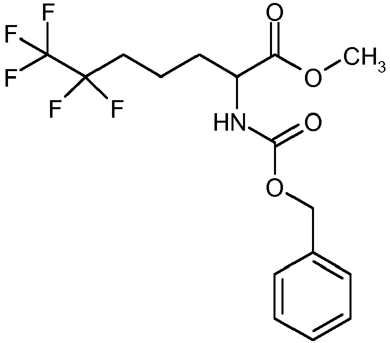

16.9 g (59.2 mmol) *rac*-Methyl-2-amino-6,6,7,7,7-pentafluorheptanoathydrochlorid (Racemat) aus Beispiel 139A wurden unter Argon in 775 ml THF und 99 ml Wasser vorgelegt und bei RT vorsichtig mit 25.37 g (183.6 mmol) Kaliumcarbonat versetzt. Anschließend wurden 11.0 ml (65.1 mmol) Benzylchlorocarbonat bei 0°C zugetropft und die Suspension wurde über Nacht bei RT gerührt. Das Reaktionsgemisch wurde eingeengt, der Rückstand mit Wasser versetzt und zweimal mit Dichlormethan extrahiert. Die vereinigten organischen Phasen wurden über Natriumsulfat getrocknet, filtriert und eingeengt. Das Rohprodukt wurde mittels Kieselgel-Chromatographie gereinigt (Laufmittel: Cyclohexan/Essigsäureethylester-Gradient: 100/0 nach 10/1 nach 8/1 nach 5/1). Die Produktfraktion wurde anschließend nochmals mittels präparativer RP-HPLC gereinigt (Acetonitril/Wasser-Gradient mit 0.1% TFA). Man erhielt 13.34 g (56% d. Th., Reinheit 94%) der Zielverbindung.
LC-MS (Methode 1): Rₜ = 1.18 min
MS (ESIpos): m/z = 384 (M+H)⁺
¹H-NMR (400 MHz, DMSO-d₆): δ = 1.51 - 1.62 (m, 2H), 1.63 - 1.74 (m, 1H), 1.76 - 1.87 (m, 1H), 2.07 - 2.31 (m, 2H), 3.64 (s, 3H), 4.06 - 4.13 (m, 1H), 5.04 (d, 2H), 7.24 - 7.40 (m, 5H), 7.82 (d, 1 H).

### Beispiel 141A

### rac-Benzyl-(7,7,8,8,8-pentafluor-2-hydroxy-2-methyloctan-3-yl)carbamat (Racemat)

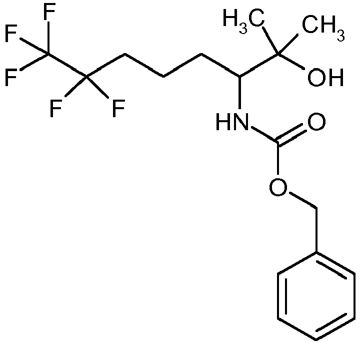

13.0 g (32.1 mmol, Reinheit 94%) *rac*-Methyl-2-{[(benzyloxy)carbonyl]amino}-6,6,7,7,7-pentafluorheptanoat (Racemat) aus Beispiel 140A wurden unter Argon in THF vorgelegt und auf 0°C abgekühlt. 37.4 ml (112.29 mmol) 3 M Methylmagnesiumbromid-Lösung in Diethylether wurden zugetropft und es wurde 15 min bei 0°C gerührt. Dann ließ man das Gemisch langsam auf RT erwärmen und rührte über Nacht bei Raumtemperatur. Die Reaktionslösung wurde auf 0°C abgekühlt und mit 10.7 ml (32.1 mmol) 3M Methylmagnesiumbromid-Lösung in Diethylether versetzt. Anschließend wurde über Nacht bei Raumtemperatur gerührt. Dann wurde vorsichtig mit gesättigter wässriger Ammoniumchlorid-Lösung versetzt und die Reaktionslösung zur Hälfte eingeengt. Der Rückstand wurde zwischen Dichlormethan und Wasser verteilt, die organische Phase einmal mit Wasser gewaschen, über Natriumsulfat getrocknet, filtriert und eingeengt. Der Rückstand wurde mittels Kieselgel-Chromatographie (Cyclohexan/Essigsäureethylester = 5/1 nach 1/1) gereinigt und im Hochvakuum getrocknet. Man erhielt 10.1 g (78% d. Th.) der Zielverbindung.
LC-MS (Methode 1): Rₜ = 1.12 min
MS (ESIpos): m/z = 384 (M+H)⁺.
¹H-NMR (400 MHz, DMSO-d₆): δ = 0.99 (s, 3H), 1.06 (s, 3H), 1.26 - 1.48 (m, 2H), 1.51 - 1.64 (m, 1H), 1.70 - 1.82 (m, 1H), 1.99 - 2.31 (m, 2H), 4.24 - 4.41 (m, 1H), 4.98 - 5.11 (m, 2H), 6.95 (d, 1H), 7.28 - 7.38 (m, 5H).

### Beispiel 142A

### rac-3-Amino-7,7,8,8,8-pentafluor-2-methyloctan-2-ol Hydrochlorid (Racemat)

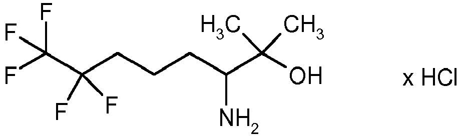

330 mg (0.86 mmol) *rac*-Benzyl-(7,7,8,8,8-pentafluor-2-hydroxy-2-methyloctan-3-yl)carbamat aus Beispiel 141A wurden in 6.0 ml Ethanol vorgelegt, mit 92 mg (0.086 mmol) 10%-igem Palladium auf Kohle sowie 2.62 ml (25.82 mmol) Cyclohexen versetzt und 3 h unter Rückfluss gerührt. Das Reaktionsgemisch wurde über einen Millipore-Filter filtriert, mit Ethanol gewaschen, das Filtrat mit 0.86 ml 2 N Chlorwasserstoff-Lösung in Diethylether versetzt, eingeengt und im Hochvakuum getrocknet. Es wurden 243 mg (99% d. Th.) der Zielverbindung erhalten.
MS (Methode 19): m/z = 250 (M-HCl+H)⁺

### Beispiel 143A

### Methyl-6,6,6-trifluor-5-hydroxynorleucinat Hydrochlorid (Stereoisomerenmischung)

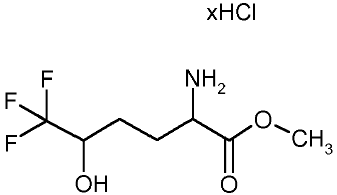

150 mg (0.7 mmol) *rac*-6,6,6-Trifluor-5-hydroxynorleucin (Stereoisomerenmischung) wurden mit 1.4 ml gesättigter Salzsäure in Methanol versetzt und über Nacht unter Rückfluss gerührt. Die Reaktionslösung wurde eingeengt, erneut in 1.5 ml gesättigter Salzsäure in Methanol versetzt und über Nacht unter Rückfluss gerührt. Die Reaktionslösung wurde eingeengt und der Rückstand am Hochvakuum getrocknet. Man erhielt 185 mg (99% d. Th.) der Zielverbindung.
MS (Methode 19): m/z = 216 (M-HCl+H)⁺
¹H-NMR (400 MHz, DMSO-d₆): δ = 1.42 - 1.55 (m, 1H), 1.69 - 1.80 (m, 1H), 1.83 - 1.94 (m, 1H), 1.95 - 2.08 (m, 1H), 3.77 (s, 3H), 3.91 - 4.03 (m, 1H), 4.12 (t, 1H), 6.33 (s, 1H), 8.34 - 8.63 (m, 2H).

### Beispiel 144A

### Methyl-6,6,6-trifluor-N-({8-[(3-fluorpyridin-2-yl)methoxy]-2,6-dimethylimidazo[1,2-a]pyridin-3-yl}carbonyl)-5-hydroxynorleucinat (Stereoisomerenmischung)

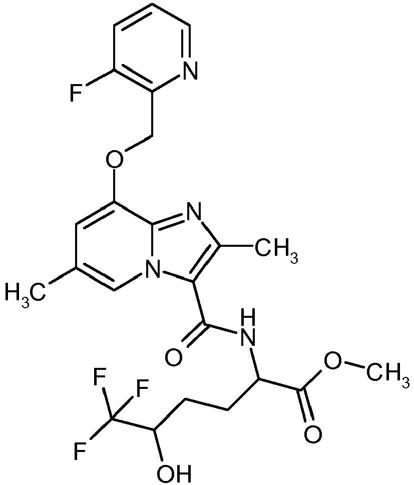

250 mg (0.71 mmol) 8-[(3-Fluorpyridin-2-yl)methoxy]-2,6-dimethylimidazo[1,2-a]pyridin-3-carbonsäure-Hydrochlorid aus Beispiel 121A wurden mit 299 mg (0.79 mmol) HATU und 0.50 ml (2.85 mmol) N,N-Diisopropylethylamin in 2.5 ml DMF vorgelegt und 20 min bei RT gerührt. 216 mg (0.86 mmol) Methyl-6,6,6-trifluor-5-hydroxynorleucinat Hydrochlorid (Stereoisomerenmischung) wurden hinzugegeben und das Gemisch wurde über Nacht bei RT gerührt. Die Reaktionslösung wurde mit Wasser und TFA versetzt und mittels präparativer RP-HPLC (Acetonitril/Wasser-Gradient unter Zusatz von 0.1% TFA) gereinigt. Die Produktfraktionen wurden eingeengt, der Rückstand wurde mit Natrium-hydrogencarbonat-Lösung versetzt und dreimal mit Dichlormethan extrahiert. Die vereinten organischen Phasen wurden über Natriumsulfat getrocknet, abfiltriert, eingeengt und am Hochvakuum getrocknet. Man erhielt 265 mg (69% d. Th.) der Zielverbindung.
LC-MS (Methode 1): Rₜ = 0.78 min
MS (ESIpos): m/z = 513 (M+H)⁺
¹H-NMR (400 MHz, DMSO-d₆): δ = 1.53 - 1.64 (m, 1H), 1.65 - 1.76 (m, 1H), 1.94 - 2.03 (m, 2H), 2.29 (s, 3H), 2.49 (s, 3H; überlagert mit Lösungsmittelpeak), 3.70 (s, 3H), 3.97 - 4.07 (m, 1H), 4.52 - 4.59 (m, 1H), 5.38 - 5.41 (m, 2H), 6.20 - 6.26 (m, 1H), 6.90 - 6.95 (m, 1H), 7.56 - 7.62 (m, 1H), 7.82 - 7.89 (m, 1H), 8.29 - 8.35 (m, 2H), 8.47 - 8.53 (m, 1H).

### Beispiel 145A

### Ethyl-2,6-dimethyl-8-[(2,3,6-trifluorbenzyl)oxy]imidazo[1,2-a]pyridin-3-carboxylat

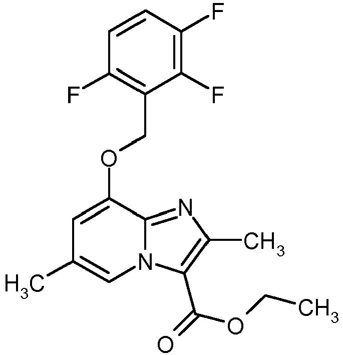

10 g (42.69 mmol) Ethyl-8-hydroxy-2,6-dimethylimidazo[1,2-a]pyridin-3-carboxylat aus Beispiel 87A, 10.9 g (46.96 mmol) 2-(Brommethyl)-1,3,4-trifluorbenzol und 30.6 g (93.91 mmol) Cäsiumcarbonat wurden in 611 ml DMF vorgelegt und für 30 min in einem vorgeheizten 60°C warmen Ölbad erhitzt. Das Reaktionsgemisch wurde auf ca. 5 1 Wasser gegossen, 30 min gerührt, der entstandene Feststoff abgesaugt, mit Wasser gewaschen und in Hochvakuum getrocknet. Es wurden 14.1 g der Zielverbindung (86% d. Th.) erhalten.
LC-MS (Methode 18): Rₜ = 2.28 min
MS (ESpos): m/z = 379 (M+H)⁺

### Beispiel 146A

### 2,6-Dimethyl-8-[(2,3,6-trifluorbenzyl)oxy]imidazo[1,2-a]pyridin-3-carbonsäure

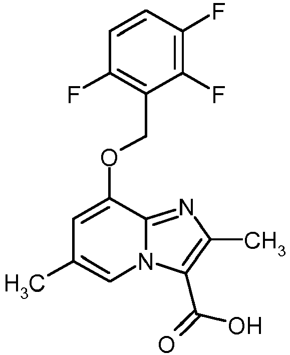

6.9 g (17.51 mmol) Ethyl-2,6-dimethyl-8-[(2,3,6-trifluorbenzyl)oxy]imidazo[1,2-a]pyridin-3-carboxylat aus Beispiel 145A wurden in 374 ml THF/Methanol (5/1) gelöst, mit 87.5 ml (87.5 mmol) 1 N wässriger Lithiumhydroxid-Lösung versetzt und 5 Stunden bei 40°C gerührt. Nach dem Abkühlen wurde unter Eiskühlung mit 6 N wässriger Salzsäure angesäuert und anschließend am Rotationsverdampfer vom organischen Lösungsmittel befreit. Der entstandene Feststoff wurde abgesaugt, mit Wasser nachgewaschen und anschließend im Hochvakuum getrocknet. Es wurden 6.7 g der Zielverbindung (108% d. Th.) erhalten.
LC-MS (Methode 1): Rₜ = 0.76 min
MS (ESpos): m/z = 351 (M+H)⁺
¹H-NMR (400 MHz, DMSO-d₆): δ = 2.37 (s, 3H), 2.53 (s, 3H; überlagert mit Lösungsmittelpeak), 5.35 (s, 2H), 7.09 (s, 1H), 7.25 - 7.35 (m, 1H), 7.61 - 7.74 (m, 1H), 8.77 (s, 1H), 12.88 - 13.23 (m, 1 H).

### Ausführungsbeispiele:

Erfindungsgemäße Beispiele sind solche Beispiele, die unter den Anspruch 1 fallen.

### Beispiel 1

### 8-[(2,6-Difluorbenzyl)oxy]-N-[(2R)-1-hydroxyhexan-2-yl]-2-methylimidazo[1,2-a]pyridin-3-carboxamid

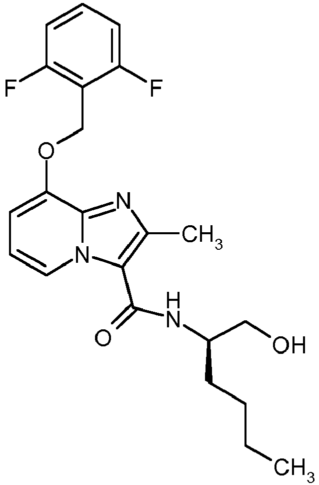

50 mg (0.172 mmol) 8-Hydroxy-N-[(2R)-1-hydroxyhexan-2-yl]-2-methylimidazo[1,2-a]pyridin-3-carboxamid, 39 mg (0.189 mmol) 2,6-Difluorbenzylbromid, 52 mg (0.378 mmol) Kaliumcarbonat, und 14 mg (0.086 mmol) Kaliumiodid wurden mit 1.2 ml 1-Methyl-2-pyrrolidon versetzt und dann wurde das Reaktionsgemisch in der Mikrowelle für 40 min bei 120°C erhitzt. Es wurde vom Feststoff abdekantiert und dieser noch zweimal mit Methanol gewaschen. Die vereinigten Lösungen wurden aufkonzentriert und der Rückstand über präparative HPLC aufgereinigt (Methode 7). Man erhielt 43.5 mg (60% d. Th.) 8-[(2,6-Difluorbenzyl)oxy]-N-[(2R)-1-hydroxyhexan-2-yl]-2-methylimidazo[1,2-a]pyridin-3-carboxamid.
LC-MS (Methode 2): Rₜ = 0.96 min
MS (ESpos): m/z = 418.2 (M+H)⁺
¹H-NMR (400 MHz, DMSO-d₆): δ = 0.88 (t, 3H), 1.25 - 1.70 (m, 6H), 2.58 (s, 3H), 3.48 (m, 2H), 3.95 (m, 1H), 4.71 (t, 1H), 5.29 (s, 2H), 6.90 (t, 1H), 6.98 (d, 1H), 7.22 (t, 2H), 7.48 (d, 1H), 7.57 (m, 1H), 8.52 (d, 1H).
Spezifischer Drehwert (589 nm, 20.1 °C, c = 0.365 g/ml) in Methanol: + 15.7°

In Analogie zu Beispiel 1 wurden die in Tabelle 1 gezeigten Beispielverbindungen hergestellt, indem 8-Hydroxy-N-[(2R)-1-hydroxyhexan-2-yl]-2-methylimidazo[1,2-a]pyridin-3-carboxamid mit den entsprechenden, kommerziell erhältlichen Benzylhalogeniden umgesetzt worden ist:

**Tabelle 1:**

| **Beispiel** | **IUPAC-Name / Struktur (Ausbeute)** | **Analytische Daten** |
|---|---|---|
| **2** | N-[(2R)-1-Hydroxyhexan-2-yl]-2-methyl-8-[(2,3,6-trifluorbenzyl)oxy]imidazo[1,2-a]pyridin-3-carboxamid | LC-MS (Methode 1): Rₜ = 0.89 min |
| | 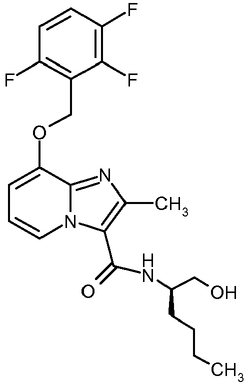 | MS (ESpos): m/z = 436.1 (M+H)⁺ |
| | | ¹H-NMR (400 MHz, DMSO-d₆): δ = 0.88 (t, 3H), 1.25 -1.70 (m, 6H), 2.58 (s, 3H), 3.48 (m, 2H), 3.95 (m, 1H), 4.71 (t, 1H), 5.35 (s, 2H), 6.92 (t,1H, 6.98 (d, 1H), 7.29 (m, 2H), 7.50 (d, 1H), 7.65 (m,1H, 8.54 (d, 1H). |
| | (61% d. Th.) | |
| **3** | 8-[(2-Chlor-6-fluorbenzyl)oxy]-N-[(2R)-1-hydroxyhexan-2-yl]-2-methylimidazo[1,2-a]pyridin-3-carboxamid | LC-MS (Methode 1): Rₜ = 0.91 min |
| | | MS (ESpos): m/z = 434.1 (M+H)⁺ |
| | 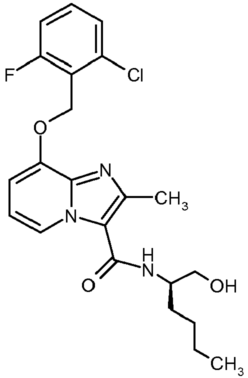 | ¹H-NMR (400 MHz, DMSO-d₆): δ = 0.88 (t, 3H), 1.25 -1.70 (m, 6H), 2.58 (s, 3H), 3.48 (m, 2H), 3.95 (m, 1H), 4.71 (t, 1H), 5.35 (s, 2H), 6.93 (t,1H, 7.01 (d, 1H), 7.34-7.40 (m, 1H), 7.45 - 7.60 (m, 3H), 8.55 (d, 1H). |
| | (56% d. Th.) | |
| **4** | 8-[(2-Chlorbenzyl)-oxy]-N-[(2R)-1-hydroxy-hexan-2-yl]-2-methylimidazo[1,2-a]pyridin-3-carboxamid | LC-MS (Methode 1): Rₜ = 0.93 min |
| | 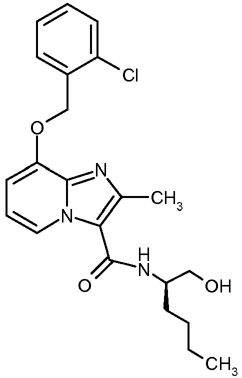 | MS (ESpos): m/z = 416.2 (M+H)⁺ |
| | (68% d. Th.) | |
| **5** | 8-[(2-Fluorbenzyl)-oxy]-N-[(2R)-1-hydroxy-hexan-2-yl]-2-methylimidazo[1,2-a]pyridin-3-carboxamid | LC-MS (Methode 1): Rₜ = 0.86 min |
| | 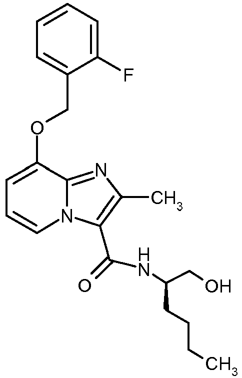 | MS (ESpos): m/z = 400.2 (M+H)⁺ |
| | (58% d. Th.) | |
| **6** | 8-[(3-Fluorbenzyl)-oxy]-N-[(2R)-1-hydroxy-hexan-2-yl]-2-methylimidazo[1,2-a]pyridin-3-carboxamid | LC-MS (Methode 1): Rₜ = 0.86 min |
| | 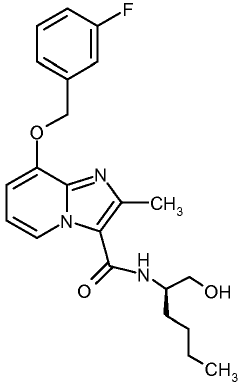 | MS (ESpos): m/z = 400.2 (M+H)⁺ |
| | (58% d. Th.) | |
| **7** | N-[(2R)-1-Hydroxy-hexan-2-yl]-2-methyl-8-[(2,3,5,6-tetrafluorbenzyl)oxy]-imidazo[1,2-a]pyridin-3-carboxamid | LC-MS (Methode 3): Rₜ = 1.80 min |
| | | MS (ESpos): m/z = 454.2 (M+H)⁺ |
| | 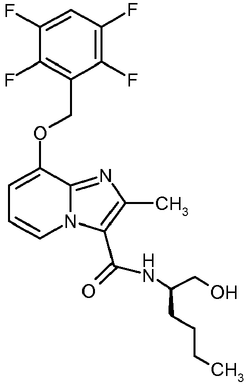 | |
| | (60% d. Th.) | |
| **8** | 8-[(2,6-Dichlorbenzyl)oxy]-N-[(2R)-1-hydroxyhexan-2-yl]-2-methyl-imidazo[1,2-a]pyridin-3-carboxamid | LC-MS (Methode 1): Rₜ = 0.96 min |
| | 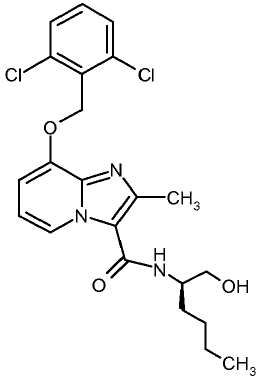 | MS (ESpos): m/z = 450.0 (M+H)⁺ |
| | (64% d. Th.) | |
| **9** | 8-[(2,3-Dichlorbenzyl)oxy]-N-[(2R)-1-hydroxy-hexan-2-yl]-2-methyl-imidazo[1,2-a]pyridin-3-carboxamid | LC-MS (Methode 1): Rₜ = 1.00 min |
| | 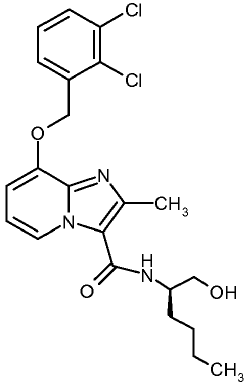 | MS (ESpos): m/z = 450.1 (M+H)⁺ |
| | | Spezifischer Drehwert (589 nm, 19.8 °C, c = 0.445 g/100 ml) in Methanol: + 14.0° |
| | (52% d. Th.) | |
| **10** | 8-[(2,3-Difluorbenzyl)oxy]-N-[(2R)-1-hydroxy-hexan-2-yl]-2-methyl-imidazo[1,2-a]pyridin-3-carboxamid | LC-MS (Methode 1): Rₜ = 0.89 min |
| | 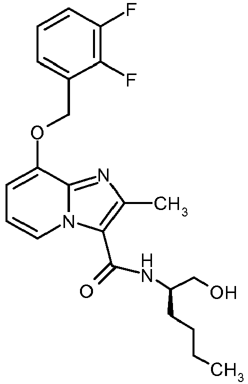 | MS (ESpos): m/z = 418.1 (M+H)⁺ Spezifischer Drehwert (589 nm, 19.7 °C) in Methanol: + 16.0° |
| | (63% d. Th.) | |
| **11** | 8-[(2-Fluor-3-methyl-benzyl)oxy]-N-[(2R)-1-hydroxyhexan-2-yl]-2-methyl-imidazo[1,2-a]pyridin-3-carboxamid | LC-MS (Methode 2): Rₜ = 1.02 min |
| | | MS (ESpos): m/z = 414.3 (M+H)⁺ |
| | 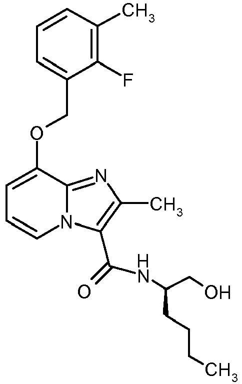 | |
| | (56% d. Th.) | |
| **12** | 8-[(2,2-Difluor-1,3-benzodioxol-4-yl)methoxy]-N-[(2R)-1-hydroxyhexan-2-yl]-2-methyl-imidazo[1,2-a]pyridin-3-carboxamid | LC-MS (Methode 1): Rₜ = 0.97 min |
| | | MS (ESpos): m/z = 462.1 (M+H)⁺ |
| | 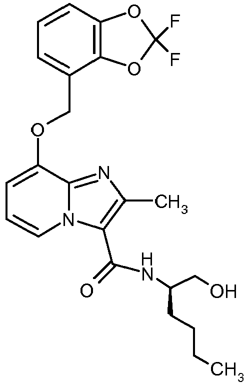 (57% d. Th.) | |
| **13** | 8-[(2,6-Dimethylbenzyl)oxy]-N-[(2R)-1-hydroxyhexan-2-yl]-2-methyl-imidazo[1,2-a]pyridin-3-carboxamid | LC-MS (Methode 1): Rₜ = 0.95 min |
| | 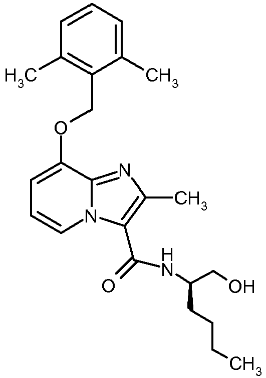 | MS (ESpos): m/z = 410.2 (M+H)⁺ |
| | (63% d. Th.) | |
| **14** | 8-{[2-Fluor-3-(trifluor-methyl)benzyl]oxy}-N-[(2R)-1-hydroxyhexan-2-yl]-2-methyl-imidazo[1,2-a]pyridin-3-carboxamid | LC-MS (Methode 1): Rₜ = 0.98 min |
| | 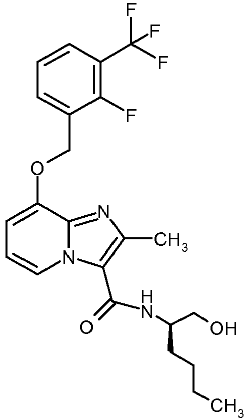 | MS (ESpos): m/z = 468.1 (M+H)⁺ |
| | (65% d. Th.) | |

### Beispiel 15

### N-[(2R)-1-Hydroxyhexan-2-yl]-2-methyl-8-[(2,4,6-trifluorbenzyl)oxy]imidazo[1,2-a]pyridin-3-carboxamid

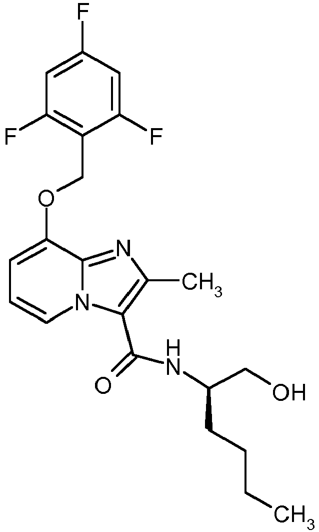

50 mg (0.172 mmol) 8-Hydroxy-N-[(2R)-1-hydroxyhexan-2-yl]-2-methylimidazo[1,2-a]pyridin-3-carboxamid, 42 mg (0.189 mmol) 2,4,6-Trifluorbenzylbromid und 123 mg (0.378 mmol) Cäsiumcarbonat wurden in 2.5 mL Dimethylformamid vorgelegt und für 15 min auf 50°C erhitzt. Nach dem Abkühlen auf Raumtemperatur wurde mit Wasser versetzt. Dann wurde mit Essigsäureethylester versetzt, die Phasen wurden getrennt und die wässrige Phase wurde zweimal mit Essigsäureethylester extrahiert. Die vereinigten organischen Phasen wurden eingeengt und der Rückstand über präparative HPLC aufgereinigt (Methode 8). Man erhielt 54.5 mg (72% d. Th.) der Zielverbindung.
LC-MS (Methode 1): Rₜ = 0.88 min
MS (ESpos): m/z = 436.3 (M+H)⁺
¹H-NMR (400 MHz, DMSO-d₆): δ = 0.88 (t, 3H), 1.25 - 1.40 (m, 4H), 1.40 - 1.51 (m, 1 H), 1.60 - 1.68 (m, 1 H), 2.55 (s, 3H; verdeckt durch DMSO-Signal), 3.40 - 3.51 (m, 2H), 3.90 - 4.02 (m, 1H), 4.71 (t, 1H), 5.29 (s, 2H), 6.90 (t, 1H). 6.98 (d, 1H), 7.31 (t, 2H), 7.52 (d, 1H), 8.52 (d, 1H).

### Allgemeine Arbeitsvorschrift 1: Amidkupplung mit TBTU als Aktivierungsreagenz

Unter Argon wurde 1 Äquivalent der zu kuppelnden Carbonsäure mit 1.0 bis 1.2 Äquivalenten des zu kuppelnden Amins sowie 5 Äquivalenten 4-Methylmorpholin in trockenem Dichlormethan (z.B. 0.1 bis 0.5 M bezogen auf die zu kuppelnde Säure) vorgelegt. Dann wurden 1.1 Äquivalente TBTU zugesetzt und über Nacht bei Raumtemperatur gerührt. Nachdem vollständiger Umsatz der zu kuppelnden Carbonsäure erreicht worden ist (üblicherweise nach 16h bei Raumtemperatur; Analytik per LC-MS), wurde der Ansatz mit zusätzlichem Dichlormethan verdünnt und extraktiv aufgearbeitet. Eine beispielhafte extraktive Aufarbeitung bestand aus dem Waschen mit 10%iger wässriger Zitronensäure-Lösung (oder alternativ gesättigter wässriger NatriumhydrogencarbonatLösung), mit Wasser, abschließend mit gesättigter wässriger Natriumchlorid-Lösung, nachfolgender Trocknung der organischen Phase z.B. mit Magnesiumsulfat, Filtration sowie Aufkonzentrierung des Filtrats. Der so erhaltene Rückstand wurde z.B. säulenchromatographisch (z.B. Biotage Isolera mit Dichlormethan/Methanol oder Cyclohexan/Essigsäureethylester-Gemischen als Eluent) oder per präparativer HPLC aufgereinigt. Beispiel 16 beschreibt eine repräsentative Ausführung dieser allgemeinen Arbeitsvorschrift.

### Beispiel 16

### 8-(Benzyloxy)-N-[(2R)-1-hydroxyhexan-2-yl]-2-methylimidazo[1,2-a]pyridin-3-carboxamid

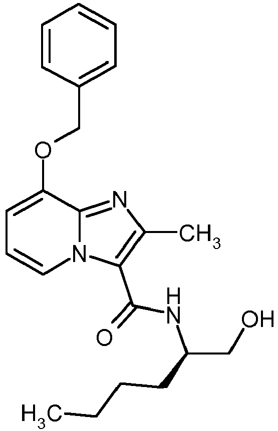

Unter Argon wurden 4 g (14.17 mmol) 8-(Benzyloxy)-2-methylimidazo[1,2-a]pyridin-3-carbonsäure, 1.83 g (15.59 mmol) (R)-(-)-2-Aminohexanol und 7.166 g (70.85 mmol) 4-Methylmorpholin in 28 ml Dichlormethan suspendiert. Dann wurden 5 g (15.59 mmol) TBTU zugesetzt und über Nacht bei Raumtemperatur gerührt. Nach 16 h wurde der Ansatz mit 200 ml Dichlormethan verdünnt und mit 10%iger wässriger Zitronensäure-Lösung, mit Wasser sowie mit gesättigter wässriger Natriumchlorid-Lösung gewaschen. Die organische Phase wurde eingeengt und der erhaltene Rückstand über eine Kieselgelkartusche gereinigt (Laufmittel Dichlormethan:Methanol = 100:3). Nach Einengen der Produktfraktionen erhielt man so 3.83 g (71% d. Th.) 8-(Benzyloxy)-N-[(2R)-1-hydroxyhexan-2-yl]-2-methylimidazo[1,2-a]pyridin-3-carboxamid.
LC-MS (Methode 1): Rₜ = 0.84 min
MS (ESpos): m/z = 381.2 (M+H)^{+ 1}H-NMR (400 MHz, DMSO-d₆): δ = 0.88 (t, 3H), 1.29 - 1.69 (m, 6H), 2.59 (s, 3H), 3.45 (m, 2H), 3.98 (m, 1H), 4.73 (t, 1H), 5.29 (s, 2H), 6.88 (m, 2H), 7.34 - 7.52 (m, 6H). 8.49 (d, 1H).
Spezifischer Drehwert (589 nm, 20.1 °C, c = 0.55 g/100 ml) in Methanol: + 15.5°

### Beispiel 17

### 8-(Benzyloxy)-N-[(2R)-1-hydroxybutan-2-yl]-2-methylimidazo[1,2-a]pyridin-3-carboxamid

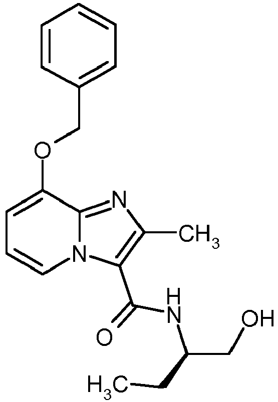

Die Herstellung erfolgte analog zur allgemeinen Arbeitsvorschrift 1 und Beispiel 16 ausgehend von 4.00 g (14.17 mmol) 8-(Benzyloxy)-2-methylimidazo[1,2-a]pyridin-3-carbonsäure. Man erhielt 3.52 g (56% d. Th.) des Zielproduktes in 80%iger Reinheit.
LC-MS (Methode 1): Rₜ = 0.71 min
MS (ESpos): m/z = 354.1 (M+H)⁺
¹H-NMR (400 MHz, DMSO-d₆): δ = 0.93 (t, 3H), 1.48 (m, 1H), 1.68 (m, 1H), 2.53 (s, 3H), 3.49 (m, 2H), 3.92 (m, 1H), 4.73 (tr br, 1H), 5.29 (s, 2H), 6.88 (m, 2H), 7.34 - 7.52 (m, 6H), 8.50 (dd, 1 H).

In Analogie zu Beispiel 17 wurden die in Tabelle 2 gezeigten Beispielverbindungen hergestellt, indem 8-[(2,6-Difluorobenzyl)oxy]-2-methylimidazo[1,2-a]pyridin-3-carbonsäure mit den entsprechenden, kommerziell erhältlichen Aminen unter den beschriebenen TBTU-Bedingungen (allgemeine Arbeitsvorschrift 1) umgesetzt wurde:

**Tabelle 2:**

| **Beispiel** | **IUPAC-Name / Struktur (Ausbeute)** | **Analytische Daten** |
|---|---|---|
| **18** | 8-[(2,6-Difluorbenzyl)oxy]-N-[(2R)-1-hydroxy-pentan-2-yl]-2-methyl-imidazo[1,2-a]pyridin-3-carboxamid | LC-MS (Methode 1): Rₜ = 0.80 min |
| | | MS (ESpos): m/z = 404.1 (M+H)⁺ |
| | 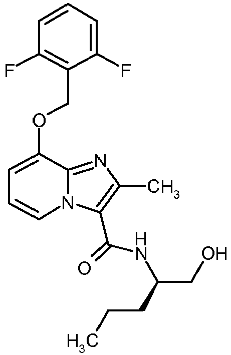 | |
| | (59% d. Th.) | |
| **19** | 8-[(2,6-Difluorbenzyl)oxy]-N-[(1S,2S)-2-hydroxy-cyclopentyl]-2-methyl-imidazo[1,2-a]pyridin-3-carboxamid | LC-MS (Methode 1): Rₜ = 0.74 min |
| | | MS (ESpos): m/z = 402.1 (M+H)⁺ |
| | 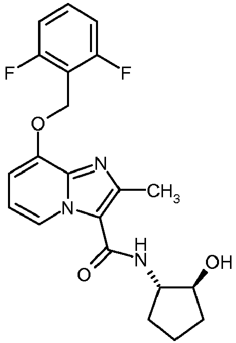 | |
| | (67% d. Th.) | |
| **20** | 8-[(2,6-Difluorbenzyl)oxy]-N-[(3S)-3-hydroxy-2-methylbutan-2-yl]-2-methyl-imidazo[1,2-a]pyridin-3-carboxamid | LC-MS (Methode 1): Rₜ = 0.82 min |
| | | MS (ESpos): m/z = 404.1 (M+H)⁺ |
| | 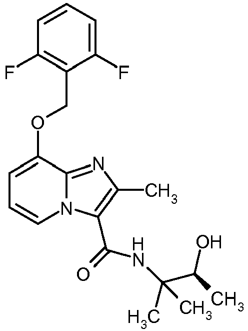 | |
| | (22% d. Th.) | |
| **21** | 8-[(2,6-Difluorbenzyl)oxy]-N-(2-hydroxyethyl)-2-methyl-imidazo[1,2-a]pyridin-3-carboxamid | LC-MS (Methode 3): Rₜ = 1.29 min |
| | | MS (ESpos): m/z = 362.1 (M+H)⁺ |
| | 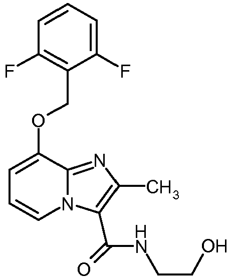 | |
| | (46% d. Th.) | |
| **22** | 8-[(2,6-Difluorbenzyl)oxy]-N-(3-hydroxy-propyl)-2-methyl-imidazo[1,2-a]pyridin-3-carboxamid | LC-MS (Methode 1): Rₜ = 0.67 min |
| | | MS (ESpos): m/z = 376.2 (M+H)⁺ |
| | 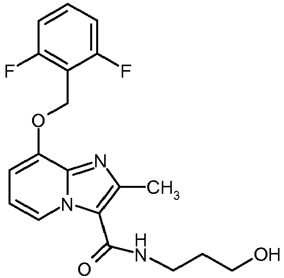 | |
| | (54% d. Th.) | |
| **23** | 8-[(2,6-Difluorbenzyl)oxy]-N-(4-hydroxybutyl)-2-methyl-imidazo[1,2-a]pyridin-3-carboxamid | LC-MS (Methode 3): Rₜ = 1.37 min |
| | | MS (ESpos): m/z = 390.2 (M+H)⁺ |
| | 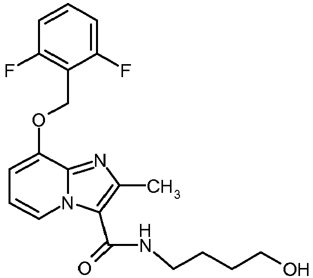 | |
| | (48% d. Th.) | |
| **24** | 8-[(2,6-Difluorbenzyl)oxy]-N-[2-(2-hydroxy-ethoxy)ethyl]-2-methyl-imidazo[1,2-a]pyridin-3-carboxamid | LC-MS (Methode 3): Rₜ = 1.33 min |
| | | MS (ESpos): m/z = 406.2 (M+H)⁺ |
| | 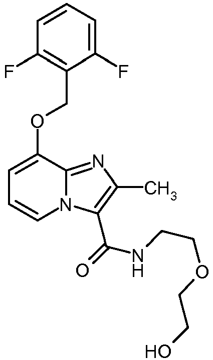 | |
| | (67% d. Th.) | |
| **25** | 8-[(2,6-Difluorbenzyl)oxy]-N-[(2R)-1-hydroxy-4-(methylsulfanyl)butan-2-yl]-2-methyl-imidazo[1,2-a]pyridin-3-carboxamid | LC-MS (Methode 3): Rₜ = 1.55 min |
| | | MS (ESpos): m/z = 436.2 (M+H)⁺ |
| | 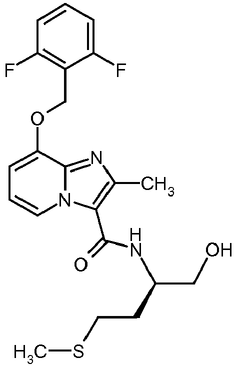 | |
| | (54% d. Th.) | |
| **26** | 8-[(2,6-Difluorbenzyl)oxy]-N-(1,3-dihydroxy-propan-2-yl)-2-methylimidazo[1,2-a]pyridin-3-carboxamid | LC-MS (Methode 1): Rt = 0.61 min |
| | | MS (ESpos): m/z = 392.0 (M+H)⁺ |
| | 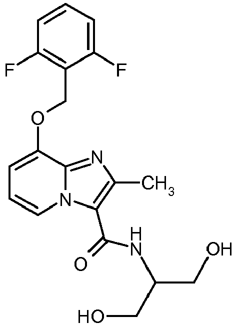 | |
| | (70% d. Th.) | |
| **27** | (rac)-Trans-8-[(2,6-Difluorbenzyl)-oxy]-N-[(2-hydroxycyclo-hexyl)methyl]-2-methylimidazo[1,2-a]pyridin-3-carboxamid | LC-MS (Methode 3): Rt = 1.66 min |
| | 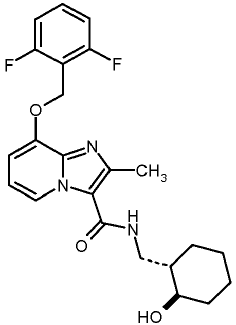 | MS (ESpos): m/z = 430.3 (M+H)⁺ |
| | | ¹H-NMR (400 MHz, DMSO-d₆): |
| | | δ = 0.95 - 1.25 (m, 4 H); 1.35 - 1.48 (m, 1 H); 1.52 - 1.85 (m, 4 H); 2.55 (s, 3 H); 3.18 - 3.22 (m, 1 H); 3.35 - 3.45 (m, 2 H); 4.82 (d, 1 H); 5.30 (s, 2 H); 6.92 (t, 1 H); 7.00 (d, 1 H); 7.21 (t, 2 H); 7.56 (quint, 1 H); 7.81 (t, 1 H); 8.70 (d, 1 H). |
| | (79 % d. Th.) | |
| **28** | (rac)-8-[(2,6-Difluorbenzyl)oxy]-N-(3-hydroxybutyl)-2-methylimidazo[1,2-a]pyridin-3-carboxamid | LC-MS (Methode 2): Rₜ = 0.78 min |
| | 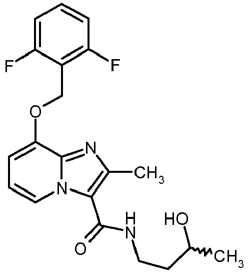 | MS (ESpos): m/z = 390.1 (M+H)⁺ |
| | | ¹H-NMR (400 MHz, DMSO-d₆): δ = 1.11 (d, 3 H); 1.53 - 1.70 (m, 2 H); 2.55 (s, 3 H, durch DMSO-Signal überlagert); 3.38 (q, 2 H); 3.68 - 3.78 (m, 1 H); 4.59 (d, 1 H); 5.30 (s, 2 H); 6.92 (t, 1 H); 7.00 (d, 1 H); 7.23 (t, 2 H); 7.58 (quint, 1 H); 7.83 (t, 1 H); 8.65 (d, 1 H). |
| | (78 % d. Th.) | |
| **29** | 8-[(2,6-Difluorbenzyl)oxy]-N-[(2R)-1-hydroxy-3-methylbutan-2-yl]-2-methylimidazo[1,2-a]pyridin-3-carboxamid | LC-MS (Methode 1): Rₜ = 0.79 min |
| | 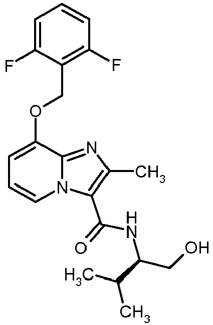 | MS (ESpos): m/z = 404.2 (M+H)⁺ |
| | | ¹H-NMR (400 MHz, DMSO-d₆): δ = 0.92 (d, 3 H), 0.96 (d, 3 H), 1.92 (dq, 1 H); 2.55 (s, 3 H, durch DMSO-Signal überlagert); 3.48 - 3.58 (m, 2 H); 3.85 - 3.95 (m, 1 H); 4.63 (t, 1 H); 5.30 (s, 2 H); 6.92 (t, 1 H); 6.99 (d, 1 H); 7.21 (t, 2 H); 7.47 (d, 1 H), 7.58 (quint, 1 H); 8.54 (d, 1 H). |
| | (81 % d. Th.) | |
| **30** | (rac)-8-[(2,6-Difluorbenzyl)oxy]-N-(2-hydroxybutyl)-2-methylimidazo[1,2-a]pyridin-3-carboxamid | LC-MS (Methode 1): Rₜ = 0.75 min |
| | 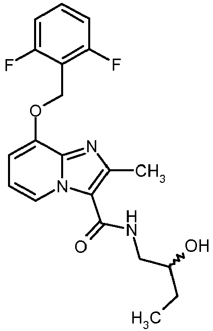 | MS (ESpos): m/z = 390.2 (M+H)⁺ |
| | | ¹H-NMR (400 MHz, DMSO-d₆): δ = 0.90 (d, 3 H), 1.32 (sept., 1 H), 1.40 - 1.55 (m, 1 H), 2.55 (s, 3 H, durch DMSO-Signal überlagert), 3.18 - 3.28 (m, 1 H), 3.33 - 3.40 (m, 1 H), 3.50 - 3.60 (m, 1 H), 4.78 (d, 1 H), 5.30 (s, 2 H), 6.91 (t, 1 H), 6.99 (d, 1 H), 7.21 (t, 2 H), 7.58 (quint, 1 H), 7.70 (t, 1 H), 8.61 (d, 1 H). |
| | (45 % d. Th.) | |
| **31** | 8-[(2,6-Difluorbenzyl)oxy]-N-[1-(hydroxymethyl)cyclopentyl]-2-methylimidazo[1,2-a]pyridin-3-carboxamid | LC-MS (Methode 1): Rₜ = 0.82 min |
| | 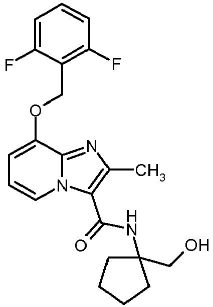 | MS (ESpos): m/z = 416.2 (M+H)⁺ |
| | | ¹H-NMR (400 MHz, DMSO-d₆): δ = 1.52 - 1.68 (m, 2 H), 1.69 - 1.78 (m, 4 H), 1.98 - 2.08 (m, 2 H), 2.55 (s, 3 H, durch DMSO-Signal überlagert), 3.61 (d, 2 H), 4.90 (t, 1 H), 5.30 (s, 2 H), 6.89 (t, 1 H), 6.95 (d, 1 H), 7.21 (t, 2 H), 7.35 (s, 1 H), 7.58 (quint, 1 H), 8.51 (d, 1 H). |
| | (67 % d. Th.) | |
| **32** | 8-[(2,6-Difluorbenzyl)oxy]-N-[(2S)-2-hydroxy-2-phenylethyl]-2-methylimidazo[1,2-a]pyridin-3-carboxamid | LC-MS (Methode 3): Rₜ = 1.67 min |
| | 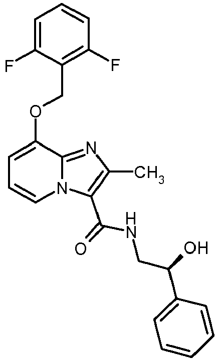 | MS (ESpos): m/z = 438.2 (M+H)⁺ |
| | | ¹H-NMR (400 MHz, DMSO-d₆): δ = 2.40 (s, 3 H, durch DMSO-Signal überlagert), 3.40 - 3.48 (m, 1 H), 3.50 - 3.60 (m, 1 H), 4.80 (dt, 1 H), 5.30 (s, 2 H), 5.59 (d, 1 H), 6.90 (t, 1 H), 7.00 (d, 1 H), 7.20 - 7.28 (m, 3 H), 7.31 (t, 2 H), 7.39 (d, 2 H), 7.58 (quint, 1 H), 7.79 (t, 1 H), 8.61 (d, 1 H). |
| | (61 % d. Th.) | |
| **33** | 8-[(2,6-Difluorbenzyl)oxy]-N-[(2R)-2-hydroxy-2-phenylethyl]-2-methylimidazo[1,2-a]pyridin-3-carboxamid | LC-MS (Methode 1): Rₜ = 0.85 min |
| | 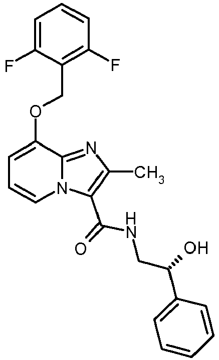 | MS (ESpos): m/z = 438.2 (M+H)⁺ |
| | | ¹H-NMR (400 MHz, DMSO-d₆): δ = 2.40 (s, 3 H, durch DMSO-Signal überlagert), 3.40 - 3.48 (m, 1 H), 3.50 - 3.60 (m, 1 H), 4.80 (dt, 1 H), 5.30 (s, 2 H), 5.59 (d, 1 H), 6.90 (t, 1 H), 7.00 (d, 1 H), 7.20 - 7.28 (m, 3 H), 7.31 (t, 2 H), 7.39 (d, 2 H), 7.58 (quint, 1 H), 7.79 (t, 1 H), 8.61 (d, 1 H). |
| | (43 % d. Th.) | |
| **34** | 8-[(2,6-Difluorbenzyl)oxy]-N-(1-hydroxy-2-methylpropan-2-yl)-2-methylimidazo[1,2-a]pyridin-3-carboxamid | LC-MS (Methode 3): Rₜ = 1.49 min |
| | 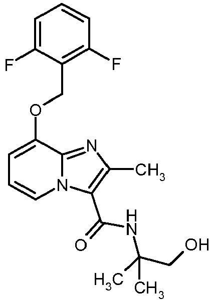 | MS (ESpos): m/z = 390.1 (M+H)⁺ |
| | | ¹H-NMR (400 MHz, DMSO-d₆): δ = 1.38 (s, 6 H), 2.56 (s, 3 H), 3.51 (d, 2 H), 5.00 (t, 1 H), 5.30 (s, 2 H), 6.90 (t, 1 H), 6.99 (d, 1 H), 7.15 (s, 1 H), 7.23 (t, 2 H), 7.59 (quint, 1 H), 8.60 (d, 1 H). |
| | (30 % d. Th.) | |
| **35** | 8-[(2,6-Difluorbenzyl)oxy]-N-[(2R)-1-hydroxy-3,3-dimethylbutan-2-yl]-2-methylimidazo[1,2-a]pyridin-3-carboxamid | LC-MS (Methode 1): Rₜ = 0.84 min |
| | 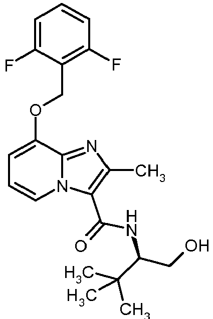 | MS (ESpos): m/z = 418.3 (M+H)⁺ |
| | | ¹H-NMR (400 MHz, DMSO-d₆): δ = 0.99 (s, 9 H), 2.56 (s, 3 H), 3.45 - 3.53 (m, 1 H), 3.68 - 3.73 (m, 1 H), 3.95 (dt, 1 H), 4.55 (t, 1 H), 5.30 (s, 2 H), 6.90 (t, 1 H), 6.99 (d, 1 H), 7.21 (t, 2 H), 7.44 (d, 1 H), 7.59 (quint, 1 H), 8.50 (d, 1 H). |
| | (51 % d. Th.) | |
| **36** | 8-[(2,6-Difluorbenzyl)oxy]-N-[(1R,2R)-2-hydroxycyclopentyl]-2-methylimidazo[1,2-a]pyridin-3-carboxamid | LC-MS (Methode 1): Rₜ = 0.72 min |
| | 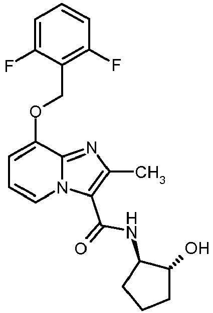 | MS (ESpos): m/z = 402.3 (M+H)⁺ |
| | | 1H-NMR (400 MHz, DMSO-d₆): δ = 1.43 - 1.57 (m, 2 H), 1.60 - 1.76 (m, 2 H), 1.81 - 1.95 (m, 1 H), 2.01 - 2.12 (m, 1 H), 2.53 (s, 3 H; überlagert durch DMSO-Signal), 3.95 - 4.10 (m, 2 H), 4.80 (d, 1 H), 5.30 (s, 2 H), 6.90 (t, 1 H), 6.99 (d, 1 H), 7.21 (t, 2 H), 7.59 (quint, 1 H), 7.72 (d, 1 H), 8.50 (d, 1 H). |
| | (33 % d. Th.) | |
| **37** | 8-[(2,6-Difluorbenzyl)oxy]-N-[(1R,2S)-2-hydroxycyclopentyl]-2-methylimidazo[1,2-a]pyridin-3-carboxamid | LC-MS (Methode 3): Rₜ = 1.47 min |
| | 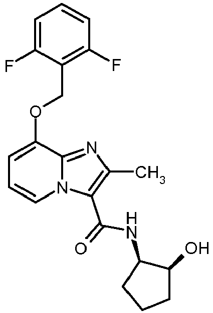 | MS (ESpos): m/z = 402.2 (M+H)⁺ |
| | | 1H-NMR (400 MHz, DMSO-d₆): δ = 1.43 - 1.57 (m, 1 H), 1.60 - 1.70 (m, 2 H), 1.70 - 1.90 (m, 2 H), 1.91 - 2.00 (m, 1 H), 2.53 (s, 3 H; überlagert durch DMSO-Signal), 4.08 - 4.18 (m, 2 H), 4.96 (d, 1 H), 5.30 (s, 2 H), 6.91 (t, 1 H), 7.00 (d, 1 H), 7.20 - 7.25 (m, 3 H), 7.59 (quint, 1 H), 8.72 (d, 1 H). |
| | (49 % d. Th.) | |
| **38** | 8-[(2,6-Difluorbenzyl)oxy]-N-[(2R)-1-hydroxy-3-phenylpropan-2-yl]-2-methylimidazo[1,2-a]pyridin-3-carboxamid | LC-MS (Methode 3): Rₜ = 1.67 min |
| | 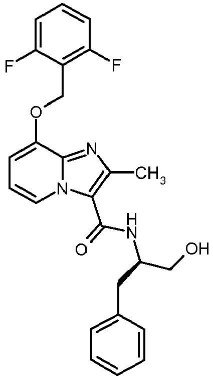 | MS (ESpos): m/z = 452.2 (M+H)⁺ |
| | | 1H-NMR (400 MHz, DMSO-d₆): δ = 2.34 (s, 3 H), 2.79 (dd, 1 H), 3.00 (dd, 1 H), 3.45 - 3.60 (m, 2 H), 4.20 - 4.30 (m, 1 H), 4.89 (t, 1 H), 5.29 (s, 2 H), 6.87 (t, 1 H), 6.97 (d, 1 H), 7.15 - 7.30 (m, 7 H), 7. 53 - 7.64 (m, 2 H), 8.39 (d, 1 H). |
| | (67 % d. Th.) | |

### Beispiel 39

### 8-[(2,6-Difluorbenzyl)oxy]-N-[(2R)-1-hydroxy-4-methylpentan-2-yl]-2-methylimidazo[1,2-a]pyridin-3-carboxamid

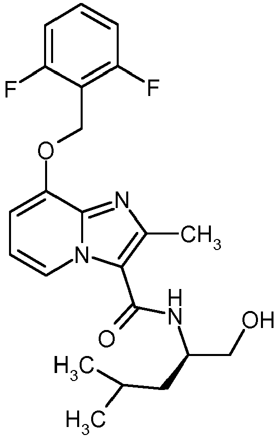

31 mg (0.1 mmol) 8-(Benzyloxy)-2-methylimidazo[1,2-a]pyridin-3-carbonsäure, 14 mg (0.12 mmol, 1.2 Äquivalente) (2R)-2-Amino-4-methylpentan-1-ol, 41 mg TBTU (0.13 mmol, 1.3 Äquivalente) und 20.2 mg (0.2 mmol, 2 Äquivalente) 4-Methylmorpholin wurden in 400 µL DMF vorgelegt und bei RT über Nacht gerührt. Die Zielverbindung wurde per präparativer HPLC (Methode 11) isoliert. Es wurden 26 mg (55% d. Th.) erhalten.
LC-MS (Methode 12): Rₜ = 0.89 min
MS (ESpos): m/z = 418.12 (M+H)⁺

In Analogie zu Beispiel 39 wurden die in Tabelle 3 gezeigten Beispielverbindungen hergestellt, indem 8-[(2,6-Difluorobenzyl)oxy]-2-methylimidazo[1,2-a]pyridin-3-carbonsäure mit den entsprechenden, kommerziell erhältlichen Aminen unter den beschriebenen Bedingungen umgesetzt wurde:

**Tabelle 3:**

| **Beispiel** | **IUPAC**-**Name / Struktur (Ausbeute)** | **Analytische Daten** |
|---|---|---|
| **40** | 8-[(2,6-Difluorbenzyl)oxy]-N-[(1S,2R)-2-hydroxy-2,3-dihydro-1H-inden-1-yl]-2-methylimidazo[1,2-a]pyridin-3-carboxamid | LC-MS (Methode 12): Rₜ = 0.95 min |
| | | MS (ESpos): m/z = 450.20 (M+H)⁺ |
| | 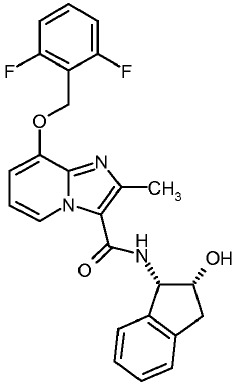 | |
| | (82% d. Th.) | |
| **41** | 8-[(2,6-Difluorbenzyl)oxy]-N-(3-hydroxy-2,2-dimethylpropyl)-2-methylimidazo[1,2-a]pyridin-3-carboxamid | LC-MS (Methode 12): Rₜ = 0.86 min |
| | | MS (ESpos): m/z = 404.14 (M+H)⁺ |
| | 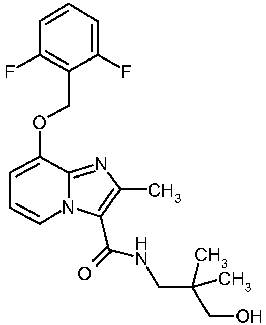 | |
| | (30% d. Th.) | |
| **42** | (rac)-8-[(2,6-Difluorbenzyl)oxy]-N-(trans-4-hydroxycyclohexyl)-2-methylimidazo[1,2-a]pyridin-3-carboxamid | LC-MS (Methode 12): Rₜ = 0.78 min |
| | | MS (ESpos): m/z = 416.20 (M+H)⁺ |
| | 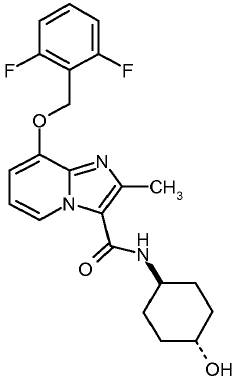 | |
| | (56% d. Th.) | |

### Beispiel 43

### 8-[(2,6-Difluorbenzyl)oxy]-N-[(2R)-1-hydroxybutan-2-yl]-2-methylimidazo[1,2-a]pyridin-3-carboxamid

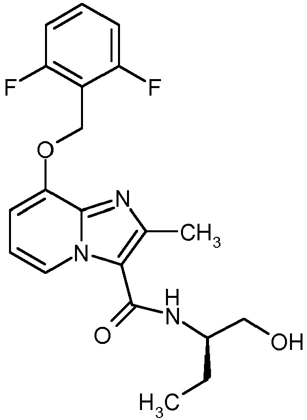

50 mg (0.190 mmol) 8-Hydroxy-N-[(2R)-1-hydroxybutan-2-yl]-2-methylimidazo[1,2-a]pyridin-3-carboxamid, 43 mg (0.209 mmol) 2,6-Difluorbenzylbromid, 58 mg (0.418 mmol) Kaliumcarbonat und 16 mg (0.095 mmol) Kaliumiodid wurden mit 1.3 ml 1-Methyl-2-pyrrolidon versetzt und es wurde in der Mikrowelle für 40 min bei 120°C erhitzt. Danach wurde vom Feststoff abdekantiert und dieser noch zweimal mit Methanol gewaschen. Die vereinigten Lösungen wurden im Hochvakuum bei 60°C zur Trockene eingeengt und der entstandene Rückstand wurde über präparative HPLC aufgereinigt (Methode 7). Man erhielt 52 mg (69% d. Th.) 8-[(2,6-Difluorbenzyl)oxy]-N-[(2R)-1-hydroxybutan-2-yl]-2-methylimidazo[1,2-a]pyridin-3-carboxamid.
LC-MS (Methode 1): Rₜ = 0.73 min
MS (ESpos): m/z = 390.1 (M+H)⁺
¹H-NMR (400 MHz, DMSO-d₆): δ = 0.94 (t, 3H), 1.46-1.50 (m, 1H), 1.66-1.70 (m, 1H), 2.53 (s, 3H), 3.47 (m, 2H), 3.87-3.90 (m, 1H), 4.72 (t, 1H), 5.30 (s, 2H), 6.90 (t, 1H). 6.97 (d, 1H). 7.22 (t, 2H). 7.48 (d, 1H). 7.53-7.58 (m, 1H). 8.53 (d, 1H).

Die in Tabelle 4 gezeigten Ausführungsbeispiele wurden ausgehend von 8-Hydroxy-N-[(2R)-1-hydroxybutan-2-yl]-2-methylimidazo[1,2-a]pyridin-3-carboxamid durch Umsetzung mit den entsprechenden, kommerziell erhältlichen Benzylbromiden in Analogie zu Beispielverbindung 43 hergestellt.

**Tabelle 4:**

| **Beispiel** | **IUPAC-Name / Struktur (Ausbeute)** | **Analytische Daten** |
|---|---|---|
| **44** | 8-[(2,3-Difluorbenzyl)oxy]-N-[(2R)-1-hydroxybutan-2-yl]-2-methylimidazo[1,2-a]pyridin-3-carboxamid | LC-MS (Methode 1): Rₜ = 0.76 min |
| | | MS (ESpos): m/z = 390.1 (M+H)⁺ |
| | 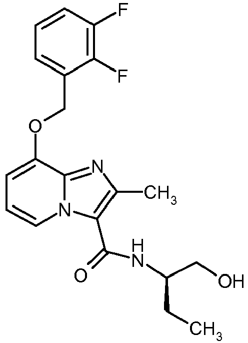 | ¹H-NMR (400 MHz, DMSO-d₆): δ = 0.94 (t, 3H), 1.40-1.54 (m, 1H), 1.61-1.75 (m, 1H), 2.53 (s, 3H), 3.39-3.55 (m, 2H), 3.85-3.96 (m, 1H), 4.72 (t, 1H), 5.39 (s, 2H), 6.90 (t, 1H). 6.96 (d, 1H). 7.24-7.33 (m, 1H). 7.41-7.54 (m, 3H), 8.53 (d, 1H). |
| | (64% d. Th.) | |
| **45** | 8-[(2,3-Dichlorbenzyl)oxy]-N-[(2R)-1-hydroxybutan-2-yl]-2-methylimidazo[1,2-a]pyridin-3-carboxamid | LC-MS (Methode 1): Rₜ = 0.88 min |
| | | MS (ESpos): m/z = 422.0 (M+H)⁺ |
| | 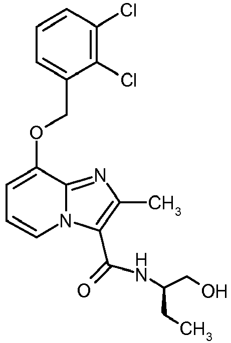 | ¹H-NMR (400 MHz, DMSO-d₆): δ = 0.94 (t, 3H), 1.40-7.54 (m, 1H), 1.62-1.75 (m, 1H), 2.53 (s, 3H), 3.40-3.55 (m, 2H), 3.85-3.96 (m, 1H), 4.75 (t, 1H), 5.40 (s, 2H), 6.87-6.96 (m, 2H). 7.46 (t, 1H). 7.53 (d, 1H). 7.65 (d, 1H), 7.70 (d, 1H), 8.53 (d, 1H). |
| | (61% d. Th.) | |

### Beispiel 46

### 8-(Benzyloxy)-N-[(2S)-1-hydroxypropan-2-yl]-2-methylimidazo[1,2-a]pyridin-3-carboxamid

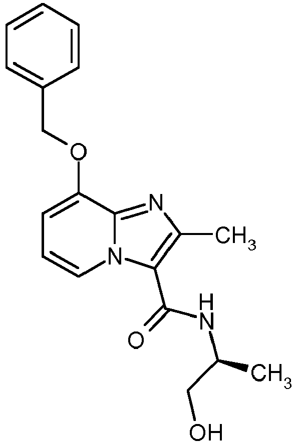

Die Herstellung erfolgte in Analogie zu Beispiel 16 ausgehend von 2.00 g (7.09 mmol) 8-(Benzyloxy)-2-methylimidazo[1,2-a]pyridin-3-carbonsäure und 0.53 g (7.09 mmol) L-Alaninol. Man erhielt 1.65 g (69% d. Th.) der Titelverbindung.
LC-MS (Methode 1): Rₜ = 0.66 min
MS (ESpos): m/z = 340.1 (M+H)⁺

Die in Tabelle 5 gezeigten Ausführungsbeispiele wurden ausgehend von 8-Hydroxy-N-[2-(1-hydroxycyclopentyl)ethyl]-2-methylimidazo[1,2-a]pyridin-3-carboxamid durch Umsetzung mit den entsprechenden, kommerziell erhältlichen Benzylbromiden hergestellt.

**Tabelle 5:**

| **Beispiel** | **IUPAC-Name / Struktur (Ausbeute)** | **Analytische Daten** |
|---|---|---|
| **47** | 8-[(2,3-Dichlorbenzyl)oxy]-N-[2-(1-hydroxycyclopentyl)ethyl]-2-methylimidazo[1,2-a]pyridin-3-carboxamid | LC-MS (Methode 1): Rₜ = 0.98 min |
| | 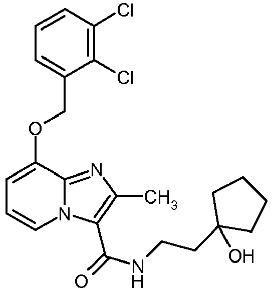 | MS (ESpos): m/z = 462.1 (M+H)⁺ |
| | (36% d. Th.) | |
| **48** | 8-(Benzyloxy)-N-[2-(1-hydroxycyclopentyl)ethyl]-2-methylimidazo[1,2-a]pyridin-3-carboxamid | LC-MS (Methode 1): Rₜ = 0.82 min |
| | 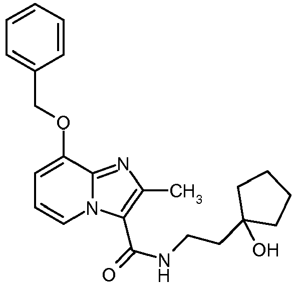 | MS (ESpos): m/z = 394.2 (M+H)⁺ |
| | (37% d. Th.) | |

Die in Tabelle 6 gezeigten weiteren Beispielverbindungen wurden ausgehend von N-(1,3-Dihydroxypropan-2-yl)-8-hydroxy-2-methylimidazo[1,2-a]pyridin-3-carboxamid durch Umsetzung mit den entsprechenden, kommerziell erhältlichen Benzylbromiden hergestellt.

**Tabelle 6:**

| **Beispiel** | **IUPAC-Name / Struktur (Ausbeute)** | **Analytische Daten** |
|---|---|---|
| **49** | 8-[(2,3-Dichlorbenzyl)oxy]-N-(1,3-dihydroxypropan-2-yl)-2-methylimidazo[1,2-a]pyridin-3-carboxamid | LC-MS (Methode 1): Rₜ = 0.77 min |
| | 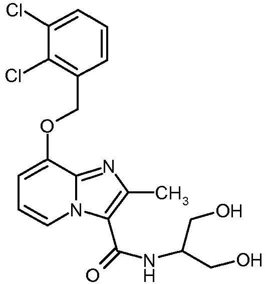 | MS (ESpos): m/z = 424.0 (M+H)⁺ |
| | (49% d. Th.) | |
| **50** | 8-[(2-Chlorbenzyl)oxy]-N-(1,3-dihydroxypropan-2-yl)-2-methylimidazo[1,2-a]pyridin-3-carboxamid | LC-MS (Methode 4): Rₜ = 0.74 min |
| | 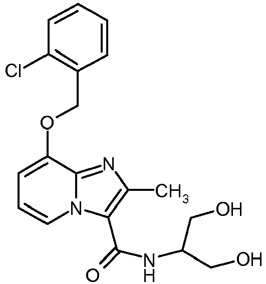 | MS (ESneg): m/z = 388.0 (M+H)⁺ |
| | (9% d. Th.) od. (36% d. Th.) | |
| **51** | N-(1,3-Dihydroxypropan-2-yl)-8-[(2-fluorbenzyl)oxy]-2-methylimidazo[1,2-a]pyridin-3-carboxamid | LC-MS (Methode 1): Rₜ = 0.61 min |
| | 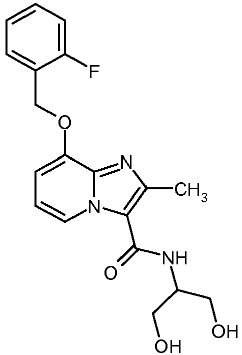 | MS (ESpos): m/z = 374.0 (M+H)⁺ |
| | (11% d. Th.) od. (47% d. Th.) | |
| **52** | 8-[(2-Chlor-3-methylbenzyl)oxy]-N-(1,3-dihydroxypropan-2-yl)-2-methylimidazo[1,2-a]pyridin-3-carboxamid | LC-MS (Methode 4): Rₜ = 0.80 min |
| | 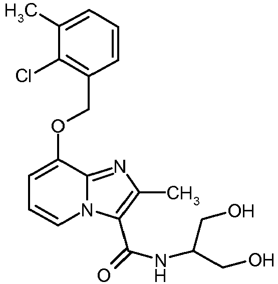 | MS (ESpos): m/z = 404.0 (M+H)⁺ |
| | (12% d. Th.) | |
| **53** | 8-[(2,6-Dichlorbenzyl)oxy]-N-(1,3-dihydroxypropan-2-yl)-2-methylimidazo[1,2-a]pyridin-3-carboxamid | LC-MS (Methode 4): Rₜ = 0.79 min |
| | 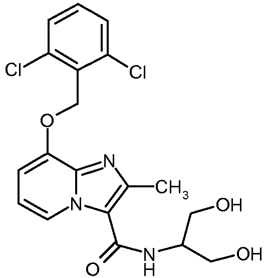 | MS (ESpos): m/z = 424.0 (M+H)⁺ |
| | (31% d. Th.) | |
| **54** | 8-[(2-Brombenzyl)oxy]-N-(1,3-dihydroxypropan-2-yl)-2-methylimidazo[1,2-a]pyridin-3-carboxamid | LC-MS (Methode 4): Rₜ = 0.76 min |
| | 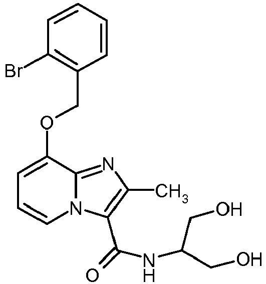 | MS (ESpos): m/z = 435.1 (M+H)⁺ |
| | (23% d. Th.) | |

### Beispiel 55

### 8-(Cyclohexylmethoxy)-2-ethyl-N-[(2R)-1-hydroxypropan-2-yl]imidazo[1,2-a]pyridin-3-carboxamid Ameisensäuresalz

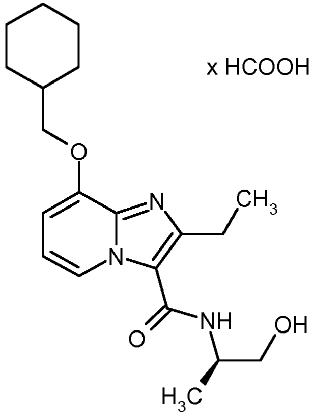

Eine Lösung von 12 mg (0.040 mmol) 8-(Cyclohexylmethoxy)-2-ethylimidazo[1,2-a]pyridin-3-carbonsäure in 750 µl DMSO wurde mit 3.3 mg (0.044 mmol) L-Alaninol, 14 mg (0.044 mmol) TBTU und 20 mg (0.198 mmol) N-Methylmorpholin versetzt und anschließend über Nacht bei Raumtemperatur geschüttelt. Anschließend wurde über präparative HPLC (Methode 6) aufgereinigt und man erhielt 12 mg (84% d.Th.) 8-(Cyclohexylmethoxy)-2-ethyl-N-[(2R)-1-hydroxypropan-2-yl]imidazo[1,2-a]pyridin-3-carboxamid als Ameisensäuresalz.
LC-MS (Methode 1): Rₜ = 0.82 min
MS (ESpos): m/z = 360.2 (M+H)⁺
¹H-NMR (400 MHz, DMSO-d₆): δ = 1.02 - 1.16 (m, 2 H), 1.19 (d, 3 H), 1.22 - 1.34 (m, 3 H), 1.24 (t, 3 H), 1.65 - 1.75 (m, 3 H), 1.85 - 1.89 (m, 3 H), 2.50 (teilweise durch DMSO-Signal überdeckt, s, 3 H), 2.91 (q, 2 H), 3.40 (dd, 1 H), 3.49 (dd, 1 H), 3.95 (d, 2 H), 4.05 (quint, 1 H), 6.76 (d, 1 H), 6.85 (t, 1 H), 7.60 (d, 1 H), 8.15 (br. s, 1 H, Ameisensäure); 8.44 (d, 1 H).

Die in der folgenden Tabelle 7 zusammengefassten weiteren Synthesebeispiele wurden in Analogie zu Beispiel 55 sowie der allgemeinen Arbeitsvorschrift 1 durch Umsetzung der entsprechenden Carbonsäure (siehe Syntheseintermediate weiter oben) mit den entsprechenden, kommerziell erhältlichen Aminen unter TBTU-Bedingungen hergestellt.

**Tabelle 7:**

| **Beispiel** | **IUPAC-Name / Struktur (Ausbeute)** | **Analytische Daten** |
|---|---|---|
| **56** | 8-(Cyclohexylmethoxy)-N-[(2S)-1-hydroxypropan-2-yl]-2-(trifluoromethyl)imidazo[1,2-a]pyridin-3-carboxamid | LC-MS (Methode 1): Rₜ = 1.09 min |
| | | MS (ESpos): m/z = 400.2 (M+H)⁺ |
| | | ¹H-NMR (400 MHz, DMSO-d₆): |
| | 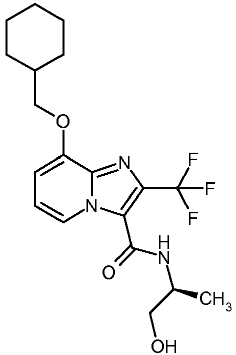 | δ = 1.02 - 1.33 (m, 6 H), 1.14 (d, 3 H), 1.65 - 1.71 (m, 3 H), 1.85 - 1.88 (m, 3 H), 3.33 - 3.41 (m 1 H), 3.43 - 3.49 (m, 1 H), 4.00 (d, 2 H), 4.04 (quint, 1 H), 4.85 (t, 1 H), 6.91 (d, 1 H), 7.04 (t, 1 H), 8.09 (d, 1 H), 8.60 (d, 1 H). |
| | (45% d. Th.) | |
| **57** | 8-(Cyclohexylmethoxy)-N-[(2S)-1-hydroxypropan-2-yl]-2-propylimidazo[1,2-a]pyridin-3-carboxamid | LC-MS (Methode 1): Rₜ = 0.85 min |
| | | MS (ESpos): m/z = 374.2 (M+H)⁺ |
| | | ¹H-NMR (400 MHz, DMSO-d₆): |
| | 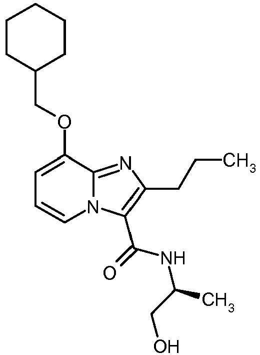 | δ = 0.90 (t, 3 H), 1.04 - 1.10 (m, 2 H), 1.16 (d, 3 H), 1.20 - 1.30 (m, 3 H), 1.65 - 1.75 (m, 5 H), 1.84 - 1.88 (m, 3 H), 2.87 (t, 2 H), 3.35 - 3.42 (m 1 H), 3.45 - 3.53 (m, 1 H), 3.95 (d, 2 H), 4.05 (quint, 1 H), 4.80 (br. s, 1 H), 6.75 (d, 1 H), 6.85 (t, 1 H), 7.63 (d, 1 H), 8.42 (d, 1 H). |
| | (58% d. Th.) | |
| **58** | 8-(Cyclobutylmethoxy)-2-ethyl-N-[(2S)-1-hydroxypropan-2-yl]imidazo[1,2-a]pyridin-3-carboxamid Ameisensäuresalz | LC-MS (Methode 1): Rₜ = 0.68 min |
| | | MS (ESpos): m/z = 332.1 (M+H)⁺ |
| | | ¹H-NMR (400 MHz, DMSO-d₆): |
| | 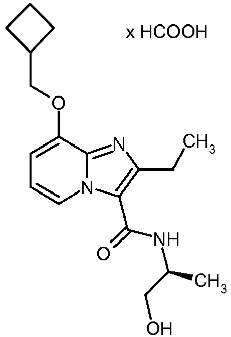 | δ = 1.16 (d, 3 H), 1.24 (t, 3 H), 1.83 - 1.96 (m, 4 H), 2.08 - 2.16 (m, 2 H), 2.81 (quint, 1 H), 2.91 (q, 2 H), 3.40 (dd, 1 H), 3.49 (dd, 1 H), 4.05 (quint, 1 H), 4.14 (d, 2 H), 4.80 (br, 1 H), 6.78 (d, 1 H), 6.85 (t, 1 H), 7.60 (d, 1 H), 8.18 (br. s, Ameisensäure), 8.45 (d, 1 H). |
| | (32% d. Th.) | |
| **59** | 8-(Cyclohexylmethoxy)-N-[(2S)-1-hydroxypropan-2-yl]imidazo[1,2-a]pyridin-3-carboxamid | LC-MS (Methode 3): Rₜ = 1.69 min |
| | | MS (ESpos): m/z = 332.3 (M+H)⁺ |
| | 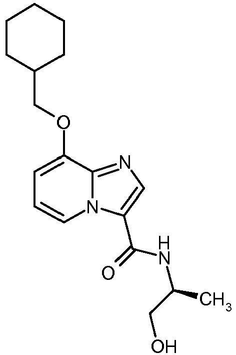 | |
| | (67% d. Th.) | |
| **60** | 8-[(2,6-Difluorbenzyl)oxy]-6-ethyl-N-[(2R)-1-hydroxyhexan-2-yl]-2-methylimidazo[1,2-a]pyridin-3-carboxamid | LC-MS (Methode 1): Rₜ = 0.94 min |
| | 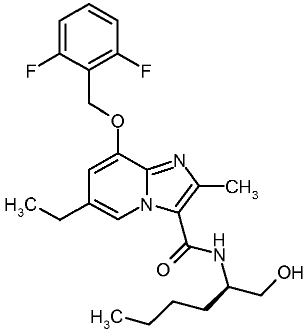 | MS (ESpos): m/z = 446.3 (M+H)+ |
| | | ¹H-NMR (400 MHz, DMSO-d6): |
| | | δ = 0.89 (t, 3 H), 1.20 (t, 3 H), 1.25 - 1.40 (m, 4 H), 1.40 - 1.52 (m, 1 H), 1.60 -1.70 (m, 1 H), 2.55 (s, 3 H), 2.65 (q, 2 H), 3.45 - 3.53 (m, 2 H), 3.94 - 4.05 (m, 1 H), 4.71 (t, 1 H), 5.30 (s, 2 H), 6.90 (s, 1 H), 7.21 (t, 2 H), 7.48 (d, 1 H), 7.59 (quint, 1 H), 8.40 (s, 1 H). |
| | (55% d. Th.) | |
| **61** | 6-Cyclopropyl-8-[(2,6-difluorbenzyl)oxy]-N-[(2R)-1-hydroxyhexan-2-yl]-2-methylimidazo[1,2-a]pyridin-3-carboxamid | LC-MS (Methode 2): Rₜ = 1.08 min |
| | 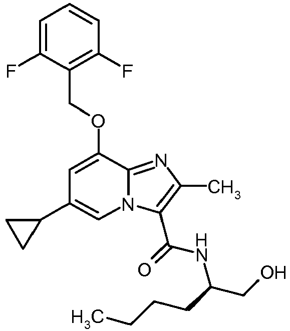 | MS (ESpos): m/z = 458.1 (M+H)+ |
| | | ¹H-NMR (400 MHz, DMSO-d₆): |
| | | δ = 0.72 (q, 2 H), 0.89 (t, 3 H), 0.95 (q, 2 H), 1.22-1.40 (m, 4 H), 1.40 - 1.52 (m, 1 H), 1.58 - 1.70 (m, 1 H), 1.98 (quint., 1 H), 2.47 (s, 3 H), 3.40 - 3.51 (m, 2 H), 3.94 - 4.02 (m, 1 H), 4.71 (t, 1 H), 5.30 (s, 2 H), 6.65 (s, 1 H), 7.21 (t, 2 H), 7.48 (d, 1 H), 7.59 (quint, 1 H), 8.40 (s, 1 H). |
| | (70% d. Th.) | |
| **62** | 8-[(2,6-Difluorbenzyl)oxy]-N-[(2R)-1-hydroxyhexan-2-yl]imidazo[1,2-a]pyridin-3-carboxamid | LC-MS (Methode 3): Rₜ = 1.92 min |
| | 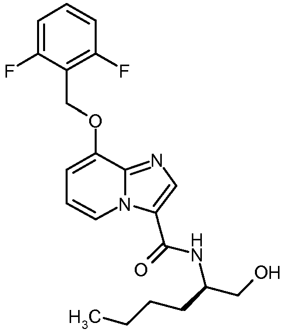 | MS (ESpos): m/z = 404.2 (M+H)+ |
| | | ¹H-NMR (400 MHz, DMSO-d₆): |
| | | δ = 0.84 (t, 3 H), 1.22-1.40 (m, 4 H), 1.40 - 1.52 (m, 1 H), 1.58 - 1.70 (m, 1 H), 3.38 - 3.51 (m, 2 H), 3 .91 - 4.02 (m, 1 H), 4.71 (t, 1 H), 5.30 (s, 2 H), 7.00 (t, 1 H), 7.08 (d, 1 H), 7.21 (t, 2 H), 7.59 (quint, 1 H), 8.02 (d, 1 H), 8.25 (s, 1 H), 9.12 (d, 1 H). |
| | (59% d. Th.) | |
| **63** | 8-[(2,6-Difluorbenzyl)oxy]-N-[(2R)-1-hydroxyhexan-2-yl]-2-methyl-6-(morpholin-4-yl)imidazo[1,2-a]pyridin-3-carboxamid | LC-MS (Methode 1): Rₜ = 0.88 min |
| | 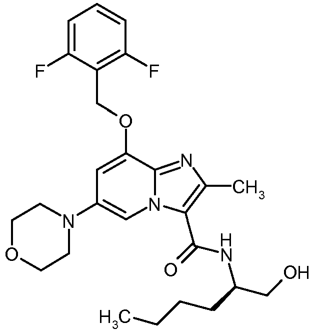 | MS (ESpos): m/z = 503.3 (M+H)+ |
| | | ¹H-NMR (400 MHz, DMSO-d₆): |
| | | δ = 0.89 (t, 3 H), 1.22 - 1.35 (m, 4 H), 1.40 - 1.52 (m, 1 H), 1.60 - 1.70 (m, 1 H), 2.48 (s, 3 H), 3.03 - 3.07 (m, 4 H), 3.38 - 3.52 (m, 2 H), 3.72 - 3.80 (m, 4 H), 3.94 - 4.01 (m, 1 H), 4.71 (t, 1 H), 5.30 (s, 2 H), 7.00 (s, 1 H), 7.21 (t, 2 H), 7.38 (d, 1 H), 7.59 (quint, 1 H), 8.09 (s, 1 H). |
| | (55% d. Th.) | |
| **64** | 8-[(2,6-Difluorbenzyl)oxy]-N-[(2R)-1-hydroxyhexan-2-yl]-2-methyl-6-(pyrrolidin-1-yl)imidazo[1,2-a]pyridin-3-carboxamid | LC-MS (Methode 1): Rₜ = 0.97 min |
| | 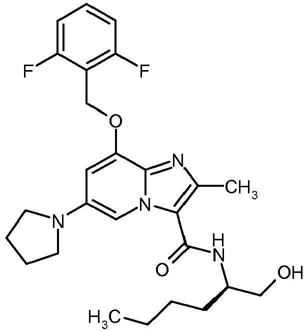 | MS (ESpos): m/z = 487.3 (M+H)+ |
| | | 1H-NMR (400 MHz, DMSO-d₆): δ = 0.87 (t, 3 H), 1.22 - 1.35 (m, 4 H), 1.40 - 1.52 (m, 1 H), 1.60 - 1.70 (m, 1 H), 1.96 - 2.03 (m, 4 H), 2.42 (s, 3 H), 3.20 - 3.28 (m, 4 H), 3.38 - 3.52 (m, 2 H), 3.91 - 4.01 (m, 1 H), 4.71 (t, 1 H), 5.32 (s, 2 H), 6.70 (s, 1 H), 7.21 (t, 2 H), 7.23 (d, 1 H), 7.59 (quint, 1 H), 7.81 (s, 1 H). |
| | (30% d. Th.) | |
| **65** | 6-Brom-8-[(2,6-difluorbenzyl)oxy]-N-[(2R)-1-hydroxyhexan-2-yl]-2-methylimidazo[1,2-a]pyridin-3-carboxamid | LC-MS (Methode 1): Rₜ = 1.13 min |
| | 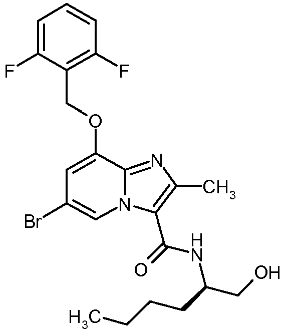 | MS (ESpos): m/z = 496.2/498.2 (M+H)+ |
| | | 1H-NMR (400 MHz, DMSO-d₆): |
| | | δ = 0.87 (t, 3 H), 1.22 - 1.35 (m, 4 H), 1.40 - 1.52 (m, 1 H), 1.60 - 1.70 (m, 1 H), 2.52 (s, 3 H; verdeckt durch DMSO-Signal), 3.38 - 3.52 (m, 2 H), 3.91 - 4.01 (m, 1 H), 4.75 (t, 1 H), |
| | (84% d. Th.) | 5.32 (s, 2 H), 7.19 - 7.25 (m, 3 H), 7.55 - 7.65 (m, 2 H), 8.71 (s, 1 H). |

Die in der folgenden Tabelle 8 zusammengefassten Ausführungsbeispiele wurden durch Umsetzung von 8-(Cyclohexylmethoxy)-2-methylimidazo[1,2-a]pyridin-3-carbonsäure mit den entsprechenden, kommerziell erhältlichen Aminen unter TBTU-Bedingungen (siehe auch allgemeine Arbeitsvorschrift 1) hergestellt.

**Tabelle 8:**

| **Beispiel** | **IUPAC**-**Name / Struktur (Ausbeute)** | **Analytische Daten** |
|---|---|---|
| **66** | 8-(Cyclohexylmethoxy)-N-[(2R)-1-hydroxyhexan-2-yl]-2-methylimidazo[1,2-a]pyridin-3-carboxamid | LC-MS (Methode 1): Rₜ = 0.94 min |
| | 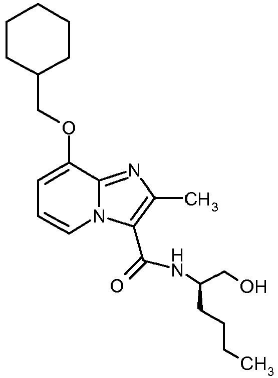 | MS (ESpos): m/z = 388.2 (M+H)⁺ |
| | (72% d. Th.) | |
| **67** | 8-(Cyclohexylmethoxy)-N-[(2R)-1-hydroxypentan-2-yl]-2-methylimidazo[1,2-a]pyridin-3-carboxamid | LC-MS (Methode 3): Rₜ = 1.69 min |
| | 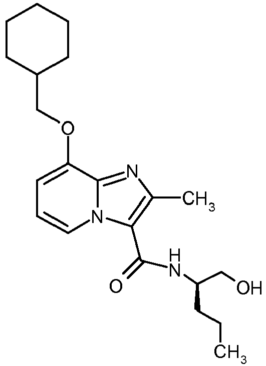 | MS (ESpos): m/z = 374.2 (M+H)⁺ |
| | (72% d. Th.) | |
| **68** | 8-(Cyclohexylmethoxy)-N-[(2S)-2,3-dihydroxypropyl]-2-methylimidazo[1,2-a]pyridin-3-carboxamid | LC-MS (Methode 3): Rₜ = 1.43 min |
| | | MS (ESpos): m/z = 362.1 (M+H)⁺ |
| | 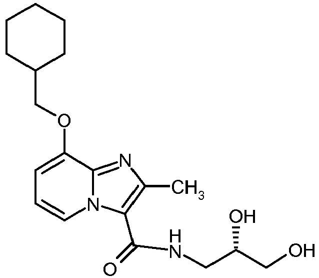 | |
| | (75% d. Th.) | |
| **69** | (rac)-8-(Cyclohexylmethoxy)-N-(1-hydroxybutan-2-yl)-2-methylimidazo[1,2-a]pyridin-3-carboxamid-Formiat | LC-MS (Methode 3): Rₜ = 1.62 min |
| | | MS (ESpos): m/z = 360.2 (M+H-HCOOH)⁺ |
| | 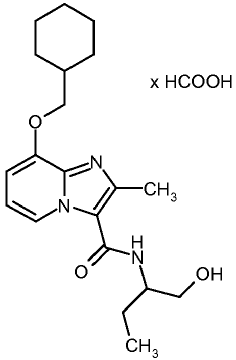 | ¹H-NMR (400 MHz, DMSO-d₆): δ = 0.92 (t, 3H), 1.0-1.15 (m, 2H), 1.15-1.36 (m, 3H), 1.4-1.55 (m, 1H), 1.6-1.78 (m, 4H), 1.8-1.92 (m, 3H), 2.52 (s, teilweise durch DMSO-Signal verborgen), 3.45, 3.50 (2m, 2x1H), 3.87-3.94 (m, 1H), 3.92 (d, 1H), 4.76-4.85 (br m, 1H), 6.75 (d, 1H), 6.83 (t, 1H), 7.48 (d, 1H), 8.12 (s, 1H), 8.48 (d, 1 H), 12.7 (br s, 1 H). |
| | (63% d. Th.) | |
| **70** | (rac)-8-(Cyclohexylmethoxy)-N-(1-hydroxypent-4-en-2-yl)-2-methylimidazo[1,2-a]pyridin-3-carboxamid | LC-MS (Methode 3): Rₜ = 1.65 min |
| | | MS (ESpos): m/z = 372.2 (M+H)⁺ |
| | 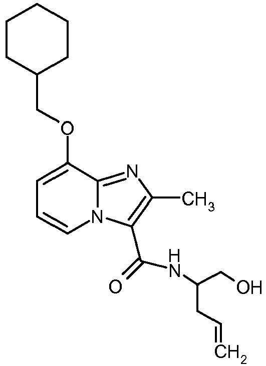 | |
| | (70% d. Th.) | |
| **71** | (rac)-8-(Cyclohexylmethoxy)-N-[trans-2-hydroxycyclopentyl]-2-methylimidazo[1,2-a]pyridin-3-carboxamid | LC-MS (Methode 1): Rₜ = 0.82 min |
| | 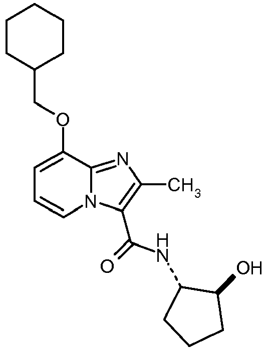 | MS (ESpos): m/z = 372.1 (M+H)⁺ |
| | (69% d. Th.) | |
| **72** | 8-(Cyclohexylmethoxy)-N-[(2R)-1-hydroxybutan-2-yl]-2-methylimidazo[1,2-a]pyridin-3-carboxamid | LC-MS (Methode 3): Rₜ = 1.57 min |
| | | MS (ESpos): m/z = 360.2 (M+H)⁺ |
| | 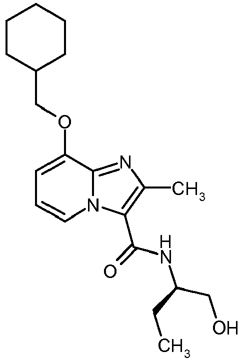 | |
| | (74% d. Th.) | |
| **73** | (rac)-8-(Cyclohexylmethoxy)-N-(1-hydroxyhexan-2-yl)-2-methylimidazo[1,2-a]pyridin-3-carboxamid | LC-MS (Methode 5): Rₜ = 1.63 min |
| | 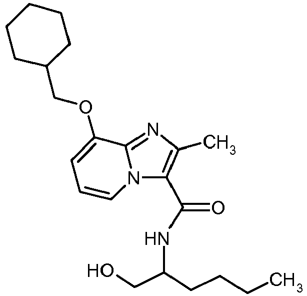 | MS (ESpos): m/z = 388.3 (M+H)⁺ |
| | (18% d. Th.) | |
| | DMSO wurde als Lösemittel verwendet | |
| **74** | 8-(Cyclohexylmethoxy)-N-(4-hydroxybutyl)-2-methylimidazo[1,2-a]pyridin-3-carboxamid | LC-MS (Methode 5): Rₜ = 1.44 min |
| | 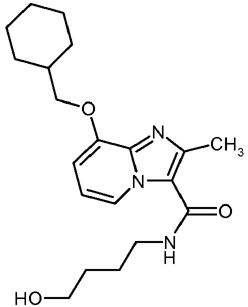 | MS (ESpos): m/z = 360.3 (M+H)⁺ |
| | (9% d. Th.) DMSO wurde als Lösemittel verwendet | |
| **75** | 8-(Cyclohexylmethoxy)-N-[(2S)-1-hydroxypropan-2-yl]-2-methylimidazo[1,2-a]pyridin-3-carboxamid | LC-MS (Methode 3): Rₜ = 1.49 min |
| | 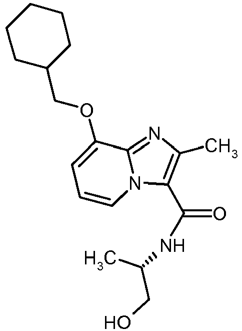 | MS (ESpos): m/z = 346.1 (M+H)⁺ |
| | (82% d. Th.) | |
| **76** | 8-(Cyclohexylmethoxy)-N-(2-hydroxyethyl)-2-methylimidazo[1,2-a]pyridin-3-carboxamid-Formiat | LC-MS (Methode 1): Rₜ = 0.76 min |
| | 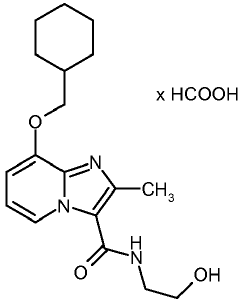 | MS (ESpos): m/z = 332.1 (M+H)⁺ |
| | | ¹H-NMR (400 MHz, DMSO-d₆): δ = 1.0-1.35 (m, 5H), 1.61-1.90 (m, 6H), 2.52 (s, teilweise durch DMSO-Signal verborgen), 3.36 (q, 2H), 3.52-3.57 (m, 2H), 3.95 (s, 2H), 4.78 (br s, 1H), 6.74 (d, 1H), 6.87 (t, 1H), 7.72 (t, 1H), 8.14 (s, 1H), 8.56 (d, 1H), 12.8 (br s, 1H). |
| | (67% d. Th.) | |
| 77 | 8-(Cyclohexylmethoxy)-N-[1,3-dihydroxy-2-(hydroxymethyl)propan-2-yl]-2-methylimidazo[1,2-a]pyridin-3-carboxamid-Formiat | LC-MS (Methode 2): Rₜ = 0.83 min |
| | | MS (ESpos): m/z = 392.2 (M+H-HCO₂H)⁺ |
| | 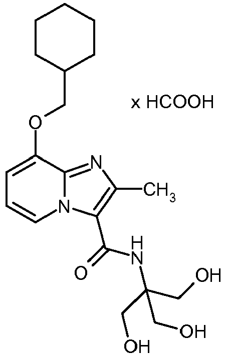 | ¹H-NMR (400 MHz, DMSO-d₆): δ = 1.0-1.15 (m, 2H), 1.15-1.38 (m, 3H), 1.6-1.78 (m, 3H), 1.78-1.90 (m, 3H), 2.53 (s, 3H), 3.7 (s, 6H), 3.95 (d, 2H), 4.85 (br s, 3H), 6.79 (d, 1H), 6.82 (s, 1H), 6.87 (t, 1H), 8.13 (s, 1H), 8.62 (d, 1H), 11.5 (br s, 1H). |
| | (41% d. Th.) | |
| **78** | (rac)-8-(Cyclohexylmethoxy)-N-[(trans)-2-hydroxycyclohexyl]-2-methylimidazo[1,2-a]pyridin-3-carboxamid | LC-MS (Methode 3): Rₜ = 1.61 min |
| | | MS (ESpos): m/z = 386.2 (M+H)⁺ |
| | 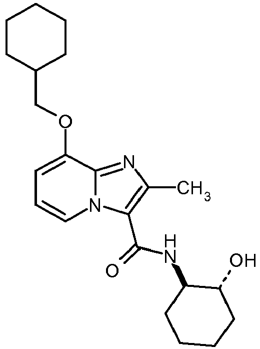 | |
| | (20% d. Th.) | |
| **79** | 8-(Cyclohexylmethoxy)-N-(2-hydroxy-3-methoxypropyl)-2-methylimidazo[1,2-a]pyridin-3-carboxamid | LC-MS (Methode 3): Rₜ = 1.53 min |
| | | MS (ESpos): m/z = 376.2 (M+H)⁺ |
| | 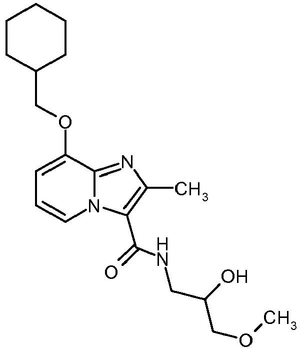 | |
| | (78% d. Th.) | |
| **80** | 8-(Cyclohexylmethoxy)-N-[(2R)-2,3-dihydroxypropyl]-2-methylimidazo[1,2-a]pyridine-3-carboxamid | LC-MS (Methode 3): Rₜ = 1.43 min |
| | | MS (ESpos): m/z = 362.1 (M+H)⁺ |
| | 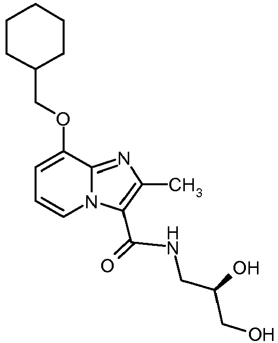 | |
| | (71% d. Th.) | |
| **81** | 8-(Cyclohexylmethoxy)-N-[cis-2-hydroxycyclopentyl]-2-methylimidazo[1,2-a]pyridin-3-carboxamid | LC-MS (Methode 1): Rₜ = 0.87 min |
| | 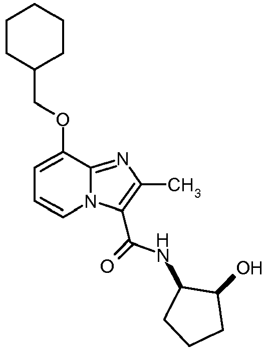 | MS (ESpos): m/z = 372.1 (M+H)⁺ |
| | (70% d. Th.) | |
| **82** | 8-(Cyclohexylmethoxy)-N-(3-hydroxypropyl)-2-methylimidazo[1,2-a]pyridin-3-carboxamid | LC-MS (Methode 5): Rₜ = 1.42 min |
| | 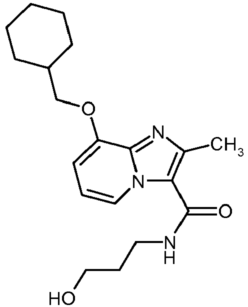 | MS (ESpos): m/z = 346.3 (M+H)⁺ |
| | (2% d. Th.) | |
| | DMSO wurde als Lösemittel verwendet | |
| **83** | 8-(Cyclohexylmethoxy)-N-(2-hydroxycyclopentyl)-2-methylimidazo[1,2-a]pyridin-3-carboxamid | LC-MS (Methode 5): Rₜ = 1.47 min |
| | 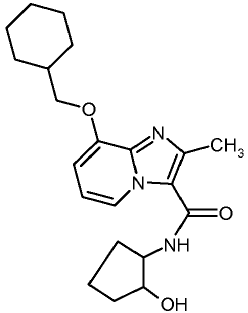 | MS (ESpos): m/z = 372.3 (M+H)⁺ |
| | (21% d. Th.) | |
| | DMSO wurde als Lösemittel verwendet | |
| **84** | 8-(Cyclohexylmethoxy)-N-(1-hydroxypropan-2-yl)-2-methylimidazo[1,2-a]pyridin-3-carboxamid | LC-MS (Methode 5): Rₜ = 1.42 min |
| | 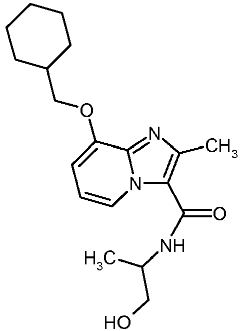 | MS (ESpos): m/z = 346.3 (M+H)⁺ |
| | (14% d. Th.) | |
| | DMSO wurde als Lösemittel verwendet | |
| **85** | 8-(Cyclohexylmethoxy)-N-(1,3-dihydroxypropan-2-yl)-2-methylimidazo[1,2-a]pyridin-3-carboxamid | LC-MS (Methode 2): Rₜ = 0.81 min |
| | 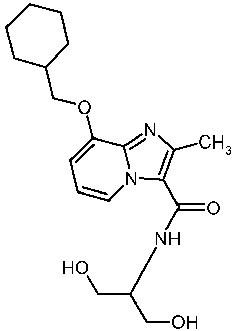 | MS (ESpos): m/z = 362.2 (M+H)⁺ |
| | (58% d. Th.) | |
| **86** | 8-(Cyclohexylmethoxy)-N-[2-(2-hydroxyethoxy)ethyl]-2-methylimidazo[1,2-a]pyridin-3-carboxamid | LC-MS (Methode 2): Rₜ = 0.87 min |
| | 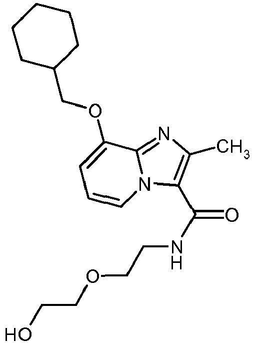 | MS (ESpos): m/z = 376.2 (M+H)⁺ |
| | (54% d. Th.) | |
| | DMSO wurde als Lösemittel verwendet | |
| **87** | 8-(Cyclohexylmethoxy)-N-(3-hydroxy-2-methylbutan-2-yl)-2-methylimidazo[1,2-a]pyridin-3-carboxamid | LC-MS (Methode 1): Rₜ = 0.91 min |
| | | MS (ESpos): m/z = 374.1 (M+H)⁺ |
| | 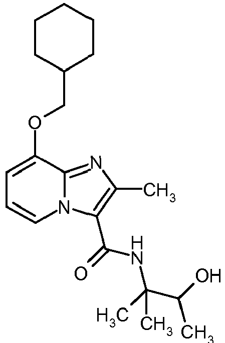 | ¹H-NMR (400 MHz, DMSO-d₆): δ = 1.0-1.09 (m, 1H), 1.1 (d, 1H), 1.15-1.35 (m, 3H), 1.35 (d, 6H), 1.61-1.09 (m, 6H), 2.51 (s, teilweise durch DMSO Signal verborgen), 3.88 (t, 1H), 3.95 (d, 1H), 5.05 (d, 1H), 6.78 (d, 1H), 6.88 (t, 1H), 7.1 (s, 1H), 8.58 (d, 1H). |
| | (40% d. Th., 88%ige Reinheit) | |
| **88** | 8-(Cyclohexylmethoxy)-N-[(2S)-1-hydroxybutan-2-yl]-2-methylimidazo[1,2-a]pyridin-3-carboxamid | LC-MS (Methode 3): Rₜ = 1.57 min |
| | 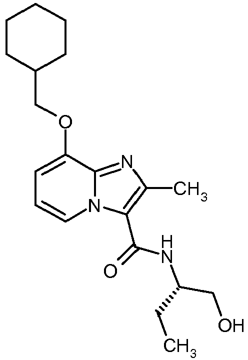 | MS (ESpos): m/z = 360.2 (M+H)⁺ |
| | (74% d. Th.) | |
| **89** | 8-(Cyclohexylmethoxy)-N-[(2S)-1-hydroxypentan-2-yl]-2-methylimidazo[1,2-a]pyridin-3-carboxamid | LC-MS (Methode 3): Rₜ = 1.69 min |
| | 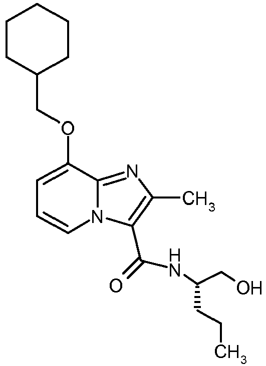 | MS (ESpos): m/z = 374.2 (M+H)⁺ |
| | (81% d. Th.) | |
| **90** | 8-(Cyclohexylmethoxy)-N-[(2S)-1-hydroxy-4-(methylsulfanyl)butan-2-yl]-2-methylimidazo[1,2-a]pyridin-3-carboxamid | LC-MS (Methode 5): Rₜ = 1.53 min |
| | 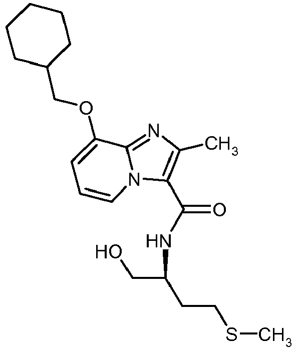 | MS (ESpos): m/z = 406.3 (M+H)⁺ |
| | (27% d. Th.) | |
| | DMSO wurde als Lösemittel verwendet | |
| **91** | 8-(Cyclohexylmethoxy)-N-[(2S,3S)-1-hydroxy-3-methylpentan-2-yl]-2-methylimidazo[1,2-a]pyridin-3-carboxamid | LC-MS (Methode 5): Rₜ = 1.58 min |
| | | MS (ESpos): m/z = 388.3 (M+H)⁺ |
| | 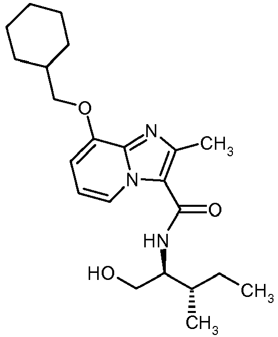 | |
| | (41% d. Th.) | |
| | DMSO wurde als Lösemittel verwendet | |
| **92** | 8-(Cyclohexylmethoxy)-N-[1-(hydroxymethyl)cyclopentyl]-2-methylimidazo[1,2-a]pyridin-3-carboxamid | LC-MS (Methode 5): Rₜ = 1.56 min |
| | 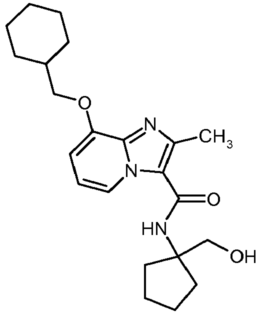 | MS (ESpos): m/z = 386.3 (M+H)⁺ |
| | (27% d. Th.) | |
| | DMSO wurde als Lösemittel verwendet | |
| **93** | 8-(Cyclohexylmethoxy)-N-[(2S)-1-hydroxy-3-methylbutan-2-yl]-2-methylimidazo[1,2-a]pyridin-3-carboxamid | LC-MS (Methode 5): Rₜ = 1.53 min |
| | 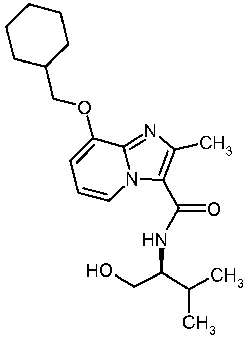 | MS (ESpos): m/z = 374.3 (M+H)⁺ |
| | (23% d. Th.) | |
| | DMSO wurde als Lösemittel verwendet | |
| **94** | N-(1-tert.-Butoxy-3-hydroxypropan-2-yl)-8-(cyclohexylmethoxy)-2-methylimidazo[1,2-a]pyridin-3-carboxamid | LC-MS (Methode 5): Rₜ = 1.62 min |
| | 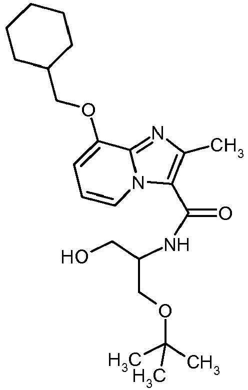 | MS (ESpos): m/z = 418.3 (M+H)⁺ |
| | (33% d. Th.) DMSO wurde als Lösemittel verwendet | |
| **95** | 8-(Cyclohexylmethoxy)-N-[(2S)-1-hydroxy-4-methylpentan-2-yl]-2-methylimidazo[1,2-a]pyridin-3-carboxamid | LC-MS (Methode 5): Rₜ = 1.60 min |
| | 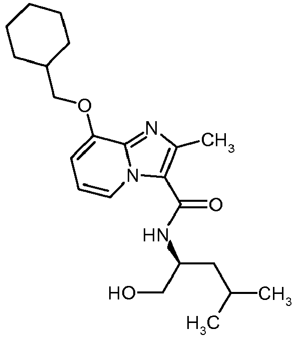 | MS (ESpos): m/z = 388.3 (M+H)⁺ |
| | (23% d. Th.) DMSO wurde als Lösemittel verwendet | |
| **96** | 8-(Cyclohexylmethoxy)-N-[(2R)-1-hydroxy-3-methylpentan-2-yl]-2-methylimidazo[1,2-a]pyridin-3-carboxamid | LC-MS (Methode 5): Rₜ = 1.60 min |
| | 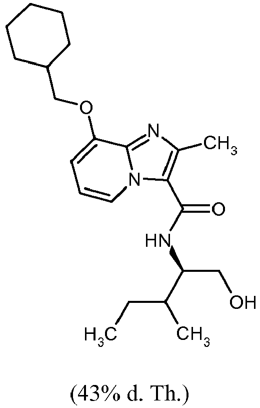 | MS (ESpos): m/z = 388.3 (M+H)⁺ |
| | DMSO wurde als Lösemittel verwendet | |

Die in der folgenden Tabelle 9 zusammengefassten weiteren Ausführungsbeispiele wurden durch Umsetzung von 8-(Cyclobutylmethoxy)-2-methylimidazo[1,2-a]pyridine-3-carbonsäure mit den entsprechenden, kommerziell erhältlichen Aminen unter TBTU-Bedingungen hergestellt.

**Tabelle 9:**

| **Beispiel** | **IUPAC-Name / Struktur (Ausbeute)** | **Analytische Daten** |
|---|---|---|
| **97** | 8-(Cyclobutylmethoxy)-N-[2-(1-hydroxycyclopentyl)ethyl]-2-methylimidazo[1,2-a]pyridin-3-carboxamid | LC-MS (Methode 1): Rₜ = 0.82 min |
| | | MS (ESpos): m/z = 372.2 (M+H)⁺ |
| | 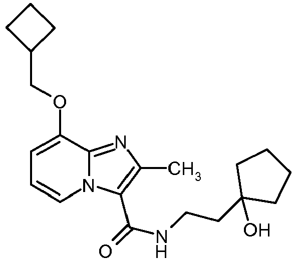 | |
| | (48% d. Th.) | |
| **98** | 8-(Cyclobutylmethoxy)-N- [2-(2-hydroxyethoxy)ethyl]-2-methylimidazo[1,2-a]pyridin-3-carboxamid | LC-MS (Methode 1): Rₜ = 0.65 min |
| | | MS (ESpos): m/z = 348.1 (M+H)⁺ |
| | 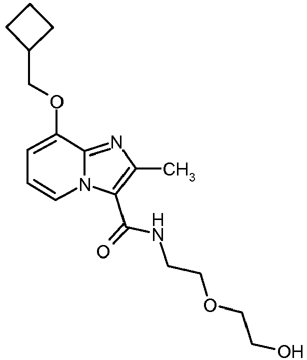 | |
| | (49% d. Th.) | |
| **99** | 8-(Cyclobutylmethoxy)-N-[2-(1-hydroxycyclohexyl)ethyl]-2-methylimidazo[1,2-a]pyridin-3-carboxamid | LC-MS (Methode 2): Rt = 0.98 min |
| | | MS (ESpos): m/z = 386.2 (M+H)+ |
| | 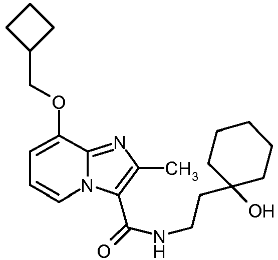 | |
| | (61% d. Th.) | |

### Repräsentative Arbeitsvorschrift 2

### Amidbildung unter Verwendung von TBTU als Kupplungsreagenz (Variante B)

1 Äquivalent der zu kuppelnden Carbonsäure, 1.1 - 1.5 Äquivalente (Benzotriazol-1-yloxy)bisdimethylaminomethyliumfluoroborat (TBTU) und 3-6 Äquivalente 4-Methylmorpholin wurden in DMF oder Dichlormethan (ca. 0.1-0.2 M bezogen auf die zu kuppelnde Carbonsäure) vorgelegt und anschließend wurden 1.1 bis 1.5 Äquivalente des zu kuppelnden Amins zugesetzt und über Nacht bei Raumtemperatur gerührt.

Beispielhafte Aufarbeitung der Reaktionsmischung: Die Reaktionslösung wurde mit Wasser versetzt, der entstandene Niederschlag noch 0.5-1.0 h ausgerührt, abfiltriert und gut mit Wasser gewaschen und über Nacht im Hochvakuum getrocknet. Alternativ wurde das Reaktionsrohgemisch direkt aufkonzentriert und per präparativer HPLC weiter aufgereinigt uns über Nacht im Hochvakuum getrocknet.

### Beispiel 100

### rac-8-[(2,6-Difluorbenzyl)oxy]-2-methyl-N-(4,4,4-trifluor-1-hydroxybutan-2-yl)imidazo[1,2-a]pyridin-3-carboxamid-Trifluoracetat

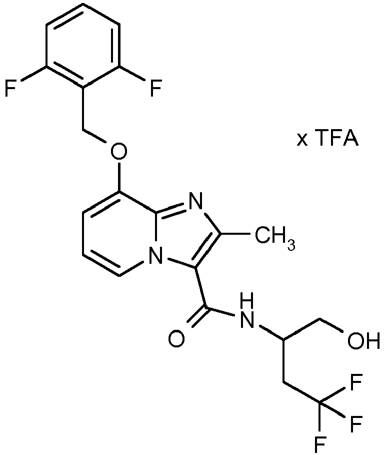

54 mg (0.17 mmol) 8-[(2,6-Difluorbenzyl)oxy]-2-methylimidazo[1,2-a]pyridin-3-carbonsäure, 59 mg (0.19 mmol) (Benzotriazol-1-yloxy)bisdimethylaminomethyliumfluoroborat (TBTU) und 85 mg (0.84 mmol) 4-Methylmorpholin wurden in 1.0 ml Dichlormethan vorgelegt. Nach 10 min bei RT wurden 27 mg (0.19 mmol) 2-Amino-4,4,4-trifluorobutan-1-ol zugesetzt und über Nacht bei Raumtemperatur gerührt. Die Reaktionslösung wurde eingedampft und mittels präparativer HPLC gereinigt (RP18 Säule, Laufmittel: Acetonitril/Wasser-Gradient unter Zusatz von 0.1% TFA). Es wurden 48 mg der Zielverbindung (49% d. Th., Reinheit 96%) erhalten.
LC-MS (Methode 1): Rₜ = 0.82 min
MS (ESpos): m/z = 444 (M-TFA+H)⁺
¹H-NMR (400 MHz, DMSO-d₆): δ = 2.50 (s, 3H), 2.55-2.72 (m, 2H), 3.38-3.62 (m, 2H), 4.29-4.40 (m, 1H), 5.39 (s, 2H), 7.10-7.38 (m, 4H), 7.60 (quint, 1H), 8.13 (br s, 1H), 8.60 (d, 1H).

In Analogie zu Beispielverbindung 100 wurden die in Tabelle 10 gezeigten Beispielverbindungen hergestellt, indem 8-[(2,6-Difluorbenzyl)oxy]-2-methylimidazo[1,2-a]pyridin-3-carbonsäure mit den entsprechenden, kommerziell erhältlichen Aminen in DMF oder Dichlormethan unter den in der repräsentativen Arbeitsvorschrift 2 beschriebenen Reaktionsbedingungen umgesetzt worden ist:

**Tabelle 10:**

| **Beispiel** | **IUPAC-Name / Struktur (Ausbeute)** | **Analytische Daten** |
|---|---|---|
| **101** | *rac*-8-[(2,6-Difluorbenzyl)oxy]-2-methyl-*N-*(5,5,5-trifluor-1-hydroxypentan-2-yl)imidazo[1,2-a]pyridin-3-carboxamid-Trifluoracetat | LC-MS (Methode 1): Rₜ = 0.85 min |
| | | MS (ESpos): m/z = 458 (M-TFA+H)⁺ |
| | | ¹H-NMR (400 MHz, DMSO-d₆): |
| | 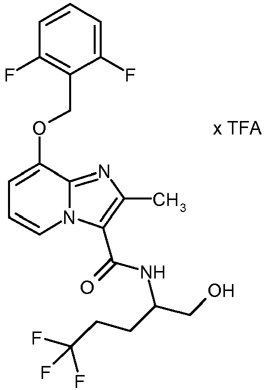 | δ = 1.69-1.79 (m, 1H), 1.83-1.98 (m, 1H), 2.29-2.45 (m, 2H), 2.50 (s, 3H), 3.46-3.60 (m, 2H), 4.00-4.12 (m, 1H), 5.40 (s, 2H), 7.18-7.28 (m, 3H), 7.38 (br s, 1H), 7.60 (quint, 1H), 8.00 (br s, 1H), 8.62 (d, 1H). |
| | (71% d. Th.) | |
| **102** | *rac*-8-[(2,6-Difluorbenzyl)oxy]-2-methyl-*N-*(6,6,6-trifluor-1-hydroxyhexan-2-yl)imidazo[1,2-a]pyridin-3-carboxamid-Trifluoracetat | LC-MS (Methode 1): Rₜ = 0.88 min |
| | | MS (ESpos): m/z = 472 (M-TFA+H)⁺ |
| | | ¹H-NMR (400 MHz, DMSO-d₆): |
| | 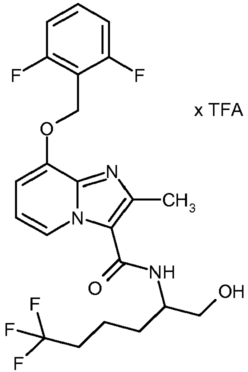 | δ = 1.51-1.79 (m, 4H), 2.19-2.40 (m, 2H), 2.50 (s, 3H), 3.44-3.59 (m, 2H), 3.98-4.09 (m, 1H), 5.40 (s, 2H), 7.18-7.28 (m, 3H), 7.35 (br s, 1H), 7.60 (quint, 1H), 7.98 (br s, 1H), 8.59 (d, 1H). |
| | (36% d. Th.) | |
| **103** | *rac*-8-[(2,6-Difluorbenzyl)oxy]-*N-*[1-(4-fluorphenyl)-2-hydroxyethyl]-2-methylimidazo-[1,2-a]pyridin-3-carboxamid-Trifluoracetat | LC-MS (Methode 1): Rₜ = 0.86 min |
| | | MS (ESpos): m/z = 456 (M-TFA+H)⁺ |
| | | ¹H-NMR (400 MHz, DMSO-d₆): |
| | 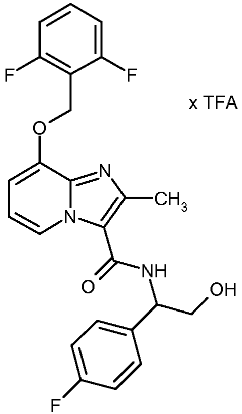 | δ = 2.61 (s, 3H), 3.70 (d, 2H), 5.10 (q, 1H), 5.38 (s, 2H), 7.00-7.32 (m, 6H), 7.48 (dd, 2H), 7.59 (quint, 1H), 8.41 (br s, 1H), 8.58 (d, 1H). |
| | (68% d. Th.) | |
| **104** | *rac*-8-[(2,6-Difluorbenzyl)oxy]-*N*-[(1R)-2-hydroxy-1-phenylethyl]-2-methylimidazo[1,2-a]pyridin-3-carboxamid-Trifluoracetat | LC-MS (Methode 2): Rₜ = 0.94 min |
| | | MS (ESpos): m/z = 456 (M-TFA+H)⁺ |
| | | ¹H-NMR (400 MHz, DMSO-d₆): |
| | 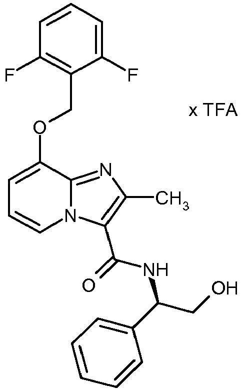 | δ = 2.61 (s, 3H), 3.70 (d, 2H), 5.11 (q, 1H), 5.39 (s, 2H), 7.12-7.30 (m, 4H), 7.33 (t, 2H), 7.40 (d, 2H), 7.60 (quint, 1H), 8.48-8.61 (m, 2H). |
| | (70% d. Th.) | |
| **105** | *rac-N-*[1-(4-Chlorphenyl)-2-hydroxyethyl]-8-[(2,6-difluorbenzyl)oxy]-2-methylimidazo[1,2-a]pyridin-3-carboxamid- | LC-MS (Methode 3): Rₜ = 1.85 min |
| | | MS (ESpos): m/z = 472 (M-TFA+H)⁺ |
| | | ¹H-NMR (400 MHz, DMSO-d₆): |
| | 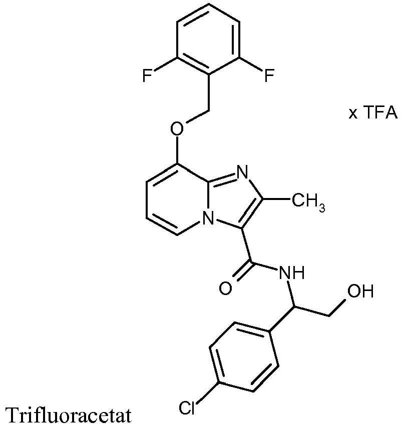 | δ = 2.61 (s, 3H), 3.70 (d, 2H), 5.10 (q, 1H), 5.40 (s, 2H), 7.15-7.28 (m, 3H), 7.31-48 (m, 4H), 7.60 (quint, 1H), 8.52-8.63 (m, 2H). |
| | (49% d. Th.) | |
| **106** | *rac*-8-[(2,6-Difluorbenzyl)oxy]-2-methyl-*N-*(3,3,3-trifluor-2-hydroxypropyl)imidazo[1,2-a]pyridin-3-carboxamid-Trifluoracetat | LC-MS (Methode 1): Rₜ = 0.82 min |
| | | MS (ESpos): m/z = 430 (M-TFA+H)⁺ |
| | | ¹H-NMR (400 MHz, DMSO-d₆): |
| | 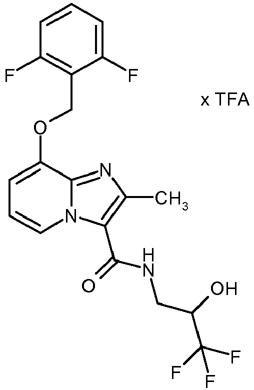 | δ = 2.50 (s, 3H), 3.60-3.70 (m, 2H), 4.20-4.30 (m, 1H), 5.38 (s, 2H), 6.60 (br s, 1H), 7.15-7.28 (m, 3H), 7.33 (br s, 1H), 7.60 (quint, 1H), 8.30 (br s, 1H), 8.59 (d, 1H). |
| | (70% d. Th.) | |

### Beispiel 107

### ent-8-[(2,6-Difluorbenzyl)oxy]-N-[1-(4-fluorphenyl)-2-hydroxyethyl]-2-methylimidazo[1,2-a]pyridin-3-carboxamid (Enantiomer B)

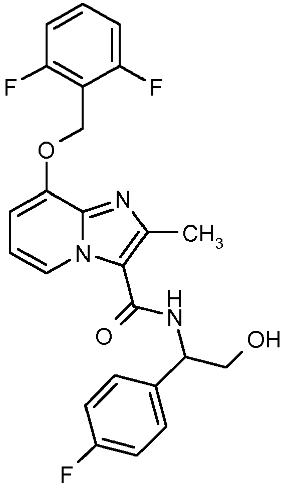

112 mg Beispiel 103 wurden in Dichlormethan und wenige Tropfen Methanol gelöst und mit gesättigter Natriumhydrogencarbonat-Lösung sowie mit Wasser gewaschen. Die organische Phase wurde eingeengt (98 mg) und durch präparative Trennung an chiraler Phase in die Enantiomere getrennt [Säule: Daicel Chiralpak AD-H, 5 µm, 250 x 20 mm, Eluent: 50% iso-Hexan, 50% Ethanol + 0.2% Diethylamin, Fluß 15 ml/min; 40°C, Detektion: 220 nm].
Ausbeute: 44 mg (99% Reinheit, >99% ee)
Enantiomer B: Rₜ = 22.01 min [Chiralpak AD-H, 5µm, 250 x 4.6 mm; Eluent: 50% iso-Hexan, 50% Ethanol + 0.2% Diethylamin; Fluss 1.0 ml/min; 40°C; Detektion: 235 nm].

### Beispiel 108

### ent-N-[1-(4-Chlorphenyl)-2-hydroxyethyl]-8-[(2,6-difluorbenzyl)oxy]-2-methylimidazo[1,2-a]pyridin-3-carboxamid (Enantiomer A)

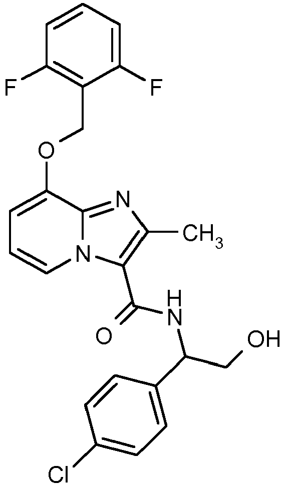

83 mg Beispiel 105 wurden in Essigsäureethylester gelöst und mit gesättigter Natriumhydrogencarbonat-Lösung sowie mit gesättigter Natriumchlorid-Lösung gewaschen. Die organische Phase wurde über Natriumsulfat getrocknet, eingeengt (53 mg) und durch präparative Trennung an chiraler Phase in die Enantiomere getrennt [Säule: Daicel Chiralpak AD-H, 5 µm, 250 x 20 mm, Eluent: 50% iso-Hexan, 50% Ethanol + 0.2% Diethylamin, Fluß 15 ml/min; 30°C, Detektion: 220 nm].
Ausbeute: 13 mg (99% Reinheit, >99% ee)
Enantiomer A: Rₜ= 6.44 min [Chirakcel OD-H, 5µm, 250 x 4.6 mm; Eluent: 50% iso-Hexan, 50% Isopropanol + 0.2% Diethylamin; Fluss 1.0 ml/min; 40°C; Detektion: 235 nm].

### Beispiel 109

### ent-8-[(2,6-Difluorbenzyl)oxy]-2-methyl-N-(3,3,3-trifluor-2-hydroxypropyl)imidazo[1,2-a]pyridin-3-carboxamid (Enantiomer B)

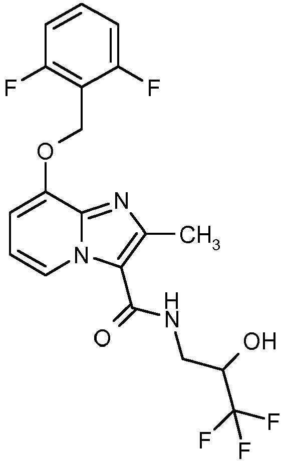

108 mg der Beispiel 106 wurden in Dichlormethan und wenigen Tropfen Methanol gelöst und mit gesättigter Natriumhydrogencarbonat-Lösung sowie mit Wasser gewaschen. Die organische Phase wurde über Natriumsulfat getrocknet, eingeengt (73 mg) und durch präparative Trennung an chiraler Phase in die Enantiomere getrennt [Säule: Daicel Chiralpak AD-H, 5 µm, 250 x 20 mm; Eluent: 50% iso-Hexan, 50% Isopropanol + 0.2% Diethylamin; Fluß 15 ml/min; Temperatur: 30°C; Detektion: 220 nm].
Ausbeute: 31 mg (99% Reinheit, >98.5% ee)
Enantiomer B: Rₜ= 6.50 min [Chiralpak AD-H, 5µm, 250 x 4.6 mm; Eluent: 50% iso-Hexan, 50% Isopropanol + 0.2% Diethylamin; Fluss 1.0 ml/min; 40°C; Detektion: 235 nm].

### Beispiel 110

### rac-8-[(2,6-Difluorbenzyl)oxy]-2-methyl-N-(1,1,1-trifluor-3-hydroxypropan-2-yl)imidazo[1,2-a]pyridin-3-carboxamid

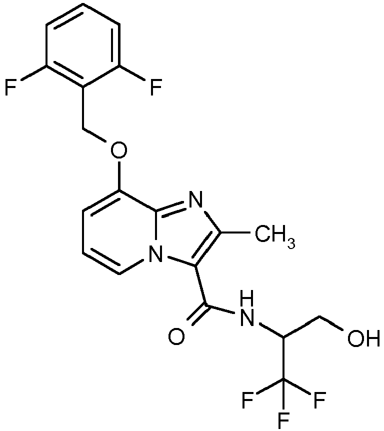

50 mg (0.16 mmol) 8-[(2,6-Difluorbenzyl)oxy]-2-methylimidazo[1,2-a]pyridin-3-carbonsäure, 55 mg (0.17 mmol) (Benzotriazol-1-yloxy)bisdimethylaminomethyliumfluoroborat (TBTU) und 79 mg (0.79 mmol) 4-Methylmorpholin wurden in 1.0 ml Dichlormethan vorgelegt. Nach 10 min bei RT wurden 28 mg (0.17 mmol) 2-Amino-4,4,4-trifluorobutan-1-ol zugesetzt und über Nacht bei Raumtemperatur gerührt. Dann wurden 1 ml DMF und 50 mg (0.39 mmol) 2-Amino-4,4,4-trifluorobutan-1-ol zugegeben und es wurde 8 h bei 50°C gerührt. Es wurden nochmals 50 mg (0.39 mmol) 2-Amino-4,4,4-trifluorobutan-1-ol zugegeben und es wurde 0.5 h bei 80°C in der Mikrowelle gerührt. Anschließend wurden nochmals 50 mg (0.39 mmol) 2-Amino-4,4,4-trifluorobutan-1-ol zugegeben und es wurde 1 h bei 80°C in der Mikrowelle gerührt. Die Reaktionslösung wurde eingedampft und mittels präparativer HPLC gereinigt (RP18 Säule, Laufmittel: Acetonitril/Wasser-Gradient unter Zusatz von 0.1% TFA). Alle produkthaltigen Fraktionen wurden vereint und eingedampft. Der Rückstand wurde in Dichlormethan gelöst und mit gesättigter wässriger Natriumhydrogencarbonat-Lösung gewaschen. Es wurden 17 mg der Zielverbindung (25% d. Th., Reinheit 100%) erhalten.
LC-MS (Methode 1): Rₜ = 0.85 min
MS (ESpos): m/z = 430 (M+H)⁺
¹H-NMR (400 MHz, DMSO-d₆): δ = 2.50 (s, 3H), 3.70-3.86 (m, 2H), 4.78-4.88 (m, 1H), 5.28 (t, 1H), 5.32 (s, 2H), 6.98 (t, 1H), 7.06 (d, 1H), 7.23 (t, 2H), 7.60 (quint, 1H), 8.29 (d, 1H), 8.50 (d, 1 H).

### Beispiel 111

### rac-8-[(2,6-Difluorbenzyl)oxy]-N-[1-(3,4-difluorphenyl)-2-hydroxyethyl]-2-methylimidazo[1,2-a]pyridin-3-carboxamid

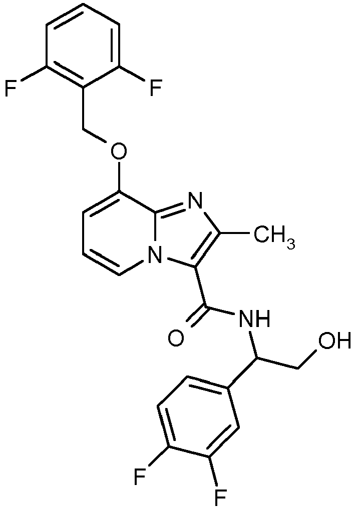

100 mg (0.31 mmol) 8-[(2,6-Difluorbenzyl)oxy]-2-methylimidazo[1,2-a]pyridin-3-carbonsäure, 111 mg (0.35 mmol) (Benzotriazol-1-yloxy)bisdimethylaminomethyliumfluoroborat (TBTU) und 159 mg (1.57 mmol) 4-Methylmorpholin wurden in 2.0 ml Dichlormethan vorgelegt. Nach 10 min bei RT wurden 142 mg (0.35 mmol) 2-Amino-2-(3,4-difluorphenyl)ethanol zugesetzt und über Nacht bei Raumtemperatur gerührt. Die Reaktionslösung wurde eingedampft und mit Acetonitril/Methanol/Wasser versetzt. Es fiel ein Feststoff aus, der abfiltriert wurde und über Chromatographie an Kieselgel gereinigt wurde (Laufmittel: Dichlormethan:THF = 10:1 -> 5:1). Die vereinten Produktfraktionen wurden nach Eindampfen mit Acetonitril verrührt und der Feststoff filtriert und mit Methanol gewaschen. Es wurden 39 mg der Zielverbindung (26% d. Th., Reinheit 95%) erhalten. Aus dem Filtrat fiel nach einiger Zeit erneut ein Feststoff aus; es wurden nochmals 5 mg der Zielverbindung (3.3% d. Th., Reinheit 99%) erhalten.
LC-MS (Methode 1): Rₜ = 0.91 min
MS (ESpos): m/z = 474 (M+H)⁺
¹H-NMR (400 MHz, DMSO-d₆): δ = 2.59 (s, 3H), 3.70 (t, 2H), 5.04-5.13 (m, 2H), 5.30 (s, 2H), 6.90 (t, 1H), 7.00 (d, 1H), 7.18-7.29 (m, 3H), 7.39 (q, 1H), 7.42-7.53 (m, 1H), 7.59 (quint, 1H), 8.12 (d, 1H), 8.52 (d, 1H).

### Beispiel 112

### ent-8-[(2,6-Difluorbenzyl)oxy]-N-[1-(3,4-difluorphenyl)-2-hydroxyethyl]-2-methylimidazo[1,2-a]pyridin-3-carboxamid (Enantiomer B)

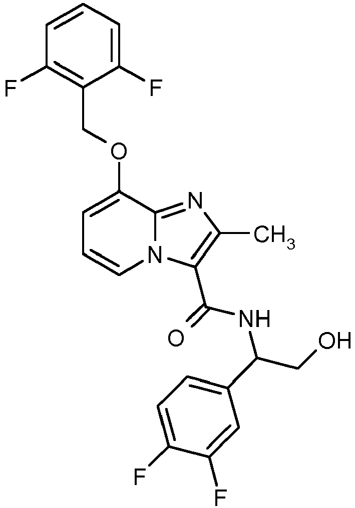

Durch präparative Trennung an chiraler Phase wurde Beispiel 111 (130 mg) in die Enantiomere getrennt [Säule: Daicel Chiralpak AD-H, 5 µm, 250 x 20 mm, Eluent: 25% iso-Hexan, 75% Ethanol + 0.2% Diethylamin, Fluß 15 ml/min; 45°C, Detektion: 220 nm]. Die Produktfraktionen wurden eingeengt und nochmals in Wasser/Acetonitril aufgenommen und gefriergetrocknet.
Ausbeute: 18 mg (99% Reinheit, >99% ee)
Enantiomer B: Rₜ = 9.32 min [Chiralpak AD-H, 5µm, 250 x 4.6 mm; Eluent: 25% iso-Hexan, 75% Ethanol + 0.2% Diethylamin; Fluss 1.0 ml/min; 45°C; Detektion: 235 nm].

### Beispiel 113

### rac-8-[(2,6-Difluorbenzyl)oxy]-2-methyl-N-(4,4,4-trifluor-1-hydroxybutan-2-yl)imidazo[1,2-a]pyridin-3-carboxamid

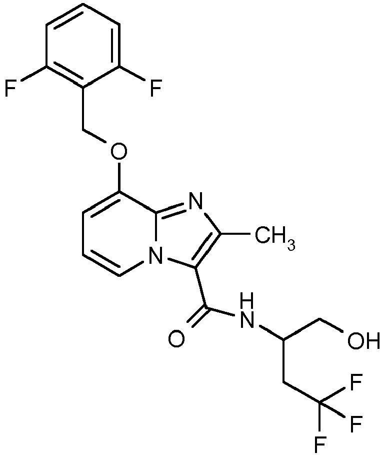

330 mg (1.04 mmol) 8-[(2,6-Difluorbenzyl)oxy]-2-methylimidazo[1,2-a]pyridin-3-carbonsäure, 399 mg (1.24 mmol) (Benzotriazol-1-yloxy)bisdimethylaminomethyliumfluoroborat (TBTU) und 524 mg (5.18 mmol) 4-Methylmorpholin wurden in 6.6 ml DMF vorgelegt. Nach 10 min bei RT wurden 371 mg (1.56 mmol, Reinheit ca. 60%) 2-Amino-4,4,4-trifluorobutan-1-ol zugesetzt und über Nacht bei Raumtemperatur gerührt. Die Reaktionslösung wurde mit ca. 200 ml Wasser versetzt, der entstandene Niederschlag noch 30 min ausgerührt, abfiltriert und gut mit Wasser gewaschen und über Nacht im Hochvakuum getrocknet. Es wurden 439 mg der Zielverbindung (96% d. Th., Reinheit 100%) erhalten.
LC-MS (Methode 3): Rₜ = 1.62 min
MS (ESpos): m/z = 444 (M+H)⁺
¹H-NMR (400 MHz, DMSO-d₆): δ = 2.50 (s, 3H), 2.55-2.72 (m, 2H), 3.38-3.47 (m, 1H), 3.51-3.62 (m, 1H), 4.29-4.40 (m, 1H), 5.12 (t, 1H), 5.30 (s, 2H), 6.92 (t, 1H), 7.02 (d, 1H), 7.23 (t, 2H), 7.59 (quint, 1H), 7.80 (d, 1H), 8.56 (d, 1H).

In Analogie zu Beispiel113 wurden die in Tabelle 11 gezeigten Beispielverbindungen hergestellt, indem 8-[(2,6-Difluorbenzyl)oxy]-2-methylimidazo[1,2-a]pyridin-3-carbonsäure mit den entsprechenden Aminen in DMF oder Dichlormethan unter den in der repräsentativen Arbeitsvorschrift 2 beschriebenen Reaktionsbedingungen umgesetzt worden ist

**Tabelle 11:**

| **Beispiel** | **IUPAC-Name / Struktur (Ausbeute)** | **Analytische Daten** |
|---|---|---|
| **114** | *rac*-8-[(2,6-Difluorbenzyl)oxy]-2-methyl-*N-*(6,6,6-trifluor-1-hydroxyhexan-2-yl)imidazo[1,2-a]pyridin-3-carboxamid | LC-MS (Methode 1): Rₜ = 0.88 min |
| | | MS (ESpos): m/z = 472 (M+H)⁺ |
| | | ¹H-NMR (400 MHz, DMSO-d₆): |
| | 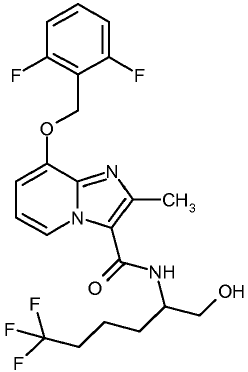 | δ = 1.51-1.79 (m, 4H), 2.19-2.40 (m, 2H), 2.50 (s, 3H), 3.41-3.57 (m, 2H), 3.96-4.08 (m, 1H), 4.82 (t, 1H), 5.30 (s, 2H), 6.91 (t, 1H), 6.99 (d, 1H), 7.22 (t, 2H), 7.56-7.62 (m, 2H), 8.52 (d, 1H). |
| | (76% d. Th.) | |
| **115** | 8-{[(2,6-Difluorphenyl)(²H₂)methyl]oxy}-*N-*[(2R)-1-hydroxyhexan-2-yl]-2-methylimidazo[1,2-a]pyridin-3-carboxamid | LC-MS (Methode 3): Rₜ = 1.66 min |
| | | MS (ESpos): m/z = 420 (M+H)⁺ |
| | | ¹H-NMR (400 MHz, DMSO-d₆): |
| | 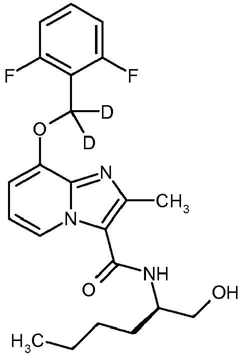 | δ = 0.88 (t, 3H), 1.22-1.51 (m, 5H), 1.59-1.70 (m, 1H), 2.50 (s, 3H), 3.39-3.54 (m, 2H), 3.93-4.04 (m, 1H), 4.74 (t, 1H), 6.91 (t, 1H), 6.99 (d, 1H), 7.22 (t, 2H), 7.50-7.63 (m, 2H), 8.52 (d, 1H). |
| | (90% d. Th.) | |
| **116** | 8-[(2,6-Difluorbenzyl)oxy]-*N*-[2-(1-hydroxycyclohexyl)ethyl]-2-methylimidazo[1,2-a]pyridin-3-carboxamid | LC-MS (Methode 1): Rₜ = 0.91 min |
| | | MS (ESpos): m/z = 444 (M+H)⁺ |
| | | ¹H-NMR (400 MHz, DMSO-d₆): |
| | 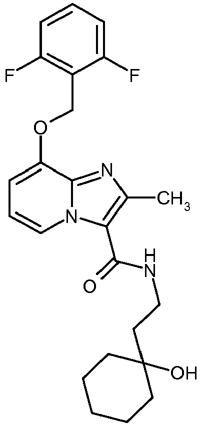 | δ = 1.20-1.63 (m, 10H, 1.68 (t, 2H), 2.50 (s, 3H), 3.41 (q, 2H), 4.23 (s, 1H), 5.30 (s, 2H), 6.91 (t, 1H), 7.00 (d, 1H), 7.23 (t, 2H), 7.59 (quintett, 1H), 7.82 (t, 1H), 8.69 (d, 1H). |
| | (46% d. Th.) | |
| **117** | 8-[(2,6-Difluorbenzyl)oxy]-*N*-[2-(1-hydroxycyclopentyl)ethyl]-2-methylimidazo[1,2-a]pyridin-3-carboxamid | LC-MS (Methode 1): Rₜ = 0.84 min |
| | | MS (ESpos): m/z = 430 (M+H)⁺ |
| | | ¹H-NMR (400 MHz, DMSO-d₆): |
| | 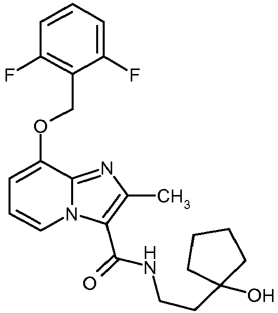 | δ = 1.43-1.76 (m, 8H), 1.78 (t, 2H), 2.50 (s, 3H), 3.44 (q, 2H), 4.31 (s, 1H), 5.30 (s, 2H), 6.92 (t, 1H), 7.00 (d, 1H), 7.23 (t, 2H), 7.59 (quintett, 1H), 7.86 (t, 1H), 8.69 (d, 1H). |
| | (83% d. Th.) | |
| **118** | 8-[(2,6-Difluorbenzyl)oxy]-*N*-[(2R)-1-hydroxypropan-2-yl]-2-methylimidazo[1,2-a]pyridin-3-carboxamid | LC-MS (Methode 1): Rₜ = 0.68 min |
| | | MS (ESpos): m/z = 376 (M+H)⁺ |
| | | ¹H-NMR (400 MHz, DMSO-d₆): |
| | 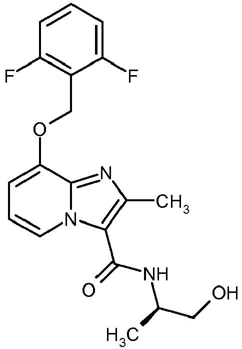 | δ = 1.18 (d, 3H), 2.50 (s, 3H), 3.37-3.54 (m, 2H), 4.00-4.10 (m, 1H), 4.78 (t, 1H), 5.30 (s, 2H), 6.90 (t, 1H), 6.99 (d, 1H), 7.22 (t, 2H), 7.50-7.63 (m, 2H), 8.58 (d, 1H). |
| | (93% d. Th.) | |
| **119** | *rac*-8-[(2,6-Difluorbenzyl)oxy]-*N*-[2-hydroxy-1-(pyridin-2-yl)ethyl]-2-methylimidazo[1,2-a]pyridin-3-carboxamid | LC-MS (Methode 1): Rₜ = 0.72 min |
| | | MS (ESpos): m/z = 439 (M+H)⁺ |
| | | ¹H-NMR (400 MHz, DMSO-d₆): |
| | 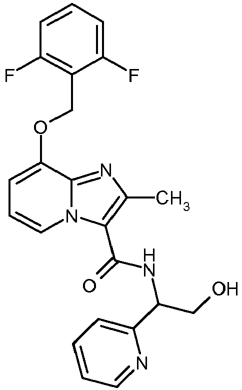 | δ = 2.62 (s, 3H), 3.75-3.88 (m, 2H), 5.01 (t, 1H), 5.19 (q, 1H), 5.32 (s, 2H), 6.92 (t, 1H), 7.03 (d, 1H), 7.19-7.32 (m, 3H), 7.48 (d, 1H), 7.59 (quintett, 1H), 7.79 (t, 1H), 8.09 (d, 1H), 8.58 (d, 1H), 8.68 (d, 1H). |
| | (78% d. Th.) | |
| **120** | *rac*-8-[(2,6-Difluorbenzyl)oxy]-*N*-{2-hydroxy-1-[4-(methylsulfonyl)phenyl]ethyl}-2-methylimidazo[1,2-a]pyridin-3-carboxamid ²⁾ | LC-MS (Methode 2): Rₜ = 0.87 min |
| | | MS (ESpos): m/z = 516 (M+H)⁺ |
| | | ¹H-NMR (400 MHz, DMSO-d₆): |
| | 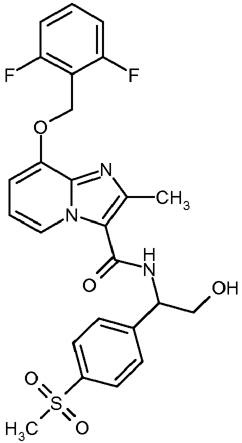 | δ = 2.62 (s, 3H), 3.22 (s, 3H), 3.74 (t, 2H), 5.10-5.19 (m, 2H), 5.31 (s, 2H), 6.90 (t, 1H), 7.01 (d, 1H), 7.22 (t, 2H), 7.59 (quintett, 1H), 7.69 (d, 2H), 7.90 (d, 2H), 8.26 (d, 1H), 8.53 (d, 1H). |
| | (58% d. Th.) | |

| | | |
|---|---|---|
| ¹⁾ Aufarbeitung und Reinigung: Das Gemisch wurde dreimal mit Essigsäureethylester extrahiert, die vereinten organischen Phasen wurden mit gesättigter Natriumchlorid-Lösung gewaschen, über Natriumsulfat getrocknet, filtriert und eingeengt. Das Rohprodukt wurde über eine Kieselgelkartusche gereinigt (Laufmittel: Dichlormethan:Methanol = 40:1 -> 10:1). ²⁾ Das erhaltene Rohprodukt wurde nochmals mittels Kieselgelchromatographie gereinigt (Laufmittel: Dichlormethan:Ethanol = 20:1). | | |

### Beispiel 121

### ent-8-[(2,6-Difluorbenzyl)oxy]-2-methyl-N-(4,4,4-trifluor-1-hydroxybutan-2-yl)imidazo[1,2-a]pyridin-3-carboxamid (Enantiomer B)

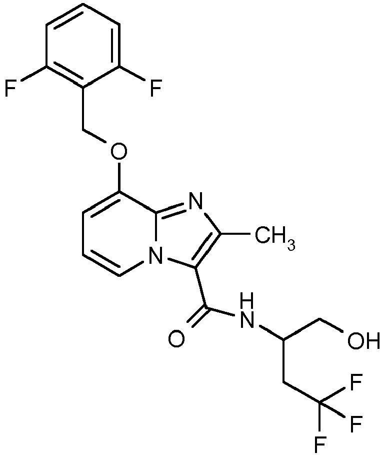

Durch präparative Trennung an chiraler Phase wurde Beispiel 113 (148 mg) in die Enantiomere getrennt [Säule: Daicel Chiralpak AD-H, 5 µm, 250 x 20 mm; Eluent: 50% iso-Hexan, 50% Ethanol + 0.2%Diethylamin; Fluß 15 ml/min; 25°C; Detektion: 220 nm].
Ausbeute: 64 mg (98% Reinheit, >99% ee)
Enantiomer B: Rₜ= 9.71 min [Chiralpak AD-H, 5µm, 250 x 4.6 mm; Eluent: 50% iso-Hexan, 50% Ethanol + 0.2% Diethylamin; Fluss 1.0 ml/min; 40°C; Detektion: 235 nm].

### Beispiel 122

### ent-8-[(2,6-Difluorbenzyl)oxy]-2-methyl-N-(5,5,5-trifluor-1-hydroxypentan-2-yl)imidazo[1,2-a]pyridin-3-carboxamid (Enantiomer B)

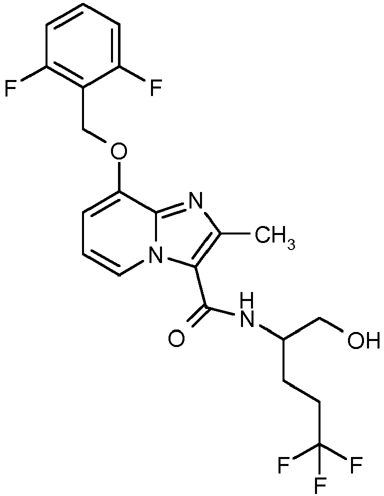

Durch präparative Trennung an chiraler Phase wurde Beispiel 101 (93 mg) in die Enantiomere getrennt [Säule: Daicel Chiralpak AD-H, 5 µm, 250 x 20 mm, Eluent: 50% iso-Hexan, 50% Ethanol + 0.2%Diethylamin, Fluß 15 ml/min; 30°C, Detektion: 220 nm].
Ausbeute: 36 mg (99% Reinheit, >99% ee)
Enantiomer B: Rₜ = 8.38 min [Chiralpak AD-H, 5µm, 250 x 4.6 mm; Eluent: 50% iso-Hexan, 50% Ethanol + 0.2% Diethylamin; Fluss 1.0 ml/min; 40°C; Detektion: 235 nm].

### Beispiel 123

### ent-8-[(2,6-Difluorbenzyl)oxy]-2-methyl-N-(6,6,6-trifluor-1-hydroxyhexan-2-yl)imidazo[1,2-a]pyridin-3-carboxamid (Enantiomer B)

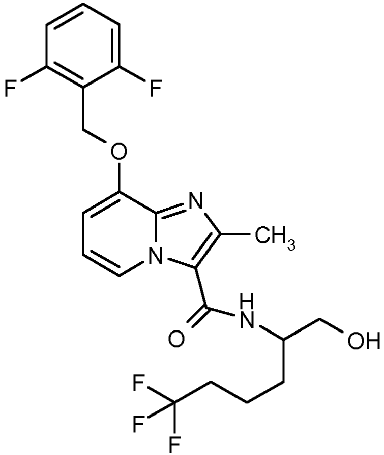

Durch präparative Trennung an chiraler Phase wurde Beispiel 114 (157 mg) in die Enantiomere getrennt [Säule: Daicel Chiralpak AD-H, 5 µm, 250 x 20 mm, Eluent: 50% iso-Hexan, 50% Ethanol + 0.2%Diethylamin, Fluß 15 ml/min; 25°C, Detektion: 220 nm].
Ausbeute: 60 mg (99% Reinheit, >99% ee)
Enantiomer B: Rₜ = 10.52 min [Chiralpak AD-H, 5µm, 250 x 4.6 mm; Eluent: 50% iso-Hexan, 50% Ethanol + 0.2% Diethylamin; Fluss 1.0 ml/min; 40°C; Detektion: 235 nm].

### Beispiel 124

### rac-8-[(2,6-Difluorbenzyl)oxy]-N-[2-hydroxy-1-(pyridin-3-yl)ethyl]-2-methylimidazo[1,2-a]pyridin-3-carboxamid

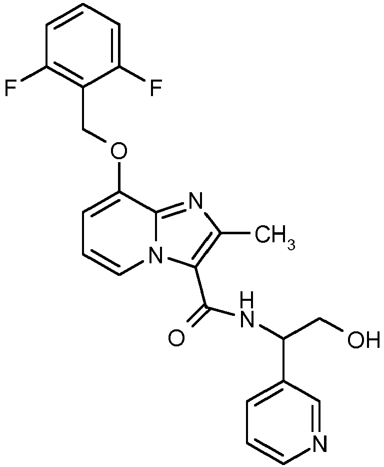

150 mg (0.47 mmol) 8-[(2,6-Difluorbenzyl)oxy]-2-methylimidazo[1,2-a]pyridin-3-carbonsäure, 227 mg (0.71 mmol) (Benzotriazol-1-yloxy)bisdimethylaminomethyliumfluoroborat (TBTU) und 238 mg (2.36 mmol) 4-Methylmorpholin wurden in 3 ml DMF vorgelegt. Nach 10 min bei RT wurden 98 mg (0.71 mmol) 2-Amino-2-(pyridin-3-yl)ethanol zugesetzt und über Nacht bei Raumtemperatur gerührt. Die Reaktionslösung wurde mit ca. 25 ml Wasser versetzt, der entstandene Niederschlag noch 30 min ausgerührt, abfiltriert, gut mit Wasser gewaschen und über Nacht im Hochvakuum getrocknet. Das Rohprodukt wurde mittels Kieselgel-Chromatographie gereinigt (Laufmittel: Dichlormethan:Methanol-Gradient). Die Produktfraktionen wurden eingedampft und mit Acetonitril verrührt. Der enthaltene Feststoff wurde abfiltriert und getrocknet. Es wurden 40 mg der Zielverbindung (20% d. Th., Reinheit 100%) erhalten.
LC-MS (Methode 1): Rₜ = 0.65 min
MS (ESpos): m/z = 439 (M+H)⁺
¹H-NMR (400 MHz, DMSO-d₆): δ = 2.58 (s, 3H), 3.70-3.80 (m, 2H), 5.08-5.15 (m, 2H), 5.30 (s, 2H), 6.90 (t, 1H), 7.00 (d, 1H), 7.22 (t, 2H), 7.37 (t, 1H), 7.59 (quint, 1H), 7.82 (d, 1H), 8.23 (d, 1H), 8.47 (d, 1H), 8.53 (d, 1H), 8.64 (s, 1H).

### Beispiel 125

### 8-[(2,6-Difluorbenzyl)oxy]-N-[1-(hydroxymethyl)cyclobutyl]-2-methylimidazo[1,2-a]pyridin-3-carboxamid

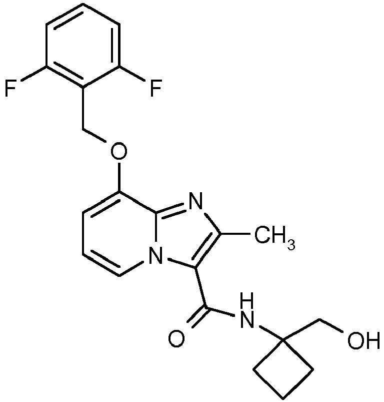

75 mg (0.24 mmol) 8-[(2,6-Difluorbenzyl)oxy]-2-methylimidazo[1,2-a]pyridin-3-carbonsäure, 91 mg (0.28 mmol) (Benzotriazol-1-yloxy)bisdimethylaminomethyliumfluoroborat (TBTU) und 143 mg (1.41 mmol) 4-Methylmorpholin wurden in 1.6 ml DMF vorgelegt. Nach 10 min bei RT wurden 79 mg (0.35 mmol, Reinheit ca. 45%) (1-Aminocyclobutyl)methanol zugesetzt und über Nacht bei Raumtemperatur gerührt. anschließend wurden nochmals 53 mg (0.24 mmol, Reinheit ca. 45%) (1-Aminocyclobutyl)methanol zu der Reaktionslösung gegeben. Das Reaktionsgemisch wurde über Nacht bei Raumtemperatur gerührt. Dann wurde mit Wasser versetzt und dreimal mit Dichlormethan extrahiert. Die vereinigten organischen Phasen wurden mit Wasser gewaschen, über Natriumhydrogensulfat getrocknet, filtriert und eingeengt. Das Rohprodukt wurde mittels präparativer HPLC (RP18 Säule, Laufmittel: Acetonitril/Wasser-Gradient unter Zusatz von 0.1% TFA) gereinigt. Die entsprechenden Fraktionen wurden weitesgehend vom Acetonitril befreit, die wässrige Phase mit Dichlormethan versetzt und mit gesättigter, wässiger Natriumhydrogencarbonat Lösung gewaschen. Die organische Phase wurde über Natriumsulfat getrocknet, filtriert und eingeengt. Es wurden 42 mg der Zielverbindung (43% d. Th., Reinheit 98%) erhalten.
LC-MS (Methode 1): Rₜ = 0.75 min
MS (ESpos): m/z = 402 (M+H)⁺
¹H-NMR (400 MHz, DMSO-d₆): δ = 1.71-1.93 (m, 2H), 2.09-2.19 (m, 2H), 2.23-2.34 (m, 2H), 2.50 (s, 3H), 3.68 (d, 2H), 4.90 (t, 1H), 5.30 (s, 2H), 6.90 (t, 1H), 6.98 (d, 1H), 7.23 (t, 2H), 7.59 (quint, 1H), 7.71 (s, 1H), 8.56 (d, 1H).

### Allgemeine Arbeitsvorschrift 3: Amidbildung unter Verwendung von HATU als Kupplungsreagenz.

1 Äquivalent der zu kuppelnden Carbonsäure, 1.3 Äquivalente *O*-(7-Azabenzotriazol-1-yl)-*N*,*N*,*N'N'*-tetramethyluroniumhexafluorphosphat (HATU) und 3 bis 4 Äquivalente *N*,*N-*Diisopropylethylamin wurden in DMF (ca. 0.2 M bezogen auf die zu kuppelnde Carbonsäure) vorgelegt, mit 1.2 bis 1.5 Äquivalenten des zu kuppelnden Amins versetzt und über Nacht bei RT gerührt. In einzelnen Fällen, in denen nach 16h kein vollständiger Umsatz der Carbonsäure erreicht worden war (z.B. basierend auf LC-MS Analyse), wurde eine zusätzliche Menge HATU und zu kuppelndes Amin zugesetzt und die Reaktion bei RT weitergerührt.

Beispielhafte Aufarbeitung der Reaktionsmischung: Die Reaktionslösung wurde mit Wasser versetzt, der entstandene Niederschlag noch 30 min ausgerührt, abfiltriert, mit Wasser gewaschen und über Nacht im Hochvakuum getrocknet. Alternativ wurde das Reaktionsrohgemisch entweder direkt nach Aufkonzentration im Vakuum oder nach extraktiver Aufarbeitung per präparativer HPLC (siehe allgemeine HPLC-Bedingungen) weiter aufgereinigt. Die folgenden Beispielverbindung 126 und 127 stellen beispielhafte Ausführungen dieser allgemeinen Arbeitsvorschrift dar.

### Beispiel 126

### 8-[(2,6-Difluorbenzyl)oxy]-6-ethinyl-N-[(2R)-1-hydroxyhexan-2-yl]-2-methylimidazo[1,2-a]pyridin-3-carboxamid

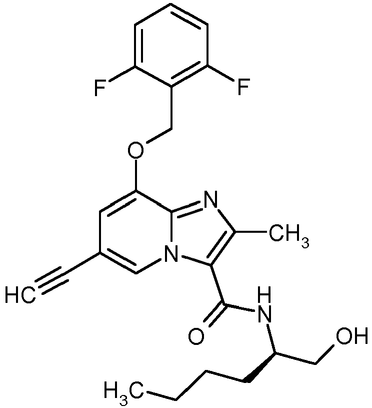

Unter Argon wurden 50 mg (0.15 mmol) 8-[(2,6-Difluorbenzyl)oxy]-6-ethinyl-2-methylimidazo[1,2-a]pyridin-3-carbonsäure in 0.5 mL trockenem Dimethylformamid gelöst, mit 20.54 mg (0.18 mmol, 1.2 Äquivalente) (R)-(-)-2-Aminohexanol, 72 µL Diisopropylethylamin (0.44 mmol, 3 Äquivalente) und 72 mg HATU (0.19 mmol, 1.3 Äquivalente) versetzt und über Nacht bei Raumtemperatur gerührt. Dann wurde mit wenig Wasser versetzt und direkt per präparativer HPLC aufgereinigt (Methode 8). Es wurden 37.5 mg der Zielverbindung (58% d. Th.) erhalten.
LC-MS (Methode 2): Rₜ = 1.17 min
MS (ESpos): m/z = 442.0 (M+H)⁺
¹H-NMR (400 MHz, DMSO-d₆): δ = 0.88 (t, 3H), 1.29 - 1.39 (m, 4H), 1.40 - 1.50 (m, 1 H), 1.60 - 1.70 (m, 1 H), 2.55 (s, 3H; überlagert durch DMSO-Signal), 3.40 - 3.52 (m, 2H), 3.90 - 4.01 (m, 1H), 4.73 (t, 1H), 5.31 (s, 2H), 7.03 (s, 1 H), 7.21 (t, 2 H), 7.59 (quint., 1 H), 7.62 (d, 1 H), 8.68 (s, 1 H).

### Beispiel 127

### 6-Chlor-8-[(2,6-difluorbenzyl)oxy]-N-[(2R)-1,5-dihydroxypentan-2-yl]-2-methylimidazo[1,2-a]pyridin-3-carboxamid

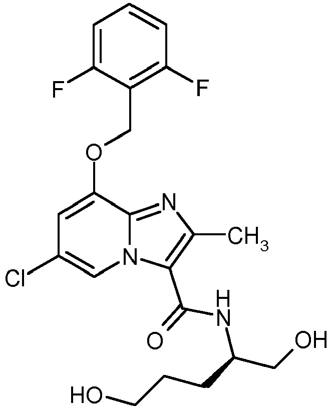

Unter Argon wurden 70 mg (0.20 mmol) 6-Chlor-8-[(2,6-difluorbenzyl)oxy]-2-methylimidazo[1,2-a]pyridin-3-carbonsäure in 0.63 mL trockenem Dimethylformamid gelöst, mit 33.1 mg (0.28 mmol, 1.4 Äquivalente) (2R)-2-Aminopentan-1,5-diol, 98 µL Diisopropylethylamin (0.60 mmol, 3 Äquivalente) und 98 mg HATU (0.26 mmol, 1.3 Äquivalente) versetzt und über Nacht bei Raumtemperatur gerührt. Dann wurde mit wenig Wasser versetzt und direkt per präparativer HPLC aufgereinigt (Methode 10). Es wurden 31.4 mg der Zielverbindung (35% d. Th.) erhalten.
LC-MS (Methode 1): Rₜ = 0.78 min
MS (ESpos): m/z = 454.2 (M+H)⁺
¹H-NMR (400 MHz, DMSO-d₆): δ = 1.40 - 1.60 (m, 3H), 1.60 - 1.70 (m, 1 H), 2.55 (s, 3H; überlagert durch DMSO-Signal), 3.35 - 3.52 (m, 4H), 3.90 - 4.01 (m, 1H), 4.40 (t, 1 H), 4.73 (t, 1H), 5.31 (s, 2H), 7.18 (s, 1 H), 7.23 (t, 2 H), 7.59 (quint., 1 H), 7.62 (d, 1 H), 8.65 (s, 1 H).

Die in der folgenden Tabelle 12 zusammengefassten weiteren Synthesebeispiele wurden in Analogie zu den Beispielen 126 und 127 sowie der allgemeinen Arbeitsvorschrift 3 durch Umsetzung der entsprechenden Carbonsäure (siehe Syntheseintermediate weiter oben) mit den entsprechenden, kommerziell erhältlichen oder wie beschrieben hergestellten Aminen hergestellt.

**Tabelle 12:**

| **Beispiel** | **IUPAC-Name / Struktur (Ausbeute)** | **Analytische Daten** |
|---|---|---|
| **128** | 6-Chlor-8-[(2,6-difluorbenzyl)oxy]-N-[(2R)-1,4-dihydroxybutan-2-yl]-2-methylimidazo[1,2-a]pyridin-3-carboxamid | LC-MS (Methode 1): Rₜ = 0.77 min |
| | | MS (ESpos): m/z = 440.2 (M+H)⁺ |
| | 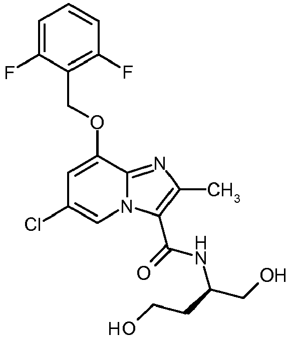 | ¹H-NMR (400 MHz, DMSO-d₆): |
| | | δ = 1.60 - 1.70 (m, 1 H), 1.75 - 1.87 (m, 1 H), 2.55 (s, 3 H; überlagert durch DMSO-Signal), 3.41 - 3.57 (m, 4 H), 4.02 - 4.15 (m, 1 H), 4.53 (t, 1 H), 4.79 (t, 1 H), 5.31 (s, 2 H), 7.20 (s, 1 H), 7.23 (t, 2 H), 7.59 (quint., 1 H), 7.68 (d, 1 H), 8.70 (s, 1 H). |
| | (78% d. Th.) | |
| **129** | 8-[(2,6-Difluorbenzyl)oxy]-N-[(2R)-1,4-dihydroxybutan-2-yl]-2-methylimidazo[1,2-a]pyridin-3-carboxamid | LC-MS (Methode 1): Rₜ = 0.53 min |
| | | MS (ESpos): m/z = 406.3 (M+H)⁺ |
| | | ¹H-NMR (400 MHz, DMSO-d₆): |
| | 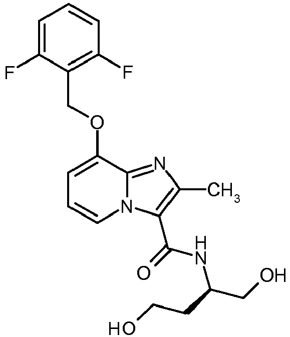 | δ = 1.60 - 1.70 (m, 1 H), 1.75 - 1.87 (m, 1 H), 2.55 (s, 3 H; überlagert durch DMSO-Signal), 3.41 - 3.57 (m, 4 H), 4.02 - 4.15 (m, 1 H), 4.53 (t, 1 H), 4.79 (t, 1 H), 5.31 (s, 2 H), 6.91 (t, 1 H), 7.00 (d, 1 H), 7.23 (t, 2 H), 7.55 - 7.65 (m, 2 H), 8.59 (d, 1 H). |
| | (69% d. Th.) | |
| **130** | 8-[(2,6-Difluorbenzyl)oxy]-N-[(2R)-1,5-dihydroxypentan-2-yl]-2-methylimidazo[1,2-a]pyridin-3-carboxamid | LC-MS (Methode 1): Rₜ = 0.54 min |
| | | MS (ESpos): m/z = 420.3 (M+H)⁺ |
| | 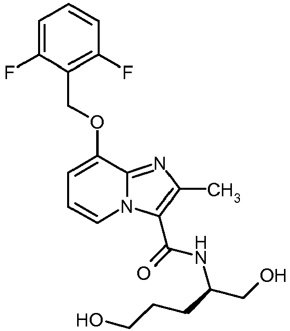 | ¹H-NMR (400 MHz, DMSO-d₆): δ = 1.40 - 1.55 (m, 3 H), 1.65 - 1.72 (m, 1 H), 2.55 (s, 3 H; überlagert durch DMSO-Signal), 3.37 - 3.52 (m, 4 H), 3.95 - 4.04 (m, 1 H), 4.42 (t, 1 H), 4.73 (t, 1 H), 5.31 (s, 2 H), 6.91 (t, 1 H), 7.00 (d, 1 H), 7.23 (t, 2 H), 7.52 (d, 1 H), 7.59 (quint., 1 H), 8.52 (d, 1 H). |
| | (37% d. Th.) | |
| **131** | 6-Chlor-8-[(2,6-difluorbenzyl)oxy]-N-[(2R)-1-hydroxyhexan-2-yl]-2-methylimidazo[1,2-a]pyridin-3-carboxamid | LC-MS (Methode 1): Rₜ = 1.05 min |
| | | MS (ESpos): m/z = 452.1/454.1 (M+H)⁺ |
| | | ¹H-NMR (400 MHz, DMSO-d₆): |
| | 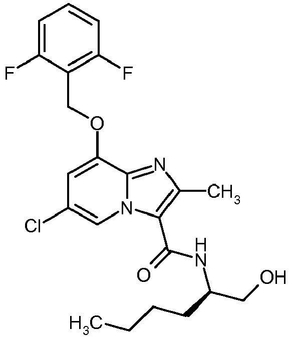 | δ = 0.88 (t, 3H), 1.25 - 1.39 (m, 4H), 1.40 - 1.50 (m, 1 H), 1.60 - 1.70 (m, 1 H), 2.55 (s, 3H; überlagert durch DMSO-Signal), 3.40 - 3.52 (m, 2H), 3.90 - 4.01 (m, 1H), 4.72 (t, 1H), 5.33 (s, 2H), 7.15 (s, 1 H), 7.21 (t, 2 H), 7.55 - 7.65 (m, 2 H), 8.62 (s, 1 H). |
| | (82% d. Th.) | |
| **132** | 8-[(2,6-Difluorbenzyl)oxy]-6-fluor-N-[(2R)-1-hydroxyhexan-2-yl]-2-methylimidazo[1,2-a]pyridin-3-carboxamid | LC-MS (Methode 1): Rₜ = 0.97 min |
| | | MS (ESpos): m/z = 436.1 (M+H)⁺ |
| | | ¹H-NMR (400 MHz, DMSO-d₆): |
| | 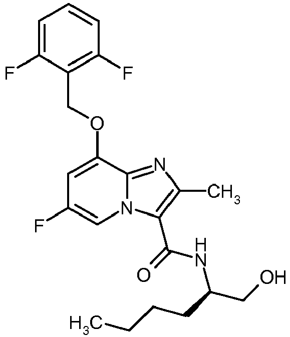 | δ = 0.86 (t, 3H), 1.21 - 1.39 (m, 4H), 1.40 - 1.52 (m, 1 H), 1.58 - 1.71 (m, 1 H), 2.55 (s, 3H; überlagert durch DMSO-Signal), 3.40 - 3.55 (m, 2H), 3.91 - 4.04 (m, 1H), 4.73 (t, 1H), 5.35 (s, 2H), 7.20 - 7.30 (m, 3 H), 7.55 (d, 1 H), 7.59 (quint., 1 H), 8.61 (d, 1 H). |
| | (92% d. Th.) | |
| **133** | 8-[(2,6-Difluorbenzyl)oxy]-N-[(2R)-1-hydroxyhexan-2-yl]-2,6-dimethylimidazo[1,2-a]pyridin-3-carboxamid | LC-MS (Methode 1): Rₜ = 0.87 min |
| | | MS (ESpos): m/z = 432.3 (M+H)⁺ |
| | | ¹H-NMR (400 MHz, DMSO-d₆): |
| | 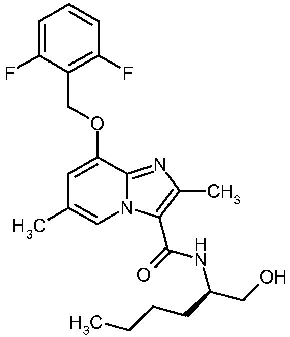 | δ = 0.88 (t, 3H), 1.21 - 1.49 (m, 5H), 1.58 - 1.71 (m, 1 H), 2.30 (s, 3 H), 2.49 (s, 3H), 3.40 - 3.55 (m, 2H), 3.91 - 4.04 (m, 1H), 4.73 (t, 1H), 5.30 (s, 2H), 6.89 (s, 1 H), 7.21 (t, 2 H), 7.50 (d, 1 H), 7.59 (quint., 1 H), 8.87 (s, 1 H). |
| | (24% d. Th.) | |
| **134** | 2-Cyclopropyl-8-[(2,6-difluorbenzyl)oxy]-N-[(2R)-1-hydroxyhexan-2-yl]imidazo[1,2-a]pyridin-3-carboxamid | LC-MS (Methode 1): Rₜ = 0.98 min |
| | | MS (ESpos): m/z = 443.3 (M+H)⁺ |
| | | ¹H-NMR (400 MHz, DMSO-d₆): |
| | 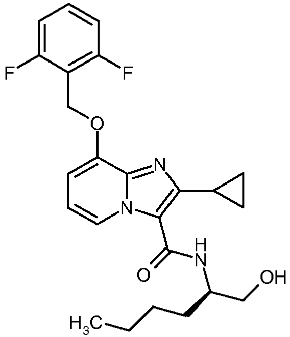 | δ = 0.70 - 1.00 (m, 7H), 1.25 - 1.39 (m, 4H), 1.40 - 1.53 (m, 1 H), 1.60 - 1.70 (m, 1 H), 2.22 - 2.31 (m, 1 H), 2.55 (s, 3H; überlagert durch DMSO-Signal), 3.43 - 3.52 (m, 2H), 3.95 - 4.07 (m, 1H), 4.78 (t, 1H), 5.30 (s, 2H), 6.90 (t, 1 H), 6.96 (d, 1 H), 7.21 (t, 2 H), 7.59 (quint.m 1 H), 7.68 (d, 1 H), 8.60 (d, 1 H). |
| | (12% d. Th.) | |
| **135** | 8-[(2,6-Difluorbenzyl)oxy]-2-methyl-*N-*[(2R,5R)-6,6,6-trifluor-1,5-dihydroxyhexan-2-yl]imidazo[1,2-a]pyridin-3-carboxamid trifluoroacetat (1:1)¹⁾ | LC-MS (Methode 1): Rₜ = 0.77 min |
| | | MS (ESpos): m/z = 488 (M+H)⁺ |
| | | ¹H-NMR (400 MHz, DMSO-d₆): |
| | 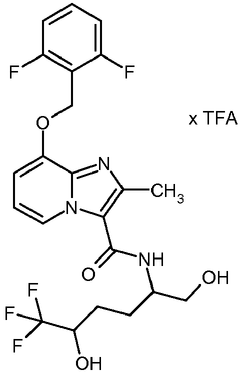 | δ = 1.49-1.80 (m, 4H), 2.50 (s, 3H), 3.39-3.58 Signal unter breitem Singulett (m, 1H), 3.97-4.12 (m, 2H), 5.39 (s, 2H), 6.12 (br s, 1H), 7.09-7.7.36 (m, 4H), 7.59 (quint, 1H),7.89 (br s, 1H), 8.58 (d, 1H). |
| | (22% d. Th.) | |
| **136** | 8-[(2,6-Difluorbenzyl)oxy]-N-[3-(4-fluorphenoxy)-2-hydroxypropyl]-2-methylimidazo[1,2-a]pyridin-3-carboxamid | LC-MS (Methode 1): Rₜ = 0.84 min |
| | | MS (ESpos): m/z = 486 (M+H)⁺ |
| | | ¹H-NMR (400 MHz, DMSO-d₆): |
| | 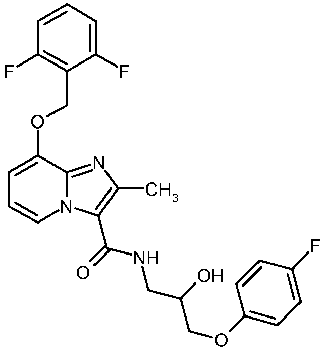 | δ = 2.52 (s, 3H; verborgen unter DMSO-Signal), 3.35-3.44 (m, 1H), 3.49-3.59 (m, 1H), 3.87-4.08 (m, 3H), 5.25-5.35 (m, 3H), 6.89-7.04 (m, 4H), 7.09-7.11 (t, 2H), 7.23 (t, 2H), 7.59 (quint, 1H),7.84 (t, 1H), 8.62 (d, 1H). |
| | (9% d. Th.) | |
| **137** | 8-[(2,6-Difluorbenzyl)oxy]-N-(2-hydroxy-3-phenoxypropyl)-2-methylimidazo[1,2-a]pyridin-3-carboxamid | LC-MS (Methode 1): Rₜ = 0.83 min |
| | | MS (ESpos): m/z = 468 (M+H)⁺ |
| | | ¹H-NMR (400 MHz, DMSO-d₆): |
| | 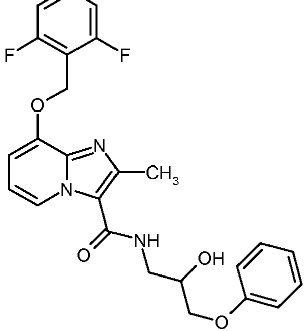 | δ = 2.52 (s, 3H; verborgen unter DMSO-Signal), 3.36-3.45 (m, 1H), 3.50-3.60 (m, 1H), 3.89-4.09 (m, 3H), 5.26-5.35 (m, 3H), 6.89-6.98 (m, 5H), 7.19-7.32 (m, 4H), 7.59 (quint, 1H),7.85 (t, 1H), 8.63 (d, 1H). |
| | (5% d. Th.) | |

| | | |
|---|---|---|
| ¹⁾ Das Produkt wurde durch zweimalige Reinigung an präparativen HPLC erhalten: 1) Präparative HPLC (RP18 Säule, Laufmittel: Acetonitril/Wasser-Gradient unter Zusatz von 0.1% TFA). 2) Säule: Sunfire C18, 5 µm, 250 x 20 mm, Eluent: 44% Wasser, 45% Methanol, 11% Waser + 1% TFA, 40°C, Detektion: 210 nm. | | |

### Beispiel 138

### 8-[(2,6-Difluorbenzyl)oxy]-2-methyl-N-(6,6,6-trifluor-1,5-dihydroxyhexan-2-yl)imidazo[1,2-a]pyridin-3-carboxamid (Stereoisomer B)

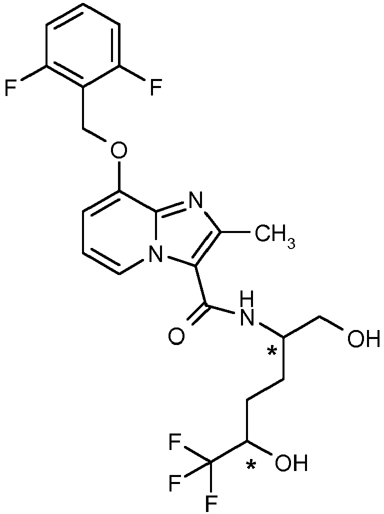

Durch präparative Trennung an chiraler Phase wurde Beispiel 135 (44 mg) in die Stereoisomere getrennt [Säule: Daicel Chiralpak AD-H, 5 µm, 250 x 20 mm, Eluent: 50% iso-Hexan, 50% Ethanol, Fluß 20 ml/min; 25°C, Detektion: 230 nm].
Ausbeute: 12.4 mg (99% Reinheit, >99% reines Stereoisomer)
Stereoisomer B: Rₜ = 3.34 min [Chiralpak AD-H, 5µm, 250 x 4 mm; Eluent: 50% iso-Hexan, 50% Ethanol; Fluss 1.0 ml/min; 25°C; Detektion: 230 nm].

### Beispiele 139 und 140

### 8-[(2,6-Difluorbenzyl)oxy]-N-[(3R)-2-hydroxyheptan-3-yl]-2-methylimidazo[1,2-a]pyridin-3-carboxamid [Diastereomerengemisch]

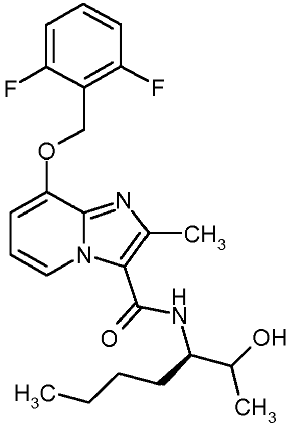

282 mg 8-[(2,6-Difluorbenzyl)oxy]-2-methyl-N-[(2R)-1-oxohexan-2-yl]imidazo[1,2-a]pyridin-3-carboxamid (0.68 mmol, 1 Äquivalent) wurden in 20 mL trockenem THF vorgelegt und bei 0 °C tropfenweise mit 450 µL Methylmagnesiumbromid-Lösung (3.0 M in Diethylether 1.36 mmol) versetzt. Die Reaktionsmischung wurde langsam auf RT erwärmt und für 5h gerührt. Die Reaktionsmischung wurde mit Essigsäureethylester verdünnt, mit gesättigter wässriger Natriumhydrogencarbonat-Lösung gewaschen, getrocknet, filtriert und eingeengt. Säulenchromatographie (Cyclohexan-Essigsäureethylester Gradient) lieferte 125 mg (43% der Theorie) des Produktes als Diastereomerengemisch (4:1) (HPLC- und NMR-Integration).

Durch Trennung per HPLC (Details siehe unten) wurden 20.4 mg Diastereomer 139 (erstes Diastereomer unter Methode 2; Rₜ = 0.99 min) und 85 mg des Diastereomers 140 (zweites Diastereomer unter Methode 2; Rₜ = 1.01 min) jeweils als Trifluoressigsäure-Salz erhalten. Die Trifluoressigsäure-Salze der reinen Diastereomere wurden in Dichlormethan aufgenommen, zweimal mit gesättigter wässriger Natriumhydrogencarbonat-Lösung gewaschen, getrocknet und wieder eingeengt und ergaben so 71 mg der freien Base des Hauptdiastereomers 140 sowie 12.6 mg der freien Base des Nebendiastereomers 139.

**HPLC-Bedingungen für Diastereomerentrennung:**

| | |
|---|---|
| Probenvorbereitung: | 125 mg in 3 ml Acetonitril/2 ml Wasser gelöst. |
| Packungsmaterial: | SUNFIRE C 18 OBD 5 µm 19*150 mm |
| Fluß | 25 ml/min |
| Wellenlänge | 210 nm |
| Injektionsvolumen | 350 µL |
| Temperatur | 40 °C |
| Eluent | 60/25/15 Milli-Q-Wasser/Acetonitril/1%ige Trifluoressigsäure. |

### Beispiel 139 Nebendiastereomer

LC-MS (Methode 2): Rₜ = 0.99 min
MS (ESpos): m/z = 432 (M+H)⁺
¹H-NMR (400 MHz, DMSO-d₆): δ = 0.87 (t, 3 H); 1.11 (d, 3 H); 1.23 - 1.42 (m, 5 H); 1.71 - 1.75 (m, 1 H); 2.52 (s, 3 H); 3.62 - 3.67 (m, 1 H); 3.82 - 3.89 (m, 1 H); 4.67 (d, 1 H); 5.31 (s, 2 H); 6.92 (t, 1 H); 6.98 (d, 1 H); 7.22 (t, 2 H); 7.51 (d, 1 H); 7.58 (quint., 1 H); 8.50 (d, 1 H).

### Beispiel 140 Hauptdiastereomer

LC-MS (Methode 2): Rₜ = 1.01 min
MS (ESpos): m/z = 432 (M+H)⁺
¹H-NMR (400 MHz, DMSO-d₆): δ = 0.87 (t, 3 H); 1.07 (d, 3 H); 1.25 - 1.36 (m, 4 H); 1.50 - 1.62 (m, 2 H); 2.52 (s, 3 H); 3.75 - 3.79 (m, 1 H); 3.89 - 3.94 (m, 1 H); 4.72 (d, 1 H); 5.31 (s, 2 H); 6.93 (t, 1 H); 6.99 (d, 1 H); 7.21 (t, 2 H); 7.27 (d, 1 H); 7.58 (quint., 1 H); 8.57 (d, 1 H).

### Beispiel 141 und 142

### 8-[(2,6-Difluorbenzyl)oxy]-2-methyl-N-[(3R)-1,1,1-trifluor-2-hydroxyheptan-3-yl]imidazo[1,2-a]pyridin-3-carboxamid

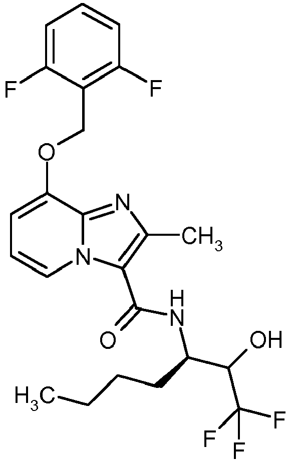

430 mg 8-[(2,6-Difluorbenzyl)oxy]-2-methyl-N-[(2R)-1-oxohexan-2-yl]imidazo[1,2-a]pyridin-3-carboxamid (1.05 mmol) wurden in 30 mL trockenem THF vorgelegt und bei 0 °C nacheinander tropfenweise mit 194 µL Trimethylsilyltrifluormethan (1.24 mmol) und 621 µL Tetrabutylammoniumfluorid-Lösung (1.0 M in THF; 0.62 mmol,) versetzt. Die Reaktionsmischung wurde langsam auf RT erwärmt und über Nacht weitergerührt. Dann wurde eingeengt, der Rückstand in Essigsäureethylester aufgenommen, mit gesättigter wässriger Natriumhydrogencarbonat-Lösung gewaschen, getrocknet, filtriert und eingeengt. Präparative HPLC-Trennung (Methode 9) lieferte 41 mg der Zielverbindung, die einer erneuten HPLC-Aufreinigung unterzogen wurden (Methode 10). Es wurden 28.4 mg der Zielverbindung erhalten. Das Diastereomerenverhältnis betrug ca. 3:1 (¹H-NMR Integration).

### Trennung der Diastereomere:

Säule: Daicel Chiralpak AD-H, 5µm 250 x 20 mm, Laufmittel: A = iso-Hexan, B = 2-Propanol + 0,2% TFA + 0,1% H₂O, 0 bis 15 min 1:1 A/B.

Die so erhaltenen TFA-Salze der reinen Diastereomere wurden mit gesättigter wässriger Natriumhydrogencarbonat-Lösung versetzt, mit Essigsäureethylester extrahiert, mit gesättigter Natriumchloridlösung gewaschen, über Magnesiumsulfat getrocknet und eingeengt.

### Beispiel 141 - Hauptdiastereomer

Ausbeute: 12.3 mg (2% d. Th., >98.5 % de)
LC (Daicel Chiralpak AD-H, 5µm 250 x 4.6 mm, Laufmittel: A = iso-Hexan, B = 2-Propanol + 0,2% TFA + 0,1% H₂O, 0 bis 15 min 1:1 A/B) Rₜ = 4.52 min
¹H-NMR (400 MHz, DMSO-d₆): δ = 0.87 (t, 3 H), 1.23 - 1.40 (m, 4 H), 1.61 - 1.75 (m, 2 H), 2.56 (s, 3 H; verdeckt durch DMSO-Signal), 4.10 (quint., 1 H), 4.40 (q, 1 H), 5.30 (s, 2 H), 6.64 (d, 1 H), 6.92 (t, 1 H), 6.98 (d, 1 H), 7.21 (t, 2 H), 7.46 (d, 1 H), 7.58 (quint., 1 H), 8.51 (d, 1 H).
spez. Drehwert: +39° (0.3 g/100 mL Methanol, 20.0°C, 100 mm Schichtdicke, 589 nm).

### Beispiel 142 - Nebendiastereomer

Ausbeute: 4.5 mg (0.9% d. Th., > 98.5% de)
LC (Daicel Chiralpak AD-H, 5µm 250 x 4.6 mm, Laufmittel: A = iso-Hexan, B = 2-Propanol + 0,2% TFA + 0,1% H₂O, 0 bis 15 min 1:1 A/B) Rₜ = 7.20 min
¹H-NMR (400 MHz, DMSO-d₆): δ = 0.87 (t, 3 H), 1.20 - 1.42 (m, 4 H), 1.57 - 1.65 (m, 1 H), 1.75 - 1.80 (m, 1 H), 2.56 (s, 3 H; verdeckt durch DMSO-Signal), 4.11 (quint., 1 H), 4.25 (q, 1 H), 5.30 (s, 2 H), 6.51 (d, 1 H), 6.91 (t, 1 H), 6.98 (d, 1 H), 7.21 (t, 2 H), 7.58 (quint., 1 H), 7.80 (d, 1 H), 8.50 (d, 1 H).
spez. Drehwert: +21° (0.225 g/100 mL Methanol, 19.9°C, 100 mm Schichtdicke, 589 nm).

### Beispiele 143, 144, 145 und 146

### 8-[(2,6-Difluorbenzyl)oxy]-2-methyl-N-(4,4,4-trifluoro-3-hydroxybutan-2-yl)imidazo[1,2-a]pyridin-3-carboxamid (Diastereomerengemisch)

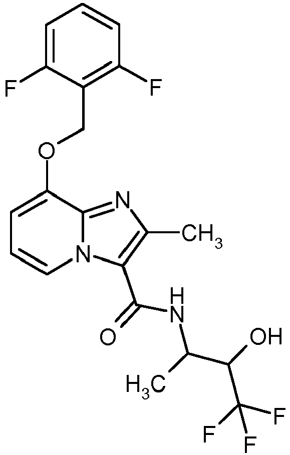

250 mg 8-[(2,6-Difluorbenzyl)oxy]-2-methylimidazo[1,2-a]pyridin-3-carbonsäure (0.785 mmol), 112 mg 3-Amino-1,1,1-trifluorbutan-2-ol (Gemisch aus vier Stereoisomeren; 0.785 mmol) und 277 mg TBTU (0.864 mmol) wurden in 1.5 mL Dichlormethan gelöst und für 48 h bei RT gerührt. Die Reaktionsmischung wurde eingeengt, der Rückstand in Methanol aufgenommen und durch präparative HPLC aufgereinigt (Methode 7). Es wurden 186 mg (53% der Theorie) der Titelverbindung erhalten, die per präparativer HPLC an chiraler Phase aufgetrennt wurden.

### HPLC-Bedingungen für Stereoisomerentrennung:

Säule 250 x 4,6 mm gefüllt mit Daicel Chiralpak AD-H, 5 µm getrennt. Fluß, 1.0 ml/min, Temperatur 45°C, Eluent, 50% iso-Hexan + 0.2% Trifluoressigsäure, 50% 2-Propanol + 2% Wasser.

Alle isolierten Stereoisomere wurden als Trifluoressigsäure-Salze bzw. mit Trifluoressigsäure-Anhaftungen erhalten. Die Stereoisomere wurden einzeln in Essigsäureethylester aufgenommen und mit gesättigter wässriger Natriumhydrogencarbonat-Lösung gewaschen, getrocknet und wieder eingeengt.

### Beispiel 143 - Stereoisomer 1

Ausbeute: 24 mg (7% d. Th., > 96% ee)
spezifischer Drehwert: -29° (598 nm, 20.4 °C, c = 0.275 g/100 ml, Methanol)
LC-MS (Methode 3): Rt = 1.65 min; Gehalt > 98% Reinheit
MS (ESpos): m/z = 443 (M+H)⁺
1H-NMR (400 MHz, DMSO-d6): δ = 1.27 (d, 3 H); 2.49 (s, 3 H, überlagert durch DMSO Signal); 4.08 - 4.16 (m, 1 H); 4.44 - 4.51 (m, 1 H); 5.31 (s, 2 H); 6.68 (d, 1 H); 6.94 (t, 1 H); 7.01 (d, 1 H); 7.23 (t, 1 H); 7.50 (d, 1 H); 7.58 (tt, 1 H); 8.63 (d, 1 H).

### Beispiel 144 - Stereoisomer 2

Ausbeute: 13 mg (4% d. Th., > 96% ee)
spezifischer Drehwert: +31° (598 nm, 20.1 °C, 0.28 g/100 ml, Methanol)
LC-MS (Methode 3): Rₜ = 1.64 min; Gehalt > 98% Reinheit
MS (ESpos): m/z = 443 (M+H)⁺
¹H-NMR (400 MHz, DMSO-d₆): δ = 1.27 (d, 3 H); 2.49 (s, 3 H, überlagert durch DMSO Signal); 4.08 - 4.16 (m, 1 H); 4.44 - 4.51 (m, 1 H); 5.31 (s, 2 H); 6.68 (d, 1 H); 6.94 (t, 1 H); 7.01 (d, 1 H); 7.23 (t, 1 H); 7.50 (d, 1 H); 7.58 (tt, 1 H); 8.63 (d, 1 H).

### Beispiel 145 - Stereoisomer 3

Ausbeute: 30 mg (9% d. Th., > 99% ee)
spezifischer Drehwert:-22.7° (598 nm, 20.5 °C, c = 0.33 g/100 ml, Methanol)
LC-MS (Methode 3): Rₜ = 1.67 min; Gehalt > 96% Reinheit
MS (ESpos): m/z = 443 (M+H)⁺
¹H-NMR (400 MHz, DMSO-d₆): δ = 1.25 (d, 3 H); 2.50 (s, 3 H, überlagert durch DMSO Signal); 4.12 - 4.22 (m, 1 H); 4.29 - 4.37 (m, 1 H); 5.31 (s, 2 H); 6.53 (d, 1 H); 6.95 (t, 1 H); 7.00 (d, 1 H); 7.23 (t, 1 H); 7.58 (tt, 1 H); 7.83 (d, 1 H); 8.55 (d, 1 H).

### Beispiel 146 - Stereoisomer 2

Ausbeute: 24 mg (7% d. Th., > 98% ee)
spezifischer Drehwert:+21.9° (598 nm, 20.8 °C, c = 0.33 g/100 ml, Methanol)
LC-MS (Methode 3): Rₜ = 1.66 min; Gehalt > 98% Reinheit
MS (ESpos): m/z = 443 (M+H)⁺
¹H-NMR (400 MHz, DMSO-d₆): δ = 1.25 (d, 3 H); 2.50 (s, 3 H, überlagert durch DMSO Signal); 4.14 - 4.20 (m, 1 H); 4.30 - 4.36 (m, 1 H); 5.31 (s, 2 H); 6.53 (d, 1 H); 6.93 (t, 1 H); 7.00 (d, 1 H); 7.23 (t, 1 H); 7.58 (tt, 1 H); 7.83 (d, 1 H); 8.55 (d, 1 H).

### Beispiel 147

### 8-[(2,6-Difluorbenzyl)oxy]-N-[(3R)-2-hydroxy-2-methylheptan-3-yl]-2-methylimidazo[1,2-a]pyridin-3-carboxamid

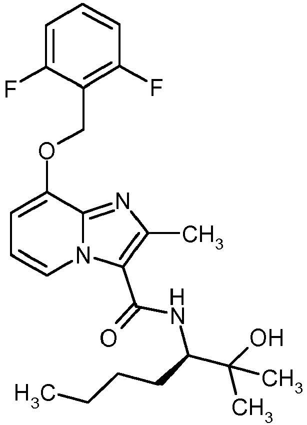

Unter Argon wurden 100 mg (0.224 mmol) Methyl-N-({8-[(2,6-difluorbenzyl)oxy]-2-methylimidazo[1,2-a]pyridin-3-yl}carbonyl)-D-norleucinat in 2 mL trockenem THF gelöst, auf 0 °C abgekühlt, tropfenweise mit 187 µL Methylmagnesiumbromid-Lösung (3.0 M in Diethylether; 0.561 mmol, 2.5 Äquivalente) versetzt und unter Rühren langsam auf RT gebracht. Nach 3 h bei RT wurde 1N Salzsäure zugesetzt sowie mit Wasser und Essigsäureethylester verdünnt. Die organische Phase wurde abgetrennt und die wässrige Phase zweimal mit Essigsäureethylester extrahiert. Die vereinten organischen Phasen wurden mit gesättigter wässriger Natriumchloridlösung gewaschen, mit Magnesiumsulfat getrocknet, filtiert und eingeengt. Der Rückstand wurde über eine 10 g Kieselgelkartusche chromatographiert (Biotage Isolera, Cyclohexan-Essigsäureethylester-Gradient als Eluent). Es wurden 39 mg der Zielverbindung (38% d. Th.) erhalten.
LC-MS (Methode 3): Rₜ = 1.88 min
MS (ESpos): m/z = 446.3 (M+H)⁺
¹H-NMR (400 MHz, DMSO-d₆): δ = 0.85 (t, 3 H), 1.10 (s, 3 H), 1.15 (s, 3 H), 1.20 - 1.47 (m, 5 H), 1.70 - 1.80 (m, 1 H), 2.55 (s, 3H; überlagert durch DMSO-Signal), 3.92 (t, 1H), 4.48 (s, 1 H), 5.31 (s, 2H), 6.92 (t, 1 H), 6.98 (d, 1 H), 7.21 (t, 2 H), 7.40 (d, 1 H), 7.60 (quint., 1 H), 8.51 (d, 1 H).

### Beispiel 148

### rac-8-[(2,6-Difluorbenzyl)oxy]-2-methyl-N-(1,1,1-trifluor-4-hydroxy-4-methylpentan-3-yl)imidazo[1,2-a]pyridin-3-carboxamid

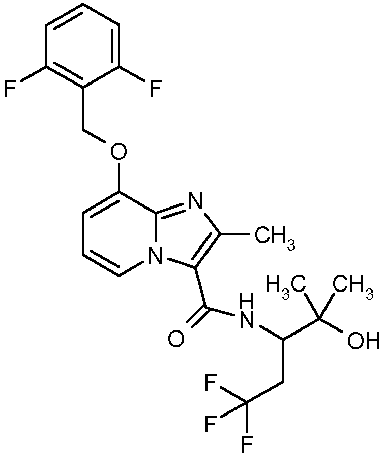

320 mg (0.68 mmol) Methyl 2-[({8-[(2,6-difluorbenzyl)oxy]-2-methylimidazo[1,2-a]pyridin-3-yl}carbonyl)amino]-4,4,4-trifluorbutanoat wurde in THF vorgelegt und die Lösung wurde auf 0°C abgekühlt. Es wurden 0.724 ml (2.17 mmol) Methylmagnesiumbromid-Lösung (3 M in Diethylether) zugetropft und 15 min bei 0°C gerührt. Das Reaktionsgemisch wurde langsam auf RT gebracht und es wurde 2 h bei dieser Temperatur gerührt. Dann wurde vorsichtig mit 1 N wässriger Salzsäure-Lösung angesäuert und die Reaktionslösung mittels präparativer HPLC gereinigt (RP18 Säule, Laufmittel: Acetonitril/Wasser-Gradient unter Zusatz von 0.1% TFA). Die Produktfraktionen wurden eingeengt und in Essigsäureethylester gelöst, mit 1 ml gesättigter, wässriger Natriumhydrogencarbonatlösung gewaschen, eingeengt und der Rückstand in Wasser/Acetonitril aufgenommen und lyophilisiert. Es wurden 94 mg der Zielverbindung (29% d. Th., Reinheit 96%) erhalten.
LC-MS (Methode 2): Rₜ = 0.94 min
MS (ESpos): m/z = 483 (M+H)⁺
¹H-NMR (400 MHz, DMSO-d₆): δ = 1.10 (s, 3H), 1.21 (s, 3H), 2.50 (s, 3H), 2.67-2.82 (m, 2H), 4.38 (t, 1H), 4.88 (s, 1H), 5.32 (s, 2H), 6.96 (t, 1H), 7.02 (d, 1H), 7.22 (t, 2H), 7.59 (quint, 1H), 7.82 (d, 1H), 8.43 (d, 1H).
In Analogie zu Beispiel 148 wurden die in Tabelle 13 gezeigten Beispielverbindungen hergestellt, indem die entsprechenden Methylester mit Methylmagnesiumbromid (2.5 bis 3.2 Äquivalente) zur Reaktion gebracht wurden.

**Tabelle 13:**

| **Beispiel** | **IUPAC-Name / Struktur (Ausbeute)** | **Analytische Daten** |
|---|---|---|
| **149** | *rac*-8-[(2,6-Difluorbenzyl)oxy]-2-methyl-*N-*(6,6,6-trifluor-2-hydroxy-2-methylhexan-3-yl)imidazo[1,2-a]pyridin-3-carboxamid ¹⁾ | LC-MS (Methode 1): Rₜ = 0.95 min |
| | | MS (ESpos): m/z = 486 (M+H)⁺ |
| | | ¹H-NMR (400 MHz, DMSO-d₆): |
| | 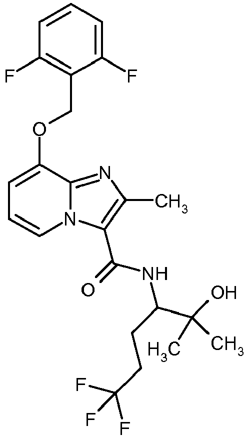 | δ = 1.12 (s, 3H), 1.20 (s, 3H), 1.60-1.74 (m, 1H), 1.95-2.07 (m, 1H), 2.18-2.33 (m, 2H), 2.50 (s, 3H), 3.98-4.07 (m, 1H), 4.69 (s, 1H), 5.31 (s, 2H), 6.93 (t, 1H), 7.02 (d, 1H), 7.23 (t, 2H), 7.53-7.63 (m, 2H), 8.56 (d, 1H). |
| | (53% d. Th.) | |
| **150** | *rac*-8-[(2,6-Difluorbenzyl)oxy]-2-methyl-*N-*(7,7,7-trifluor-2-hydroxy-2-methylheptan-3-yl)imidazo[1,2-a]pyridin-3-carboxamid | LC-MS (Methode 1): Rₜ = 0.97 min |
| | | MS (ESpos): m/z = 500 (M+H)⁺ |
| | | ¹H-NMR (400 MHz, DMSO-d₆): |
| | 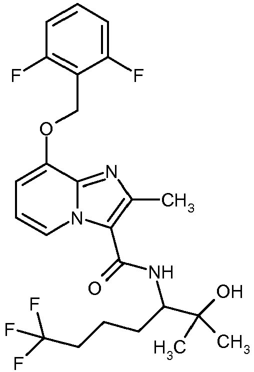 | δ = 1.12 (s, 3H), 1.19 (s, 3H), 1.42-1.65 (m, 3H), 1.79-1.88 (m, 1H), 2.13-2.29 (m, 1H), 2.33-2.46 (m, 1H), 2.50 (s, 3H), 3.94-4.02 (m, 1H), 4.57 (s, 1H), 5.31 (s, 2H), 6.94 (t, 1H), 7.02 (d, 1H), 7.22 (t, 2H), 7.48 (d, 1H), 7.59 (quint, 1H), 8.50 (d, 1H). |
| | (30% d. Th.) | |
| **151** | *rac-N-*[1-(4-Chlorphenyl)-2-hydroxy-2-methylpropyl]-8-[(2,6-difluorbenzyl)oxy]-2-methylimidazo[1,2-a]pyridin-3-carboxamid ²⁾ | LC-MS (Methode 1): Rₜ = 1.15 min |
| | | MS (ESpos): m/z = 500 (M+H)⁺ |
| | | ¹H-NMR (400 MHz, DMSO-d₆): |
| | 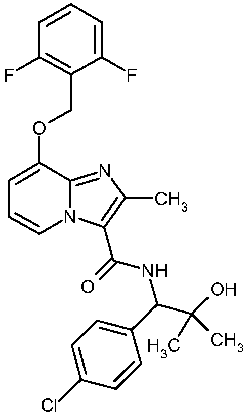 | δ = 1.03 (s, 3H), 1.25 (s, 3H), 2.61 (s, 3H), 4.89 (s, 1H), 4.92 (d, 1H), 5.31 (s, 2H), 6.91 (t, 1H), 7.02 (d, 1H), 7.24 (t, 2H), 7.38 (d, 2H), 7.48 (d, 2H), 7.59 (quint, 1H), 7.91 (d, 1H), 8.58 (d, 1H). |
| | (38% d. Th.) | |
| **152** | *rac*-8-[(2,6-Difluorbenzyl)oxy]-*N*-[1-(4-fluorphenyl)-2-hydroxy-2-methylpropyl]-2-methylimidazo[1,2-a]pyridin-3-carboxamid ²⁾ | LC-MS (Methode 1): Rₜ = 0.97 min |
| | | MS (ESpos): m/z = 484 (M+H)⁺ |
| | | ¹H-NMR (400 MHz, DMSO-d₆): |
| | 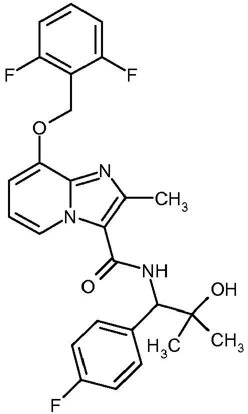 | δ = 1.02 (s, 3H), 1.25 (s, 3H), 2.61 (s, 3H), 4.87 (s, 1H), 4.93 (d, 1H), 5.31 (s, 2H), 6.90 (t, 1H), 7.02 (d, 1H), 7.14 (t, 2H), 7.22 (t, 2H), 7.45-7.51 (m, 2H), 7.59 (quint, 1H), 7.89 (d, 1H), 8.58 (d, 1H). |
| | (42% d. Th.) | |
| **153** | 8-[(2,6-Difluorbenzyl)oxy]-*N*-(3-hydroxy-3-methylbutyl)-2-methylimidazo[1,2-a]pyridin-3-carboxamid ²⁾ | LC-MS (Methode 1): Rₜ = 0.77 min |
| | | MS (ESpos): m/z = 404 (M+H)⁺ |
| | | ¹H-NMR (400 MHz, DMSO-d₆): |
| | 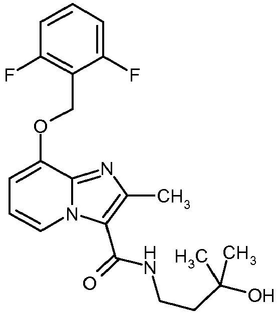 | δ = 1.16 (s, 6H), 1.69 (t, 2H), 2.50 (s, 3H), 3.41 (q, 2H), 4.48 (s, 1H), 5.31 (s, 2H), 6.91 (t, 1H), 7.00 (d, 1H), 7.22 (t, 2H), 7.59 (quint, 1H), 7.84 (t, 1H), 8.68 (d, 1H). |
| | (70% d. Th.) | |

| | | |
|---|---|---|
| ¹⁾ Aufarbeitung: Das Reaktionsgemisch wurde vorsichtig mit 1 N wässriger Salzsäure-Lösung angesäuert und dreimal mit Essigsäureethylester extrahiert. Die vereinten organischen Phasen wurden mit gesättigter Natriumchlorid-Lösung gewaschen, über Natriumsulfat getrocknet, filtriert und das Filtrat wurde eingeengt. Das Rohprodukt wurde mittels Kieselgel-Chromatographie gereinigt (Laufmittel: Dichlormethan:Methanol = 100:1 isokratisch). ²⁾ Aufarbeitung und Reinigung: Das Reaktionsgemisch wurde vorsichtig mit 1 N wässriger Salzsäure-Lösung angesäuert und anschließend mit Wasser und Essigsäureethylester versetzt. Nach Phasentrennung wurde die wässrige Phase zweimal mit Essigsäureethylester extrahiert und die vereinten organischen Phasen wurden mit gesättigter, wässriger Natriumchlorid-Lösung gewaschen. Es wurde über Natriumsulfat getrocknet, filtriert und eingeengt. Der Rückstand wurde mittels Kieselgel-Chromatographie gereinigt (Laufmittel: Cyclohexan:Essigsäureethylester-Gradient). | | |

### Beispiel 154

### ent-8-[(2,6-Difluorbenzyl)oxy]-2-methyl-N-(1,1,1-trifluor-4-hydroxy-4-methylpentan-3-yl)imidazo[1,2-a]pyridin-3-carboxamid (Enantiomer B)

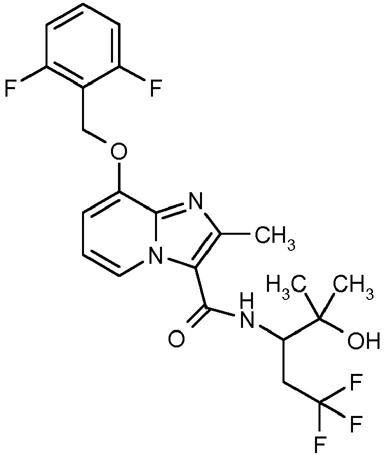

Durch präparative Trennung an chiraler Phase wurde Beispiel 148 (94 mg) in die Enantiomere getrennt [Säule: Daicel Chiralpak AD-H, 5 µm, 250 x 20 mm, Eluent: 50% iso-Hexan, 50% Isopropanol, Fluß 15 ml/min; 30°C, Detektion: 220 nm].
Ausbeute: 37 mg (99% Reinheit, >99% ee)
Enantiomer B: Rₜ = 6.23 min [Chiralpak AD-H, 5µm, 250 x 4.6 mm; Eluent: 50% iso-Hexan, 50% Isopropanol; Fluss 1.0 ml/min; 30°C; Detektion: 220 nm].

### Beispiel 155

### ent-8-[(2,6-Difluorbenzyl)oxy]-2-methyl-N-(6,6,6-trifluor-2-hydroxy-2-methylhexan-3-yl)imidazo[1,2-a]pyridin-3-carboxamid (Enantiomer B)

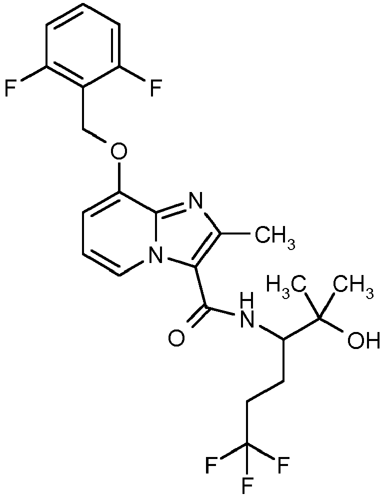

Durch präparative Trennung an chiraler Phase wurde Beispiel 149 (133 mg) in die Enantiomere getrennt [Säule: Daicel Chiralpak AD-H, 5 µm, 250 x 20 mm, Eluent: 50% iso-Hexan, 50% Isopropanol + 0.2% Diethylamin, Fluß 15 ml/min; 30°C, Detektion: 220 nm].
Ausbeute: 51 mg (99% Reinheit, >99% ee)
Enantiomer B: Rₜ = 5.41 min [Chiralpak AD-H, 5µm, 250 x 4.6 mm; Eluent: 50% iso-Hexan, 50% Isopropanol + 0.2% Diethylamin; Fluss 1.0 ml/min; 40°C; Detektion: 235 nm].

### Beispiel 156

### ent-8-[(2,6-Difluorbenzyl)oxy]-2-methyl-N-(7,7,7-trifluor-2-hydroxy-2-methylheptan-3-yl)imidazo[1,2-a]pyridin-3-carboxamid (Enantiomer B)

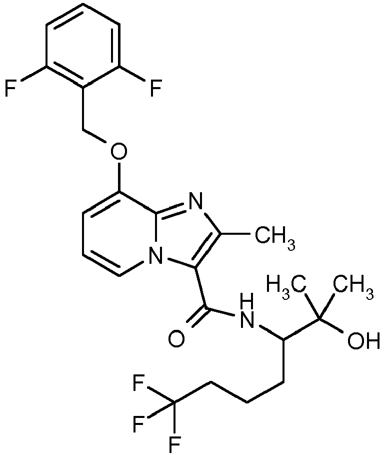

Durch präparative Trennung an chiraler Phase wurde Beispiel 150 (83 mg) in die Enantiomere getrennt [Säule: Daicel Chiralpak AD-H, 5 µm, 250 x 20 mm, Eluent: 50% iso-Hexan, 50% Isopropanol, Fluß 15 ml/min; 30°C, Detektion: 220 nm].
Ausbeute: 28 mg (99% Reinheit, >99% ee)
Enantiomer B: Rₜ = 6.26 min [Chiralpak AD-H, 5µm, 250 x 4.6 mm; Eluent: 50% iso-Hexan, 50% Isopropanol; Fluss 1.0 ml/min; 30°C; Detektion: 220 nm].

### Beispiel 157

### 6-Cyan-8-[(2,6-difluorbenzyl)oxy]-N-[(2R)-1-hydroxyhexan-2-yl]-2-methylimidazo[1,2-a]pyridin-3-carboxamid

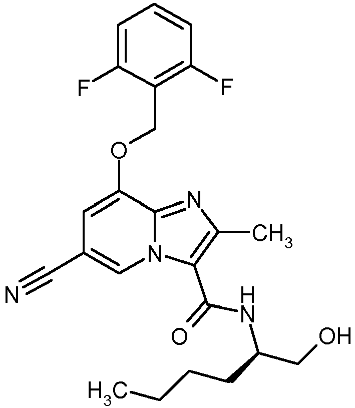

Unter Argon wurden 500 mg (1.0 mmol) 6-Brom-8-[(2,6-difluorbenzyl)oxy]-N-[(2R)-1-hydroxyhexan-2-yl]-2-methylimidazo[1,2-a]pyridin-3-carboxamid, 70.9 mg Zink(II)cyanid (0.6 mmol, 0.6 Äquivalente), 30.4 µL 1,2-Bis(dimethylamino)ethan (TMEDA; 0.2 mmol, 0.2 Äquivalente), 65 mg Tris(dibenzylidenaceton)dipalladium Pd₂dba₃ (0.07 mmol, 7 mol%) und 29 mg Xantphos-Ligand (0.05 mmol, 5 mol%) in 2 mL DMF gelöst und verteilt auf 5 Mikrowellen-Reaktionsgefäße für 5 min auf 160 °C erhitzt (CEM Discovery Mikrowelle, 300 Watt Einstrahlung). Die vereinigten Reaktionsgemische wurden mit Essigsäureethylester verdünnt und durch Kieselgur filtiert. Der Filterkuchen wurde gut nachgewaschen, das Filtrat wurde mit Wasser gewaschen, über Magnesiumsulfat getrocknet, filtiert und eingeengt. Der Rückstand wurde über eine 50g Kieselgelkartusche chromatographiert (Biotage Isolera, Cyclohexan/Essigsäureethylester-Gradient als Eluent). Es wurden 289 mg der Zielverbindung (64% d. Th.) erhalten.
LC-MS (Methode 1): Rₜ = 1.05 min
MS (ESpos): m/z = 443.3 (M+H)⁺
¹H-NMR (400 MHz, DMSO-d₆): δ = 0.85 (t, 3 H), 1.20 - 1.42 (m, 4 H), 1.45 - 1.57 (m, 1 H), 1.60 - 1.70 (m, 1 H), 2.55 (s, 3H; überlagert durch DMSO-Signal), 3.42 - 3.53 (m, 2 H), 3.90 - 4.02 (m, 1 H), 4.75 (t, 1 H), 5.31 (s, 2H), 7.21 (t, 2 H), 7.33 (s, 1 H), 7.60 (quint., 1 H), 7.79 (s, 1 H), 9.08 (s, 1 H).

### Beispiel 158

### 8-(Cyclobutylmethoxy)-N-[(2R)-1-hydroxyhexan-2-yl]-2-methylimidazo[1,2-a]pyridin-3-carboxamid

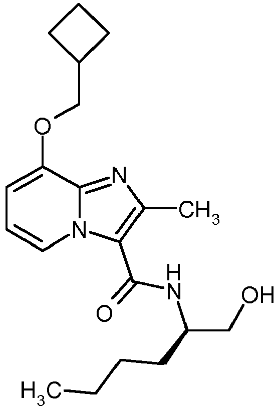

100 mg (0.34 mmol) 8-Hydroxy-*N-*[(2R)-1-hydroxyhexan-2-yl]-2-methylimidazo[1,2-a]pyridin-3-carboxamid, 246 mg (0.76 mmol) Cäsiumcarbonat und 56 mg (0.38 mmol) (Brommethyl)cyclobutan wurden in 4.9 ml trockenem DMF gegeben und für 60 min in einem vorgeheizten 60°C warmen Ölbad gerührt. Nach Abkühlen wurde das Reaktionsgemisch mit ca. 40 ml Wasser versetzt und die entstandene Lösung wurde 3x mit Essigsäureethylester extrahiert. Die vereinten organischen Phasen wurden eingeengt und der Rückstand wurde über eine präparative HPLC gereinigt (RP18 Säule, Laufmittel: Wasser/Methanol-Gradient unter Zusatz von 0.1% TFA). Alle produkthaltigen Fraktionen wurden vereint und eingedampft. Der Rückstand wurde mit gesättigter, wässriger Natriumhydrogencarbonat-Lösung versetzt und dreimal mit Essigsäureethylester extrahiert. Die vereinten organischen Phasen wurden über Natriumsulfat getrocknet, filtriert, eingeengt und im Hochvakuum getrocknet. Es wurden 78 mg der Zielverbindung (63% d. Th., Reinheit 100%) erhalten.
LC-MS (Methode 1): Rₜ = 0.81 min
MS (ESpos): m/z = 360 (M+H)⁺
¹H-NMR (400 MHz, DMSO-d₆): δ = 0.88 (t, 3H), 1.21-1.53 (m, 5H), 1.58-1.71 (m, 1H), 1.81-1.99 (m, 4H), 2.09-2.18 (m, 2H), 2.50 (s, 3H), 2.73-2.84 (m, 1H), 3.39-3.54 (m, 2H), 3.92-4.03 (m, 1H), 4.12 (d, 2H), 4.72 (t, 1H), 6.78 (d, 1H), 6.84 (t, 1H). 7.48 (d, 1H), 8.48 (d, 1H).

### Beispiel 159

### N-[(2R)-1-Hydroxyhexan-2-yl]-2-methyl-8-(3-methylbutoxy)imidazo[1,2-a]pyridin-3-carboxamid

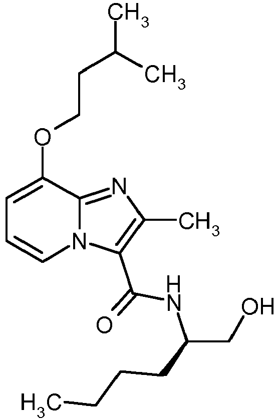

100 mg (0.34 mmol) 8-Hydroxy-*N-*[(2R)-1-hydroxyhexan-2-yl]-2-methylimidazo[1,2-a]pyridin-3-carboxamid, 246 mg (0.76 mmol) Cäsiumcarbonat und 75 mg (0.38 mmol) 1-Iod-3-methylbutan wurden in 4.9 ml trockenes DMF gegeben und für 30 min in einem vorgeheizten 60°C warmen Ölbad gerührt. Nach dem Abkühlen wurde das Reaktionsgemisch mit ca. 40 ml Wasser versetzt und die entstandene Lösung wurde dreimal mit Essigsäureethylester extrahiert. Die vereinten organischen Phasen wurden eingeengt und der Rückstand wurde über eine präparative HPLC gereinigt (RP18 Säule, Laufmittel: Wasser/Methanol-Gradient unter Zusatz von 0.1% TFA). Alle produkthaltigen Fraktionen wurden vereint und eingedampft. Der Rückstand wurde mit gesättigter, wässriger Natriumhydrogencarbonat-Lösung versetzt und dreimal mit Essigsäureethylester extrahiert. Die vereinten organischen Phasen wurden über Natriumsulfat getrocknet, filtriert, eingeengt und im Hochvakuum getrocknet. Es wurden 82 mg der Zielverbindung (66% d. Th., Reinheit 100%) erhalten.
LC-MS (Methode 1): Rₜ = 0.85 min
MS (ESpos): m/z = 362 (M+H)⁺
¹H-NMR (400 MHz, DMSO-d₆): δ = 0.88 (t, 3H), 0.96 (d, 6H), 1.24-1.53 (m, 5H), 1.59-1.73 (m, 3H), 1.78-1.90 (m, 1H), 2.50 (s, 3H), 3.39-3.53 (m, 2H), 3.92-4.03 (m, 1H), 4.18 (t, 2H), 4.72 (t, 1H), 6.78 (d, 1H), 6.86 (t, 1H). 7.48 (d, 1H), 8.48 (d, 1H).

### Beispiel 160

### rac-8-[(2,6-Difluorbenzyl)oxy]-N-(1-ethoxy-3-hydroxypropan-2-yl)-2-methylimidazo[1,2-a]pyridin-3-carboxamid

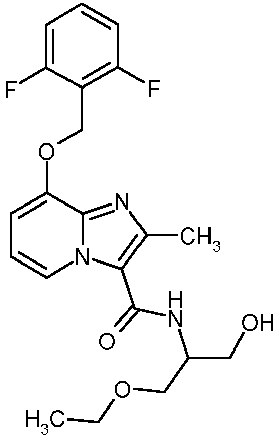

750 mg (1.92 mmol) 8-[(2,6-Difluorbenzyl)oxy]-*N*-(1,3-dihydroxy-propan-2-yl)-2-methylimidazo[1,2-a]pyridin-3-carboxamid wurden in 19 ml DMF bei 0°C vorgelegt, mit 52 mg (2.11 mmol) Natriumhydrid (95%ig) versetzt und 30 min bei 0°C gerührt. Anschließend wurde bei 0°C langsam eine Lösung von 239 mg (1.53 mmol) Iodethan in 2 ml DMF zugetropft und es wurde über Nacht bei RT gerührt. Dann wurden bei Raumtemperatur noch 9.4 mg (0.38 mmol) Natriumhydrid (95%ig) zugegeben, 30 min gerührt und anschließend nochmals 59.8 mg (0.38 mmol) Iodethan zugetropft und über Nacht bei Raumtemperatur gerührt. Das Reaktionsgemisch wurde mit etwas Methanol versetzt, auf 20 ml Wasser gegossen und zweimal mit Essigsäureethylester extrahiert. Die vereinten organischen Phasen wurden mit wässriger, gesättigter Natriumchlorid-Lösung gewaschen, über Natriumsulfat getrocknet, filtriert und eingeengt. Der Rückstand wurde mittels Kieselgelchromatographie gereinigt (Laufmittel Dichlormethan:Methanol = 40:1, 20:1). Es wurden 280 mg der Zielverbindung (33% d. Th., Reinheit 96%) erhalten.
LC-MS (Methode 2): Rₜ = 0.82 min
MS (ESpos): m/z = 420 (M+H)⁺
¹H-NMR (400 MHz, DMSO-d₆): δ = 1.12 (t, 3H), 2.50 (s, 3H), 3.42-3.60 (m, 6H), 4.09-4.18 (m, 1H), 4.72 (t, 1H), 5.31 (s, 2H), 6.93 (t, 1H), 7.00 (d, 1H), 7.22 (t, 2H), 7.53 (d, 1H), 7.59 (quint, 1H), 8.59 (d, 1H).

### Beispiel 161

### ent-8-[(2,6-Difluorbenzyl)oxy]-N-(1-ethoxy-3-hydroxypropan-2-yl)-2-methylimidazo[1,2-a]pyridin-3-carboxamid (Enantiomer A)

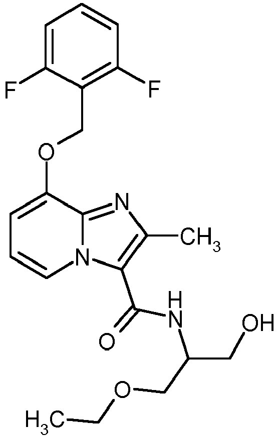

Durch präparative Trennung an chiraler Phase wurde Beispiel 160 (280 mg) in die Enantiomere getrennt [Säule: Daicel Chiralcel OD-H, 5 µm, 250 x 20 mm; Eluent: 50% iso-Hexan, 50% Isopropanol; Fluß 15 ml/min; 40°C; Detektion: 220 nm]
Ausbeute: 116 mg (99% Reinheit, >99% ee)
Enantiomer A: Rₜ= 4.88 min [Chiralcel OD-H, 5µm, 250 x 4.6 mm; Eluent: 50% iso-Hexan, 50% Isopropanol + 0.2% TFA + 1% Wasser; Fluss 1.0 ml/min; 40°C; Detektion: 220 nm].

### Beispiel 162

### rac-8-[(2,6-Difluorbenzyl)oxy]-N-(1-hydroxy-3-propoxypropan-2-yl)-2-methylimidazo[1,2-a]pyridin-3-carboxamid

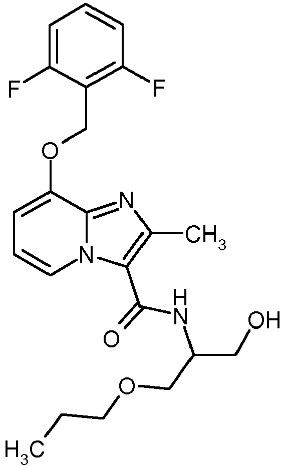

100 mg (0.26 mmol) 8-[(2,6-Difluorbenzyl)oxy]-N-(1,3-dihydroxy-propan-2-yl)-2-methylimidazo[1,2-a]pyridin-3-carboxamid wurden in 2.4 ml DMF bei 0°C vorgelegt, mit 7.1 mg (0.28 mmol) Natriumhydrid (95%ig) versetzt und 30 min bei 0°C gerührt. Anschließend wurde bei 0°C langsam eine Lösung von 35 mg (0.20 mmol) Iodpropan in 0.4 ml DMF zugetropft und über Nacht bei RT gerührt. Bei Raumtemperatur wurden noch 1.6 mg (0.064 mmol) Natriumhydrid (95%ig) zugegeben, 30 min gerührt und anschließend nochmals 10.6 mg (0.064 mmol) Iodpropan zugetropft und über Nacht bei Raumtemperatur gerührt. Dann wurden abermals bei RT 1.6 mg (0.064 mmol) Natriumhydrid (95%ig) zugegeben, 30 min gerührt und anschließend 10.6 mg (0.064 mmol) Iodpropan zugetropft und über Nacht bei Raumtemperatur gerührt. Das Reaktionsgemisch wurde mit etwas Methanol versetzt, auf 20 ml Wasser gegossen und zweimal mit Essigsäureethylester extrahiert. Die vereinten organischen Phasen wurden mit wässriger, gesättigter Natriumchlorid-Lösung gewaschen, über Natriumsulfat getrocknet, filtriert und eingeengt. Der Rückstand wurde mittels Kieselgelchromatographie gereinigt (Laufmittel: Cyclohexan:Essigsäureethylester = 7:3, dann Dichlormethan:Methanol = 40:1). Das erhaltene Rohprodukt wurde nochmals mittel präparativer Dickschichtchromatographie (Dichlormethan:Methanol = 20:1) gereinigt. Es wurden 44 mg der Zielverbindung (38% d. Th., Reinheit 95%) erhalten.
LC-MS (Methode 1): Rₜ = 0.82 min
MS (ESpos): m/z = 434 (M+H)⁺
¹H-NMR (400 MHz, DMSO-d₆): δ = 0.87 (t, 3H), 1.51 (sextett, 2H), 2.50 (s, 3H), 3.35-3.42 (m, 2H), 3.46-3.60 (m, 4H), 4.10-4.19 (m, 1H), 4.72 (t, 1H), 5.31 (s, 2H), 6.93 (t, 1H), 7.00 (d, 1H), 7.22 (t, 2H), 7.52 (d, 1H), 7.59 (quint, 1H), 8.59 (d, 1H).

### Beispiel 163

### rac-8-[(2,6-Difluorbenzyl)oxy]-N-(1-hydroxy-3-isobutoxypropan-2-yl)-2-methylimidazo[1,2-a]pyridin-3-carboxamid

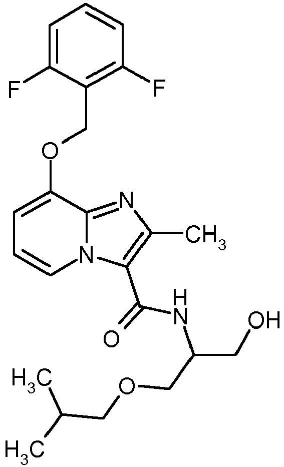

250 mg (0.64 mmol) 8-[(2,6-Difluorbenzyl)oxy]-N-(1,3-dihydroxy-propan-2-yl)-2-methylimidazo[1,2-a]pyridin-3-carboxamid und 4.94 g (26.83 mmol) 1-Iod-2-methylpropan wurden in trockenem Toluol vorgelegt, mit 1.87 g (7.66 mmol) Silber(I)oxid und 118 mg (0.32 mmol) Tetra-n-butyl-ammoniumiodid versetzt und über Nacht bei 40°C gerührt. Das Reaktionsgemisch wurde über Celite filtriert, gut gewaschen, das Filtrat eingeengt und der Rückstand mittels präparativer HPLC gereinigt (RP18 Säule, Laufmittel: Acetonitril/Wasser-Gradient unter Zusatz von 0.1% TFA). Das Rohprodukt wurde anschließend über ein präparative Dickschichtchromatographie gereinigt (Laufmittel: Dichlormethan:Methanol = 20:1). Es wurden 13 mg der Zielverbindung (4.5% d. Th., Reinheit 100%) erhalten.
LC-MS (Methode 1): Rₜ = 0.92 min
MS (ESpos): m/z = 448 (M+H)⁺
¹H-NMR (400 MHz, DMSO-d₆): δ = 0.85 (d, 6H), 1.75-1.87 (m, 1H), 2.50 (s, 3H), 3.18-3.24 (m, 2H), 3.46-3.60 (m, 4H), 4.10-4.19 (m, 1H), 4.72 (t, 1H), 5.31 (s, 2H), 6.93 (t, 1H), 7.00 (d, 1H), 7.22 (t, 2H), 7.52 (d, 1H), 7.59 (quint, 1H), 8.59 (d, 1H).

### Beispiel 164

### Methyl-N-({8-[(2,6-difluorbenzyl)oxy]-2-methylimidazo[1,2-a]pyridin-3-yl}carbonyl)-L-serinat

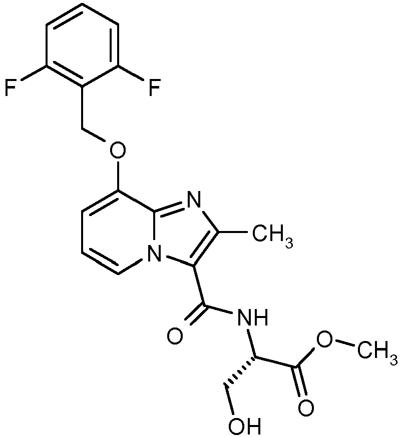

500 mg (1.57 mmol) 8-[(2,6-Difluorbenzyl)oxy]-2-methylimidazo[1,2-a]pyridin-3-carbonsäure Beispiel 6A, 756 mg (2.36 mmol) (Benzotriazol-1-yloxy)bisdimethylaminomethyliumfluoroborat (TBTU) und 794 mg (7.89 mmol) 4-Methylmorpholin wurden in 3 ml DMF vorgelegt. Nach 10 min bei RT wurden 293 mg (1.89 mmol) (S)-Serinmethylester Hydrochlorid zugesetzt und es wurde über Nacht bei RT gerührt. Die Reaktionslösung wurde mit ca. 80 ml Wasser versetzt, der entstandene Niederschlag noch 30 min ausgerührt, ab filtriert, gut mit kaltem Diethylether gewaschen und über Nacht im Hochvakuum getrocknet. Es wurden 601 mg der Zielverbindung (89% d. Th., Reinheit 98%) erhalten.
LC-MS (Methode 1): Rₜ = 0.74 min
MS (ESpos): m/z = 420 (M+H)⁺
¹H-NMR (400 MHz, DMSO-d₆): δ = 2.58 (s, 3H), 3.70 (s, 3H), 3.78-3.88 (m, 2H), 4.59-4.65 (m, 1H), 5.13 (t, 1H), 5.30 (s, 2H), 6.96 (t, 1H), 7.04 (d, 1H), 7.22 (t, 2H), 7.59 (quint, 1H), 7.92 (d, 1H), 8.62 (d, 1H).

### Beispiel 165

### 8-[(2,6-Difluorbenzyl)oxy]-N-[(2S)-1,3-dihydroxy-3-methylbutan-2-yl]-2-methylimidazo[1,2-a]pyridin-3-carboxamid

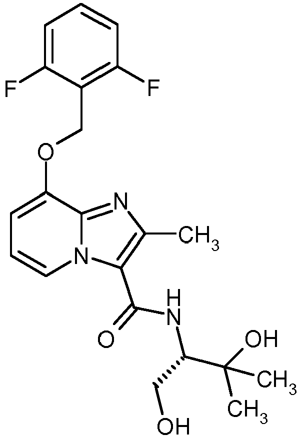

Unter Argon wurden 100 mg (0.24 mmol) Methyl-N-({8-[(2,6-difluorbenzyl)oxy]-2-methylimidazo[1,2-a]pyridin-3-yl}carbonyl)-L-serinat in 2.3 mL trockenem THF suspendiert, auf 0°C abgekühlt, tropfenweise mit 0.20 ml Methylmagnesiumbromid-Lösung (3.0 M in Diethylether; 0.60 mmol, 2.5 Äquivalente) versetzt und unter Rühren langsam auf RT gebracht. Nach Rühren bei RT über Nacht wurden bei 0°C nochmals 0.1 ml Methylmagnesiumbromid-Lösung (3.0 M in Diethylether) zugetropft und es wurde 6 h bei RT gerührt. Anschließend wurden nochmals 0.1 ml Methylmagnesiumbromid-Lösung (3.0 M in Diethylether) zugetropft und über Nacht bei RT gerührt. Es wurde 1N wässrige Salzsäure zugesetzt und mit Essigsäureethylester verdünnt. Die organische Phase wurde abgetrennt und die wässrige Phase dreimal mit Essigsäureethylester extrahiert. Die vereinten organischen Phasen wurden mit gesättigter Natriumchloridlösung gewaschen, mit Natriumsulfat getrocknet, filtiert und eingeengt. Der Rückstand wurde über eine Kieselgelkartusche chromatographiert (Dichlormethan:Methanol = 40:1 als Eluent). Es wurden 46 mg der Zielverbindung (45% d. Th.) erhalten.
LC-MS (Methode 1): Rₜ = 0.68 min
MS (ESpos): m/z = 420.3 (M+H)⁺
¹H-NMR (400 MHz, DMSO-d₆): δ = 1.12 (s, 3 H), 1.20 (s, 3 H), 2.55 (s, 3H; überlagert durch DMSO-Signal), 3.56-3.61 (m, 1 H), 3.73-3.80 (m, 1 H), 3.96-4.03 (m, 1 H), 4.57 (s, 1 H), 4.61 (t, 1H), 5.31 (s, 2 H), 6.92 (t, 1 H), 6.99 (d, 1 H), 7.23 (t, 2 H), 7.30 (d, 1 H), 7.59 (quint., 1 H), 8.62 (d, 1 H).

### Beispiel 166

### N-[(2R)-1-Hydroxyhexan-2-yl]-2,6-dimethyl-8-(3-methylbutoxy)imidazo[1,2-a]pyridin-3-carboxamid

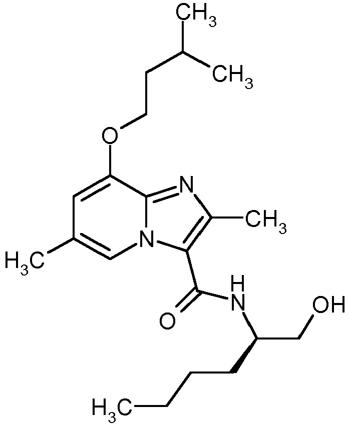

75 mg (0.27 mmol) 2,6-Dimethyl-8-(3-methylbutoxy)imidazo[1,2-a]pyridin-3-carbonsäure Beispiel 89A, 96 mg (0.30 mmol) TBTU und 110 mg (1.09 mmol) 4-Methylmorpholin wurden in 1.73 ml DMF vorgelegt. Dann wurden 35 mg (0.30 mmol) (R)-(-)-2-Aminohexanol hinzugegeben und über Nacht bei Raumtemperatur gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1% TFA). Die eingeengten Fraktionen wurden in Dichlormethan aufgenommen und zweimal mit gesättigter, wässriger Natriumhydrogen-carbonatlösung gewaschen. Die wässrige Phase wurde noch zweimal mit Dichlormethan extrahiert, die vereinigten organischen Phasen über Natriumsulfat getrocknet, filtriert, das Filtrat eingeengt und lyophilisiert. Es wurden 91 mg der Zielverbindung (87.5% d. Th., Reinheit 98%) erhalten.
LC-MS (Methode 1): Rₜ = 0.88 min
MS (ESpos): m/z = 376.5 (M+H)⁺
¹H-NMR (400 MHz, DMSO-d₆): δ = 0.88 (t, 3H), 0.96 (d, 6H), 1.23-1.50 (m, 5H), 1.59-1.73 (m, 3H), 1.78-1.88 (m, 1H), 2.28 (s, 3H), 2.56 (s, 3H), 3.38-3.53 (m, 2H), 3.90-4.02 (m, 1H), 4.15 (t, 2H), 4.74 (t, 1H), 6.69 (s, 1H), 7.48 (d, 1H), 8.30 (s, 1H).

### Beispiel 167

### ent-N-(2-Hydroxy-2-methylheptan-3-yl)-2,6-dimethyl-8-(3-methylbutoxy)imidazo[1,2-a]pyridin-3-carboxamid

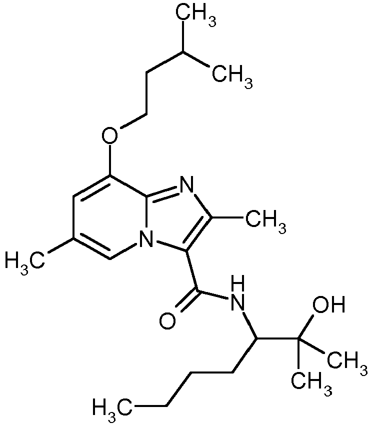

60 mg (0.22 mmol) 2,6-Dimethyl-8-(3-methylbutoxy)imidazo[1,2-a]pyridin-3-carbonsäure Beispiel 89A, 77 mg (0.24 mmol) TBTU und 88 mg (0.87 mmol) 4-Methylmorpholin wurden in 1.38 ml DMF vorgelegt. Dann wurden 43 mg (0.24 mmol) *ent*-3-Amino-2-methylheptan-2-olhydrochlorid aus Beispiel 98A hinzugegeben und über Nacht bei Raumtemperatur gerührt. Anschließend wurden nochmals 8 mg (0.04 mmol) *ent*-3-Amino-2-methylheptan-2-olhydrochlorid hinzugegeben und die Reaktionslösung wurde über Nacht bei RT gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1% TFA). Die eingeengten Fraktionen wurden in Dichlormethan aufgenommen und zweimal mit gesättigter, wässriger Natriumhydrogencarbonatlösung gewaschen. Die wässrige Phase wurde noch zweimal mit Dichlormethan extrahiert, die vereinigten organischen Phasen über Natriumsulfat getrocknet, filtriert, das Filtrat eingeengt und lyophilisiert. Es wurden 48 mg der Zielverbindung (54% d. Th., Reinheit 99%) erhalten.
LC-MS (Methode 17): Rₜ = 0.91 min
MS (ESpos): m/z = 404 (M+H)⁺
¹H-NMR (400 MHz, DMSO-d₆): δ = 0.88 (t, 3H), 0.96 (d, 6H), 1.11 (s, 3H), 1.18 (s, 3H), 1.20-1.48 (m, 5H), 1.66-1.88 (m, 4H), 2.28 (s, 3H), 2.54 (s, 3H), 3.88-3.95 (m, 1H), 4.18 (t, 2H), 4.49 (s, 1H), 6.72 (s, 1H), 7.33 (d, 1H), 8.29 (s, 1H).

### Beispiel 168

### ent-2,6-Dimethyl-8-(3-methylbutoxy)-N-(7,7,7-trifluor-2-hydroxy-2-methylheptan-3-yl)imidazo[1,2-a]pyridin-3-carboxamid

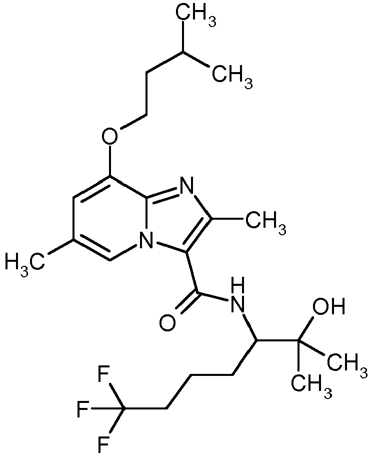

50 mg (0.18 mmol) 2,6-Dimethyl-8-(3-methylbutoxy)imidazo[1,2-a]pyridin-3-carbonsäure Beispiel 89A, 61 mg (0.19 mmol) TBTU und 55 mg (0.54 mmol) 4-Methylmorpholin wurden in 0.64 ml DMF vorgelegt. Dann wurden 47 mg (0.20 mmol) *ent*-3-Amino-7,7,7-trifluor-2-methylheptan-2-olhydrochlorid Beispiel 104A hinzugegeben und es wurde über Nacht bei Raumtemperatur gerührt. Anschließend wurden nochmals 17 mg (0.07 mmol) *ent*-3-Amino-7,7,7-trifluor-2-methylheptan-2-olhydrochlorid hinzugegeben und die Reaktionslösung wurde über Nacht bei RT gerührt. Dann wurde mit einigen Tropfen Wasser/TFA verdünnt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand mit gesättigter, wässriger Natriumhydrogencarbonat-Lösung versetzt und dreimal mit Dichlormethan extrahiert. Die vereinigten organischen Phasen wurden über Natriumsulfat getrocknet, filtriert, das Filtrat eingeengt und im Hochvakuum getrocknet. Es wurden 66 mg der Zielverbindung (78% d. Th., Reinheit 98%) erhalten.
LC-MS (Methode 17): Rₜ = 0.92 min
MS (ESpos): m/z = 458 (M+H)⁺
¹H-NMR (400 MHz, DMSO-d₆): δ = 0.96 (d, 6H), 1.11 (s, 3H), 1.18 (s, 3H), 1.42-1.67 (m, 3H), 1.67-1.73 (m, 2H), 1.77-1.88 (m, 2H), 2.12-2.29 (m, 4H), 2.30-2.48 (m, 1H), 2.54 (s, 3H), 3.90-3.99 (m, 1H), 4.18 (t, 2H), 4.56 (s, 1H), 6.72 (s, 1H), 7.43 (d, 1H), 8.28 (s, 1H).

### Beispiel 169

### N-[(2R)-1-Hydroxyhexan-2-yl]-2,6-dimethyl-8-[4,4,4-trifluor-3-(trifluormethyl)butoxy]imidazo[1,2-a]pyridin-3-carboxamid

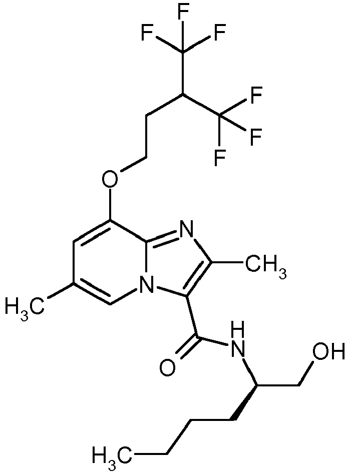

150 mg (0.39 mmol) 2,6-Dimethyl-8-[4,4,4-trifluor-3-(trifluormethyl)butoxy]imidazo[1,2-a]pyridin-3-carbonsäure Beispiel 91A und 163 mg (0.43 mmol) HATU wurden in 2.5 ml DMF vorgelegt. Dann wurden 0.2 ml (1.17 mmol) *N*,*N*-Diisopropylethylamin und 50.3 mg (0.43 mmol) (R)-(-)-2-Amino-1-hexanol zugegeben und es wurde über Nacht bei RT gerührt. Die Reaktionslösung wurde mit 10 ml Wasser versetzt, zweimal mit 20 ml Essigsäureethylester extrahiert, die vereinigten organischen Phasen mit 20 ml Wasser und mit 20 ml gesättigter wässriger Natriumchlorid-Lösung gewaschen, über Magnesiumsulfat getrocknet, filtriert und eingeengt. Der Rückstand wurde über die präparative RP-HPLC (Acetonitril-Wasser + 0.1% Ameisensäure) gereinigt, die Produktfraktionen wurden vereinigt, vollständig eingeengt und der Rückstand in einer Mischung aus 1.5 ml tert-Butanol und 2 ml Wasser gelöst und über Nacht lyophilisiert. Es wurden 42 mg der Zielverbindung (95% Reinheit, 21% d. Th.) erhalten.
LC-MS (Methode 1): Rₜ = 0.95 min
MS (ESpos): m/z = 484 (M+H)⁺
¹H-NMR (400 MHz, DMSO-d₆): δ = 0.89 (t, 3H), 1.26-1.41 (m, 4H), 1.41-1.54 (m, 1H), 1.58-1.68 (m, 1H), 2.32-2.42 (m, 5H), 2.59 (s, 3H), 3.48 (t, 2H), 3.94-4.02 (m, 1H), 4.24-4.34 (m, 1H), 4.41 (t, 2H), 7.23 (br. s, 1H), 8.05 (br. s, 1H), 8.42 (s, 1H), OH nicht sichtbar.

### Beispiel 170

### rac-2,6-Dimethyl-N-(7,7,7-trifluor-2-hydroxy-2-methylheptan-3-yl)-8-[4,4,4-trifluor-3-(trifluormethyl)butoxy]imidazo[1,2-a]pyridin-3-carboxamid

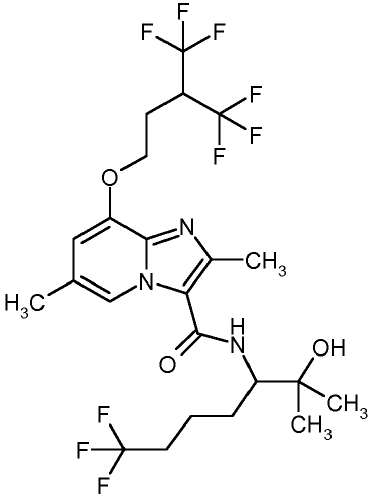

150 mg (0.390 mmol) 2,6-Dimethyl-8-[4,4,4-trifluor-3-(trifluormethyl)butoxy]imidazo[1,2-a]pyridin-3-carbonsäure Beispiel 91A und 163 mg (0.429 mmol) HATU wurden in 2 ml DMF vorgelegt. Dann wurden 0.2 ml (1.17 mmol) N,N-Diisopropylethylamin und 101 mg (0.429 mmol) *rac*-3-Amino-7,7,7-trifluor-2-methylheptan-2-olhydrochlorid Beispiel 103A zugegeben und es wirde über Nacht bei RT gerührt. Die Reaktionslösung wurde mit 10 ml Wasser versetzt, zweimal mit 20 ml Essigsäureethylester extrahiert, die vereinigten organischen Phasen mit 20 ml Wasser und mit 20 ml ges. wässriger NaCl-Lsg. gewaschen, über MgSO4 getrocknet, filtriert und eingeengt. Der Rückstand wurde über präparative RP-HPLC (ACN:Wasser + 0,1% HCOOH) gereinigt. Die Produktfraktionen wurden vereinigt, vollständig eingeengt, der Rückstand in einer Mischung aus 1.5 ml tert-Butanol und 2 ml Wasser gelöst und über Nacht lyophilisiert. Es wurden 91 mg der Zielverbindung (98% Reinheit, 40% d. Th.) erhalten.
LC-MS (Methode 1): Rₜ = 1.03 min
MS (ESpos): m/z = 566 (M+H)⁺
¹H-NMR (400 MHz, DMSO-d₆): δ = 1.12 (s, 3H), 1.19 (s, 3H), 1.40-1.67 (m, 3H), 1.79-1.88 (m, 1H) 2.13-2.29 (m, 2H), 2.29-2.44 (m, 5H), 2.58 (s, 3H), 3.97 (t, 1H), 4.18-4.33 (m, 1H), 4.38 (t, 2H), 4.60 (br. s, 1H), 7.01 (br. s, 1H) 7.70 (br. s, 1H) 8.34 (s, 1H).

### Beispiel 171

### ent-8-[1-(2,6-Difluorphenyl)ethoxy]-N-(2-hydroxy-2-methylheptan-3-yl)-2,6-dimethylimidazo[1,2-a]pyridin-3-carboxamid

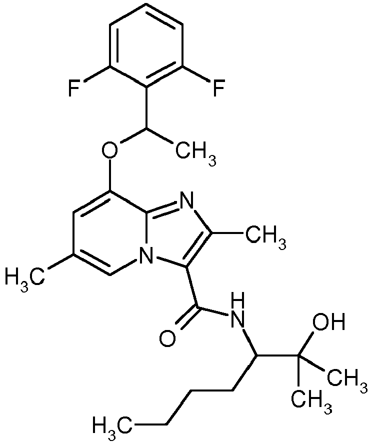

40 mg (0.12 mmol) ent-8-[1-(2,6-Difluorphenyl)ethoxy]-2,6-dimethylimidazo[1,2-a]pyridin-3-carbonsäure Beispiel 94A, 45 mg (0.14 mmol) TBTU und 47 mg (0.46 mmol) 4-Methylmorpholin wurden in 0.77 ml DMF vorgelegt. Dann wurden 25 mg (0.14 mmol) *ent*-3-Amino-2-methylheptan-2-olhydrochlorid Beispiel 98A hinzugegeben und über Nacht bei Raumtemperatur gerührt. Anschließend wurden nochmals 4 mg (0.02 mmol) *ent*-3-Amino-2-methylheptan-2-olhydrochlorid hinzugegeben und die Reaktionslösung wurde über Nacht bei RT gerührt. Dann wurden nochmals 4 mg (0.02 mmol) *ent*-3-Amino-2-methylheptan-2-olhydrochlorid hinzugegeben und die Reaktionslösung wurde über Nacht bei RT gerührt. Die Reaktionslösung wurde mit einigen Tropfen Wasser/TFA verdünnt und mittels präparativer HPLC gereinigt (RP18 Säule, Laufmittel: Acetonitril/Wasser-Gradient unter Zusatz von 0.1% TFA). Die eingeengten Fraktionen wurden in Dichlormethan und gesättigter, wässriger Natriumhydrogencarbonatlösung aufgenommen und die Phasen wurden getrennt. Die wässrige Phase wurde dreimal mit Dichlormethan extrahiert, die vereinigten organischen Phasen über Natriumsulfat getrocknet, filtriert, das Filtrat eingeengt und lyophilisiert. Es wurden 48 mg der Zielverbindung (87% d. Th., Reinheit 98%) erhalten.
LC-MS (Methode 1): Rₜ = 0.91 min
MS (ESpos): m/z = 474 (M+H)⁺
¹H-NMR (400 MHz, DMSO-d₆): δ = 0.85 (t, 3H), 1.11 (s, 3H), 1.17 (s, 3H), 1.20-1.47 (m, 5H), 1.69-1.81 (m, 4H), 2.19 (s, 3H), 2.52 (s, 3H), 3.86-3.93 (m, 1H), 4.42 (br. s, 1H), 6.22 (q, 1H), 6.58 (br. s, 1H), 7.09 (t, 2H), 7.28-7.37 (m, 1H), 7.40 (quintett, 1H), 8.30 (s, 1H).

### Beispiel 172

### N-(2-Hydroxy-2,3-dihydro-1H-inden-1-yl)-2,6-dimethyl-8-[4,4,4-trifluor-3-(trifluormethyl)butoxy]imidazo[1,2-a]pyridin-3-carboxamid

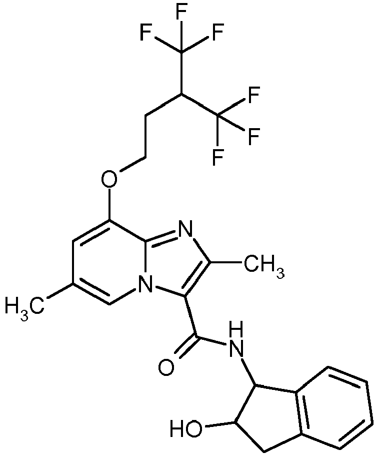

12 mg (0.08 mmol) 1-Aminoindan-2-ol wurden vorgelegt und mit 31 mg (0.08 mmol) 2,6-Dimethyl-8-[4,4,4-trifluor-3-(trifluormethyl)butoxy]imidazo[1,2-a]pyridin-3-carbonsäure Beispiel 91A, gelöst in 0.3 ml DMF, sowie mit 40 mg (0.104 mmol) HATU, gelöst in 0.3 ml DMF, und anschließend mit 16 mg (0.16 mmol) 4-Methylmorpholin versetzt und bei RT über Nacht geschüttelt. Die Zielverbindung wurde per präparativer HPLC (Methode 11) isoliert. Es wurden 15 mg (36% d. Th.) erhalten.
LC-MS (Methode 12): Rₜ = 0.99 min
MS (ESpos): m/z = 516 (M+H)⁺

In Analogie zu Beispiel 172 wurden die in Tabelle 14 gezeigten Beispielverbindungen hergestellt, indem 2,6-Dimethyl-8-[4,4,4-trifluor-3-(trifluormethyl)butoxy]imidazo[1,2-a]pyridin-3-carbon-säure mit den entsprechenden, kommerziell erhältlichen Aminen unter den beschriebenen Bedingungen umgesetzt wurde:

**Tabelle 14:**

| **Beispiel** | **IUPAC**-**Name / Struktur (Ausbeute)** | **Analytische Daten** |
|---|---|---|
| **173** | *N*-(2-Hydroxycyclopentyl)-2,6-dimethyl-8-[4,4,4-trifluor-3-(trifluormethyl)butoxy]imidazo[1,2-a]pyridin-3-carboxamid | LC-MS (Methode 12): Rₜ = 0.86 min |
| | | MS (ESpos): m/z = 468 (M+H)⁺ |
| | 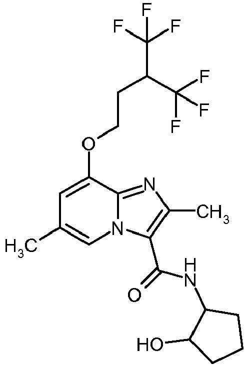 | |
| | (9% d. Th.) | |
| **174** | *rac-N*-(2-Hydroxybutyl)-2,6-dimethyl-8-[4,4,4-trifluor-3-(trifluormethyl)butoxy]imidazo[1,2-a]pyridin-3-carboxamid | LC-MS (Methode 12): Rₜ = 0.87 min |
| | | MS (ESpos): m/z = 456 (M+H)⁺ |
| | 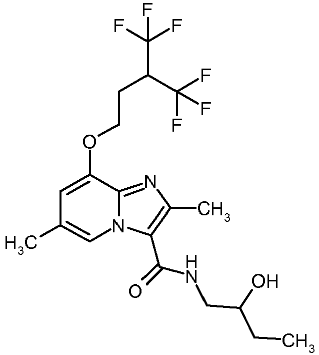 | |
| | (12% d. Th.) | |
| **175** | *rac*-N*-*[2-(4-Chlorphenyl)-2-hydroxyethyl]-2,6-dimethyl-8-[4,4,4-trifluor-3-(trifluormethyl)butoxy]imidazo[1,2-a]pyridin-3-carboxamid | LC-MS (Methode 12): Rt = 0.99 min |
| | | MS (ESpos): m/z = 538 (M+H)+ |
| | 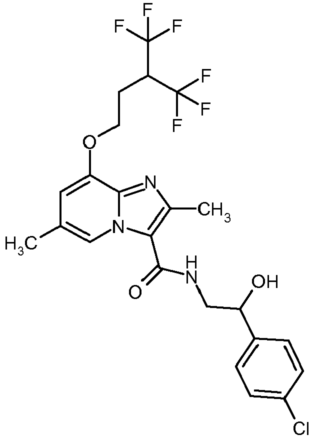 | |
| | (3% d. Th.) | |

### Beispiel 176

### rac-8-[(3,3-Difluorcyclobutyl)methoxy]-2,6-dimethyl-N-(7,7,7-trifluor-2-hydroxy-2-methylheptan-3-yl)imidazo[1,2-a]pyridin-3-carboxamid

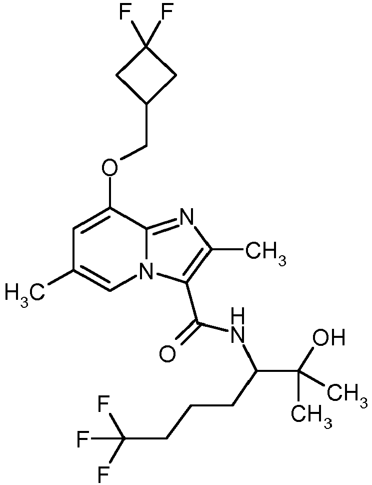

100 mg (0.32 mmol) 8-[(3,3-Difluorcyclobutyl)methoxy]-2,6-dimethylimidazo[1,2-a]pyridin-3-carbonsäure Beispiel 107A und 135 mg (0.35 mmol) HATU wurden in 2 ml DMF vorgelegt. Dann wurden 0.16 ml (0,96 mmol) *N,N*-Diisopropylethylamin und 70.6 mg (0.354 mmol) *rac*-3-Amino-7,7,7-trifluor-2-methylheptan-2-olhydrochlorid Beispiel 103A zugegeben und es wurde über Nacht bei RT gerührt. Die Reaktionslösung wurde mit 7 ml Wasser versetzt und zweimal mit 15 ml Essigsäureethylester extrahiert. Die vereinigten organischen Phasen wurden mit 15 ml Wasser und mit 10 ml gesättigter, wässriger Natriumchlorid-Lösung gewaschen, über Magnesiumsulfat getrocknet, filtriert und eingeengt. Der Rückstand wurde über präparative RP-HPLC (Acetonitril-Wasser + 0.1% Ameisensäure) gereinigt, die Produktfraktionen vereinigt und vollständig eingeengt. Es wurden 86 mg der Zielverbindung (55% d. Th.) erhalten.
LC-MS (Methode 1): Rₜ =0.89 min
MS (ESpos): m/z = 492 (M+H)⁺
¹H-NMR (400 MHz, DMSO-d₆): δ = 1.13 (s, 3H), 1.18 (s, 3H), 1.41-1.65 (m, 3H), 1.79-1.88 (m, 1H) 2.14-2.29 (m, 2H), 2.29-2.43 (m, 4H), 2.56 (s, 3H), 2.64-2.84 (m, 4H), 3.93-4.00 (m, 1H), 4.28 (d, 2H), 4.57 (br. s, 1H), 6.95 (br. s, 1H), 7.63 (br. s, 1H), 8.33 (s, 1H).

### Beispiel 177

### 8-[(2,6-Difluorbenzyl)oxy]-7-fluor-N-[(2R)-1-hydroxyhexan-2-yl]-2-methylimidazo[1,2-a]pyridin-3-carboxamid

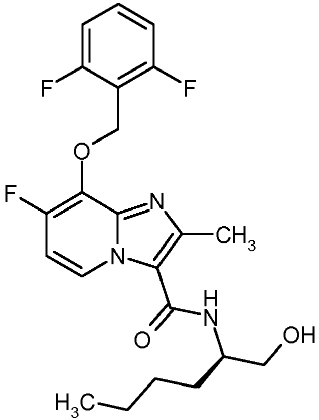

14 mg (0.04 mmol) 8-[(2,6-Difluorbenzyl)oxy]-7-fluor-2-methylimidazo[1,2-a]pyridin-3-carbonsäure Beispiel 112A, 20 mg (0.06 mmol) (Benzotriazol-1-yloxy)bisdimethylamino-methyliumfluorborat (TBTU) und 17 mg (0.17 mmol) 4-Methylmorpholin wurden in 0.28 ml DMF vorgelegt. Anschließend wurden 7.5 mg (0.06 mmol) (2R)-2-Aminohexan-1-ol zugesetzt und über Nacht bei RT gerührt. Es wurden nochmals 2.6 mg TBTU, 4 mg 4-Methylmorpholin und 1 mg (2R)-2-Aminohexan-1-ol hinzugegeben und 2 h bei RT gerührt. Das Reaktionsgemisch wurde eingeengt und mittels Dickschichtchromatographie (Laufmittel: Dichlormethan/Methanol = 10/1) gereinigt. Es wurden 12 mg (66% d. Th.; Reinheit 99%) der Titelverbindung erhalten.
LC-MS (Methode 1): Rₜ = 1.01 min
MS (ESpos): m/z = 436 (M+H)⁺
¹H-NMR (400 MHz, DMSO-d₆): δ = 0.88 (t, 3H), 1.23 - 1.56 (m, 5H), 1.58 - 1.70 (m, 1H), 2.57 (s, 3H), 3.38 - 3.55 (m, 2H), 3.91 - 4.04 (m, 1H), 4.73 (t, 1H), 5.57 - 5.63 (m, 2H), 6.96 - 7.06 (m, 1H), 7.08 - 7.18 (m, 2H), 7.46 - 7.62 (m, 2H), 8.63 - 8.68 (m, 1H).

### Beispiel 178:

### rac-8-[(3-Fluorpyridin-2-yl)methoxy]-2,6-dimethyl-N-(7,7,7-trifluor-2-hydroxy-2-methylheptan-3-yl)imidazo[1,2-a]pyridin-3-carboxamid Trifluoracetat

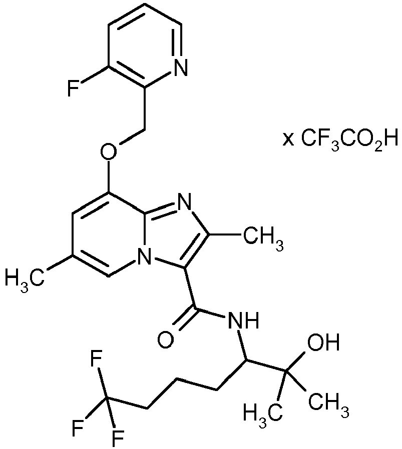

117 mg (0.24 mmol) *rac*-Methyl-6,6,6-trifluor-*N-*({8-[(3-fluorpyridin-2-yl)methoxy]-2,6-dimethylimidazo[1,2-a]pyridin-3-yl}carbonyl)norleucinat aus Beispiel 122A wurden unter Argon in 2.3 ml THF vorgelegt und bei 0°C wurden 0.2 ml (0.59 mmol) einer 3 M Methylmagnesiumbromid-Lösung in Diethylether zugetropft. Das Reaktionsgemisch wurde 15 min bei 0°C gerührt. Dann ließ man langsam auf RT erwärmen. Nach 3.5 h wurde vorsichtig mit 1 N wässriger Salzsäure angesäuert und anschließend mittels präparativer HPLC (RP18 Säule, Laufmittel: Acetonitril/Wasser-Gradient unter Zusatz von 0.1% TFA) gereinigt. Es wurden 119 mg (75% d. Th.; Reinheit 91%) der Titelverbindung erhalten.
LC-MS (Methode 1): Rₜ = 0.78 min
MS (ESpos): m/z = 497 (M-TFA+H)⁺
¹H-NMR (400 MHz, DMSO-d₆): δ = 1.11 (s, 3 H), 1.17 (s, 3 H), 1.40 - 1.65 (m, 3 H), 1.76 - 1.88 (m, 1 H), 2.12 - 2.24 (m, 1 H), 2.28 (s, 3 H), 2.32 - 2.45 (m, 1 H), 3.90 - 4.01 (m, 1 H), 4.56 (s, 1 H), 5.40 (s, 2 H), 6.87 (s, 1 H), 7.43 (d, 1 H), 7.55 - 7.64 (m, 1 H), 7.86 (t, 1 H), 8.31 (s, 1 H), 8.49 (d, 1 H) [weiteres Signal unter DMSO-Peak verborgen].

In Analogie zu Beispiel 178 wurden die in Tabelle 15 gezeigten Beispielverbindungen hergestellt, indem die entsprechenden Methylester (Beispiel 124A und Beispiel 123A) mit Methylmagnesiumbromid (2.5 bis 3.2 Äquivalente) zur Reaktion gebracht wurden.

### Beispielhafte Aufarbeitungen der Reaktionsmischung:

Der Niederschlag der Reaktion oder das Reaktionsgemisch wurden verdünnt (Wasser/TFA) und direkt per präparativer HPLC (RP18 Säule, Eluent: Acetonitril/Wasser-Gradient unter Zusatz von 0.1% TFA oder 0.1% Ameisensäure) gereinigt und über Nacht im Hochvakuum getrocknet. Die Produkt-Fraktionen wurden eingeengt, gegebenenfalls in Dichlormethan oder Essigsäureethylester aufgenommen und zweimal mit gesättigter, wässriger Natriumhydrogencarbonat-Lösung gewaschen. Die vereinigten wässrigen Phasen wurden zweimal mit Dichlormethan oder Essigsäureethylester extrahiert. Die vereinigten organischen Phasen wurden über Natriumsulfat getrocknet, filtriert und eingeengt.

Alternativ wurde die Reaktionslösung mit Dichlormethan verdünnt. Die Reaktionslösung wurde dann zweimal mit gesättigter, wässriger Natriumhydrogencarbonat-Lösung, einmal mit Wasser und einmal mit wässriger, gesättigter Natriumchlorid-Lösung gewaschen, über Natriumsulfat getrocknet, filtriert und eingeengt. Den Rückstand reinigte man über eine Kieselgelkartusche (Eluenten: Cyclohexan/Essigsäureethylester-Gradient oder Dichlormethan/Methanol-Gradient).

**Tabelle 15:**

| **Beispiel** | **IUPAC**-**Name / Struktur (Ausbeute)** | **Analytische Daten** |
|---|---|---|
| **179** | *rac*-8-[(3-Fluorpyridin-2-yl)methoxy]-2-methyl-*N-*(7,7,7-trifluor-2-hydroxy-2-methylheptan-3-yl)imidazo[1,2-a]pyridin-3-carboxamid | LC-MS (Methode 1): Rₜ = 0.76 min |
| | | MS (ESpos): m/z = 483 (M+H)⁺ |
| | 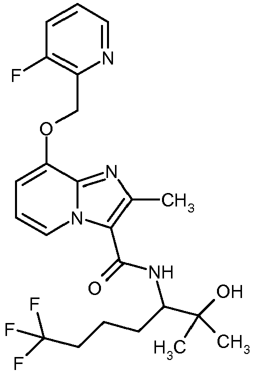 | ¹H-NMR (400 MHz, DMSO-d₆): δ = 1.12 (s, 3H), 1.18 (s, 3H), 1.41-1.67 (m, 3H), 1.78-1.90 (m, 1H), 2.13-2.28 (m, 1H), 2.32-2.45 (m, 1H), 2.50 (s, 3H; überlagert durch Lösungsmittel-Signal), 3.93-4.02 (m, 1H), 4.57 (s, 1H), 5.42 (s, 2H), 6.90 (t, 1H), 6.98 (d, 1H), 7.48 (d, 1H), 7.55-7.60 (m, 1H), 7.86 (t, 1H), 8.46-8.51 (m, 2H). |
| | (62% d. Th.) | |
| **180** | *rac*-8-[(3-Fluorpyridin-2-yl)methoxy]-2-methyl-*N-*(1,1,1-trifluor-4-hydroxy-4-methylpentan-3-yl)imidazo[1,2-a]pyridin-3-carboxamid | LC-MS (Methode 1): Rₜ = 0.73 min |
| | | MS (ESpos): m/z = 455 (M+H)⁺ |
| | 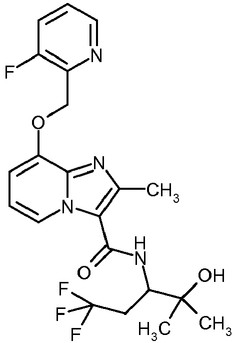 | ¹H-NMR (400 MHz, DMSO-d₆): δ = 1.11 (s, 3 H), 1.19 (s, 3 H), 2.52 (s, 3 H; überlagert durch Lösungsmittel-Signal), 2.64 - 2.81 (m, 1 H), 4.32 - 4.42 (m, 1 H), 5.42 (d, 2 H), 6.90 (t, 1 H), 6.98 (d, 1H), 7.54 - 7.62 (m, 1H), 7.80 - 7.88 (m, 2 H), 8.42 (d, 1 H), 8.49 (d, 1 H), [weiteres Signal unter Lösungsmittel-Peak verborgen]. |
| | (52% d. Th.) | |

### Beispiel 181:

### ent-8-[(3-Fluorpyridin-2-yl)methoxy]-2,6-dimethyl-N-(7,7,7-trifluor-2-hydroxy-2-methylheptan-3-yl)imidazo[1,2-a]pyridin-3-carboxamid (Enantiomer B)

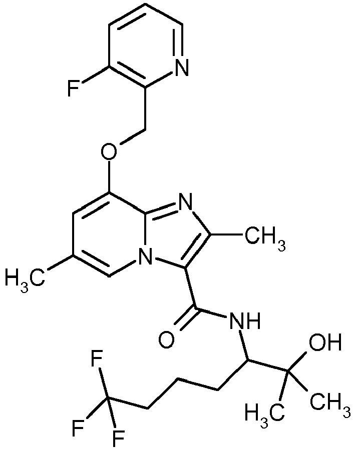

169 mg der Beispielverbindung 178 wurden an chiraler Phase in die Enantiomere getrennt [Säule: Daicel Chiralpak AD-H, 5 µm, 250 x 20 mm, Eluent: 50% iso-Hexan, 50% Ethanol + 0.2% Diethylamin, Fluß: 15 ml/min; 40°C, Detektion: 220 nm]. Zur Entfernung von Lösungsmittelresten wurde das Produkt in Acetonitril/Wasser aufgenommen und lyophilisiert.
Enantiomer B: 53 mg (99% ee)
Rt= 8.57 min [Säule: Daicel Chiralpak AD-H, 5 µm, 250 x 4.6 mm, Eluent: 50% iso-Hexan, 50% Ethanol + 0.2% Diethylamin, Fluß: 1.0 ml/min; 40°C, Detektion: 220 nm].

### Beispiel 182:

### ent-8-[(3-Fluorpyridin-2-yl)methoxy]-2-methyl-N-(7,7,7-trifluor-2-hydroxy-2-methylheptan-3-yl)imidazo[1,2-a]pyridin-3-carboxamid (Enantiomer A)

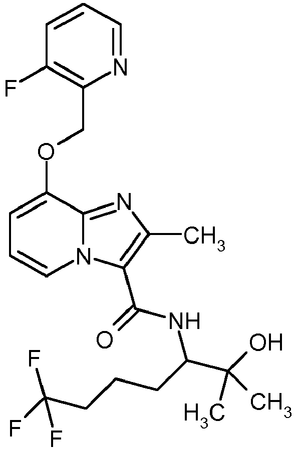

287 mg der Beispielverbindung 179 wurden an chiraler Phase in die Enantiomere getrennt [Säule: Daicel Chiralpak AD-H, 5 µm, 250 x 20 mm, Eluent: 50% iso-Hexan, 50% Ethanol + 0.2% TFA, Fluß: 15 ml/min; 45°C, Detektion: 220 nm]. Die produkthaltigen Fraktionen wurden eingeengt, der Rückstand mit gesättigter, wässriger Natriumhydrogencarbonat-Lösung versetzt, dreimal mit Essigsäureethylester extrahiert, die vereinten organischen Phasen über Natriumsulfat getrocknet, filtriert, das Filtrat eingeengt und lyophilisiert.
Enantiomer A: 125 mg (99% ee)
Rt= 3.78 min [Säule: Daicel Chiralpak AD-H, 5 µm, 250 x 4.6 mm, Eluent: 50% iso-Hexan, 50% Ethanol + 0.2% TFA, Fluß: 1.0 ml/min; 40°C, Detektion: 220 nm].

### Beispiel 183:

### ent-8-[(3-Fluorpyridin-2-yl)methoxy]-2-methyl-N-(7,7,7-trifluor-2-hydroxy-2-methylheptan-3-yl)imidazo[1,2-a]pyridin-3-carboxamid (Enantiomer B)

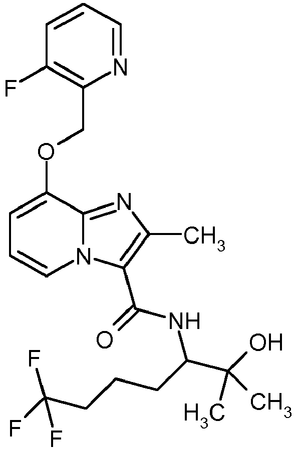

287 mg der Beispielverbindung 179 wurden an chiraler Phase in die Enantiomere getrennt [Säule: Daicel Chiralpak AD-H, 5 µm, 250 x 20 mm, Eluent: 50% iso-Hexan, 50% Ethanol + 0.2% TFA, Fluß 15 ml/min; 45°C, Detektion: 220 nm]. Die produkthaltigen Fraktionen wurden eingeengt, der Rückstand mit gesättigter, wässriger Natriumhydrogencarbonat-Lösung versetzt, dreimal mit Essigsäureethylester extrahiert, die vereinten organischen Phasen über Natriumsulfat getrocknet, filtriert, das Filtrat eingeengt und lyophilisiert.
Enantiomer B: 104 mg (99% ee)
Rₜ = 5.14 min [Säule: Daicel Chiralpak AD-H, 5 µm, 250 x 4.6 mm, Eluent: 50% iso-Hexan, 50% Ethanol + 0.2% TFA, Fluß: 1.0 ml/min; 40°C, Detektion: 220 nm].

### Beispiel 184

### rac-8-[(3-Fluorpyridin-2-yl)methoxy]-2,6-dimethyl-N-(1,1,1-trifluor-4-hydroxy-4-methylpentan-3-yl)imidazo[1,2-a]pyridin-3-carboxamid (Racemat)

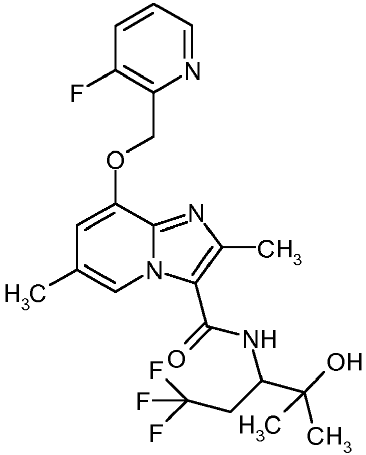

298 mg (0.48 mmol) *rac*-Methyl-4,4,4-trifluor-2-[({8-[(3-fluorpyridin-2-yl)methoxy]-2,6-dimethylimidazo[1,2-a]pyridin-3-yl}carbonyl)amino]butanoat aus Beispiel 125A wurden unter Argon in 4.68 ml abs. THF vorgelegt und bei 0°C wurden 0.4 ml (1.2 mmol) einer 3 M Methylmagnesiumbromid-Lösung in Diethylether zugetropft. Das Reaktionsgemisch wurde zuerst 15 min bei 0°C gerührt, dann ließ man auf RT erwärmen. Nach 3.5 h wurde vorsichtig mit 1 N wässriger Salzsäure angesäuert und es wurde mittels präparativer HPLC (RP18 Säule, Laufmittel: Acetonitril/Wasser-Gradient unter Zusatz von 0.1% TFA) gereinigt. Das Rohprodukt wurde in Dichlormethan gelöst und mit gesättigter, wässriger Natriumhydrogencarbonat-Lösung gewaschen. Die wässrige Phase zweimal mit Dichlormethan extrahiert. Die vereinten organischen Phasen wurden über Natriumsulfat getrocknet, filtriert, das Filtrat eingeengt, mit Wasser/Acetonitril versetzt und lyophilisiert. Es wurden 87 mg (38% d. Th.) der Titelverbindung erhalten.
LC-MS (Methode 1): Rₜ = 0.78 min
MS (ESIpos): m/z = 469 (M+H)⁺.
¹H-NMR (400 MHz, DMSO-d₆): δ = 1.10 (s, 3H), 1.19 (s, 3H), 2.29 (s, 3H), 2.52 (s, 3H; überlagert mit Lösungsmittelpeak), 2.60 - 2.82 (m, 2H), 4.37 (t, 1H), 4.88 (s, 1H), 5.39 (s, 2H), 6.90 (s, 1H), 7.55 - 7.62 (m, 1H), 7.77 (d, 1H), 7.82 - 7.89 (m, 1H), 8.26 (s, 1H), 8.47 - 8.52 (m, 1H).

### Beispiel 185

### ent-8-[(3-Fluorpyridin-2-yl)methoxy]-2,6-dimethyl-N-(1,1,1-trifluor-4-hydroxy-4-methylpentan-3-yl)imidazo[1,2-a]pyridin-3-carboxamid (Enantiomer B)

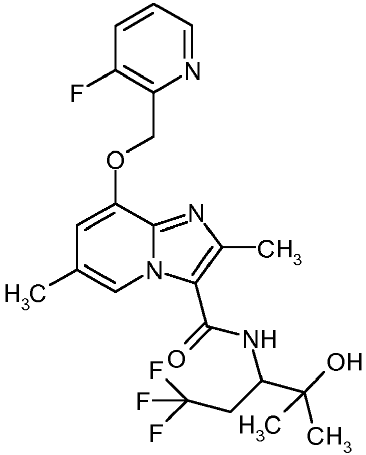

82 mg von Beispiel 184 wurden durch präparative Trennung an chiraler Phase in die Enantiomere getrennt [Säule: Daicel Chiralcel OZ-H, 5 µm, 250 x 20 mm, Eluent: 50% iso-Hexan, 50% Ethanol + 0.2% Essigsäure, Fluß: 15 ml/min; 40°C, Detektion: 220 nm].
Enantiomer B: 32 mg (99% ee)
Rₜ = 5.12 min [Daicel Chiralcel OZ-H, 5µm, 250 x 4.6 mm; Eluent: 50% iso-Hexan, 50% Ethanol + 0.2% TFA + 1% H₂O; Fluss: 1.0 ml/min; 45°C; Detektion: 235 nm].

### Beispiel 186

### rac-8-[(3-Fluorpyridin-2-yl)methoxy]-2,6-dimethyl-N-(6,6,7,7,7-pentafluor-2-hydroxy-2-methylheptan-3-yl)imidazo[1,2-a]pyridin-3-carboxamid (Racemat)

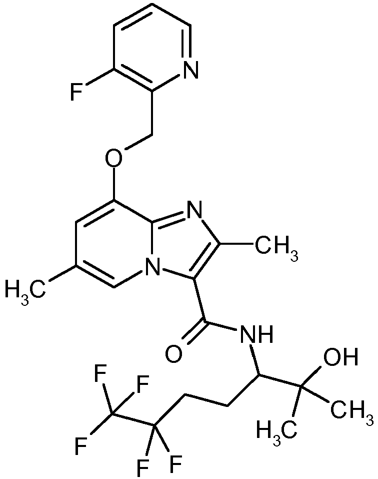

71 mg (0.20 mmol) 8-[(3-Fluorpyridin-2-yl)methoxy]-2,6-dimethylimidazo[1,2-a]pyridin-3-carbonsäure-Hydrochlorid aus Beispiel 121A wurden mit 78 mg (0.24 mmol) TBTU und 143 mg (1.42 mmol) 4-Methylmorpholin in 1.3 ml abs. DMF vorgelegt. Anschließend wurden 66 mg (0.24 mmol) *rac*-3-Amino-6,6,7,7,7-pentafluor-2-methylheptan-2-olhydrochlorid aus Beispiel 136A hinzugegeben und das Reaktionsgemisch wurde über Nacht bei RT gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1 % TFA). Die vereinten Produktfraktionen wurden eingeengt, der Rückstand in Dichlormethan aufgenommen und zweimal mit gesättigter, wässriger Natriumhydrogencarbonat-Lösung gewaschen. Die wässrige Phase wurde zweimal mit Dichlormethan extrahiert. Die vereinigten organischen Phasen wurden über Natriumsulfat getrocknet, filtriert, das Filtrat eingeengt und lyophilisiert. Es wurden 73 mg (66% d. Th.) der Zielverbindung erhalten.
LC-MS (Methode 1): Rₜ = 0.85 min
MS (ESIpos): m/z = 533 (M+H)⁺.
¹H-NMR (400 MHz, DMSO-d₆): δ = 1.13 (s, 3H), 1.19 (s, 3H), 1.65 - 1.78 (m, 1H), 2.00 - 2.11 (m, 1H), 2.12 - 2.27 (m, 2H), 2.29 (s, 3H), 3.97 - 4.06 (m, 1H), 4.71 (s, 1H), 5.36 - 5.43 (m, 2H), 6.88 - 6.92 (m, 1H), 7.53 - 7.62 (m, 2H), 7.85 (t, 1H), 8.34 (s, 1H), 8.50 (d, 1H), [weiteres Signal unter Lösungsmittel-Peak verborgen].

### Beispiel 187

### ent-8-[(3-Fluorpyridin-2-yl)methoxy]-2,6-dimethyl-N-(6,6,7,7,7-pentafluor-2-hydroxy-2-methylheptan-3-yl)imidazo[1,2-a]pyridin-3-carboxamid (Enantiomer B)

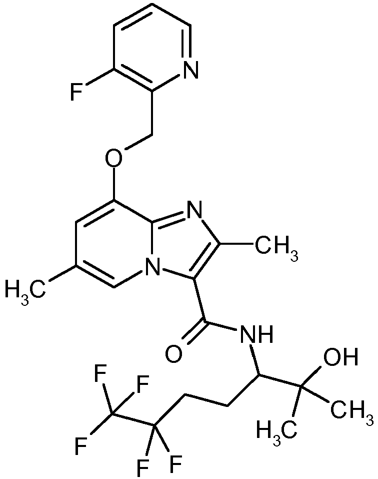

70 mg von Beispiel 186 wurden durch präparative Trennung an chiraler Phase in die Enantiomere getrennt [Säule: Daicel Chiralcel OD-H, 5 µm, 250 x 20 mm, Eluent: 80% iso-Hexan, 20% Isopropanol, Fluß: 15 ml/min; 25°C, Detektion: 220 nm].
Enantiomer B: 23 mg (99% ee)
Rₜ = 7.27 min [Daicel Chiralpak AD-H, 5µm, 250 x 4.6 mm; Eluent: 80% iso-Hexan, 20% Isopropanol; Fluss 1.0 ml/min; 40°C; Detektion: 220 nm].

### Beispiel 188

### ent-6-Chlor-8-[(3-fluorpyridin-2-yl)methoxy]-2-methyl-N-(7,7,7-trifluor-2-hydroxy-2-methylheptan-3-yl)imidazo[1,2-a]pyridin-3-carboxamid (Enantiomer A)

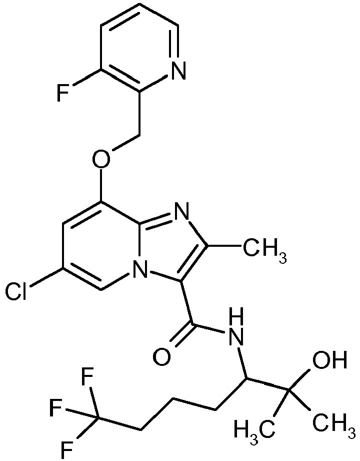

50 mg (0.15 mmol) 6-Chlor-8-[(3-fluorpyridin-2-yl)methoxy]-2-methylimidazo[1,2-a]pyridin-3-carbonsäure aus Beispiel 129A wurden mit 50 mg (0.16 mmol) TBTU und 45 mg (0.45 mmol) 4-Methylmorpholin in 0.52 ml abs. DMF vorgelegt. Anschließend wurden 38 mg (0.16 mmol) *ent*-3-Amino-7,7,7-trifluor-2-methylheptan-2-ol-Hydrochlorid (Enantiomer A) aus Beispiel 104A hinzugegeben und das Reaktionsgemisch wurde über Nacht bei RT gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1% TFA). Die vereinten Produktfraktionen wurden eingeengt, der Rückstand in Dichlormethan aufgenommen und zweimal mit gesättigter, wässriger Natriumhydrogencarbonat-Lösung gewaschen. Die wässrige Phase wurde dreimal mit Dichlormethan extrahiert. Die vereinigten organischen Phasen wurden über Natriumsulfat getrocknet, filtriert, das Filtrat eingeengt und lyophilisiert. Es wurden 54 mg (70% d. Th.) der Zielverbindung erhalten.
LC-MS (Methode 1): Rₜ = 0.92 min
MS (ESIpos): m/z = 517 (M+H)⁺.
¹H-NMR (400 MHz, DMSO-d₆): δ = 1.12 (s, 3H), 1.17 (s, 3H), 1.40 - 1.50 (m, 1H), 1.51 - 1.64 (m, 2H), 1.77 - 1.89 (m, 1H), 2.13 - 2.27 (m, 1H), 2.31 - 2.45 (m, 1H), 2.52 (s, 3H; überlagert mit Lösungsmittelpeak), 3.92 - 4.01 (m, 1H), 4.58 (s, 1H), 5.43 - 5.50 (m, 2H), 7.15 - 7.19 (m, 1H), 7.55 - 7.63 (m, 2H), 7.83 - 7.90 (m, 1H), 8.50 (d, 1H), 8.55 - 8.59 (m, 1H).

### Beispiel 189

### ent-8-[(3,5-Difluorpyridin-4-yl)methoxy]-2,6-dimethyl-N-(7,7,7-trifluor-2-hydroxy-2-methylheptan-3-yl)imidazo[1,2-a]pyridin-3-carboxamid (Enantiomer A)

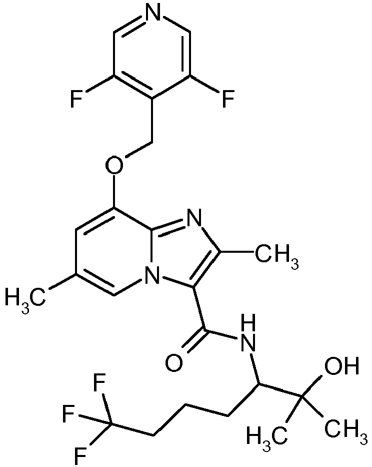

50 mg (0.15 mmol) 8-[(3,5-Difluorpyridin-4-yl)methoxy]-2,6-dimethylimidazo[1,2-a]pyridin-3-carbonsäure aus Beispiel 131A wurden mit 50 mg (0.16 mmol) TBTU und 61 mg (0.60 mmol) 4-Methylmorpholin in 0.83 ml abs. DMF vorgelegt. Anschließend wurden 45 mg (0.17 mmol) *ent*-3-Amino-7,7,7-trifluor-2-methylheptan-2-ol-Hydrochlorid (Enantiomer A) aus Beispiel 104A hinzugegeben und das Reaktionsgemisch wurde über Nacht bei RT gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1 % TFA). Die vereinten Produktfraktionen wurden eingeengt, der Rückstand in Dichlormethan aufgenommen und zweimal mit gesättigter, wässriger Natriumhydrogencarbonat-Lösung gewaschen. Die wässrige Phase wurde dreimal mit Dichlormethan extrahiert. Die vereinigten organischen Phasen wurden über Natriumsulfat getrocknet, filtriert, das Filtrat eingeengt und lyophilisiert. Es wurden 68 mg (85% d. Th.) der Zielverbindung erhalten.
LC-MS (Methode 17): Rₜ = 0.83 min
MS (ESIpos): m/z = 515 (M+H)⁺.
¹H-NMR (400 MHz, DMSO-d₆): δ = 1.12 (s, 3H), 1.17 (s, 3H), 1.40 - 1.50 (m, 1H), 1.51 - 1.65 (m, 2H), 1.78 - 1.90 (m, 1H), 2.13 - 2.27 (m, 1H), 2.32 (s, 3H), 2.36 - 2.44 (m, 1H), 2.52 (s, 3H; überlagert mit Lösungsmittelpeak), 3.92 - 4.01 (m, 1H), 4.45 - 4.71 (m, 1H), 5.45 (s, 2H), 6.95 - 7.16 (m, 1H), 7.49 - 7.74 (m, 1H), 8.33 - 8.39 (m, 1H), 8.65 - 8.70 (m, 2H).

### Beispiel 190

### rac-8-[(3-Fluorpyridin-2-yl)methoxy]-2,6-dimethyl-N-(7,7,8,8,8-pentafluor-2-hydroxy-2-methyloctan-3-yl)imidazo[1,2-a]pyridin-3-carboxamid (Racemat)

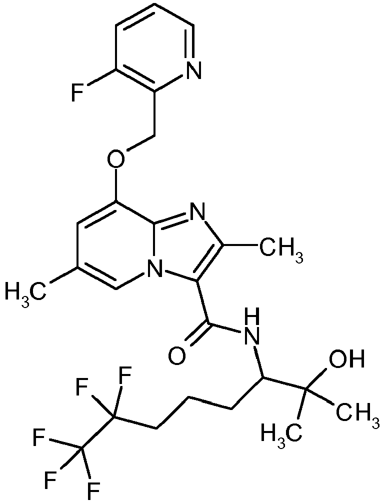

210 mg (0.60 mmol) 8-[(3-Fluorpyridin-2-yl)methoxy]-2,6-dimethylimidazo[1,2-a]pyridin-3-carbonsäure-Hydrochlorid aus Beispiel 121A wurden in 3.8 ml abs. DMF vorgelegt und mit 211 mg (0.66 mmol) TBTU und 241 mg (2.39 mmol) 4-Methylmorpholin versetzt. Anschließend wurden 188 mg (0.66 mmol) *rac*-3-Amino-7,7,8,8,8-pentafluor-2-methyloctan-2-ol Hydrochlorid (Racemat) aus Beispiel 142A zugegeben und die Reaktionsmischung wurde über Nacht bei RT gerührt. Dann wurde mit 34 mg (0.12 mmol) *rac*-3-Amino-7,7,8,8,8-pentafluor-2-methyloctan-2-olhydrochlorid (Racemat) aus Beispiel 142A versetzt und über Nacht bei RT gerührt. Das Gemisch wurde mit Wasser versetzt, der enstandene Feststoff abfiltriert, in Acetonitril/Methanol/TFA gelöst und mittels präparativer RP-HPLC (Acetonitril/Wasser-Gradient unter Zusatz von 0.1% TFA) gereinigt. Die Produktfraktionen wurden eingeengt, der Rückstand in Dichlormethan aufgenommen und zweimal mit gesättigter wässriegr Natriumhydrogencarbonat-Lösung gewaschen. Die wässrige Phase wurde zweimal mit Dichlormethan extrahiert, die vereinigten organischen Phasen über Natriumsulfat getrocknet, abfiltriert, eingeengt und lyophilisiert. Man erhielt 247 mg (74% d. Th.) der Zielverbindung.
LC-MS (Methode 1): Rₜ = 0.90 min
MS (ESIpos): m/z = 547 (M+H)⁺.
¹H-NMR (400 MHz, DMSO-d₆): δ = 1.12 (s, 3H), 1.18 (s, 3H), 1.44 - 1.69 (m, 3H), 1.78 - 1.92 (m, 1H), 2.05 - 2.24 (m, 1H), 2.28 (s, 3H), 2.32 - 2.46 (m, 1H), 2.52 (s, 3H; überlagert mit Lösungsmittelpeak), 3.93 - 4.05 (m, 1H), 4.50 - 4.62 (m, 1H), 5.41 (s, 2H), 6.94 (s, 1H), 7.46 - 7.55 (m, 1H), 7.56 - 7.63 (m, 1H), 7.81 - 7.90 (m, 1H), 8.29 (s, 1H), 8.47 - 8.53 (m, 1H).

### Beispiel 191

### ent-8-[(3-Fluorpyridin-2-yl)methoxy]-2,6-dimethyl-N-(7,7,8,8,8-pentafluor-2-hydroxy-2-methyloctan-3-yl)imidazo[1,2-a]pyridin-3-carboxamid (Enantiomer A)

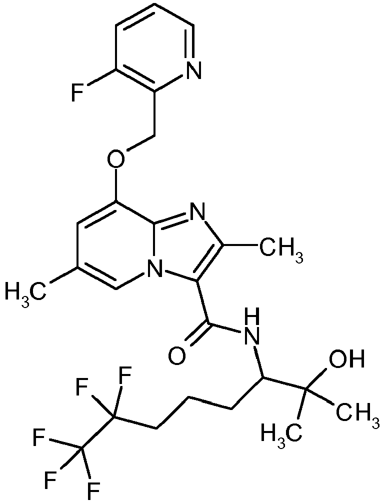

240 mg von Beispiel 190 wurden durch präparative Trennung an chiraler Phase in die Enantiomere getrennt [Säule: Daicel Chiralpak OZ-H, 5 µm, 250 x 20 mm, Eluent: 100% Ethanol, Fluß: 12 ml/min; 40°C, Detektion: 220 nm].
Enantiomer A: 114 mg (99% ee)
Rt= 3.82 min [Daicel Chiralpak OZ-H, 5µm, 250 x 4.6 mm; Eluent: 25% Iso-Hexan, 75% Ethanol; Fluss 1.0 ml/min; 45°C; Detektion: 220 nm].

### Beispiel 192

### ent-8-[(3-Fluorpyridin-2-yl)methoxy]-2,6-dimethyl-N-(7,7,8,8,8-pentafluor-2-hydroxy-2-methyloctan-3-yl)imidazo[1,2-a]pyridin-3-carboxamid (Enantiomer B)

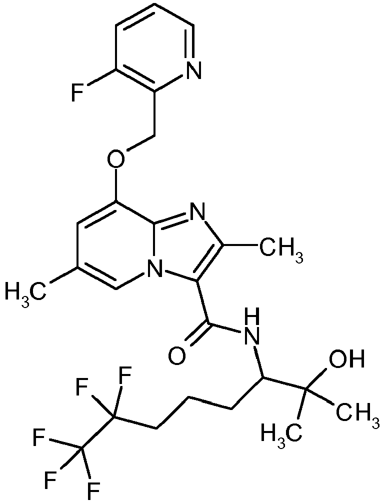

240 mg von Beispiel 190 wurden durch präparative Trennung an chiraler Phase in die Enantiomere getrennt [Säule: Daicel Chiralpak OZ-H, 5 µm, 250 x 20 mm, Eluent: 100% Ethanol, Fluß: 12 ml/min; 40°C, Detektion: 220 nm].
Enantiomer B: 105 mg (99% ee)
Rt= 6.77 min [Daicel Chiralpak OZ-H, 5µm, 250 x 4.6 mm; Eluent: 25% Iso-Hexan, 75% Ethanol; Fluss 1.0 ml/min; 45°C; Detektion: 220 nm].

### Beispiel 193

### ent-8-[(3,5-Difluorpyridin-4-yl)methoxy]-2,6-dimethyl-N-(6,6,7,7,7-pentafluor-2-hydroxy-2-methylheptan-3-yl)imidazo[1,2-a]pyridin-3-carboxamid (Enantiomer A)

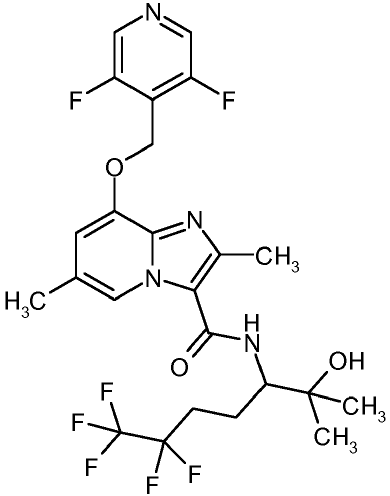

45 mg (0.14 mmol) 8-[(3,5-Difluorpyridin-4-yl)methoxy]-2,6-dimethylimidazo[1,2-a]pyridin-3-carbonsäure aus Beispiel 131A wurden in 1 ml DMF vorgelegt und mit 52 mg (0.16 mmol) TBTU und 54 mg (0.54 mmol) 4-Methylmorpholin versetzt. Anschließend wurden 40 mg (0.15 mmol) *ent*-3-Amino-6,6,7,7,7-pentafluor-2-methylheptan-2-olhydrochlorid (Enantiomer A) aus Beispiel 138A zugegeben und die Reaktionsmischung wurde über Nacht bei RT gerührt. Dann wurde mit 3.7 mg (0.01 mmol) *ent*-3-Amino-6,6,7,7,7-pentafluor-2-methylheptan-2-olhydrochlorid (Enantiomer A) aus Beispiel 138A versetzt und 4 h bei RT gerührt. Das Gemisch wurde mit Wasser/TFA versetzt und mittels präparativer RP-HPLC (Acetonitril/Wasser-Gradient unter Zusatz von 0.1% TFA) gereinigt. Die Produktfraktion wurde eingeengt, der Rückstand in Dichlormethan aufgenommen und zweimal mit gesättigter wässriger Natriumhydrogencarbonat-Lösung gewaschen. Die wässrige Phase wurde zweimal mit Dichlormethan extrahiert, die vereinigten organischen Phasen über Natriumsulfat getrocknet, abfiltriert, eingeengt und lyophilisiert. Man erhielt 64 mg (83% d. Th.) der Zielverbindung.
LC-MS (Methode 1): Rₜ = 0.91 min.
MS (ESIpos): m/z = 551 (M+H)⁺.
¹H-NMR (400 MHz, DMSO-d₆): δ = 1.13 (s, 3H), 1.19 (s, 3H), 1.65 - 1.78 (m, 1H), 2.00 - 2.11 (m, 1H), 2.13 - 2.28 (m, 2H), 2.31 (s, 3H), 2.52 (s, 3H; überlagert mit Lösungsmittelpeak), 3.98 - 4.06 (m, 1H), 4.71 (s, 1H), 5.39 - 5.46 (m, 2H), 6.93 (s, 1H), 7.59 (d, 1H), 8.38 (s, 1H), 8.67 (s, 2H).

### Beispiel 194

### ent-6-Chlor-8-[(3-fluorpyridin-2-yl)methoxy]-2-methyl-N-(6,6,7,7,7-pentafluor-2-hydroxy-2-methylheptan-3-yl)imidazo[1,2-a]pyridin-3-carboxamid (Enantiomer A)

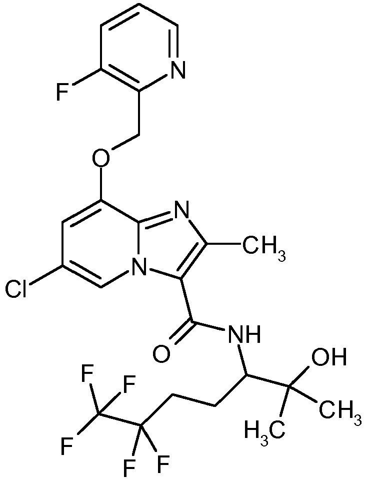

45 mg (0.13 mmol) 6-Chlor-8-[(3-fluorpyridin-2-yl)methoxy]-2-methylimidazo[1,2-a]pyridin-3-carbonsäure aus Beispiel 129A wurden mit 52 mg (0.16 mmol) TBTU und 68 mg (0.67 mmol) 4-Methylmorpholin in 0.85 ml abs. DMF vorgelegt. Anschließend wurden 40 mg (0.15 mmol) *ent*-3-Amino-6,6,7,7,7-pentafluor-2-methylheptan-2-olhydrochlorid (Enantiomer A) aus Beispiel 138A hinzugegeben und das Reaktionsgemisch wurde über Nacht bei RT gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1 % TFA). Die vereinten Produktfraktionen wurden eingeengt, der Rückstand in Dichlormethan aufgenommen und zweimal mit gesättigter, wässriger Natriumhydrogencarbonat-Lösung gewaschen. Die wässrige Phase wurde zweimal mit Dichlormethan extrahiert. Die vereinigten organischen Phasen wurden über Natriumsulfat getrocknet, filtriert, das Filtrat eingeengt und lyophilisiert. Es wurden 47 mg (63% d. Th.) der Zielverbindung erhalten.
LC-MS (Methode 1): Rₜ = 1.07 min
MS (ESIpos): m/z = 553 (M+H)⁺.
¹H-NMR (400 MHz, DMSO-d₆): δ = 1.13 (s, 3H), 1.19 (s, 3H), 1.65 - 1.79 (m, 1H), 1.99 - 2.10 (m, 1H), 2.13 - 2.31 (m, 2H), 2.53 (s, 3H; überlagert mit Lösunsmittelpeak), 3.97 - 4.07 (m, 1H), 4.71 (s, 1H), 5.43 - 5.50 (m, 2H), 7.16 - 7.20 (m, 1H), 7.56 - 7.63 (m, 1H), 7.67 (d, 1H), 7.83 - 7.90 (m, 1H), 8.48 - 8.53 (m, 1H), 8.59 - 8.63 (m, 1H).

### Beispiel 195

### 8-[(3-Fluorpyridin-2-yl)methoxy]-2,6-dimethyl-N-(7,7,7-trifluor-2,6-dihydroxy-2-methylheptan-3-yl)imidazo[1,2-a]pyridin-3-carboxamid (Stereoisomerengemisch)

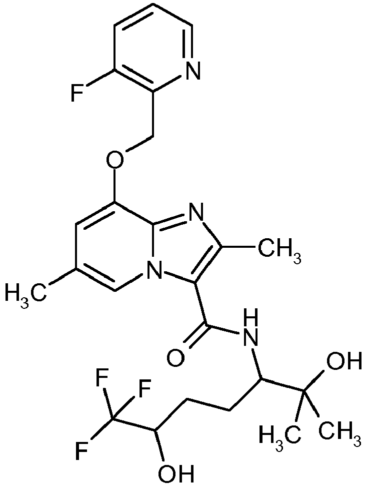

262 mg (0.51 mmol) Methyl-6,6,6-trifluor-N-({8-[(3-fluorpyridin-2-yl)methoxy]-2,6-dimethylimidazo[1,2-a]pyridin-3-yl}carbonyl)-5-hydroxynorleucinat (Stereoisomerengemisch) aus Beispiel 144A wurden unter Argon in 4.9 ml abs. THF vorgelegt und auf 0°C abgekühlt. 0.85 ml (2.56 mmol) einer 3 M Methylmagnesiumbromid-Lösung in Diethylether wurden zugegeben, 15 min bei 0°C gerührt und weiter über Nacht bei RT gerührt. Bei 0°C wurden 0.43 ml (1.28 mmol) einer 3 M Methylmagnesiumbromid-Lösung in Diethylether zur Reaktionsmischung gegeben und es wurde weiter über Nacht bei RT gerührt. Das Reaktionsgemisch wurde mit gesättigter wässriger Ammoniumchlorid-Lösung versetzt und die Reaktionslösung wurde zur Hälfte eingeengt. Der Rückstand wurde zwischen Dichlormethan und Wasser verteilt. Die organische Phase wurde zweimal mit Wasser gewaschen, über Natriumsulfat getrocknet, abfiltriert und eingeengt. Der Rückstand wurde mittels präparativer HPLC (Acetonitril/Wasser-Gradient unter Zusatz von 0.1% TFA) gereinigt. Die Produktfraktionen wurden eingeengt, mit gesättigter wässriger Natriumhydrogencarbonat-Lösung versetzt und dreimal mit Dichlormethan extrahiert. Die vereinigten organischen Phasen wurden über Natriumsulfat getrocknet, abfiltriert und eingeengt. Der Rückstand wurde im Hochvakuum getrocknet. Man erhielt 146 mg (51% d. Th., Reinheit 91%) der Zielverbindung.
LC-MS (Methode 17): Rₜ = 0.68 min
MS (ESIpos): m/z = 513 (M+H)⁺.
¹H-NMR (400 MHz, DMSO-d₆): δ = 1.13 (s, 3H), 1.18 (s, 3H), 1.48 - 1.58 (m, 2H), 1.59 - 1.69 (m, 1H), 1.85 - 1.95 (m, 1H), 2.27 (s, 3H), 2.52 (s, 3H; überlagert mit Lösungsmittelpeak), 3.94 - 4.02 (m, 1H), 4.02 - 4.12 (m, 1H), 4.56 (s, 1H), 5.37 - 5.41 (m, 2H), 6.11 (d, 1H), 6.89 (s, 1H), 7.46 (d, 1H), 7.55 - 7.62 (m, 1H), 7.82 - 7.89 (m, 1H), 8.28 (s, 1H), 8.47 - 8.53 (m, 1H).

### Beispiel 196

### ent-8-[(3-Fluorpyridin-2-yl)methoxy]-2,6-dimethyl-N-(7,7,7-trifluor-2,6-dihydroxy-2-methylheptan-3-yl)imidazo[1,2-a]pyridin-3-carboxamid (Enantiomer D)

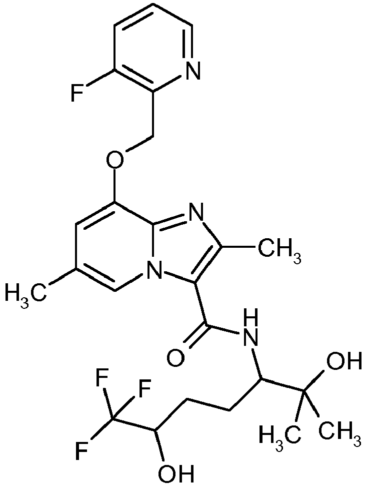

140 mg von Beispiel 195 wurden durch zwei präparative Trennungen an chiraler Phase in die Enantiomere getrennt.

Erste Trennung: Daicel Chiralcel OZ-H, 5 µm, 250 x 20 mm, Eluent: 70% iso-Hexan, 30% Ethanol, Fluß: 20 ml/min; 25°C, Detektion: 220 nm. Der dritte Peak dieser chiralen Chromatographie [analytische Daten: Rₜ = 22.22 min, Daicel IA, 5 µm, 250 x 20 mm, Eluent: 50% Acetonitril, 50% tert.-Butyl-Methylether, Fluß: 20 ml/min; 25°C, Detektion: 220 nm] wurde durch folgende Trennung in die zwei Streoisomere getrennt:
Zweite Trennung: Daicel Chiralcel AD-H, 5 µm, 250 x 20 mm, Eluent: 70% iso-Hexan, 15% Methanol, 15% Isopropanol; Fluß: 20 ml/min; 25°C, Detektion: 220 nm.
Enantiomer D: 6.4 mg (>99% ee)
Rₜ = 18.85 min [Daicel Chiralpak OZ-H, 5 µm, 250 x 4.6 mm; Eluent: 70% iso-Hexan, 30% Ethanol; Fluss 1.0 ml/min; 30°C; Detektion: 220 nm].

### Beispiel 197

### rac-8-[(3-Fluorpyridin-2-yl)methoxy]-2,6-dimethyl-N-(3,3,3-trifluor-2-hydroxypropyl)imidazo[1,2-a]pyridin-3-carboxamid

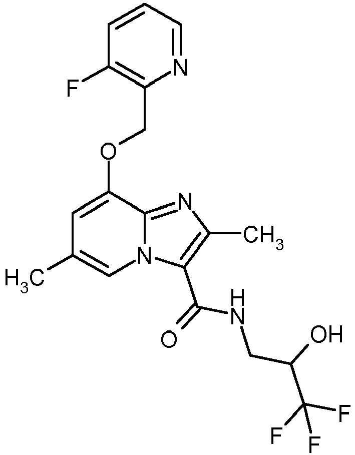

120 mg (0.34 mmol) 8-[(3-Fluorpyridin-2-yl)methoxy]-2,6-dimethylimidazo[1,2-a]pyridin-3-carbonsäure-Hydrochlorid aus Beispiel 121A wurden zusammen mit 169 mg (0.44 mmol) *O*-(7-Azabenzotriazol-1-yl)-*N,N,N'N'*-tetramethyluroniumhexafluorophosphat (HATU) und 176 mg (1.37 mmol) *N,N*-Diisopropylethylamin in 2.2 ml DMF vorgelegt, 50 min gerührt, anschließend mit 66 mg (0.51 mmol) *rac*-3-Amino-1,1,1-trifluorpropan-2-ol versetzt und 1.5 h bei RT gerührt. Die Reaktionslösung wurde mit einigen Tropfen Wasser und TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt und der Rückstand mit Dichlormethan und gesättigter, wässriger Natriumhydrogencarbonat-Lösung versetzt. Die wässrige Phase wurde dreimal mit Dichlormethan extrahiert, die vereinten organischen Phasen über Natriumsulfat getrocknet, filtriert, das Filtrat eingeengt und lyophilisiert. Es wurden 49 mg (32% d. Th.) der Zielverbindung erhalten.
LC-MS (Methode 1): Rₜ = 0.72 min
MS (ESpos): m/z = 427 (M+H)⁺
¹H-NMR (400 MHz, DMSO-d₆): δ = 2.28 - 2.32 (m, 3H), 3.59-3.70 (m, 1H), 4.18-4.28 (m, 1H), 5.36 - 5.40 (m, 2H), 6.56 (d, 1H), 6.91 (s, 1H), 7.57-7.61 (m, 1H), 7.82 - 7.89 (m, 1H), 7.95 (t, 1H), 8.44-8.52 (m, 2H), [weitere Signale unter Lösungsmittel-Peaks verborgen].

### Beispiel 198

### ent-2,6-Dimethyl-N-(6,6,7,7,7-pentafluor-2-hydroxy-2-methylheptan-3-yl)-8-[(2,3,6-trifluorbenzyl)oxy]imidazo[1,2-a]pyridin-3-carboxamid

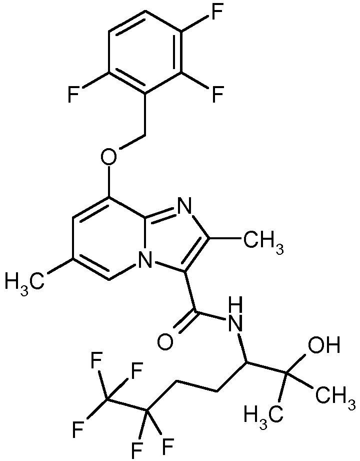

40 mg (0.11 mmol) 2,6-Dimethyl-8-[(2,3,6-trifluorbenzyl)oxy]imidazo[1,2-a]pyridin-3-carbonsäure aus Beispiel 146A wurden mit 44 mg (0.14 mmol) (Benzotriazol-1-yloxy)bisdimethylaminomethyliumfluoroborat (TBTU) und 0.05 ml (0.46 mmol) 4-Methylmorpholin in 0.8 ml DMF vorgelegt, mit *ent*-3-Amino-6,6,7,7,7-pentafluor-2-methylheptan-2-ol (Enantiomer A) aus Beispiel 138A versetzt und über Nacht bei RT gerührt. Die Reaktionslösung wurde mit Wasser/TFA verdünnt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und mit gesättigter, wässriger Natriumhydrogencarbonat-Lösung gewaschen. Die wässrige Phase wurde noch zweimal mit Dichlormethan extrahiert. Die vereinten organischen Phasen wurden über Natriumsulfat getrocknet, filtriert, das Filtrat eingeengt und lyophilisiert. Es wurden 41 mg der Zielverbindung (62% d. Th.) erhalten.
LC-MS (Methode 1): Rₜ = 1.02 min
MS (ESpos): m/z = 568 (M+H)⁺
¹H-NMR (400 MHz, DMSO-d₆): δ = 1.13 (s, 3H), 1.20 (s, 3H), 1.64 - 1.78 (m, 1H), 2.00 - 2.10 (m, 1H), 2.13 - 2.28 (m, 2H), 2.32 (s, 3H), 2.52 (s., 3H; überlagert mit Lösungsmittelpeak), 3.97 - 4.07 (m, 1H), 4.66 - 4.76 (m, 1H), 5.36 (s, 2H), 6.91 - 7.07 (m, 1H), 7.25 - 7.34 (m, 1H), 7.56 - 7.73 (m, 2H), 8.38 (s, 1H).

### B. Bewertung der pharmakologischen Wirksamkeit

Es werden die folgenden Abkürzungen verwendet:
- ATP: Adenosintriphosphat
- Brij35: Polyoxyethylen(23)laurylether
- BSA: Rinderserumalbumin
- DTT: Dithiothreitol
- TEA: Triethanolamin

Die pharmakologische Wirkung der erfindungsgemäßen Verbindungen kann in folgenden Assays gezeigt werden:

### B-1. Vermessung von sGC Enzymaktivität mittels PPi Nachweis

Lösliche Guanylylcyclase (sGC) setzt unter Stimulation GTP zu cGMP und Pyrophosphat (PPi) um. PPi wird mit Hilfe des in WO 2008/061626 beschriebenen Verfahrens nachgewiesen. Das im Test entstehende Signal nimmt mit fortschreitender Umsetzung zu und dient als Maß für die sGC-Enzymaktivität. Mit Hilfe einer PPi Referenzkurve kann das Enzym in bekannter Weise charakterisiert werden, z.B. hinsichtlich Umsatzrate, Stimulierbarkeit oder Michaelis Konstante.

### Durchführung des Tests

Zur Durchführung des Tests wurden 29 µL Enzymlösung (0-10 nM lösliche Guanylylcyclase (hergestellt nach Hönicka et al., Journal of Molecular Medicine 77(1999)14-23), in 50 mM TEA, 2 mM Magnesiumchlorid, 0.1% BSA (FraktionV), 0.005% Brij 35, pH 7.5) in die Mikroplatte vorgelegt und 1 µL der Stimulatorlösung (0-10 µM 3-Morpholinosydnonimine, SIN-1, Merck in DMSO) hinzugegeben. Es wurde 10 min bei RT inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT (Sigma) in 50 mM TEA, 2 mM Magnesiumchlorid, 0.1% BSA (Fraktion V), 0.005% Brij 35, pH 7,5) zugegeben. Die Enzymreaktion wurde durch Zugabe von 20 µl Substratlösung (1.25 mM Guanosin-5'-triphosphat (Sigma) in 50 mM TEA, 2 mM Magnesiumchlorid, 0.1% BSA (Fraktion V), 0.005% Brij 35, pH 7.5) gestartet und kontinuierlich luminometrisch vermessen.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative MEC-Werte (MEC = minimal effektive Konzentration) für die erfindungsgemäßen Verbindungen sind in der nachstehenden Tabelle wiedergegeben (zum Teil als Mittelwerte aus Einzelbestimmungen):

**Tabelle A:**

| Beispiel | MEC [µM] | Beispiel | MEC [µM] |
|---|---|---|---|
| 1 | 0.05 | 101 | 0.1 |
| 2 | 0.03 | 102 | 0.1 |
| 3 | 0.1 | 103 | 0.1 |
| 4 | 0.1 | 104 | 0.1 |
| 5 | 0.1 | 105 | 0.03 |
| 6 | 0.1 | 106 | 0.3 |
| 7 | 0.3 | 107 | 0.1 |
| 8 | 0.3 | 108 | 0.1 |
| 9 | 0.5 | 109 | 0.1 |
| 10 | 0.3 | 110 | 0.3 |
| 11 | 1.5 | 111 | 0.01 |
| 12 | 3.0 | 112 | 0.03 |
| 13 | 3.0 | 113 | 0.03 |
| 14 | 3.0 | 114 | 0.03 |
| 15 | 0.1 | 115 | 0.01 |
| 16 | 1.0 | 116 | 0.3 |
| 17 | 3.0 | 117 | 0.03 |
| 18 | 0.1 | 118 | 0.3 |
| 19 | 1.0 | 119 | 0.3 |
| 20 | 1.0 | 120 | 1.0 |
| 21 | 1.0 | 121 | 0.03 |
| 22 | 1.0 | 122 | 0.03 |
| 23 | 0.3 | 123 | 0.03 |
| 24 | 1.0 | 124 | 0.3 |
| 25 | 0.1 | 125 | 0.3 |
| 26 | 2.0 | 126 | 0.1 |
| 27 | 0.3 | 127 | 0.3 |
| 28 | 0.65 | 128 | 0.3 |
| 29 | 0.1 | 129 | 3.0 |
| 30 | 0.3 | 130 | 1.0 |
| 31 | 1.0 | 131 | 0.01 |
| 32 | 1.0 | 132 | 0.03 |
| 33 | 0.3 | 133 | 0.01 |
| 34 | 0.3 | 134 | 0.3 |
| 35 | 0.3 | 135 | 0.1 |
| 36 | 0.1 | 136 | 3.0 |
| 37 | 0.3 | 137 | 3.0 |
| 38 | 0.3 | 138 | 0.1 |
| 39 | 0.03 | 139 | 0.03 |
| 40 | 0.1 | 140 | 0.03 |
| 41 | 3.0 | 141 | 0.1 |
| 42 | 1.0 | 142 | 0.1 |
| 43 | 0.16 | 143 | 0.03 |
| 44 | 0.3 | 144 | 0.3 |
| 45 | 0.65 | 145 | > 10 |
| 46 | 3.0 | 146 | 1.0 |
| 47 | 0.3 | 147 | 0.1 |
| 48 | 1.0 | 148 | 0.03 |
| 49 | 2.0 | 149 | 0.1 |
| 50 | 6.5 | 150 | 0.03 |
| 51 | 5.5 | 151 | 0.3 |
| 52 | 10 | 152 | 0.3 |
| 53 | 10 | 153 | 0.3 |
| 54 | 10 | 154 | 0.03 |
| 55 | 0.1 | 155 | 0.1 |
| 56 | 1.0 | 156 | 0.03 |
| 57 | 1.0 | 157 | 0.065 |
| 58 | 3.0 | 158 | 1.0 |
| 59 | 3.0 | 159 | 1.0 |
| 60 | 0.1 | 160 | 0.3 |
| 61 | 1.0 | 161 | 1.0 |
| 62 | 0.3 | 162 | 0.3 |
| 63 | 3.0 | 163 | 1.0 |
| 64 | 3.0 | 164 | 0.2 |
| 65 | 0.03 | 165 | 1.0 |
| 66 | 0.01 | 166 | 1.0 |
| 67 | 0.03 | 167 | 2.0 |
| 68 | 0.03 | 168 | 3.0 |
| 69 | 0.1 | 169 | 0.3 |
| 70 | 0.1 | 170 | 3.0 |
| 71 | 0.1 | 171 | 1.0 |
| 72 | 0.1 | 172 | 1.0 |
| 73 | 0.1 | 173 | 3.0 |
| 74 | 0.2 | 174 | 3.0 |
| 75 | 0.22 | 175 | 3.0 |
| 76 | 0.3 | 176 | 3.0 |
| 77 | 0.3 | 177 | 1.0 |
| 78 | 0.3 | 178 | 1 |
| 79 | 0.3 | 179 | 1 |
| 80 | 0.3 | 180 | 1 |
| 81 | 0.65 | 181 | 0.3 |
| 82 | 0.3 | 182 | 0.65 |
| 83 | 0.3 | 183 | 0.3 |
| 84 | 0.3 | 184 | 1 |
| 85 | 0.38 | 185 | 0.3 |
| 86 | 0.65 | 186 | 1 |
| 87 | 1.0 | 187 | 1 |
| 88 | 1.0 | 188 | 0.3 |
| 89 | 1.0 | 189 | 1 |
| 90 | 1.0 | 190 | 1 |
| 91 | 1.0 | 191 | 3 |
| 92 | 1.0 | 192 | 2 |
| 93 | 1.0 | 193 | 1 |
| 94 | 3.0 | 194 | 0.53 |
| 95 | 3.0 | 195 | 10 |
| 96 | 3.0 | 196 | 3 |
| 97 | 1.0 | 197 | 3 |
| 98 | 3.0 | 198 | 0.03 |
| 99 | 3.0 | | |
| 100 | 0.03 | | |

### B-3. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

### B-4. Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht (Stasch et al. Br. J. Pharmacol. 2002; 135: 344-355).

### B-5. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

### Das System besteht aus 3 Hauptkomponenten:

- Implantierbare Sender (Physiotel® Telemetrietransmitter)
- Empfänger (Physiotel® Receiver), die über einen Multiplexer (DSI Data ExchangeMatrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5% iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen
Systolischer Blutdruck (SBP)
Diastolischer Blutdruck (DBP)
Arterieller Mitteldruck (MAP)
Herzfrequenz (HR)
Aktivität (ACT)

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-6. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wister-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

Tabelle B zeigt Daten repräsentativer Verbindungen der vorliegenden Erfindung nach intravenöser sowie peroraler Gabe in Ratten:

**Tabelle B:**

| Beispiel | AUCₙₒᵣₘ | CL_{Blut} | t_{1/2} | MRT |
|---|---|---|---|---|
| | [kg·h/L] | [L/h/kg] | [h] | [h] |
| 181 | 2.5 | 0.56 | 3.4 | 3.3 |
| 187 | 2.0 | 0.71 | 4.6 | 4.0 |
| 188 | 2.3 | 0.53 | 1.7 | 2.5 |
| 194 | 2.9 | 0.44 | 6.2 | 8.4 |

### B-7. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-8. Caco-2 Permeabilitäts-Test

Die Permeabilität einer Testsubstanz wurde mit Hilfe der Caco-2 Zelllinie, einem etablierten *in vitro* Modell für Permeabilitätsvorhersagen an der gastrointestinalen Barriere, bestimmt (Artursson, P. and Karlsson, J. (1991). Correlation between oral drug absorption in humans and apparent drug permeability coefficients in human intestinal epithelial (Caco-2) cells. Biochem. Biophys.175 (3), 880-885). Die Caco-2 Zellen (ACC No. 169, DSMZ, Deutsche Sammlung von Mikroorganismen und Zellkulturen, Braunschweig, Deutschland) wurden in 24-Well Platen mit Einsatz ausgesät und 14 bis 16 Tage kultiviert. Für die Permeabilitätsstudien wurde die Testsubstanz in DMSO gelöst und mit Transportpuffer (Hanks Buffered Salt Solution, Gibco/Invitrogen, mit 19.9 mM Glukose und 9.8 mM HEPES) auf die finale Testkonzentration verdünnt. Um die Permeabilität von apikal nach basolateral (PₐₚₚA-B) der Testsubstanz zu bestimmen, wurde die Lösung mit der Testsubstanz auf die apikale Seite des Caco-2 Zellmonolayers gegeben und Transportpuffer auf die basolaterale Seite. Um die Permeabilität von basolateral nach apikal (PₐₚₚB-A) der Testsubstanz zu bestimmen, wurde die Lösung mit der Testsubstanz auf die basolaterale Seite des Caco-2 Zellmonolayers gegeben und Transportpuffer auf die apikale Seite. Zu Beginn des Experiments wurden Proben aus dem jeweiligen Donor-Kompartiment genommen, um die Massenbilanz sicher zu stellen. Nach einer Inkubation von zwei Stunden bei 37° C wurden Proben aus beiden Kompartimenten genommen. Die Proben wurden mittels LC-MS/MS analysiert und die apparenten Permeabilitätskoeffizienten (Pₐₚₚ) berechnet. Die Permeabilität von Lucifer Yellow wurde für jeden Zellmonolayer bestimmt, um die Integrität der Zellschicht sicher zu stellen. Die Permeabilität von Atenolol (Marker für niedrige Permeabilität) und Sulfasalazin (Marker für aktive Exkretion) wurde in jedem Testlauf als Qualitätskontrolle mitbestimmt.

### C. Ausführunssbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die erhaltene Lösung wird steril filtriert und in sterile und pyrogenfreie Injektionsbehältnisse abgefüllt.

## Patentansprüche

1. Verbindung der Formel (I) 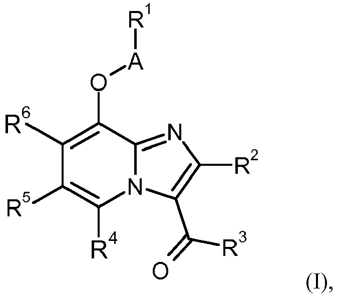 in welcher
A für CH₂, CD₂ oder CH(CH₃) steht,
R¹ für (C₄-C₆)-Alkyl, (C₃-C₇)-Cycloalkyl, Pyridyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 bis 3 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann oder an 2 benachbarten Kohlenstoffatomen des Phenyls mit einer Difluormethylendioxy-Brücke substituiert sein kann,
R² für Wasserstoff, (C₁-C₄)-Alkyl, Cyclopropyl, Monofluormethyl, Difluormethyl oder Trifluormethyl steht,
R³ für eine Gruppe der Formel 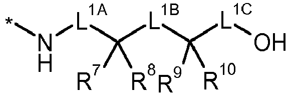 steht, wobei
* für die Anknüpfstelle an die Carbonylgruppe steht,
L^{1A} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für (C₁-C₆)-Alkyl oder (C₂-C₆)-Alkinyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy und Benzyloxy substituiert ist,
worin Benzyloxy mit 1 bis 3 Substituenten Halogen substituiert ist, und
worin darüber hinaus (C₁-C₆)-Alkyl mit Hydroxy substituiert sein kann,
R⁸ für Wasserstoff oder (C₁-C₄)-Alkyl steht,
worin (C₁-C₄)-Alkyl mit Hydroxy substituiert sein kann,
R⁹ für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkylsulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl und (C₁-C₄)-Alkoxy substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
R¹⁰ für Wasserstoff oder (C₁-C₄)-Alkyl steht,
worin (C₁-C₄)-Alkyl mit Hydroxy substituiert sein kann,
oder
R⁹ und R¹⁰ zusammen mit dem Kohlenstoffatom, an das sie gebunden sind, einen 3- bis 7-gliedrigen Carbocyclus oder einen 4- bis 7-gliedriger Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁴ für Wasserstoff steht,
R⁵ für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

2. Verbindung der Formel (I) in welcher
A für CH₂, CD₂ oder CH(CH₃) steht,
R¹ für (C₄-C₆)-Alkyl, (C₃-C₇)-Cycloalkyl, Pyridyl oder Phenyl steht,
wobei (C₄-C₆)-Alkyl bis zu sechsmal mit Fluor substituiert sein kann,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Pyridyl mit 1 bis 3 Substituenten unabhängig voneinander ausgewählt aus der Gruppe Fluor, Trifluormethyl und (C₁-C₄)-Alkyl substituiert sein kann,
und
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₃-C₆)-Cycloalkyl, (C₁-C₄)-Alkoxy, Difluormethoxy und Trifluormethoxy substituiert sein kann oder an 2 benachbarten Kohlenstoffatomen des Phenyls mit einer Difluormethylendioxy-Brücke substituiert sein kann,
R² für Wasserstoff, (C₁-C₄)-Alkyl, Cyclopropyl, Monofluormethyl, Difluormethyl oder Trifluormethyl steht,
R³ für eine Gruppe der Formel 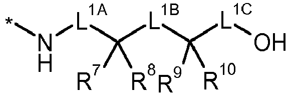 steht, wobei
* für die Anknüpfstelle an die Carbonylgruppe steht,
L^{1A} für eine Bindung oder (C₁-C₄)-Alkandiyl steht, worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
L^{1B} für eine Bindung oder (C₁-C₄)-Alkandiyl steht,
L^{1C} für eine Bindung oder (C₁-C₄)-Alkandiyl steht,
worin (C₁-C₄)-Alkandiyl mit 1 bis 3 Substituenten unabhängig voneinander ausgewählt aus der Gruppe Fluor, Trifluormethyl, (C₁-C₄)-Alkyl, (C₃-C₇)-Cycloalkyl, Hydroxy und (C₁-C₄)-Alkoxy substituiert sein kann,
R⁷ für Wasserstoff, (C₁-C₆)-Alkyl, (C₂-C₆)-Alkenyl, (C₂-C₆)-Alkinyl, (C₃-C₇)-Cycloalkyl, (C₁-C₄)-Alkoxycarbonyl, 5- oder 6-gliedriges Heteroaryl oder Phenyl steht,
worin (C₁-C₆)-Alkyl mit 1 bis 3 Substituenten unabhängig voneinander ausgewählt aus der Gruppe Fluor, Trifluormethyl, Difluormethoxy, Trifluormethoxy, Hydroxy, (C₁-C₄)-Alkoxy, (C₁-C₄)-Alkoxycarbonyl, (C₁-C₄)-Alkylthio, (C₁-C₄)-Alkyl-sulfonyl, Phenyl, Phenoxy und Benzyloxy substituiert sein kann,
worin Phenyl, Phenoxy und Benzyloxy ihrerseits mit 1 bis 3 Substituenten Halogen substituiert sein können,
worin (C₃-C₇)-Cycloalkyl mit 1 oder 2 Substituenten unabhängig voneinander ausgewählt aus der Gruppe Fluor, Trifluormethyl, (C₁-C₄)-Alkyl, (C₁-C₄)-Alkoxy und (C₁-C₄)-Alkylsulfonyl substituiert sein kann,
und
worin Phenyl und 5- oder 6-gliedriges Heteroaryl mit 1 bis 3 Substituenten unabhängig voneinander ausgewählt aus der Gruppe Halogen, Cyano, Trifluormethyl, (C₁-C₄)-Alkyl, (C₁-C₄)-Alkoxy und (C₁-C₄)-Alkylsulfonyl substituiert sein können,
R⁸ für Wasserstoff oder (C₁-C₄)-Alkyl steht,
worin (C₁-C₄)-Alkyl mit Hydroxy substituiert sein kann,
oder
R⁷ und R⁸ zusammen mit dem Kohlenstoffatom, an das sie gebunden sind, einen 3- bis 7-gliedrigen Carbocyclus oder einen 4- bis 7-gliedrigen Heterocyclus bilden,
worin der 3- bis 7-gliedrige Carbocyclus und der 4- bis 7-gliedrige Heterocyclus ihrerseits mit 1 oder 2 Substituenten unabhängig voneinander ausgewählt aus der Gruppe Fluor und (C₁-C₄)-Alkyl substituiert sein können,
R⁹ für (C₁-C₆)-Alkyl steht,
worin (C₁-C₆)-Alkyl mit 1 bis 3 Substituenten unabhängig voneinander ausgewählt aus der Gruppe Fluor, Difluormethyl, Trifluormethyl, Difluormethoxy, Trifluormethoxy, Phenoxy und Benzyloxy substituiert ist,
worin Phenoxy mit 1 bis 3 Substituenten Halogen substituiert ist, worin Benzyloxy mit 1 bis 3 Substituenten Halogen substituiert ist,
R¹⁰ für Wasserstoff oder (C₁-C₄)-Alkyl steht,
R⁴ für Wasserstoff steht,
R⁵ für Wasserstoff, Halogen, Cyano, Monofluormethyl, Difluormethyl, Trifluormethyl, (C₁-C₄)-Alkyl, (C₃-C₇)-Cycloalkyl, (C₂-C₄)-Alkinyl, Difluormethoxy, Trifluormethoxy, (C₁-C₄)-Alkoxy, Amino, 4- bis 7-gliedriges Heterocyclyl oder 5- oder 6-gliedriges Heteroaryl steht,
R⁶ für Wasserstoff, Cyano oder Halogen steht,
sowie ihre *N*-Oxide, Salze, Solvate, Salze der *N*-Oxide und Solvate der *N*-Oxide und Salze.

3. Verfahren zur Herstellung von Verbindungen der Formel (I), wie in den Ansprüchen 1 bis 2 definiert, **dadurch gekennzeichnet, dass** man
[A] eine Verbindung der Formel (II) 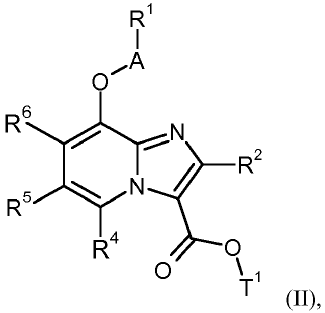
in welcher A, R¹, R², R⁴, R⁵ und R⁶ jeweils die oben angegebenen Bedeutungen haben und
T¹ für (C₁-C₄)-Alkyl oder Benzyl steht,
in einem inerten Lösungsmittel in Gegenwart einer geeigneten Base oder Säure zu einer Carbonsäure der Formel (III) 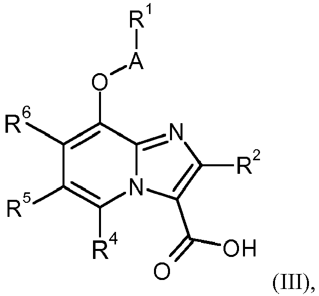 in welcher A, R¹, R², R⁴, R⁵ und R⁶ jeweils die oben angegebenen Bedeutungen haben,
umsetzt und diese in der Folge in einem inerten Lösungsmittel unter Amidkupplungsbedingungen mit einem Amin der Formel (IV-A) 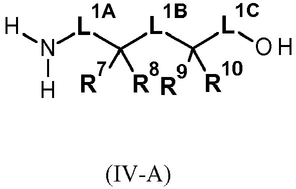 in welchem L^{1A}, L^{1B}, L^{1C}, R⁷, R⁸, R⁹ und R¹⁰ jeweils die oben angegebenen Bedeutungen haben umsetzt
oder
[B] eine Verbindung der Formel (III-B) 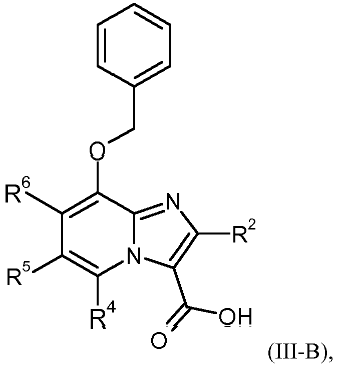 in welcher R², R⁴, R⁵ und R⁶ jeweils die oben angegebenen Bedeutungen haben,
in einem inerten Lösungsmittel unter Amidkupplungsbedingungen mit einem Amin der Formel (IV-A) zu einer Verbindung der Formel (I-A) 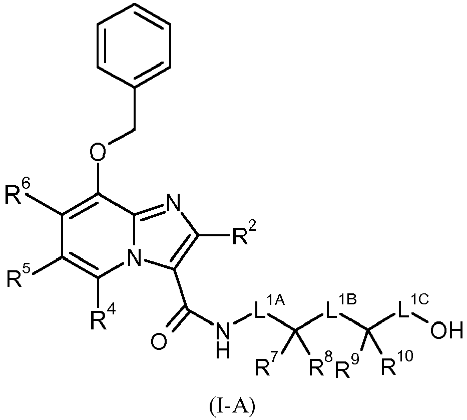 in welcher R², R⁴, R⁵, R⁶, L^{1A}, L^{1B}, L^{1C}, R⁷, R⁸, R⁹ und R¹⁰ jeweils die oben angegebenen Bedeutungen haben,
umsetzt, von dieser im Folgenden nach dem Fachmann bekannten Methoden die Benzylgruppe abspaltet und die resultierende Verbindung der Formel (V-A) 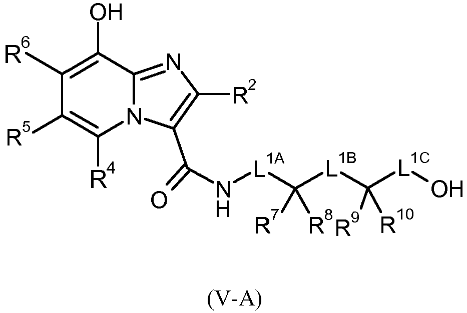 in welcher R², R⁴, R⁵, R⁶, L^{1A}, L^{1B}, L^{1C}, R⁷, R⁸, R⁹ und R¹⁰ jeweils die oben angegebenen Bedeutungen haben,
in einem inerten Lösungsmittel in Gegenwart einer geeigneten Base mit einer Verbindung der Formel (VI) 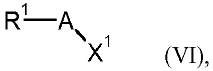 in welcher A und R¹ die oben angegebene Bedeutung haben und
X¹ für eine geeignete Abgangsgruppe, insbesondere Chlor, Brom, Iod, Mesylat, Triflat oder Tosylat, steht,
umsetzt,
und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

4. Verbindung der Formel (I), wie in einem der Ansprüche 1 bis 2 definiert, zur Behandlung und/oder Prophylaxe von Krankheiten.

5. Verwendung einer Verbindung der Formel (I), wie in einem der Ansprüche 1 bis 2 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

6. Arzneimittel enthaltend eine Verbindung der Formel (I), wie in einem der Ansprüche 1 bis 2 definiert, in Kombination mit einem inerten, nicht-toxischen, pharmazeutisch geeigneten Hilfsstoff.

7. Arzneimittel enthaltend eine Verbindung der Formel (I), wie in einem der Ansprüche 1 bis 2 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

8. Arzneimittel nach Anspruch 6 oder 7 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

## Claims

1. Compound of the formula (I) 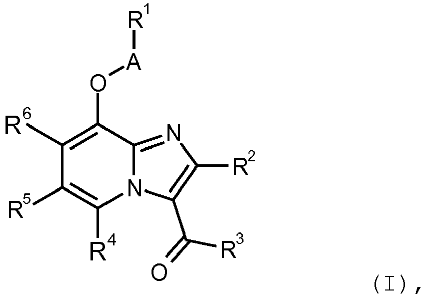 in which
R¹ represents (C₄-C₆)-alkyl, (C₃-C₇)-cycloalkyl, pyridyl or phenyl,
where (C₄-C₆)-alkyl may be substituted up to six times by fluorine,
where (C₃-C₇)-cycloalkyl may be substituted by 1 to 4 substituents independently of one another selected from the group consisting of fluorine, trifluoromethyl and (C₁-C₄)-alkyl, where pyridyl may be substituted by 1 to 3 substituents independently of one another selected from the group consisting of fluorine, trifluoromethyl and (C₁-C₄)-alkyl, and
where phenyl may be substituted by 1 to 4 substituents independently of one another selected from the group consisting of halogen, cyano, monofluoromethyl, difluoromethyl, trifluoromethyl, (C₁-C₄)-alkyl, (C₃-C₆)-cycloalkyl, (C₁-C₄)-alkoxy, difluoromethoxy and trifluoromethoxy or at two adjacent carbon atoms of the phenyl group by a difluoromethylenedioxy bridge,
R² represents hydrogen, (C₁-C₄)-alkyl, cyclopropyl, monofluoromethyl, difluoromethyl or trifluoromethyl,
R³ represents a group of the formula 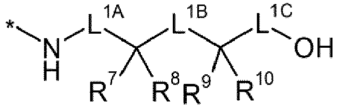 where
* represents the point of attachment to the carbonyl group,
L^{1A} represents a bond or (C₁-C₄)-alkanediyl, where (C₁-C₄)-alkanediyl may be substituted by 1 to 3 substituents independently of one another selected from the group consisting of fluorine, trifluoromethyl, (C₁-C₄)-alkyl, (C₃-C₇)-cycloalkyl, hydroxy and (C₁-C₄)-alkoxy,
L^{1B} represents a bond or (C₁-C₄)-alkanediyl,
L^{1C} represents a bond or (C₁-C₄)-alkanediyl, where (C₁-C₄)-alkanediyl may be substituted by 1 to 3 substituents independently of one another selected from the group consisting of fluorine, trifluoromethyl, (C₁-C₄)-alkyl, (C₃-C₇)-cycloalkyl, hydroxy and (C₁-C₄)-alkoxy,
R⁷ represents (C₁-C₆)-alkyl or (C₂-C₆)-alkynyl,
where (C₁-C₆)-alkyl is substituted by 1 to 3 substituents independently of one another selected from the group consisting of fluorine, trifluoromethyl, difluoromethoxy, trifluoromethoxy and benzyloxy,
where benzyloxy is substituted by 1 to 3 halogen substituents,
and
where furthermore (C₁-C₆)-alkyl may be substituted by hydroxy,
R⁸ represents hydrogen or (C₁-C₄)-alkyl, where (C₁-C₄)-alkyl may be substituted by hydroxy,
R⁹ represents hydrogen, (C₁-C₆)-alkyl, (C₂-C₆)-alkenyl, (C₂-C₆)-alkynyl, (C₃-C₇)-cycloalkyl, (C₁-C₄)-alkoxycarbonyl, 5- or 6-membered heteroaryl or phenyl,
where (C₁-C₆)-alkyl may be substituted by 1 to 3 substituents independently of one another selected from the group consisting of fluorine, trifluoromethyl, difluoromethoxy, trifluoromethoxy, hydroxy, (C₁-C₄)-alkoxy, (C₁-C₄)-alkoxycarbonyl, (C₁-C₄)-alkylthio, (C₁-C₄)-alkylsulphonyl, phenyl, phenoxy and benzyloxy,
where phenyl, phenoxy and benzyloxy for their part may be substituted by 1 to 3 halogen substituents,
where (C₃-C₇)-cycloalkyl may be substituted by 1 or 2 substituents independently of one another selected from the group consisting of fluorine, trifluoromethyl, (C₁-C₄)-alkyl and (C₁-C₄)-alkoxy,
and
where phenyl and 5- or 6-membered heteroaryl may be substituted by 1 to 3 substituents independently of one another selected from the group consisting of halogen, cyano, trifluoromethyl, (C₁-C₄)-alkyl, (C₁-C₄)-alkoxy and (C₁-C₄)-alkylsulphonyl,
R¹⁰ represents hydrogen or (C₁-C₄)-alkyl,
where (C₁-C₄)-alkyl may be substituted by hydroxy,
or
R⁹ and R¹⁰ together with the carbon atom to which they are attached form a 3-to 7-membered carbocycle or a 4- to 7-membered heterocycle,
where the 3- to 7-membered carbocycle and the 4- to 7-membered heterocycle for their part may be substituted by 1 or 2 substituents independently of one another selected from the group consisting of fluorine and (C₁-C₄)-alkyl,
R⁴ represents hydrogen,
R⁵ represents hydrogen, halogen, cyano, monofluoromethyl, difluoromethyl, trifluoromethyl, (C₁-C₄)-alkyl, (C₃-C₇)-cycloalkyl, (C₂-C₄)-alkynyl, difluoromethoxy, trifluoromethoxy, (C₁-C₄)-alkoxy, amino, 4- to 7-membered heterocyclyl or 5- or 6-membered heteroaryl,
R⁶ represents hydrogen, cyano or halogen, and its *N*-oxides, salts, solvates, salts of the *N-*oxides and solvates of the *N*-oxides and salts.

2. Compound of the formula (I) in which
A represents CH₂, CD₂ or CH(CH₃),
R¹ represents (C₄-C₆)-alkyl, (C₃-C₇)-cycloalkyl, pyridyl or phenyl,
where (C₄-C₆)-alkyl may be substituted up to six times by fluorine,
where (C₃-C₇)-cycloalkyl may be substituted by 1 to 4 substituents independently of one another selected from the group consisting of fluorine, trifluoromethyl and (C₁-C₄)-alkyl, where pyridyl may be substituted by 1 to 3 substituents independently of one another selected from the group consisting of fluorine, trifluoromethyl and (C₁-C₄)-alkyl, and
where phenyl may be substituted by 1 to 4 substituents independently of one another selected from the group consisting of halogen, cyano, monofluoromethyl, difluoromethyl, trifluoromethyl, (C₁-C₄)-alkyl, (C₃-C₆)-cycloalkyl, (C₁-C₄)-alkoxy, difluoromethoxy and trifluoromethoxy or at two adjacent carbon atoms of the phenyl group by a difluoromethylenedioxy bridge,
R² represents hydrogen, (C₁-C₄)-alkyl, cyclopropyl, monofluoromethyl, difluoromethyl or trifluoromethyl,
R³ represents a group of the formula 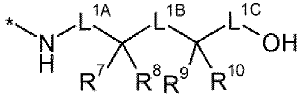 where
* represents the point of attachment to the carbonyl group,
L^{1A} represents a bond or (C₁-C₄) -alkanediyl, where (C₁-C₄)-alkanediyl may be substituted by 1 to 3 substituents independently of one another selected from the group consisting of fluorine, trifluoromethyl, (C₁-C₄)-alkyl, (C₃-C₇)-cycloalkyl, hydroxy and (C₁-C₄)-alkoxy,
L^{1B} represents a bond or (C₁-C₄)-alkanediyl,
L^{1C} represents a bond or (C₁-C₄)-alkanediyl, where (C₁-C₄)-alkanediyl may be substituted by 1 to 3 substituents independently of one another selected from the group consisting of fluorine, trifluoromethyl, (C₁-C₄)-alkyl, (C₃-C₇)-cycloalkyl, hydroxy and (C₁-C₄)-alkoxy,
R⁷ represents hydrogen, (C₁-C₆)-alkyl, (C₂-C₆)-alkenyl, (C₂-C₆)-alkynyl, (C₃-C₇)-cycloalkyl, (C₁-C₄)-alkoxycarbonyl, 5- or 6-membered heteroaryl or phenyl,
where (C₁-C₆)-alkyl may be substituted by 1 to 3 substituents independently of one another selected from the group consisting of fluorine, trifluoromethyl, difluoromethoxy, trifluoromethoxy, hydroxy, (C₁-C₄)-alkoxy, (C₁-C₄)-alkoxy-carbonyl, (C₁-C₄)-alkylthio, (C₁-C₄)-alkylsulphonyl, phenyl, phenoxy and benzyloxy,
where phenyl, phenoxy and benzyloxy for their part may be substituted by 1 to 3 halogen substituents,
where (C₃-C₇-cycloalkyl may be substituted by 1 or 2 substituents independently of one another selected from the group consisting of fluorine, trifluoromethyl, (C₁-C₄)-alkyl, (C₁-C₄)-alkoxy and (C₁-C₄) -alkylsulphonyl,
and
where phenyl and 5- or 6-membered heteroaryl may be substituted by 1 to 3 substituents independently of one another selected from the group consisting of halogen, cyano, trifluoromethyl, (C₁-C₄)-alkyl, (C₁-C₄)-alkoxy and (C₁-C₄)-alkylsulphonyl,
R⁸ represents hydrogen or (C₁-C₄)-alkyl,
where (C₁-C₄)-alkyl may be substituted by hydroxy,
or
R⁷ and R⁸ together with the carbon atom to which they are attached form a 3-to 7-membered carbocycle or a 4- to 7-membered heterocycle,
where the 3- to 7-membered carbocycle and the 4- to 7-membered heterocycle for their part may be substituted by 1 or 2 substituents independently of one another selected from the group consisting of fluorine and (C₁-C₄)-alkyl,
R⁹ represents (C₁-C₆)-alkyl,
where (C₁-C₆)-alkyl is substituted by 1 to 3 substituents independently of one another selected from the group consisting of fluorine, difluoromethyl, trifluoromethyl, difluormethoxy, trifluoromethoxy, phenoxy and benzyloxy,
where phenoxy is substituted by 1 to 3 halogen substituents, where benzyloxy is substituted by 1 to 3 halogen substituents,
R¹⁰ represents hydrogen or (C₁-C₄)-alkyl,
R⁴ represents hydrogen,
R⁵ represents hydrogen, halogen, cyano, monofluoromethyl, difluoromethyl, trifluoromethyl, (C₁-C₄)-alkyl, (C₃-C₇)-cycloalkyl, (C₂-C₄)-alkynyl, difluoromethoxy, trifluoromethoxy, (C₁-C₄)-alkoxy, amino, 4- to 7-membered heterocyclyl or 5- or 6-membered heteroaryl,
R⁶ represents hydrogen, cyano or halogen, and its *N*-oxides, salts, solvates, salts of the *N-*oxides and solvates of the *N*-oxides and salts.

3. Process for preparing compounds of the formula (I) as defined in Claims 1 to 2, **characterized in that**
[A] a compound of the formula (II) 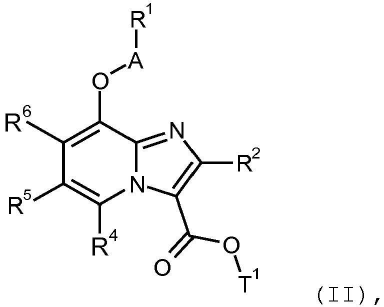
in which A, R¹, R², R⁴, R⁵ and R⁶ each have the meanings given above and
T¹ represents (C₁-C₄)-alkyl or benzyl,
is reacted in an inert solvent in the presence of a suitable base or acid to give a carboxylic acid of the formula (III) 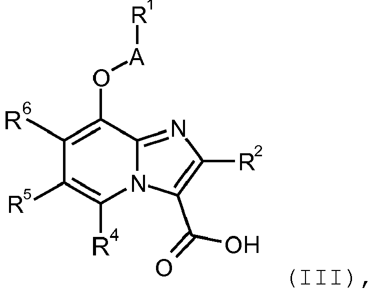 in which A, R¹, R², R⁴, R⁵ and R⁶ each have the meanings given above,
and this is subsequently reacted in an inert solvent under amide coupling conditions with an amine of the formula (IV-A) 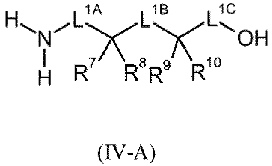 in which L^{1A}, L^{1B}, L^{1C}, R⁷, R⁸, R⁹ and R¹⁰ each have the meanings given above
or
[B] a compound of the formula (III-B) 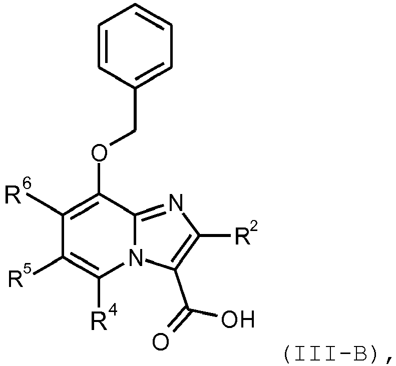 in which R², R⁴, R⁵ and R⁶ each have the meanings given above,
is reacted in an inert solvent under amide coupling conditions with an amine of the formula (IV-A) to give a compound of the formula (I-A) 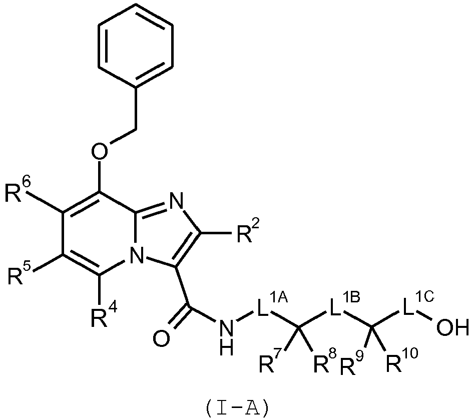 in which R², R⁴, R⁵, R⁶, L^{1A}, L^{1B}, L^{1C}, R⁷, R⁸, R⁹ and R¹⁰ each have the meanings given above,
from this compound, the benzyl group is subsequently removed using methods known to the person skilled in the art and the resulting compound of the formula (V-A) 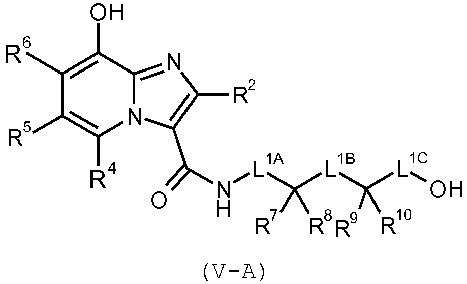 in which R², R⁴, R⁵, R⁶, L^{1A}, L^{1B}, L^{1C}, R⁷, R⁸, R⁹ and R¹⁰ each have the meanings given above,
is reacted in an inert solvent in the presence of a suitable base with a compound of the formula (VI) 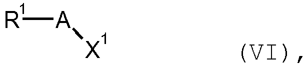 in which A and R¹ have the meanings given above and
X¹ represents a suitable leaving group, in particular chlorine, bromine, iodine, mesylate, triflate or tosylate,
and the resulting compounds of the formula (I) are optionally converted with the appropriate (i) solvents and/or (ii) acids or bases into their solvates, salts and/or solvates of the salts.

4. Compound of the formula (I) as defined in any of Claims 1 to 2 for the treatment and/or prophylaxis of diseases.

5. Use of a compound of the formula (I) as defined in any of Claims 1 to 2 for producing a medicament for the treatment and/or prophylaxis of heart failure, angina pectoris, hypertension, pulmonary hypertension, ischaemias, vascular disorders, kidney failure, thromboembolic disorders and arteriosclerosis.

6. Medicament, comprising a compound of the formula (I) as defined in any of Claims 1 to 2 in combination with an inert, non-toxic, pharmaceutically suitable auxiliary.

7. Medicament, comprising a compound of the formula (I) as defined in any of Claims 1 to 2 in combination with a further active compound selected from the group consisting of organic nitrates, NO donors, cGMP-PDE inhibitors, agents having antithrombotic activity, agents lowering blood pressure, and agents altering lipid metabolism.

8. Medicament according to Claim 6 or 7 for the treatment and/or prophylaxis of heart failure, angina pectoris, hypertension, pulmonary hypertension, ischaemias, vascular disorders, kidney failure, thromboembolic disorders, fibrotic disorders and arteriosclerosis.

## Revendications

1. Composé de formule (I) 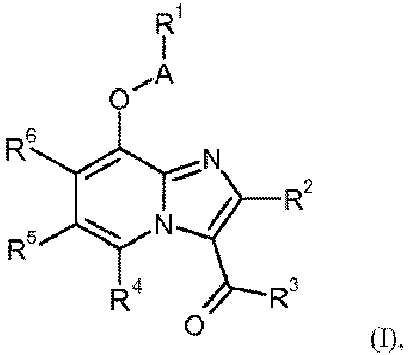 dans laquelle
A représente CH₂, CD₂ ou CH(CH₃),
R¹ représente alkyle en (C₄-C₆), cycloalkyle en (C₃-C₇), pyridyle ou phényle,
l'alkyle en (C₄-C₆) pouvant être substitué jusqu'à six fois avec fluor,
le cycloalkyle en (C₃-C₇) pouvant être substitué avec 1 à 4 substituants choisis indépendamment les uns des autres dans le groupe constitué par fluor, trifluorométhyle et alkyle en (C₁-C₄),
le pyridyle pouvant être substitué avec 1 à 3 substituants choisis indépendamment les uns des autres dans le groupe constitué par fluor, trifluorométhyle et alkyle en (C₁-C₄),
et
le phényle pouvant être substitué avec 1 à 4 substituants choisis indépendamment les uns des autres dans le groupe constitué par halogène, cyano, monofluorométhyle, difluorométhyle, trifluorométhyle, alkyle en (C₁-C₄), cycloalkyle en (C₃-C₆), alcoxy en (C₁-C₄), difluorométhoxy et trifluorométhoxy, ou pouvant être substitué sur 2 atomes de carbone voisins du phényle avec un pont difluorométhylène-dioxy,
R² représente hydrogène, alkyle en (C₁-C₄), cyclopropyle, monofluorométhyle, difluorométhyle ou trifluorométhyle,
R³ représente un groupe de formule 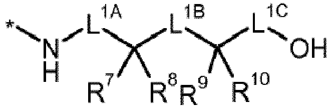 dans laquelle
* représente l'emplacement de liaison au groupe carbonyle,
L^{1A} représente une liaison ou alcanediyle en (C₁-C₄), l'alcanediyle en (C₁-C₄) pouvant être substitué avec 1 à 3 substituants choisis indépendamment les uns des autres dans le groupe constitué par fluor, trifluorométhyle, alkyle en (C₁-C₄), cycloalkyle en (C₃-C₇), hydroxy et alcoxy en (C₁-C₄),
L^{1B} représente une liaison ou alcanediyle en (C₁-C₄),
L^{1C} représente une liaison ou alcanediyle en (C₁-C₄), l'alcanediyle en (C₁-C₄) pouvant être substitué avec 1 à 3 substituants choisis indépendamment les uns des autres dans le groupe constitué par fluor, trifluorométhyle, alkyle en (C₁-C₄), cycloalkyle en (C₃-C₇), hydroxy et alcoxy en (C₁-C₄),
R⁷ représente alkyle en (C₁-C₆) ou alcynyle en (C₂-C₆), l'alkyle en (C₁-C₆) étant substitué avec 1 à 3 substituants choisis indépendamment les uns des autres dans le groupe constitué par fluor, trifluorométhyle, difluorométhyle, trifluorométhoxy et benzyloxy,
le benzyloxy étant substitué avec 1 à 3 substituants halogène,
et
l'alkyle en (C₁-C₆) peut en outre être substitué avec hydroxy,
R⁸ représente hydrogène ou alkyle en (C₁-C₄), l'alkyle en (C₁-C₄) pouvant être substitué avec hydroxy,
R⁹ représente hydrogène, alkyle en (C₁-C₆), alcényle en (C₂-C₆), alcynyle en (C₂-C₆), cycloalkyle en (C₃-C₇), alcoxycarbonyle en (C₁-C₄), hétéroaryle à 5 ou 6 éléments, ou phényle,
l'alkyle en (C₁-C₆) pouvant être substitué avec 1 à 3 substituants choisis indépendamment les uns des autres dans le groupe constitué par fluor, trifluorométhyle, difluorométhoxy, trifluorométhoxy, hydroxy, alcoxy en (C₁-C₄), alcoxycarbonyle en (C₁-C₄), alkylthio en (C₁-C₄), alkylsulfonyle en (C₁-C₄), phényle, phénoxy et benzyloxy,
le phényle, le phénoxy et le benzyloxy pouvant de leur côté être substitués avec 1 à 3 substituants halogène,
le cycloalkyle en (C₃-C₇) pouvant être substitué avec 1 ou 2 substituants choisis indépendamment l'un de l'autre dans le groupe constitué par fluor, trifluorométhyle, alkyle en (C₁-C₄) et alcoxy en (C₁-C₄),
et
le phényle et l'hétéroaryle à 5 ou 6 éléments pouvant être substitués avec 1 à 3 substituants choisis indépendamment les uns des autres dans le groupe constitué par halogène, cyano, trifluorométhyle, alkyle en (C₁-C₄), alcoxy en (C₁-C₄) et alkylsulfonyle en (C₁-C₄),
R¹⁰ représente hydrogène ou alkyle en (C₁-C₄),
l'alkyle en (C₁-C₄) pouvant être substitué avec hydroxy,
ou
R⁹ et R¹⁰ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
R⁴ représente hydrogène,
R⁵ représente hydrogène, halogène, cyano, monofluorométhyle, difluorométhyle, trifluorométhyle, alkyle en (C₁-C₄), cycloalkyle en (C₃-C₇), alcynyle en (C₂-C₄), difluorométhoxy, trifluorométhoxy, alcoxy en (C₁-C₄), amino, hétérocyclyle de 4 à 7 éléments, ou hétéroaryle à 5 ou 6 éléments,
R⁶ représente hydrogène, cyano ou halogène,
ainsi que ses N-oxydes, sels, solvates, sels des N-oxydes et solvates des N-oxydes et des sels.

2. Composé de formule (I), dans laquelle
A représente CH₂, CD₂ ou CH(CH₃),
R¹ représente alkyle en (C₄-C₆), cycloalkyle en (C₃-C₇), pyridyle ou phényle,
l'alkyle en (C₄-C₆) pouvant être substitué jusqu'à six fois avec fluor,
le cycloalkyle en (C₃-C₇) pouvant être substitué avec 1 à 4 substituants choisis indépendamment les uns des autres dans le groupe constitué par fluor, trifluorométhyle et alkyle en (C₁-C₄),
le pyridyle pouvant être substitué avec 1 à 3 substituants choisis indépendamment les uns des autres dans le groupe constitué par fluor, trifluorométhyle et alkyle en (C₁-C₄),
et
le phényle pouvant être substitué avec 1 à 4 substituants choisis indépendamment les uns des autres dans le groupe constitué par halogène, cyano, monofluorométhyle, difluorométhyle, trifluorométhyle, alkyle en (C₁-C₄), cycloalkyle en (C₃-C₆), alcoxy en (C₁-C₄), difluorométhoxy et trifluorométhoxy, ou pouvant être substitué sur 2 atomes de carbone voisins du phényle avec un pont difluorométhylène-dioxy,
R² représente hydrogène, alkyle en (C₁-C₄), cyclopropyle, monofluorométhyle, difluorométhyle ou trifluorométhyle,
R³ représente un groupe de formule 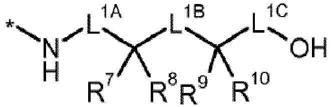 dans laquelle
* représente l'emplacement de liaison au groupe carbonyle,
L^{1A} représente une liaison ou alcanediyle en (C₁-C₄), l'alcanediyle en (C₁-C₄) pouvant être substitué avec 1 à 3 substituants choisis indépendamment les uns des autres dans le groupe constitué par fluor, trifluorométhyle, alkyle en (C₁-C₄), cycloalkyle en (C₃-C₇), hydroxy et alcoxy en (C₁-C₄),
L^{1B} représente une liaison ou alcanediyle en (C₁-C₄),
L^{1C} représente une liaison ou alcanediyle en (C₁-C₄), l'alcanediyle en (C₁-C₄) pouvant être substitué avec 1 à 3 substituants choisis indépendamment les uns des autres dans le groupe constitué par fluor, trifluorométhyle, alkyle en (C₁-C₄), cycloalkyle en (C₃-C₇), hydroxy et alcoxy en (C₁-C₄),
R⁷ représente hydrogène, alkyle en (C₁-C₆), alcényle en (C₂-C₆), alcynyle en (C₂-C₆), cycloalkyle en (C₃-C₇), alcoxycarbonyle en (C₁-C₄), hétéroaryle à 5 ou 6 éléments, ou phényle,
l'alkyle en (C₁-C₆) pouvant être substitué avec 1 à 3 substituants choisis indépendamment les uns des autres dans le groupe constitué par fluor, trifluorométhyle, difluorométhoxy, trifluorométhoxy, hydroxy, alcoxy en (C₁-C₄), alcoxycarbonyle en (C₁-C₄), alkylthio en (C₁-C₄), alkylsulfonyle en (C₁-C₄), phényle, phénoxy et benzyloxy,
le phényle, le phénoxy et le benzyloxy pouvant être substitués de leur côté avec 1 à 3 substituants halogène,
le cycloalkyle en (C₃-C₇) pouvant être substitué avec 1 ou 2 substituants choisis indépendamment l'un de l'autre dans le groupe constitué par fluor, trifluorométhyle, alkyle en (C₁-C₄), alcoxy en (C₁-C₄) et alkylsulfonyle en (C₁-C₄),
et
le phényle et l'hétéroaryle à 5 ou 6 éléments pouvant être substitués avec 1 à 3 substituants choisis indépendamment les uns des autres dans le groupe constitué par halogène, cyano, trifluorométhyle, alkyle en (C₁-C₄) , alcoxy en (C₁-C₄) et alkylsulfonyle en (C₁-C₄),
R⁸ représente hydrogène ou alkyle en (C₁-C₄),
l'alkyle en (C₁-C₄) pouvant être substitué avec hydroxy,
ou
R⁷ et R⁸ forment ensemble avec l'atome de carbone auquel ils sont reliés un carbocycle de 3 à 7 éléments ou un hétérocycle de 4 à 7 éléments,
le carbocycle de 3 à 7 éléments et l'hétérocycle de 4 à 7 éléments pouvant de leur côté être substitués avec 1 ou 2 substituants choisis indépendamment l'un de l'autre dans le groupe constitué par fluor et alkyle en (C₁-C₄),
R⁹ représente alkyle en (C₁-C₆),
l'alkyle en (C₁-C₆) pouvant être substitué avec 1 à 3 substituants choisis indépendamment les uns des autres dans le groupe constitué par fluor, difluorométhyle, trifluorométhyle, difluorométhoxy, trifluorométhoxy, phénoxy et benzyloxy,
le phénoxy étant substitué avec 1 à 3 substituants halogène,
le benzyloxy étant substitué avec 1 à 3 substituants halogène,
R¹⁰ représente hydrogène ou alkyle en (C₁-C₄),
R⁴ représente hydrogène,
R⁵ représente hydrogène, halogène, cyano, monofluorométhyle, difluorométhyle, trifluorométhyle, alkyle en (C₁-C₄), cycloalkyle en (C₃-C₇), alcynyle en (C₂-C₄), difluorométhoxy, trifluorométhoxy, alcoxy en (C₁-C₄), amino, hétérocyclyle de 4 à 7 éléments, ou hétéroaryle à 5 ou 6 éléments,
R⁶ représente hydrogène, cyano ou halogène,
ainsi que ses N-oxydes, sels, solvates, sels des N-oxydes et solvates des N-oxydes et des sels.

3. Procédé de fabrication de composés de formule (I), tels que définis dans les revendications 1 à 2, **caractérisé en ce que**
[A] un composé de formule (II) 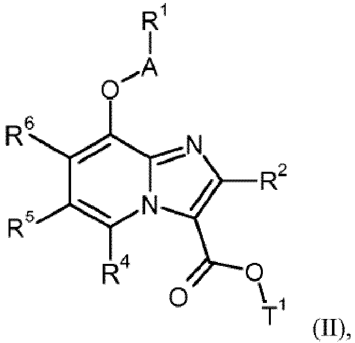
dans laquelle A, R¹, R², R⁴, R⁵ et R⁶ ont chacun les significations indiquées précédemment, et
T¹ représente alkyle en (C₁-C₄) ou benzyle,
est mis en réaction dans un solvant inerte en présence d'une base ou d'un acide approprié pour former un acide carboxylique de formule (III) 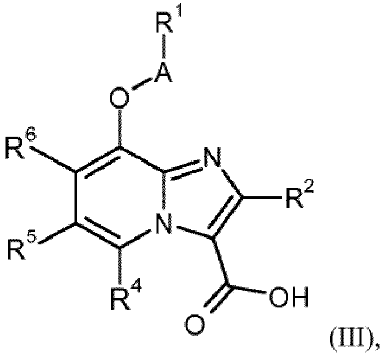 dans laquelle A, R¹, R², R⁴, R⁵ et R⁶ ont chacun les significations indiquées précédemment,
puis celui-ci est mis en réaction dans un solvant inerte dans des conditions de couplage d'amide avec une amine de formule (IV-A) 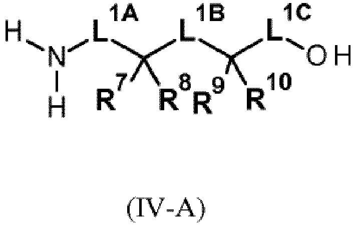 dans laquelle L^{1A}, L^{1B}, L^{1C}, R⁷, R⁸, R⁹ et R¹⁰ ont chacun les significations indiquées précédemment,
ou
[B] un composé de formule (III-B) 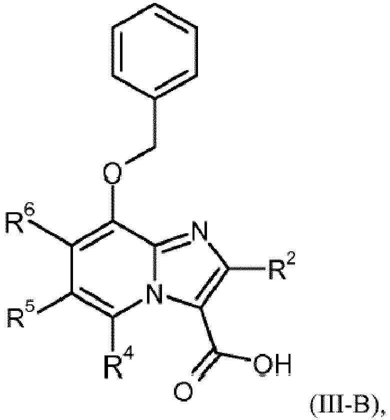 dans laquelle R², R⁴, R⁵ et R⁶ ont chacun les significations indiquées précédemment,
est mis en réaction dans un solvant inerte dans des conditions de couplage d'amide avec une amine de formule (IV-A) pour former un composé de formule (I-A) 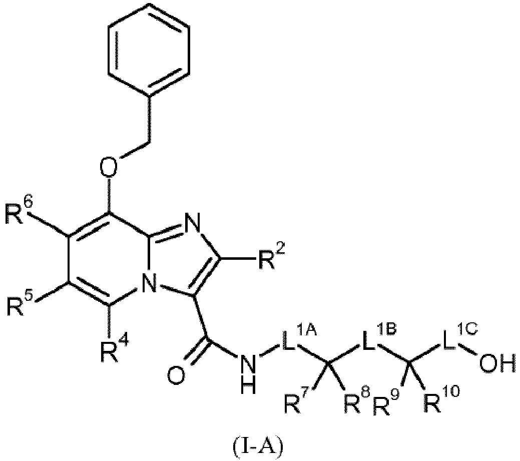 dans laquelle R², R⁴, R⁵, R⁶, L^{1A}, L^{1B}, L^{1C}, R⁷, R⁸, R⁹ et R¹⁰ ont chacun les significations indiquées précédemment,
puis le groupe benzyle de celui-ci est clivé par des méthodes connues de l'homme du métier et le composé de formule (V-A) résultant 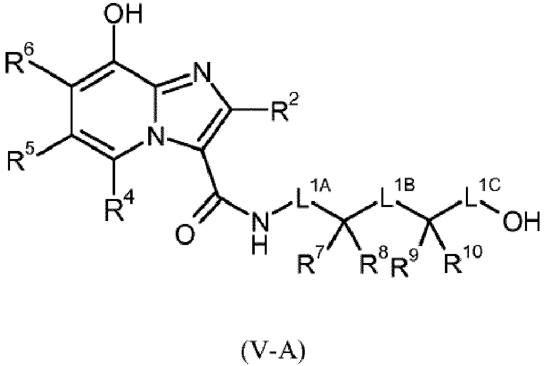 dans laquelle R², R⁴, R⁵, R⁶, L^{1A}, L^{1B}, L^{1C}, R⁷, R⁸, R⁹ et R¹⁰ ont chacun les significations indiquées précédemment,
est mis en réaction dans un solvant inerte en présence d'une base appropriée avec un composé de formule (VI) 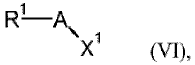 dans laquelle A et R¹ ont la signification indiquée précédemment, et
X¹ représente un groupe partant approprié, notamment chlore, brome, iode, mésylate, triflate ou tosylate,
et les composés de formule (I) résultants sont éventuellement transformés avec (i) les solvants et/ou (ii) les acides ou les bases appropriés en leurs solvates, sels et/ou solvates des sels.

4. Composé de formule (I), tel que défini dans l'une quelconque des revendications 1 à 2, pour le traitement et/ou la prophylaxie de maladies.

5. Utilisation d'un composé de formule (I), tel que défini dans l'une quelconque des revendications 1 à 2, pour la fabrication d'un médicament pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques et de l'artériosclérose.

6. Médicament contenant un composé de formule (I), tel que défini dans l'une quelconque des revendications 1 à 2, en combinaison avec un adjuvant inerte, non toxique, pharmaceutiquement approprié.

7. Médicament contenant un composé de formule (I), tel que défini dans l'une quelconque des revendications 1 à 2, en combinaison avec un autre agent actif choisi dans le groupe constitué par les nitrates organiques, les donneurs de NO, les inhibiteurs de cGMP-PDE, les agents à effet antithrombotique, les agents abaissant la tension artérielle et les agents modifiant le métabolisme des lipides.

8. Médicament selon la revendication 6 ou 7 pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques et de l'artériosclérose.
